(19) Europäisches Patentamt
European Patent Office
Office européen des brevets

(11) EP 0 392 317 B1

(12) **EUROPÄISCHE PATENTSCHRIFT**

(45) Veröffentlichungstag und Bekanntmachung des Hinweises auf die Patenterteilung:
**03.01.1996 Patentblatt 1996/01**

(51) Int. Cl.6: **C07D 235/06**, C07D 235/08, C07D 235/16, C07D 235/18, C07D 403/10, C07D 405/12

(21) Anmeldenummer: **90106322.2**

(22) Anmeldetag: **03.04.1990**

(54) **Benzimidazole, diese Verbindungen enthaltende Arzneimittel und Verfahren zu ihrer Herstellung**

Benzimidazoles, drugs containing these compounds and process for their preparation

Benzimidazoles, médicaments les contenant, et procédé pour leur préparation

(84) Benannte Vertragsstaaten:
**AT BE CH DE DK ES FR GB GR IT LI LU NL SE**

(30) Priorität: **08.04.1989 DE 3911603**
**25.08.1989 DE 3928177**

(43) Veröffentlichungstag der Anmeldung:
**17.10.1990 Patentblatt 1990/42**

(73) Patentinhaber: **Dr. Karl Thomae GmbH**
**D-88397 Biberach (DE)**

(72) Erfinder:
- **Narr, Berthold, Dr.-Dipl.-Chem.**
  **D-7950 Biberach 1 (DE)**
- **Bomhard, Andreas, Dr.-Dipl.-Chem.**
  **D-4000 Düsseldorf 13 (DE)**
- **Hauel, Norbert, Dr.-Dipl.-Chem.**
  **D-7950 Biberach 1 (DE)**
- **van Meel, Jacques, Dr.**
  **D-7951 Mittelbiberach (DE)**
- **Wienen, Wolfgang, Dr.-Dipl.-Biol.**
  **D-7951 Äpfingen (DE)**
- **Entzeroth, Michael, Dr.-Dipl.-Chem.**
  **D-7951 Warthausen (DE)**

(56) Entgegenhaltungen:

| | |
|---|---|
| **EP-A- 0 132 606** | **EP-A- 0 142 754** |
| **EP-A- 0 253 310** | **EP-A- 0 291 969** |
| **EP-A- 0 425 921** | **US-A- 4 880 804** |

Bemerkungen:
Die Akte enthält technische Angaben, die nach dem Eingang der Anmeldung eingereicht wurden und die nicht in dieser Patentschrift enthalten sind.

EP 0 392 317 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

Printed by Rank Xerox (UK) Business Services
2.6/3.4

## Beschreibung

In der EP-A-0,132,606 werden bereits 4'-(Benzimidazol-1-yl)-methyl-biphenyle beschrieben, welche Insektizide und Akarizide darstellen. Außerdem werden in der US-A-4,880,804 ebenfalls 4'-(Benzimidazol-1-yl)-methyl-biphenyle beschrieben, welche Angiotensin-II-Antagonisten darstellen, wobei jedoch die Verbindungen der vorliegenden Erfindung, denen nicht das Recht auf die in Anspruch genommenen Prioritäten zukommt, nicht unter den Schutzumfang der US-A-4,880,804 fallen, da in der US-A-4,880,804 keine Verbindungen beansprucht werden, die im Phenylkern des Benzimidazolrestes durch eine gegebenenfalls substituierte Aminogruppe substituiert sind.

Überraschenderweise wurde gefunden, daß 4'-(Benzimidazol-1-yl)-methyl-biphenyle, die im Phenylkern des Benzimidazolrestes durch eine acylierte Amino- oder Iminogruppe substituiert sind, überlegene Angiotensin-II-Antagonisten darstellen.

Gegenstand der vorliegenden Erfindung sind daher 1- und 3-Isomere der Benzimidazole der Formel

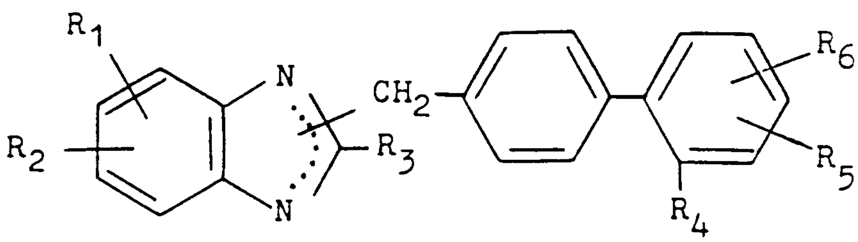

,(I)

und, sofern $R_1$ und $R_2$ nicht jeweils gleichzeitig Wasserstoffatom darstellen oder in 4- und 7-Stellung oder in 5- und 6-Stellung nicht jeweils die gleichen Bedeutungen aufweisen, deren 1-, 3-Isomerengemische sowie deren Salze, insbesondere für die pharmazeutische Anwendung deren physiologisch verträglichen Salze mit anorganischen oder organischen Säuren oder Basen.

In der obigen Formel I bedeutet

$R_1$ ein Wasserstoff-, Fluor-, Chlor- oder Bromatom,

eine Alkylgruppe mit 1 bis 4 Kohlenstoffatomen, die durch eine Hydroxy-, Alkoxy-, Amino-, Alkylamino-, Dialkylamino- oder Acylaminogruppe substituiert sein kann,

eine Alkoxygruppe mit 1 bis 4 Kohlenstoffatomen, die in 2-, 3- oder 4-Stellung durch eine Hydroxy-, Alkoxy-, Amino-, Alkylamino-, Dialkylamino- oder Imidazolylgruppe substituiert sein kann,

eine Hydroxy-, Phenylalkoxy-, Acyloxy-, Trifluormethylsulfonyloxy-, Alkylaminocarbonyloxy-, Dialkylaminocarbonyloxy-, Cycloalkylaminocarbonyloxy-, Cycloalkylalkylaminocarbonyloxy-, Arylaminocarbonyloxy-, Aralkylaminocarbonyloxy-, Alkoxycarbonyloxy-, Cycloalkoxycarbonyloxy-, Cycloalkylalkoxycarbonyloxy-, Aryloxycarbonyloxy-, Aralkoxycarbonyloxy-, Trifluormethyl-, Cyano-, Nitro-, Alkylmercapto-, Alkylsulfinyl-, Alkylsulfonyl-, Aminosulfonyl-, Alkylaminosulfonyl-, Dialkylaminosulfonyl-, Arylaminosulfonyl-, Acylaminosulfonyl- oder Acylgruppe,

eine Aminogruppe, die durch eine Imidazolylalkyl-, Dialkylaminoalkanoyl-, Acyl-, Cycloalkoxycarbonyl-, Cycloalkylalkoxycarbonyl-, Aryloxycarbonyl-, Aralkoxycarbonyl- oder Trifluoracetylgruppe, durch eine bi- oder tricyclische Alkylgruppe mit 7 bis 11 Kohlenstoffatomen, durch eine Alkylsulfonylgruppe mit 1 bis 4 Kohlenstoffatomen, durch eine Alkoxycarbonylgruppe mit insgesamt 2 bis 7 Kohlenstoffatomen oder durch eine durch eine Alkoxycarbonylgruppe substituierte Thiazolidin-3-yl-carbonylgruppe monosubstituiert sein kann,

eine Alkylaminogruppe mit 1 bis 6 Kohlenstoffatomen, die am Stickstoffatom durch eine Alkyl-, Alkylsulfonyl- oder Acylgruppe substituiert sein kann, wobei, falls die Acylgruppe eine Alkanoylgruppe darstellt, diese zusätzlich durch eine Alkoxygruppe substituiert sein kann, und der Alkylsubstituent ab Position 2 durch eine Hydroxy-, Alkoxy- oder Arylaminogruppe substituiert sein kann,

eine durch eine Cycloalkyl-, Cycloalkylalkyl-, Phenylalkyl-oder Phenylgruppe mono- oder disubstituierte Aminogruppe, wobei die Substituenten gleich oder verschieden sein können,

eine N-Alkyl-cycloalkylamino-, N-Alkyl-cycloalkylalkylamino-, N-Alkyl-phenylalkylamino- oder N-Alkyl-phenylaminogruppe,

eine gegebenenfalls durch eine Alkyl-, Cycloalkyl- oder Phenylgruppe substituierte Pyrrolidino-, Piperidino- oder Hexamethyleniminogruppe,

eine N-Alkoxycarbonyl-alkylamino-, N-Cycloalkoxycarbonylalkylamino-, N-Cycloalkylalkoxycarbonyl-alkylamino-, N-Aryloxycarbonyl-alkylamino- oder N-Aralkoxycarbonyl-alkylaminogruppe, in der die Alkylgruppe jeweils 1 bis 6 Kohlenstoffatome enthalten kann, eine am Stickstoffatom durch eine Cycloalkyl-, Cycloalkylalkyl- oder Aralkylgruppe substituierte Alkoxycarbonylamino-, Cycloalkoxycarbonylamino-, Cycloalkylalkoxycarbonylamino-, Aryloxycarbonylamino-,

Aralkoxycarbonylamino-, Acylamino- oder Alkylsulfonylaminogruppe,

eine Carbonylgruppe, die durch eine Alkylgruppe, die in 2-oder 3-Stellung durch eine Hydroxy-, Alkanoyloxy- oder Alkylaminogruppe substituiert ist, durch eine Hydroxycarbonylalkyl-, Hydroxy-, Alkoxy-, Amino-, Cycloalkylamino-, Cycloalkylalkylamino-, Arylamino- oder Aralkylaminogruppe, durch eine in 2- oder 3-Stellung durch eine Arylaminogruppe substituierte Alkylaminogruppe oder durch eine gegebenenfalls im Alkylteil durch eine Carboxygruppe substituierte Alkylaminogruppe mit 1 bis 5 Kohlenstoffatomen, die jeweils zusätzlich am Stickstoffatom durch eine Alkylgruppe substituiert sein kann, substituiert ist,

eine gegebenenfalls durch eine Alkoxycarbonylgruppe mit insgesamt 2 bis 5 Kohlenstoffatomen substituierte Aminoacetylaminogruppe,

eine Aminocarbonylamino- oder Aminothiocarbonylaminogruppe, welche durch eine Alkylgruppe mit 1 bis 20 Kohlenstoffatomen, durch eine Alkenyl- oder Alkinylgruppe mit jeweils 3 bis 5 Kohlenstoffatomen, durch eine bi- oder tricyclische Alkylgruppe mit 7 bis 11 Kohlenstoffatomen, durch eine Cycloalkyl-, Cycloalkylalkyl-, Aralkyl- oder Arylgruppe mono-, di- oder trisubstituiert sein kann, wobei die Substituenten gleich oder verschieden sein können sowie eine Methylengruppe in einem Cycloalkylrest mit 5 bis 7 Kohlenstoffatomen durch ein Sauerstoffatom ersetzt und eine Alkylgruppe in 4-, 5- oder 6-Stellung durch eine Hydroxy-, Alkanoyl- oder Trifluoracetylgruppe substituiert sein kann,

eine Cycloalkyleniminocarbonylaminogruppe mit 4 bis 6 Kohlenstoffatomen im Cycloalkyleniminoteil oder eine Morpholinocarbonylaminogruppe, die beide am Aminostickstoff durch eine Alkylgruppe mit 1 bis 20 Kohlenstoffatomen, durch eine Cycloalkyl-, Cycloalkylalkyl-, Aralkyl- oder Arylgruppe substituiert sein können,

eine Phthalimino-, Homophthalimino-, 2-Carboxyphenylcarbonylamino-, 2-Carboxyphenylmethylamino-, 2-Carboxyphenylmethylencarbonylamino- oder 2-Carboxymethylenphenylcarbonylaminogruppe, wobei eine Carbonylgruppe in einer Phthaliminogruppe durch eine Methylengruppe ersetzt sowie eine Methylengruppe in einer Homophthalimino-, 2-Carboxyphenylmethylencarbonylamino- oder 2-Carboxymethylenphenylcarbonylaminogruppe durch eine oder zwei Alkylgruppen substituiert sein kann, und zusätzlich die vorstehend erwähnten Phenylkerne durch Alkyl- oder Alkoxygruppen mono- oder disubstituiert, wobei die Substituenten gleich oder verschieden sein können, und gleichzeitig ganz oder teilweise hydriert sein können, eine in 2-Stellung durch eine Carboxygruppe substituierte Bicycloalkan-3-carbonsäureamino- oder Bicycloalken-3-carbonsäureaminogruppe, eine Bicycloalkan-2,3-dicarbonsäureimino- oder Bicycloalken-2,3-dicarbonsäureiminogruppe, in denen der Bicycloalkan- und Bicycloalkenteil jeweils 9 oder 10 Kohlenstoffatome enthalten, durch 1, 2 oder 3 Methylgruppen substituiert und eine Endomethylengruppe durch ein Sauerstoffatom ersetzt sein kann, eine Glutarsäureimino- oder 3-Carboxy-n-propylencarbonylgruppe, in denen jeweils die n-Propylengruppe perfluoriert, durch ein oder zwei Alkylgruppen oder durch eine Tetramethylen- oder Pentamethylengruppe substituiert sein kann, oder

eine 5,7-Dioxa-1H,3H-imidazo[1,5-c]thiazolylgruppe,

$R_2$ die für $R_1$ vorstehend erwähnten Bedeutungen, eine gegebenfalls in 3-Stellung durch eine Alkyl-, Cycloalkyl-, Cycloalkylalkyl- oder Aralkylgruppe substituierte 2-Imidazolidon-1-yl- oder 3,4,5,6-Tetrahydro-2-pyrimidon-1-yl-gruppe oder eine Tetrazolylgruppe oder

$R_1$ und $R_2$ zusammen mit 2 Kohlenstoffatomen des benachbarten Phenylringes eine Phenyl- oder 1-Alkyl-3,3-dialkyl-2,3-dihydro-pyrrol-2-on-gruppe,

$R_3$ ein Wasserstoff-, Fluor-, Chlor- oder Bromatom,

eine Alkylgruppe mit 1 bis 6 Kohlenstoffatomen, in welcher eine Methylengruppe durch ein Sauerstoff- oder Schwefelatom, eine Sulfinyl-, Sulfonyl- oder Alkylaminogruppe ersetzt und eine Methylgruppe durch eine Hydroxy-, Alkoxy-, Amino-, Alkylamino- oder Dialkylaminogruppe substituiert sein kann, wobei jedoch die zum Benzimidazolring benachbarte Methylengruppe durch keine Sulfinyl- oder Sulfonylgruppe ersetzt sein kann und bei gleichzeitigem Ersatz einer Methylengruppe und Substitution einer Methylgruppe diese durch mindestens 2 Kohlenstoffatome voneinander getrennt sein müssen,

eine Alkenyl- oder Alkinylgruppe mit jeweils 3 bis 5 Kohlenstoffatomen,

eine Phenylalkylgruppe,

eine Cycloalkyl- oder Cycloalkyl-alkylgruppe, in welcher der Cycloalkylteil jeweils 5 bis 7 Kohlenstoffatome enthalten kann,

eine Aryl-, Hydroxy- oder Imidazolylalkylaminogruppe,

eine Alkylaminogruppe mit 1 bis 4 Kohlenstoffatomen,

eine Aminocarbonylgruppe, welche durch eine Alkyl- oder Cycloalkylgruppe mit 5 bis 7 Kohlenstoffatomen substituiert sein kann,

einen 5-gliedrigen heteroaromatischen Ring, welcher eine NH-Gruppe, ein Sauerstoff- oder Schwefelatom enthält, wobei dieser vorstehend erwähnte 5-gliedrige heteroaromatische Ring zusätzlich noch 1 bis 3 N-Atome enthalten kann, oder einen 6-gliedrigen heteroaromatischen Ring, welcher 1 oder 2 Stickstoffatome enthalten kann, wobei die vorstehend erwähnten 5- und 6-gliedrigen heteroaromatischen Ringe durch eine oder mit Ausnahme der Tetrazolylgruppe durch zwei Alkylgruppen substituiert sein können,

$R_4$ eine Amino-, Phthalimino-, Aminomethyl-, Cyano-, tert.-Butoxycarbonyl- oder 1-(Triphenylmethyl)-tetrazolylgruppe, eine eine Brönsted-Säure enthaltende Gruppe oder einen Rest, der in vivo in eine eine Brönsted-Säure enthaltende

Gruppe übergeführt werden kann,
$R_5$ und $R_6$ jeweils ein Wasserstoffatom oder
$R_5$ und $R_6$ zusammen mit den beiden ortho-ständigen Kohlenstoffatomen eine Phenylgruppe.

Unter dem vorstehend erwähnten Begriff "eine eine Brönsted-Säure enthaltende Gruppe" ist hierbei insbesondere eine Carboxy-, Aminoacetylamino-, Trifluormethylcarbonylamino-, Trifluormethylcarbonylaminomethyl-, Trifluormethylsulfonylamino-, Trifluormethylsulfonylaminomethyl- oder 1H-Tetrazolylgruppe, eine Alkylcarbonylamino-, Alkylcarbonylaminomethyl-, Arylcarbonylamino-, Arylcarbonylaminomethyl-, Aralkylcarbonylamino-, Aralkylcarbonylaminomethyl-, Alkylsulfonylamino-, Alkylsulfonylaminomethyl-, Arylsulfonylamino-, Arylsulfonylaminomethyl-, Aralkylsulfonylamino-, Aralkylsulfonylaminomethyl-, Arylsulfonylaminocarbonyl- oder Benzylsulfonylaminocarbonylgruppe, eine Alkylsulfonylaminocarbonyl- oder Perfluoralkylsulfonylaminocarbonylgruppe mit jeweils 1 bis 6 Kohlenstoffatomen im Alkylteil,
unter "einem Rest, der in vivo in eine eine Brönsted-Säure enthaltende Gruppe übergeführt werden kann" mit Ausnahme der tert.Butoxycarbonylgruppe eine Alkoxycarbonylgruppe mit insgesamt 2 bis 7 Kohlenstoffatomen wie die Methoxycarbonyl-, Äthoxycarbonyl-, n-Propoxycarbonyl-, Isopropoxycarbonyl-, n-Butoxycarbonyl-, Isobutoxycarbonyl-, n-Pentoxycarbonyl- oder n-Hexoxycarbonylgruppe, eine Aralkoxycarbonylgruppe wie die Benzyloxycarbonyl-, 1-Phenyläthoxycarbonyl-, 2-Phenyläthoxycarbonyl- oder 3-Phenylpropoxycarbonylgruppe oder die Pivaloyloxymethoxycarbonyl-, Phthalidylmethoxycarbonyl-, Ethoxycarbonyloxyethoxycarbonyl-, Methoxymethoxycarbonyl-, Cyclohexyloxycarbonylmethoxycarbonyl- oder (1,3-Di-oxa-2-oxo-4-methyl-cyclopenten-5-yl)-methoxycarbonylgruppe,
unter "eine Acylgruppe" eine Alkanoylgruppe mit 1 bis 7 Kohlenstoffatomen, eine Cycloalkylcarbonylgruppe mit insgesamt 4 bis 8 Kohlenstoffatomen, eine Cycloalkylalkanoylgruppe mit insgesamt 5 bis 10 Kohlenstoffatomen oder eine gegebenenfalls durch ein Fluor-, Chlor- oder Bromatom, eine Alkyl- oder Alkoxygruppe substituierte Arylcarbonyl-, Aralkanoyl- oder Phenylsulfonylgruppe,
unter "eine Arylgruppe" eine gegebenenfalls durch ein Fluor-, Chlor- oder Bromatom, durch eine Hydroxy-, Alkyl-, Alkoxy-, Phenylalkoxy- oder Trifluormethylgruppe substituierte Phenylgruppe, wobei der Alkylteil jeweils 1 bis 4 Kohlenstoffatome enthalten kann, oder eine Naphthylgruppe und
unter "eine Cycloalkylgruppe" eine Cycloalkylgruppe mit 3 bis 7 Kohlenstoffatomen, welche durch eine oder zwei Alkylgruppen substituiert sein kann, zu verstehen, wobei, sofern nichts anderes erwähnt wurde, die bei der Definition der Reste $R_1$ bis $R_3$ erwähnten Alkyl- und Alkanoylteile sowie die vorstehend erwähnten Alkylteile jeweils 1 bis 3 Kohlenstoffatome enthalten können.

Die neuen Verbindungen der obigen Formel I weisen wertvolle Eigenschaften auf. So weisen die Verbindungen der Formel I, in der $R_4$ eine eine Brönsted-Säure enthaltende Gruppe oder einen Rest, der in vivo in eine eine Brönsted-Säure enthaltende Gruppe übergeführt werden kann, bedeutet, wertvolle pharmakologische Eigenschaften auf, insbesondere stellen diese Verbindungen Angiotensin-II-Antagonisten dar.

Die übrigen Verbindungen der Formel I stellen wertvolle Zwischenprodukte dar, da diese sich chemisch in eine der vorstehend erwähnten pharmakologisch wirksamen Verbindungen der Formel I überführen lassen.

Ein weiterer Gegenstand der vorliegenden Erfindung sind somit neue Arzneimittel, welche eine der oben erwähnten pharmakologisch wirksamen Verbindungen der Formel I oder ein entsprechendes physiologisch verträgliches Salz enthalten und insbesondere zur Behandlung der Hypertonie und Herzinsuffizienz, ferner zur Behandlung ischämischer peripherer Durchblutungsstörungen, der myokardialen Ischämie (Angina), zur Prävention der Herzinsuffizienzprogression nach Myokard-Infarkt, zur Behandlung der diabetischen Nephropathie, des Glaukoms, von gastrointestinalen Erkrankungen und Blasenerkrankungen geeignet sind.

Für die bei der Definition der Reste $R_1$, $R_2$, $R_3$ und $R_4$ erwähnten Bedeutungen kommt beispielsweise für $R_1$ die Bedeutung des Wasserstoff-, Fluor-, Chlor- oder Bromatoms, der Methyl-, Ethyl-, n-Propyl-, Isopropyl-, n-Butyl-, Hydroxymethyl-, 2-Hydroxy-ethyl-, 2-Hydroxy-isopropyl-, 3-Hydroxy-propyl-, Methoxymethyl-, 2-Methoxy-ethyl-, 2-Ethoxy-ethyl-, 2-Methoxy-isopropyl-, 2-n-Propoxy-ethyl-, Aminomethyl-, 1-Aminoethyl-, 2-Aminoethyl-, 3-Aminopropyl-, Methylaminomethyl-, Dimethylaminomethyl-, Ethylaminomethyl-, Diethylaminomethyl-, N-Methyl-isopropylaminomethyl-, 2-Methylaminoethyl-, 2-Dimethylaminoethyl-, 2-Isopropylaminoethyl-, 2-Diisopropylaminoethyl-, 3-Methylaminopropyl-, 3-Dimethylaminopropyl-, Acetaminomethyl-, Propionylaminomethyl-, Butanoylaminomethyl-, Pentanoylaminomethyl-, Benzoylaminomethyl-, Benzolsulfonylaminomethyl-, 2-Acetaminoethyl-, 2-Propionylaminoethyl-, 2-Butanoylaminoethyl-, 2-Benzoylaminoethyl-, 3-Acetaminopropyl-, Methoxy-, Ethoxy-, n-Propoxy-, Isopropoxy-, n-Butoxy-, 2-Hydroxy-ethoxy-, 2-Hydroxy-isopropoxy-, 3-Hydroxy-propoxy-, 2-Methoxy-ethoxy-, 2-Ethoxy-ethoxy-, 2-Methoxy-isopropoxy-, 3-Methoxy-propoxy-, 3-n-Propoxy-propoxy-, 2-Amino-ethoxy-, 2-Methylamino-ethoxy-, 2-Dimethylamino-ethoxy-, 2-Ethylamino-ethoxy-, 2-Diethylamino-ethoxy-, 2-Isopropylamino-ethoxy-, 3-Amino-propoxy-, 3-Methylamino-propoxy-, 3-Dimethylamino-propoxy-, 2-(Imidazol-1-yl)-ethoxy-, 2-(Imidazol-2-yl)-ethoxy-, 2-(Imidazol-4-yl)-ethoxy-, 3-(Imidazol-1-yl)-propoxy-, 3-(Imidazol-2-yl)-propoxy-, 3-(Imidazol-4-yl)-propoxy-, Hydroxy-, Benzyloxy-, 2-Phenyl-ethoxy-, 3-Phenylpropoxy-, Acetoxy-, Propionyloxy-, n-Butanoyloxy-, n-Pentanoyloxy-, Trifluormethylsulfonyloxy-, Methylaminocarbonyloxy-, Ethylaminocarbonyloxy-, Isopropylaminocarbonyloxy-, Dimethylaminocarbonyloxy-, Diethylaminocarbonyloxy-, Di-n-propylaminocarbonyloxy-, N-Methyl-ethylaminocarbonyloxy-, Cyclopropylaminocarbonyloxy-, Cyclobutylaminocarbonyloxy-, Cyclopentylaminocarbonyloxy-, Cyclohexylaminocarbonyloxy-, Cycloheptylaminocarbonyloxy-, Cyclopropylmethylaminocarbonyloxy-, Cyclobutylmethylaminocarbonyloxy-, Cyclopentylmethylaminocarbonyloxy-,

Cyclohexylmethylaminocarbonyloxy-, Cycloheptylmethylaminocarbonyloxy-, (2-Cyclopropylethyl)-aminocarbonyloxy-, (2-Cyclobutylethyl)-aminocarbonyloxy-, (2-Cyclopentylethyl)-aminocarbonyloxy-, (2-Cyclohexylethyl)-aminocarbonyloxy-, (2-Cycloheptylethyl)-aminocarbonyloxy-, (3-Cyclopropylpropyl)-aminocarbonyloxy-, (3-Cyclobutylpropyl)-aminocarbonyloxy-, (3-Cyclopentylpropyl)-aminocarbonyloxy-, (3-Cyclohexylpropyl)-aminocarbonyloxy-, (3-Cycloheptylpropyl)-aminocarbonyloxy-, Methoxycarbonyloxy-, Ethoxycarbonyloxy-, n-Propoxycarbonyloxy-, Isopropoxycarbonyloxy-, Cyclopropoxycarbonyloxy-, Cyclobutoxycarbonyloxy-, Cyclopentoxycarbonyloxy-, Cyclohexoxycarbonyloxy-, Cycloheptoxycarbonyloxy-, Cyclopropylmethoxycarbonyloxy-, Cyclobutylmethoxycarbonyloxy-, Cyclopentylmethoxycarbonyloxy-, Cyclohexylmethoxycarbonyloxy-, Cycloheptylmethoxycarbonyloxy-, (2-Cyclopropylethoxy)-carbonyloxy-, (2-Cyclobutylethoxy)-carbonyloxy-, (2-Cyclopentylethoxy)-carbonyloxy-, (2-Cyclohexylethoxy)-carbonyloxy-, (2-Cycloheptylethoxy)-carbonyloxy-, (3-Cyclopropylpropoxy)-carbonyloxy-, (3-Cyclobutylpropoxy)-carbonyloxy-, (3-Cyclopentylpropoxy)-carbonyloxy-, (3-Cyclohexylpropoxy)-carbonyloxy-, (3-Cycloheptylpropoxy)-carbonyloxy-, Phenyloxycarbonyloxy-, Benzyloxycarbonyloxy-, (2-Phenylethoxy)-carbonyloxy-, Trifluormethyl-, Phenylaminocarbonyloxy-, Benzylaminocarbonyloxy-, (2-Phenylethyl)-aminocarbonyloxy-, Cyano-, Nitro-, Methylmercapto-, Ethylmercapto-, n-Propylmercapto-, Isopropylmercapto-, Methylsulfinyl-, Ethylsulfinyl-, n-Propylsulfinyl-, Isopropylsulfinyl-, Methylsulfonyl-, Ethylsulfonyl-, n-Propylsulfonyl-, Isopropylsulfonyl-, Phenylsulfonyl-, Fluorphenylsulfonyl-, Chlorphenylsulfonyl-, Bromphenylsulfonyl-, Methylphenylsulfonyl-, Ethylphenylsulfonyl-, Isopropylphenylsulfonyl-, Methoxyphenylsulfonyl-, Ethoxyphenylsulfonyl-, n-Propoxyphenylsulfonyl-, Aminosulfonyl-, Methylaminosulfonyl-, Ethylaminosulfonyl-, n-Propylaminosulfonyl-, Isopropylaminosulfonyl-, Dimethylaminosulfonyl-, Diethylaminosulfonyl-, Di-n-Propylaminosulfonyl-, N-Methyl-ethylaminosulfonyl-, Phenylaminosulfonyl-, Fluorphenylaminosulfonyl-, Chlorphenylaminosulfonyl-, Bromphenylaminosulfonyl-, Methylphenylaminosulfonyl-, Ethylphenylaminosulfonyl-, Isopropylphenylaminosulfonyl-, Methoxyphenylaminosulfonyl-, Ethoxyphenylaminosulfonyl-, n-Propoxyphenylaminosulfonyl-, Acetaminosulfonyl-, Propionylaminosulfonyl-, n-Butanoylaminosulfonyl-, Benzoylaminosulfonyl-, Fluorbenzoylaminosulfonyl-, Chlorbenzoylaminosulfonyl-, Brombenzoylaminosulfonyl-, Methylbenzoylaminosulfonyl-, Methoxybenzoylaminosulfonyl-, Acetyl-, Propionyl-, n-Butanoyl-, n-Pentanoyl-, n-Hexanoyl-, n-Heptanoyl-, Phenylacetyl-, 2-Phenylpropionyl-, Cyclopropylcarbonyl-, Cyclobutylcarbonyl-, Cyclopentylcarbonyl-, Cyclohexylcarbonyl-, Cycloheptylcarbonyl-, Cyclopropylmethylcarbonyl-, Cyclobutylmethylcarbonyl-, Cyclopentylmethylcarbonyl-, Cyclohexylmethylcarbonyl-, Cycloheptylmethylcarbonyl-, (2-Cyclopropylethyl)-carbonyl-, (2-Cyclobutylethyl)-carbonyl-, (2-Cyclopentylethyl)-carbonyl-, (2-Cyclohexylethyl)-carbonyl-, (2-Cycloheptylethyl)-carbonyl-, (3-Cyclopropylpropyl)-carbonyl-, (3-Cyclobutylpropyl)-carbonyl-, (3-Cyclopentylpropyl)-carbonyl-, (3-Cyclohexylpropyl)-carbonyl-, (3-Cycloheptylpropyl)carbonyl-, Benzoyl-, Fluorbenzoyl-, Chlorbenzoyl-, Brombenzoyl-, Methylbenzoyl-, Methoxybenzoyl-, Phenylsulfonyl-, Fluorphenylsulfonyl-, Chlorphenylsulfonyl-, Bromphenylsulfonyl-, Methylphenylsulfonyl-, Methoxyphenylsulfonyl-, Amino-, 2-(Imidazol-1-yl)-ethylamino-, 2-(Imidazol-1-yl)-isopropylamino-, 3-(Imidazol-1-yl)-propylamino-, (Imidazol-4-yl)-methylamino-, 2-(Imidazol-4-yl)-ethylamino-, 2-(Imidazol-4-yl)-isopropylamino-, 3-(Imidazol-4-yl)-propylamino-, Acetylamino-, Propionylamino-, n-Butanoylamino-, Isobutanoylamino-, n-Pentanoylamino-, n-Hexanoylamino-, n-Heptanoylamino-, Cyclopropylcarbonylamino-, Cyclobutylcarbonylamino-, Cyclopentylcarbonylamino-, Cyclohexylcarbonylamino-, Cycloheptylcarbonylamino-, Cyclopropylmethylcarbonylamino-, Cyclobutylmethylcarbonylamino-, Cyclopentylmethylcarbonylamino-, Cyclohexylmethylcarbonylamino-, Cycloheptylmethylcarbonylamino-, (2-Cyclopropylethyl)-carbonylamino-, (2-Cyclobutylethyl)-carbonylamino-, (2-Cyclopentylethyl)-carbonylamino-, (2-Cyclohexylethyl)-carbonylamino-, (2-Cycloheptylethyl)-carbonylamino-, (3-Cyclopropylpropyl)-carbonylamino-, (3-Cyclobutylpropyl)-carbonylamino-, (3-Cyclopentylpropyl)-carbonylamino-, (3-Cyclohexylpropyl)-carbonylamino-, (3-Cycloheptylpropyl)-carbonylamino-, Benzoylamino-, Fluorbenzoylamino-, Chlorbenzoylamino-, Brombenzoylamino-, Methylbenzoylamino-, Hydroxybenzoylamino-, Methoxybenzoylamino-, Phenylacetylamino-, Phenylpropionylamino-, Naphthylcarbonylamino-, Methoxycarbonylamino-, Ethoxycarbonylamino-, n-Propoxycarbonylamino-, Cyclopropyloxycarbonylamino-, Cyclobutyloxycarbonylamino-, Cyclopentoxycarbonylamino-, Cyclohexoxycarbonylamino-, Cycloheptoxycarbonylamino-, Cyclopropylmethoxycarbonylamino-, Cyclobutylmethoxycarbonylamino-, Cyclopentylmethoxycarbonylamino-, Cyclohexylmethoxycarbonylamino-, Cycloheptylmethoxycarbonylamino-, (2-Cyclopropylethoxy)-carbonylamino-, (2-Cyclobutylethoxy)-carbonylamino-, (2-Cyclopentylethoxy)-carbonylamino-, (2-Cyclohexylethoxy)-carbonylamino-, (2-Cycloheptylethoxy)-carbonylamino-, (3-Cyclopropylpropoxy)-carbonylamino-, (3-Cyclobutylpropoxy)-carbonylamino-, (3-Cyclopentylpropoxy)-carbonylamino-, (3-Cyclohexylpropoxy)-carbonylamino-, (3-Cycloheptylpropoxy)-carbonylamino-, Phenoxycarbonylamino-, Fluorphenoxycarbonylamino-, Chlorphenoxycarbonylamino-, Bromphenoxycarbonylamino-, Methylphenoxycarbonylamino-, Hydroxyphenoxycarbonylamino-, Methoxyphenoxycarbonylamino-, Benzyloxyphenoxycarbonylamino-, Benzyloxycarbonylamino-, (2-Phenylethoxy)-carbonylamino-, Trifluoracetylamino-, Decalinylamino-, Adamantylamino-, Methylsulfonylamino-, Ethylsulfonylamino-, n-Propylsulfonylamino-, n-Butylsulfonylamino-, Methylamino-, Ethylamino-, n-Propylamino-, Isopropylamino-, n-Butylamino-, Isobutylamino-, n-Pentylamino-, n-Hexylamino-, Dimethylamino-, Diethylamino-, Di-n-propylamino-, Methyl-ethylamino-, Methyl-isopropylamino-, Methyl-n-butylamino-, Ethyl-n-propylamino-, N-n-Propylsulfonyl-methylamino-, Methyl-n-pentylamino-, Ethyl-n-hexylamino-, N-Methylsulfonyl-methylamino-, N-Ethylsulfonyl-methylamino-, N-n-Propylsulfonyl-methylamino-, N-Methylsulfonyl-ethylamino-, N-Ethylsulfonyl-ethylamino-, N-n-Propylsulfonylethylamino-, N-Methylsulfonyl-n-propylamino-, N-Ethylsulfonyl-n-propylamino-, N-n-Propylsulfonyl-n-propylamino-, N-Methylsulfonyl-n-butylamino-, N-Ethylsulfonyl-n-pentylamino-, N-n-Propylsulfonyl-n-hexylamino-, N-Acetyl-methylamino-, N-Acetyl-ethylamino-, N-Acetyl-n-hexylamino-, N-Propionylmethylamino-, N-Propionyl-ethylamino-, N-Propionyl-n-butylamino-, N-n-Butanoyl-methylamino-, N-n-Butanoyl-ethylamino-, N-n-Butanoyl-n-pentylamino-, N-Isobutanoyl-methylamino-, N-Isobutanoyl-ethylamino-, N-Isobutanoyl-isopropylamino-, N-Isobutanoyl-n-pentylamino-, N-n-Pentanoyl-methylamino-, N-n-Pentanoyl-ethylamino-, N-n-Pentanoyl-isopropylamino-, N-n-Pentanoyl-n-pentylamino-, N-n-Hexanoyl-methylamino-, N-n-Hexanoyl-ethylamino-, N-n-Hexanoyl-isopropylamino-, N-n-Hexanoyl-n-pentylamino-, N-n-Heptanoyl-methylamino-, N-n-Heptanoyl-ethylamino-, N-n-Heptanoyl-isopropylamino-, N-n-Heptanoyl-n-pentylamino-, N-Cyclopropylcarbonylmethylamino-, N-Cyclobutylcarbonyl-methylamino-, N-Cyclopentylcarbonyl-methylamino-, N-Cyclohexylcarbonyl-methylamino-, N-Cycloheptylcarbonyl-methylamino-, N-Cyclopropylmethylcarbonyl-methylamino-, N-Cyclobutylmethylcarbonyl-methylamino-, N-Cyclopentylmethylcarbonyl-methylamino-, N-Cyclohexylmethylcarbonyl-methylamino-, N-Cycloheptylmethylcarbonyl-methylamino-, N-(2-Cyclopropylethylcarbonyl)-methylamino-, N-(2-Cyclobutylethylcarbonyl)-methylamino-, N-(2-Cyclopentylethylcarbonyl)-methylamino-, N-(2-Cyclohexylethylcarbonyl)-methylamino-, N-(2-Cycloheptylethylcarbonyl)-methylamino-, N-(3-Cyclopropylpropylcarbonyl)-methylamino-, N-(3-Cyclobutylpropylcarbonyl)-methylamino-, N-(3-Cyclopentylpropylcarbonyl)-methylamino-, N-(3-Cyclohexylpropylcarbonyl)-methylamino-, N-(3-Cycloheptylpropylcarbonyl)-methylamino-, N-Benzoyl-methylamino-, N-Benzoyl-ethylamino-, N-Benzoyl-isopropylamino-, N-Benzoyl-n-butylamino-, N-Benzoyl-n-pentylamino-, N-Benzoyl-n-hexylamino-, N-Fluorbenzoyl-methylamino-, N-Methylbenzoyl-ethylamino-, N-Methoxybenzoyl-isopropylamino-, N-Chlorbenzoyl-n-butylamino-, N-Fluorbenzoyl-n-pentylamino-, N-Brombenzoyl-n-hexylamino-, N-Phenylacetyl-methylamino-, N-Phenylsulfonyl-methylamino-, N-(2-Hydroxyethyl)-methylamino-, N-(2-Hydroxyethyl)-ethylamino-, N-(2-Methoxyethyl)-methylamino-, N-(2-Methoxyethyl)-ethylamino-, N-Hydroxyacetyl-methylamino-, N-Hydroxyacetyl-ethylamino-, N-Methoxyacetyl-methylamino-, N-Methoxyacetyl-ethylamino-, Cyclopropylamino-, Cyclobutylamino-, Cyclopentylamino-, Cyclohexylamino-, Cycloheptylamino-, Cyclopropylmethylamino-, Cyclobutylmethylamino-, Cyclopentylmethylamino-, Cyclohexylmethylamino-, Cycloheptylmethylamino-, (2-Cyclopropylethyl)-amino-, (2-Cyclobutylethyl)-amino-, (2-Cyclopentylethyl)-amino-, (2-Cyclohexylethyl)-amino-, (2-Cycloheptylethyl)-amino-, (3-Cyclopropylpropyl)-amino-, (3-Cyclopentylpropyl)-amino-, (3-Cyclohexylpropyl)-amino-, (3-Cycloheptylpropyl)-amino-, Benzylamino-, 2-Phenylethylamino-, 3-Phenylpropylamino-, Phenylamino-, Dicyclohexylamino-, Dicyclohexylmethylamino-, Dibenzylamino-, N-Methyl-cyclopropylamino-, N-Isopropyl-cyclopropylamino-, N-Ethyl-cyclobutylamino-, N-Methyl-cyclopentylamino-, N-Ethyl-cyclohexylamino-, N-(n-Propyl)-cycloheptylamino-, N-Methyl-cyclopropylmethylamino-, N-Isopropyl-cyclopropylmethylamino-, N-Ethyl-cyclobutylmethylamino-, N-Methyl-cyclopentylmethylamino-, N-Ethyl-cyclohexylmethylamino-, N-(n-Propyl)-cycloheptylmethylamino-, N-Methyl-(2-cyclopropylethyl)-amino-, N-Isopropyl-(2-cyclopropylethyl)-amino-, N-Ethyl-(2-cyclobutylethyl)-amino-, N-Methyl-(2-cyclopentylethyl)-amino-, N-Ethyl-(2-cyclohexylethyl)-amino-, N-(n-Propyl)-(2-cycloheptylethyl)-amino-, N-Methyl-(3-cyclopropylpropyl)-amino-, N-Isopropyl-(3-cyclopropylpropyl)-amino-, N-Ethyl-(3-cyclobutylpropyl)-amino-, N-Methyl-(3-cyclopentylpropyl)-amino-, N-Ethyl-(3-cyclohexylpropyl)-amino-, N-(n-Propyl)-(3-cycloheptylpropyl)-amino-, N-Methyl-benzylamino-, N-Ethyl-benzylamino-, N-Isopropyl-benzylamino-, N-Methyl-(2-phenylethyl)-amino-, N-Methyl-phenylamino-, N-(n-Propyl)-phenylamino-, Pyrrolidino-, Methylpyrrolidino-, Ethylpyrrolidino-, Isopropylpyrrolidino-, Cyclopentylpyrrolidino-, Cyclohexylpyrrolidino-, Cycloheptylpyrrolidino-, Phenylpyrrolidino-, Piperidino-, Methylpiperidino-, Ethylpiperidino-, Isopropylpiperidino-, Cyclopentylpiperidino-, Cyclohexylpiperidino-, Cycloheptylpiperidino-, Phenylpiperidino-, Hexamethylenimino-, Methylhexamethylenimino-, Ethylhexamethylenimino-, Isopropylhexamethylenimino-, Cyclopentylhexamethylenimino-, Cyclohexylhexamethylenimino-, Cycloheptylhexamethylenimino-, Phenylhexamethylenimino, N-Methoxycarbonyl-methylamino-, N-Methoxycarbonyl-N-ethylamino-, N-Methoxycarbonyl-n-pentyl-amino-, N-Methoxycarbonyl-n-hexylamino-, N-Ethoxycarbonyl-methylamino-, N-Ethoxycarbonyl-ethylamino-, N-Cyclopropyloxycarbonyl-methylamino-, N-Cyclopropyloxycarbonyl-ethylamino-, N-Cyclopropyloxycarbonyl-n-propylamino-, N-Cyclobutyloxycarbonyl-methylamino-, N-Cyclobutyloxycarbonyl-isopropylamino-, N-Cyclopentyloxycarbonyl-methylamino-, N-Cyclopentyloxycarbonyl-ethylamino-, N-Cyclohexyloxycarbonyl-methylamino-, N-Cyclohexyloxycarbonyl-ethylamino-, N-Cycloheptyloxycarbonyl-methylamino-, N-Cyclopentylmethyloxycarbonyl-methylamino-, N-Cyclopentylmethyloxycarbonyl-ethylamino-, N-Cyclohexylmethyloxycarbonyl-methylamino-, N-Cyclohexylmethyloxycarbonyl-ethylamino-, N-Cycloheptylmethyloxycarbonyl-methylamino-, N-Cycloheptylmethyloxycarbonyl-ethylamino-, N-(2-Cyclopentylethyloxy)-carbonyl-methylamino-, N-(2-Cyclopentylethyloxy)-carbonyl-ethylamino-, N-(2-Cyclohexylethyloxy)-carbonyl-methylamino-, N-(2-Cyclohexylethyloxy)-carbonyl-ethylamino-, N-(2-Cycloheptylethyloxy)-carbonyl-methylamino-, N-(2-Cycloheptylethyloxy)-carbonyl-ethylamino-, N-Phenoxycarbonyl-methylamino-, N-Phenoxycarbonyl-ethylamino-, N-Phenoxycarbonyl-isopropylamino-, N-Benzyloxycarbonyl-methylamino-, N-Benzyloxycarbonyl-ethylamino-, N-Benzyloxycarbonyl-isopropylamino-, N-(2-Phenylethoxy)-carbonyl-methylamino-, N-(2-Phenylethoxy)-carbonyl-ethylamino-, N-(2-Phenylethoxy)-carbonyl-isopropylamino-, N-(3-Phenylpropoxy)-carbonyl-methylamino-, N-(3-Phenylpropoxy)-carbonyl-ethylamino-, N-(3-Phenylpropoxy)-carbonyl-isopropylamino-, N-Methoxycarbonyl-cyclopropylamino-, N-Methoxycarbonyl-cyclobutylamino-, N-Methoxycarbonyl-cyclopentylamino-, N-Methoxycarbonyl-cyclohexylamino-, N-Methoxycarbonyl-cycloheptylamino-, N-Ethoxycarbonyl-cyclopropylamino-, N-Ethoxycarbonyl-cyclobutylamino-, N-Ethoxycarbonyl-cyclopentylamino-, N-Ethoxycarbonylcyclohexylamino-, N-Ethoxycarbonyl-cycloheptylamino-, N-Methoxycarbonyl-cyclopropylmethylamino-, N-Methoxycarbonyl-cyclobutylmethylamino-, N-Methoxycarbonyl-cyclopentylmethylamino-, N-Methoxycarbonyl-cyclohexylmethylamino-, N-Methoxycarbonyl-cycloheptylmethylamino-, N-Ethoxycarbonyl-cyclopropylmethylamino-, N-Ethoxycarbonyl-cyclobutylmethylamino-, N-Ethoxycarbonyl-cyclopentylmethylamino-, N-Ethoxycarbonyl-cyclohexylmethylamino-, N-Ethoxycarbonyl-cycloheptylmethylamino-, N-Methoxycarbonyl-(2-cyclopropylethyl)-amino-, N-Methoxycarbonyl-(2-cyclobutylethyl)-amino-, N-Methoxycarbonyl-(2-cyclopentylethyl)-amino-, N-Methoxycarbonyl-(2-cyclohexylethyl)-amino-, N-Methoxycarbonyl-(2-cycloheptylethyl)-amino-, N-Ethoxycarbonyl-(2-cyclopropylethyl)-amino-, N-Ethoxycarbonyl-(2-cyclobutylethyl)-amino-, N-Ethoxycarbonyl-(2-cyclopentylethyl)-amino-, N-Ethoxycarbonyl-(2-cyclohexylethyl)-amino-, N-Ethoxycarbonyl-(2-cycloheptylethyl)-amino-, N-Methoxycarbonyl-(3-cyclopropylpropyl)-amino-, N-Methoxycarbonyl-(3-cyclobutylpropyl)-amino-, N-Methoxycarbonyl-(3-cyclopentylpropyl)-amino-, N-Methoxycarbonyl-(3-cyclohexylpropyl)-amino-, N-Methoxycarbonyl-(3-cycloheptylpropyl)-amino-, N-Ethoxycarbonyl-(3-cyclopropylpropyl)-amino-, N-Ethoxycarbonyl-(3-cyclobutylpropyl)-amino-, N-Ethoxycarbonyl-(3-cyclopentylpropyl)-amino-, N-Ethoxycarbonyl-(3-cyclohexylpropyl)-amino-, N-Ethoxycarbonyl-(3-cycloheptylpropyl)-amino-, N-Phenoxycarbonyl-cyclopropylamino-, N-Phenoxycarbonyl-cyclobutylamino-, N-Phenoxycarbonyl-cyclopentylamino-, N-Phenoxycarbonyl-cyclohexylamino-, N-Phenoxycarbonyl-cycloheptylamino-, N-Benzyloxycarbonyl-cyclopropylamino-, N-Benzyloxycarbonyl-cyclobutylamino-, N-Benzyloxycarbonyl-cyclopentylamino-, N-Benzyloxycarbonyl-cyclohexylamino-, N-Benzyloxycarbonyl-cycloheptylamino-, N-(2-Phenylethoxy)-carbonyl-cyclopropylamino-, N-(2-Phenylethoxy)-carbonyl-cyclobutylamino-, N-(2-Phenylethoxy)-carbonyl-cyclopentylamino-, N-(2-Phenylethoxy)-carbonyl-cyclohexylamino-, N-(2-Phenylethoxy)-carbonyl-cycloheptylamino-, N-(3-Phenylpropoxy)-carbonyl-cyclopropylamino-, N-(3-Phenylpropoxy)-carbonyl-cyclobutylamino-, N-(3-Phenylpropoxy)-carbonyl-cyclopentylamino-, N-(3-Phenylpropoxy)-carbonyl-cyclohexylamino-, N-(3-Phenylpropoxy)-carbonyl-cycloheptylamino-, N-Methylsulfonyl-cyclopropylamino-, N-Ethylsulfonyl-cyclobutylamino-, N-n-Propylsulfonyl-cyclopentylamino-, N-Ethylsulfonyl-cyclohexylamino-, N-Methylsulfonyl-cycloheptylamino-, N-Phenoxycarbonyl-cyclopropylmethylamino-, N-Phenoxycarbonyl-cyclobutylmethylamino-, N-Phenoxycarbonyl-cyclopentylmethylamino-, N-Phenoxycarbonyl-cyclohexylmethylamino-, N-Phenoxycarbonyl-cycloheptylmethylamino-, N-Benzyloxycarbonyl-cyclopropylmethylamino-, N-Benzyloxycarbonyl-cyclobutylmethylamino-, N-Benzyloxycarbonyl-cyclopentylmethylamino-, N-Benzyloxycarbonyl-cyclohexylmethylamino-, N-Benzyloxycarbonyl-cycloheptylmethylamino-, N-(2-Phenylethoxycarbonyl)-cyclopropylmethylamino-, N-(2-Phenylethoxycarbonyl)-cyclobutylmethylamino-, N-(2-Phenylethoxycarbonyl)-cyclopentylmethylamino-, N-(2-Phenylethoxycarbonyl)-cyclohexylmethylamino-, N-(2-Phenylethoxycarbonyl)-cycloheptylmethylamino-, N-(3-Phenylpropoxycarbonyl)-cyclopropylmethylamino-, N-(3-Phenylpropoxycarbonyl)-cyclobutylmethylamino-, N-(3-Phenylpropoxycarbonyl)-cyclopentylmethylamino-, N-(3-Phenylpropoxycarbonyl)-cyclohexylmethylamino-, N-(3-Phenylpropoxycarbonyl)-cycloheptylmethylamino-, N-Methylsulfonyl-cyclopropylmethylamino-, N-Ethylsulfonyl-cyclobutylmethylamino-, N-Methylsulfonyl-cyclopentylmethylamino-, N-Ethylsulfonyl-cyclohexylmethylamino-, N-Isopropylsulfonyl-cycloheptylmethylamino-, N-Phenoxycarbonyl-(2-cyclopropylethyl)-amino-, N-Phenoxycarbonyl-(2-cyclobutylethyl)-amino-, N-Phenoxycarbonyl-(2-cyclopentylethyl)-amino-, N-Phenoxycarbonyl-(2-cyclohexylethyl)-amino-, N-Phenoxycarbonyl-(2-cycloheptylethyl)-amino-, N-Benzyloxycarbonyl-(2-cyclopropylethyl)-amino-, N-Benzyloxycarbonyl-(2-cyclobutylethyl)-amino-, N-Benzyloxycarbonyl-(2-cyclopentylethyl)-amino-, N-Benzyloxycarbonyl-(2-cyclohexylethyl)-amino-, N-Benzyloxycarbonyl-(2-cycloheptylethyl)-amino-, N-(2-Phenylethoxy-carbonyl)-(2-cyclopropylethyl)-amino-, N-(2-Phenylethoxy-carbonyl)-(2-cyclobutylethyl)-amino-, N-(2-Phenylethoxy-carbonyl)-(2-cyclopentylethyl)-amino-, N-(2-Phenylethoxy-carbonyl)-(2-cyclohexylethyl)-amino-, N-(2-Phenylethoxy-carbonyl)-(2-cycloheptylethyl)-amino-, N-(3-Phenylpropoxy-carbonyl)-(2-cyclopropylethyl)-amino-, N-(3-Phenylpropoxy-carbonyl)-(2-cyclobutylethyl)-amino-, N-(3-Phenylpropoxy-carbonyl)-(2-cyclopentylethyl)-amino-, N-(3-Phenylpropoxy-carbonyl)-(2-cyclohexylethyl)-amino-, N-(3-Phenylpropoxy-carbonyl)-(2-cycloheptylethyl)-amino-, N-Methylsulfonyl-(2-cyclopropylethyl)-amino-, N-Ethylsulfonyl-(2-cyclobutylethyl)-amino-, N-Isopropylsulfonyl-(2-cyclopentylethyl)-amino-, N-Methylsulfonyl-(2-cyclohexylethyl)-amino-, N-Methylsulfonyl-(2-cycloheptylethyl)-amino-, N-Phenoxycarbonyl-(3-cyclopropylpropyl)-amino-, N-Phenoxycarbonyl-(3-cyclobutylpropyl)-amino-, N-Phenoxycarbonyl-(3-cyclopentylpropyl)-amino-, N-Phenoxycarbonyl-(3-cyclohexylpropyl)-amino-, N-Phenoxycarbonyl-(3-cycloheptylpropyl)-amino-, N-Benzyloxycarbonyl-(3-cyclopropylpropyl)-amino-, N-Benzyloxycarbonyl-(3-cyclobutylpropyl)-amino-, N-Benzyloxycarbonyl-(3-cyclopentylpropyl)-amino-, N-Benzyloxycarbonyl-(3-cyclohexylpropyl)-amino-, N-Benzyloxycarbonyl-(3-cycloheptylpropyl)-amino-, N-(2-Phenylethoxycarbonyl)-(3-cyclopropylpropyl)-amino-, N-(2-Phenylethoxycarbonyl)-(3-cyclobutylpropyl)-amino-, N-(2-Phenylethoxycarbonyl)-(3-cyclopentylpropyl)-amino-, N-(2-Phenylethoxycarbonyl)-(3-cyclohexylpropyl)-amino-, N-(2-Phenylethoxycarbonyl)-(3-cycloheptylpropyl)-amino-, N-(3-Phenylpropoxycarbonyl)-(3-cyclopropylpropyl)-amino-, N-(3-Phenylpropoxycarbonyl)-(3-cyclobutylpropyl)-amino-, N-(3-Phenylpropoxycarbonyl)-(3-cyclopentylpropyl)-amino-, N-(3-Phenylpropoxycarbonyl)-(3-cyclohexylpropyl)-amino-, N-(3-Phenylpropoxycarbonyl)-(3-cycloheptylpropyl)-amino-, N-Methylsulfonyl-(3-cyclopropylpropyl)-amino-, N-Ethylsulfonyl-(3-cyclobutylpropyl)-amino-, N-Isopropylsulfonyl-(3-cyclopentylpropyl)-amino-, N-Methylsulfonyl-(3-cyclohexylpropyl)-amino-, N-Methylsulfonyl-(3-cycloheptylpropyl)-amino-, N-Benzoyl-cyclopropylamino-, N-Benzoyl-cyclobutylamino-, N-Benzoyl-cyclopentylamino-, N-Benzoyl-cyclohexylamino-, N-Benzoyl-cycloheptylamino-, N-Phenylacetyl-cyclopropylamino-, N-Phenylacetyl-cyclobutylamino-, N-Phenylacetyl-cyclopentylamino-, N-Phenylacetyl-cyclohexylamino-, N-Phenylacetyl-cycloheptylamino-, N-Phenylsulfonyl-cyclopropylamino-, N-Phenylsulfonyl-cyclobutylamino-, N-Phenylsulfonyl-cyclopentylamino-, N-Phenylsulfonyl-cyclohexylamino-, N-Phenylsulfonyl-cycloheptylamino-, N-Benzoyl-cyclopropylmethylamino-, N-Benzoyl-cyclobutylmethylamino-, N-Benzoyl-cyclopentylmethylamino-, N-Benzoyl-cyclohexylmethylamino-, N-Benzoyl-cycloheptylmethylamino-, N-Phenylacetyl-cyclopropylmethylamino-, N-Phenylacetyl-cyclobutylmethylamino-, N-Phenylacetyl-cyclopentylmethylamino-, N-Phenylacetyl-cyclohexylmethylamino-, N-Phenylacetyl-cycloheptylmethylamino-, N-Phenylsulfonyl-cyclopropylmethylamino-, N-Phenylsulfonyl-cyclobutylmethylamino-, N-Phenylsulfonyl-cyclopentylmethylamino-, N-Phenylsulfonyl-cyclohexylmethylamino-, N-Phenylsulfonyl-cycloheptylmethylamino-, N-Benzoyl-(2-cyclopropylethyl)-amino-, N-Benzoyl-(2-cyclobutylethyl)-amino-, N-Benzoyl-(2-cyclopentylethyl)-amino-, N-Benzoyl-(2-cyclohexylethyl)-amino-, N-Benzoyl-(2-cycloheptylethyl)-amino-, N-Phenylacetyl-(2-cyclopropylethyl)-amino-, N-Phenylacetyl-(2-cyclobutylethyl)-amino-, N-Phenylacetyl-(2-cyclopentylethyl)-amino-, N-Phenylacetyl-(2-cyclohexylethyl)-amino-, N-Phenylacetyl-(2-cycloheptylethyl)-amino-, N-Phenylsulfonyl-(2-cyclopropylethyl)-amino-, N-Phenylsulfonyl-(2-cyclobutylethyl)-amino-, N-Phenylsulfonyl-(2-cyclopentylethyl)-amino-, N-Phenylsulfonyl-(2-cyclohexylethyl)-amino-, N-Phenylsulfonyl-(2-cycloheptylethyl)-amino-, N-Benzoyl-(3-cyclopropylpropyl)-amino-, N-Benzoyl-(3-cyclobutylpropyl)-amino-, N-Benzoyl-(3-cyclopentylpropyl)-amino-, N-Benzoyl-(3-cyclohexylpropyl)-amino-, N-Benzoyl-(3-cycloheptylpropyl)-amino-, N-Phenylacetyl-(3-cyclopropylpropyl)-amino-, N-Phenylacetyl-(3-cyclobutylpropyl)-amino-, N-Phenylacetyl-(3-cyclopentylpropyl)-amino-, N-Phenylacetyl-(3-cyclohexylpropyl)-amino-, N-Phenylacetyl-(3-cycloheptylpropyl)-amino-, N-Phenylsulfonyl-(3-cyclopropylpropyl)-amino-, N-Phenylsulfonyl-(3-cyclobutylpropyl)-amino-, N-Phenylsulfonyl-(3-cyclopentylpropyl)-amino-, N-Phenylsulfonyl-(3-cyclohexylpropyl)-amino-, N-Phenylsulfonyl-(3-cycloheptylpropyl)-amino-, N-Acetyl-cyclopropylamino-, N-Acetyl-cyclobutylamino-, N-Acetyl-cyclopentylamino-, N-Acetyl-cyclohexylamino-, N-Acetyl-cycloheptylamino-, N-Acetyl-cyclopropylmethylamino-, N-Acetyl-cyclobutylmethylamino-, N-Acetyl-cyclopentylmethylamino-, N-Acetyl-cyclohexylmethylamino-, N-Acetyl-cycloheptylmethylamino-, N-Acetyl-(2-cyclopropylethyl)-amino-, N-Acetyl-(2-cyclobutylethyl)-amino-, N-Acetyl-(2-cyclopentylethyl)-amino-, N-Acetyl-(2-cyclohexylethyl)-amino-, N-Acetyl-(2-cycloheptylethyl)-amino-, N-Acetyl-(3-cyclopropylpropyl)-amino-, N-Acetyl-(3-cyclobutylpropyl)-amino-, N-Acetyl-(3-cyclopentylpropyl)-amino-, N-Acetyl-(3-cyclohexylpropyl)-amino-, N-Acetyl-(3-cycloheptylpropyl)-amino-, N-Acetyl-benzylamino-, N-Acetyl-(2-phenylethyl)-amino-, N-Acetyl-(3-phenylpropyl)-amino-, N-Benzoyl-benzylamino-, N-Benzoyl-(2-phenylethyl)-amino-, N-Benzoyl-(3-phenylpropyl)-amino-, N-Methylsulfonyl-benzylamino-, N-Methylsulfonyl-(2-phenylethyl)-amino-, N-Methylsulfonyl-(3-phenylpropyl)-amino-, Carboxy-, Methoxycarbonyl-, Ethoxycarbonyl-, n-Propoxycarbonyl-, Aminocarbonyl-, Methylaminocarbonyl-, Ethylaminocarbonyl-, n-Propylaminocarbonyl-, n-Butylaminocarbonyl-, n-Pentylaminocarbonyl-, Cyclopropylaminocarbonyl-, Cyclobutylaminocarbonyl-, Cyclopentylaminocarbonyl-, Cyclohexylaminocarbonyl-, Cycloheptylaminocarbonyl-, Cyclopentylmethylaminocarbonyl-, Cyclohexylmethylaminocarbonyl-, Cycloheptylmethylaminocarbonyl-, 2-Cyclohexylethylaminocarbonyl-, Phenylaminocarbonyl-, Fluorphenylaminocarbonyl-, Chlorphenylaminocarbonyl-, Bromphenylaminocarbonyl-, Methylphenylaminocarbonyl-, Ethylphenylaminocarbonyl-, Isopropylphenylaminocarbonyl-, Methoxyphenylaminocarbonyl-, Ethoxyphenylaminocarbonyl-, Isopropoxyphenylaminocarbonyl-, n-Butoxyphenylaminocarbonyl-, Benzylaminocarbonyl-, (2-Phenylethyl)-aminocarbonyl-, Dimethylaminocarbonyl-, Diethylaminocarbonyl-, Diisopropylaminocarbonyl-, N-Methyl-ethylaminocarbonyl-, N-Methyl-n-propylaminocarbonyl-, N-Methyl-n-butylaminocarbonyl-, Aminoacetylamino-, N-Methoxycarbonyl-aminoacetylamino-, N-Ethoxycarbonyl-aminoacetylamino-, N-Isopropoxycarbonyl-aminoacetylamino-, Aminocarbonylamino-, Methylaminocarbonylamino-, Dimethylaminocarbonylamino-, N-Methylaminocarbonyl-methylamino-, N-(Dimethylaminocarbonyl)-methylamino-, N-Dimethylaminocarbonyl-ethylamino-, N-Dimethylaminocarbonyl-isopropylamino-, N-(Dimethylaminocarbonyl)-n-pentylamino-, N-Methylaminocarbonyl-ethylamino-, N-Methylaminocarbonyl-n-pentylamino-, N-Methylaminocarbonyl-n-hexylamino-, N-Methylaminocarbonyl-n-octylamino-, N-Methylaminocarbonyl-n-dodecylamino-, N-Methylaminocarbonyl-cyclohexylamino-, Ethylaminocarbonylamino-, N-Ethylaminocarbonylmethylamino-, N-Ethylaminocarbonyl-ethylamino-, N-Ethylaminocarbonyl-n-hexylamino-, N-Ethylaminocarbonyl-n-octylamino-, N-Ethylaminocarbonyl-n-dodecylamino-, N-Ethylaminocarbonylcyclohexylamino-, Diethylaminocarbonylamino-, N-(Diethylaminocarbonyl)-methylamino-, N-(Diethylaminocarbonyl)-ethylamino-, N-(Diethylaminocarbonyl)-n-butylamino-, N-(Diethylaminocarbonyl)-n-hexylamino-, N-(Diethylaminocarbonyl)-n-octylamino-, N-(Diethylaminocarbonyl)-n-dodecylamino-, Isopropylaminocarbonylamino-, N-Isopropylaminocarbonyl-methylamino-, n-Butylaminocarbonylamino-, N-(n-Butylaminocarbonyl)-methylamino-, N-(n-Butylaminocarbonyl)-ethylamino-, N-(n-Butylaminocarbonyl)-isopropylamino-, N-(n-Butylaminocarbonyl)-n-butylamino-, N-(n-Butylaminocarbonyl)-n-hexylamino-, N-(n-Butylaminocarbonyl)-n-octylamino-, N-(n-Butylaminocarbonyl)-n-dodecylamino-, N-(n-Butylaminocarbonyl)-cyclohexylamino-, N-(Di-(n-Butyl)-aminocarbonyl)-amino-, N-(Di-(n-Butyl)-aminocarbonyl)-methylamino-, N-(Di-(n-Butyl)-aminocarbonyl)-ethylamino-, N-(Di-(n-Butyl)-aminocarbonyl)-n-butylamino-, N-(Di-(n-Butyl)-aminocarbonyl)-n-hexylamino-, N-(Di-(n-Butyl)-aminocarbonyl)-n-octylamino-, N-(Di-(n-Butyl)-aminocarbonyl)-n-dodecylamino-, N-(n-Pentylaminocarbonyl)-ethylamino-, N-(n-Hexylaminocarbonyl)-ethylamino-, N-(n-Octylaminocarbonyl)-ethylamino-, N-(n-Dodecylaminocarbonyl)-ethylamino-, n-Hexylaminocarbonylamino-, n-Octylaminocarbonylamino-, n-Dodecylaminocarbonylamino-, N-(n-Hexylaminocarbonyl)-n-butylamino-, N-(n-Hexylaminocarbonyl)-n-pentylamino-, N-(n-Hexylaminocarbonyl)-n-hexylamino-, N-(n-Hexylaminocarbonyl)-n-octylamino-, N-(n-Hexylaminocarbonyl)-n-dodecylamino-, N-(n-Octylaminocarbonyl)-n-butylamino-, N-(n-Octylaminocarbonyl)-n-pentylamino-, N-(n-Octylaminocarbonyl)-n-hexylamino-, N-(n-Octylaminocarbonyl)-n-octylamino-, N-(n-Octylaminocarbonyl)-n-dodecylamino-, N-(n-Dodecylaminocarbonyl)-n-butylamino-, N-(n-Dodecylaminocarbonyl)-n-pentylamino-, N-(n-Dodecylaminocarbonyl)-n-hexylamino-, N-(n-Dodecylaminocarbonyl)-n-octylamino-, N-(n-Dodecylaminocarbonyl)-n-dodecylamino-, N-(n-Hexylaminocarbonyl)-cyclohexylamino-, N-(n-Octylaminocarbonyl)-cyclohexylamino-, N-(n-Dodecylaminocarbonyl)-cyclohexylamino-, Di-(n-Hexyl)-aminocarbonylamino-, N-(Di-(n-Hexyl)-aminocarbonyl)-methylamino-, N-((n-Hexyl)-methylaminocarbonyl)-amino-, N-Cyclohexylaminocarbonyl-n-pentylamino-, N-Cyclohexylaminocarbonyl-n-hexylamino-, N-Cyclohexylaminocarbonyl-n-octylamino-, N-Cyclohexylaminocarbonyl-n-dodecylamino-, N-Cyclohexylaminocarbonyl-cyclohexylamino-, N-(Ethyl-cyclohexylaminocarbonyl)-methylamino-, N-(Propyl-cyclohexylaminocarbonyl)-methylamino-, N-(n-Butylcyclohexylaminocarbonyl)-methylamino-, Adamantyl-(1)-aminocarbonylamino-, 4-Hydroxybutylaminocarbonylamino-, 5-Hydroxypentylaminocarbonylamino-, 6-Hydroxyhexylaminocarbonylamino-, Allylaminocarbonylamino-, But-2-enylaminocarbonylamino-, Pent-2-enylaminocarbonylamino-, Pent-3-enylaminocarbonylamino-, Crotylaminocarbonylamino-, But-2-inylaminocarbonylamino-, Pent-2-inylaminocarbonylamino-, Pent-3-inylaminocarbonylamino-, Tetrahydrofuran-2-yl-aminocarbonylamino-, Tetrahydropyran-2-yl-aminocarbonylamino-, N-(Ethyl-(n-pentyl)-aminocarbonyl)-ethylamino-, N-(Methyl-cyclopentylaminocarbonyl)-methylamino-, N-(Methyl-cyclohexylaminocarbonyl)-ethylamino-, Cyclopropylaminocarbonylamino-, N-Cyclopropylaminocarbonyl-methylamino-, Cyclobutylaminocarbonylamino-, N-Cyclobutylaminocarbonyl-methylamino-, Cyclopentylaminocarbonylamino-, N-Cyclopentylaminocarbonyl-methylamino-, Cyclohexylaminocarbonylamino-, N-Cyclohexylaminocarbonyl-methylamino-, N-Cyclohexylaminocarbonyl-ethylamino-, N-Cyclohexylaminocarbonyl-n-propylamino-, N-Cyclohexylaminocarbonyl-n-butylamino-, N-Cyclohexylaminocarbonyl-n-pentylamino-, N-Cyclohexylaminocarbonyl-n-hexylamino-, N-Cyclohexylaminocarbonyl-n-octylamino-, N-Cyclohexylaminocarbonyl-n-dodecylamino-, Cycloheptylaminocarbonylamino-, N-Cycloheptylaminocarbonyl-methylamino-, Cyclopentylmethylaminocarbonylamino-, N-Cyclopentylmethylaminocarbonyl-methylamino-, Cyclohexylmethylaminocarbonylamino-, N-Cyclohexylmethylaminocarbonyl-methylamino-, Benzylaminocarbonylamino-, (2-Phenylethyl)-aminocarbonylamino-, Phenylaminocarbonylamino-, Fluorphenylaminocarbonylamino-, Chlorphenylaminocarbonylamino-, Bromphenylaminocarbonylamino-, Methylphenylaminocarbonylamino-, Ethylphenylaminocarbonylamino-, Isopropylphenylaminocarbonylamino-, Methoxyphenylaminocarbonylamino-, Ethoxyphenylaminocarbonylamino-, Isopropoxyphenylaminocarbonylamino-, n-Butoxyphenylaminocarbonylamino-, Aminothiocarbonylamino-, Methylaminothiocarbonylamino-, N-Methylaminocarbonyl-methylamino-, N-Methylaminocarbonyl-ethylamino-, Ethylaminothiocarbonylamino-, N-Ethylaminothiocarbonyl-methylamino-, N-Ethylaminothiocarbonyl-ethylamino-, N-Ethylaminothiocarbonyl-n-hexylamino-, Isopropylaminothiocarbonylamino-, N-Isopropylaminothiocarbonyl-methylamino-, Allylaminothiocarbonylamino-, But-2-enylaminothiocarbonylamino-, Pent-2-enylaminothiocarbonylamino-, Pent-3-enylaminothiocarbonylamino-, Crotylaminothiocarbonylamino-, But-2-inylaminothiocarbonylamino-, Pent-2-inylaminothiocarbonylamino-, Pent-3-inylaminothiocarbonylamino-, Tetrahydrofuran-2-yl-aminothiocarbonylamino-, Tetrahydropyran-2-yl-aminothiocarbonylamino-, Adamantyl-(1)-aminothiocarbonylamino-, Cyclopropylaminothiocarbonylamino-, N-Cyclopropylaminothiocarbonyl-methylamino-, Cyclobutylaminothiocarbonylamino-, N-Cyclobutylaminothiocarbonyl-methylamino-, Cyclopentylaminothiocarbonylamino-, N-Cyclopentylaminothiocarbonyl-methylamino-, Cyclohexylaminothiocarbonylamino-, N-Cyclohexylaminothiocarbonyl-methylamino-, Cycloheptylaminothiocarbonylamino-, N-Cycloheptylaminothiocarbonyl-methylamino-, Cyclopentylmethylaminothiocarbonylamino-, N-Cyclopentylmethylaminothiocarbonyl-methylamino-, Cyclohexylmethylaminothiocarbonylamino-, N-Cyclohexylmethylaminothiocarbonyl-methylamino-, Dimethylaminothiocarbonylamino-, Diethylaminothiocarbonylamino-, N-((n-Hexyl)-aminothiocarbonyl)-amino-, N-((n-Hexyl)-methylaminothiocarbonyl)-amino-, N-(Dimethylaminothiocarbonyl)-methylamino-, N-(Dimethylaminothiocarbonyl)-n-pentylamino-, N-(Diethylaminothiocarbonyl)-methylamino-, N-(Diethylaminothiocarbonyl)-ethylamino-, N-(Di-(n-hexyl)-aminothiocarbonyl)-methylamino-, N-(Di-(n-butyl)-aminothiocarbonyl)-n-butylamino-, N-((n-Hexyl)-methylaminothiocarbonyl)-methylamino-, N-(Ethyl-(n-pentyl)-aminothiocarbonyl)-ethylamino-, N-(Methyl-cyclopentylaminothiocarbonyl)-methylamino-, N-(Methyl-cyclohexylaminothiocarbonyl)-ethylamino-, Benzylaminothiocarbonylamino-, Phenylaminothiocarbonylamino-, Fluorphenylaminothiocarbonylamino-, Chlorphenylaminothiocarbonylamino-, Bromphenylaminothiocarbonylamino-, Methylphenylaminothiocarbonylamino-, Ethylphenylaminothiocarbonylamino-, Isopropylphenylaminothiocarbonylamino-, Methoxyphenylaminothiocarbonylamino-, Ethoxyphenylaminothiocarbonylamino-, Isopropoxyphenylaminothiocarbonylamino-, n-Butoxyphenylaminothiocarbonylamino-, Pyrrolidinocarbonylamino-, Piperidinocarbonylamino-, Hexamethyleniminocarbonylamino-, N-Pyrrolidinocarbonyl-methylamino-, N-Pyrrolidinocarbonyl-ethylamino-, N-Pyrrolidinocarbonyl-isopropylamino-, N-Pyrrolidinocarbonyl-n-butylamino-, N-Pyrrolidinocarbonyl-n-pentylamino-, N-Pyrrolidinocarbonyl-n-hexylamino-, N-Pyrrolidinocarbonyl-n-octylamino-, N-Pyrrolidinocarbonyl-n-dodecylamino-, N-Pyrrolidinocarbonyl-cyclopropylamino-, N-Pyrrolidinocarbonyl-cyclobutylamino-, N-Pyrrolidinocarbonyl-cyclopentylamino-, N-Pyrrolidinocarbonyl-cyclohexylamino, N-Pyrrolidinocarbonyl-cycloheptylamino-, N-Pyrrolidinocarbonyl-cyclopropylmethylamino-, N-Pyrrolidinocarbonyl-cyclobutylmethylamino-, N-Pyrrolidinocarbonyl-cyclopentylmethylamino-, N-Pyrrolidinocarbonyl-cyclohexylmethylamino-, N-Pyrrolidinocarbonyl-cycloheptylmethylamino-, N-Pyrrolidinocarbonyl-(2-cyclopropylethyl)-amino-, N-Pyrrolidinocarbonyl-(2-cyclobutylethyl)-amino-, N-Pyrrolidinocarbonyl-(2-cyclopentylethyl)-amino-, N-Pyrrolidinocarbonyl-(2-cyclohexylethyl)-amino-, N-Pyrrolidinocarbonyl-(2-cycloheptylethyl)-amino-, N-Pyrrolidinocarbonyl-(3-cyclopropylpropyl)-amino-, N-Pyrrolidinocarbonyl-(3-cyclobutylpropyl)-amino-, N-Pyrrolidinocarbonyl-(3-cyclopentylpropyl)-amino-, N-Pyrrolidinocarbonyl-(3-cyclohexylpropyl)-amino-, N-Pyrrolidinocarbonyl-(3-cycloheptylpropyl)-amino-, N-Pyrrolidinocarbonyl-phenylamino-, N-Pyrrolidinocarbonyl-benzylamino-, N-Piperidinocarbonyl-methylamino-, N-Piperidinocarbonyl-ethylamino-, N-Piperidinocarbonyl-isopropylamino-, N-Piperidinocarbonyl-n-butylamino-, N-Piperidinocarbonyl-n-pentylamino-, N-Piperidinocarbonyl-n-hexylamino-, N-Piperidinocarbonyl-n-octylamino-, N-Piperidinocarbonyl-n-dodecylamino-, N-Piperidinocarbonyl-cyclopropylamino-, N-Piperidinocarbonyl-cyclobutylamino-, N-Piperidinocarbonyl-cyclopentylamino-, N-Piperidinocarbonyl-cyclohexylamino-, N-Piperidinocarbonyl-cycloheptylamino-, N-Piperidinocarbonyl-cyclopropylmethylamino-, N-Piperidinocarbonyl-cyclobutylmethylamino-, N-Piperidinocarbonyl-cyclopentylmethylamino-, N-Piperidinocarbonyl-cyclohexylmethylamino-, N-Piperidinocarbonyl-cycloheptylmethylamino-, N-Piperidinocarbonyl-(2-cyclopropylethyl)-amino-, N-Piperidinocarbonyl-(2-cyclobutylethyl)-amino-, N-Piperidinocarbonyl-(2-cyclopentylethyl)-amino-, N-Piperidinocarbonyl-(2-cyclohexylethyl)-amino-, N-Piperidinocarbonyl-(2-cycloheptylethyl)-amino-, N-Piperidinocarbonyl-(3-cyclopropylpropyl)-amino-, N-Piperidinocarbonyl-(3-cyclobutylpropyl)-amino-, N-Piperidinocarbonyl-(3-cyclopentylpropyl)-amino-, N-Piperidinocarbonyl-(3-cyclohexylpropyl)-amino-, N-Piperidinocarbonyl-(3-cycloheptylpropyl)-amino-, N-Piperidinocarbonyl-phenylamino-, N-Piperidinocarbonyl-benzylamino-, N-Hexamethyleniminocarbonyl-methylamino-, N-Hexamethyleniminocarbonyl-ethylamino-, N-Hexamethyleniminocarbonyl-isopropylamino-, N-Hexamethyleniminocarbonyl-n-butylamino-, N-Hexamethyleniminocarbonyl-n-pentylamino-, N-Hexamethyleniminocarbonyl-n-hexylamino-, N-Hexamethyleniminocarbonyl-n-octylamino-, N-Hexamethyleniminocarbonyl-n-dodecylamino-, N-Hexamethyleniminocarbonyl-cyclopropylamino-, N-Hexamethyleniminocarbonyl-cyclobutylamino-, N-Hexamethyleniminocarbonyl-cyclopentylamino-, N-Hexamethyleniminocarbonyl-cyclohexylamino-, N-Hexamethyleniminocarbonyl-cycloheptylamino-, N-Hexamethyleniminocarbonyl-cyclopropylmethylamino-, N-Hexamethyleniminocarbonyl-cyclobutylmethylamino-, N-Hexamethyleniminocarbonyl-cyclopentylmethylamino-, N-Hexamethyleniminocarbonyl-cyclohexylmethylamino-, N-Hexamethyleniminocarbonyl-cycloheptylmethylamino-, N-Hexamethyleniminocarbonyl-(2-cyclopropylethyl)-amino-, N-Hexamethyleniminocarbonyl-(2-cyclobutylethyl)-amino-, N-Hexamethyleniminocarbonyl-(2-cyclopentylethyl)-amino-, N-Hexamethyleniminocarbonyl-(2-cyclohexylethyl)-amino-, N-Hexamethyleniminocarbonyl-(2-cycloheptylethyl)-amino-, N-Hexamethyleniminocarbonyl-(3-cyclopropylpropyl)-amino-, N-Hexamethyleniminocarbonyl-(3-cyclobutylpropyl)-amino-, N-Hexamethyleniminocarbonyl-(3-cyclopentylpropyl)-amino-, N-Hexamethyleniminocarbonyl-(3-cyclohexylpropyl)-amino-, N-Hexamethyleniminocarbonyl-(3-cycloheptylpropyl)-amino-, N-Hexamethyleniminocarbonyl-phenylamino-, N-Hexamethyleniminocarbonyl-benzylamino-, N-Morpholinocarbonyl-methylamino-, N-Morpholinocarbonyl-ethylamino-, N-Morpholinocarbonyl-isopropylamino-, N-Morpholinocarbonyl-n-butylamino-, N-Morpholinocarbonyl-n-pentylamino-, N-Morpholinocarbonyl-n-hexylamino-, N-Morpholinocarbonyl-n-octylamino-, N-Morpholinocarbonyl-n-dodecylamino-, N-Morpholinocarbonyl-cyclopropylamino-, N-Morpholinocarbonyl-cyclobutylamino-, N-Morpholinocarbonyl-cyclopentylamino-, N-Morpholinocarbonyl-cyclohexylamino-, N-Morpholinocarbonyl-cycloheptylamino-, N-Morpholinocarbonyl-cyclopropylmethylamino-, N-Morpholinocarbonyl-cyclobutylmethylamino-, N-Morpholinocarbonyl-cyclopentylmethylamino-, N-Morpholinocarbonyl-cyclohexylmethylamino-, N-Morpholinocarbonyl-cycloheptylmethylamino-, N-Morpholinocarbonyl-(2-cyclopropylethyl)-amino-, N-Morpholinocarbonyl-(2-cyclobutylethyl)-amino-, N-Morpholinocarbonyl-(2-cyclopentylethyl)-amino-, N-Morpholinocarbonyl-(2-cyclohexylethyl)-amino-, N-Morpholinocarbonyl-(2-cycloheptylethyl)-amino-, N-Morpholinocarbonyl-(3-cyclopropylpropyl)-amino-, N-Morpholinocarbonyl-(3-cyclobutylpropyl)-amino-, N-Morpholinocarbonyl-(3-cyclopentylpropyl)-amino-, N-Morpholinocarbonyl-(3-cyclohexylpropyl)-amino-, N-Morpholinocarbonyl-(3-cycloheptylpropyl)-amino-, N-Morpholinocarbonyl-phenylamino-, N-Morpholinocarbonyl-benzylamino-, Phthalimino-, 5-Methoxy-phthalimino-, 5,6-Dimethoxy-phthalimino-, 6-Methoxy-phthalimino-, Homophthalimino-, 4,4-Dimethyl-homophthalimino-, 7-Methoxy-homophthalimino-, 6,7-Dimethoxy-homophthalimino-, 7-Methoxy-4,4-dimethyl-homophthalimino-, 6,7-Dimethoxy-4,4-dimethyl-homophthalimino-, 1,2,3,6-Tetrahydrophthalimino-, Hexahydrophthalimino-, 1-Oxo-isoindolin-2-yl-, 3,4-Dimethyl-phthalimino-, 4,5-Dimethyl-1,2,3,6-tetrahydrophthalimino-, 4,5-Dimethyl-hexahydrophthalimino-, 4,5-Dimethyl-1-oxo-isoindolin-2-yl-, 3,4-Dimethoxy-phthalimino-, 4,5-Dimethoxy-1,2,3,6-tetrahydrophthalimino-, 4,5-Dimethoxy-hexahydrophthalimino-, 4,5-Dimethoxy-1-oxo-isoindolin-2-yl-, Glutarimino-, 3,3-Tetramethylen-glutarimino-, 3,3-Pentamethylen-glutarimino-, 2,2-Dimethyl-glutarimino-, 3-Methyl-glutarimino-, 3,3-Dimethyl-glutarimino-, 3-Ethyl-glutarimino-, Hexafluor-glutarimino-, 3-Ethyl-3-methyl-glutarimino-, 1,3-Cyclopentandicarbonylimino-, 2,4-Dimethyl-glutarimino-, 2,4-Di-n-propyl-glutarimino-, Endo-bicyclo[2.2.2]-oct-5-en-2,3-dicarbonsäureimino-, Methyl-5-norbornen-2,3-dicarbonsäureimino-, 3.6-Endoxo-1,2,3,6-tetrahydro-phthalimino- oder 5-Norbornen-endo-2,3-dicarbonsäureiminogruppe,

für $R_2$ die für $R_1$ vorstehend erwähnten Bedeutungen oder die der 1H-Tetrazol-5-yl-, 2-Imidazolidon-1-yl-, 3-Methyl-2-imidazolidon-1-yl-, 3-Ethyl-2-imidazolidon-1-yl-, 3-n-Propyl-2-imidazolidon-1-yl-, 3-Isopropyl-2-imidazolidon-1-yl-, 3-Cyclopropyl-2-imidazolidon-1-yl-, 3-Cyclobutyl-2-imidazolidon-1-yl-, 3-Cyclopentyl-2-imidazolidon-1-yl-, 3-Cyclohexyl-2-imidazolidon-1-yl-, 3-Cycloheptyl-2-imidazolidon-1-yl-, 3-Cyclopropylmethyl-2-imidazolidon-1-yl-, 3-Cyclobutylmethyl-2-imidazolidon-1-yl-, 3-Cyclopentylmethyl-2-imidazolidon-1-yl-, 3-Cyclohexylmethyl-2-imidazolidon-1-yl-, 3-Cycloheptylmethyl-2-imidazolidon-1-yl-, 3-(2-Cyclopropylethyl)-2-imidazolidon-1-yl-, 3-(2-Cyclobutylethyl)-2-imidazolidon-1-yl-, 3-(2-Cyclopentylethyl)-2-imidazolidon-1-yl-, 3-(2-Cyclohexylethyl)-2-imidazolidon-1-yl-, 3-(2-Cycloheptylethyl)-2-imidazolidon-1-yl-, 3-(3-Cyclopropylpropyl)-2-imidazolidon-1-yl-, 3-(3-Cyclobutylpropyl)-2-imidazolidon-1-yl-, 3-(3-Cyclopentylpropyl)-2-imidazolidon-1-yl-, 3-(3-Cyclohexylpropyl)-2-imidazolidon-1-yl-, 3-(3-Cycloheptylpropyl)-2-imidazolidon-1-yl-, 3-Benzyl-2-imidazolidon-1-yl-, 3-(2-Phenylethyl)-2-imidazolidon-1-yl-, 3-(3-Phenylpropyl)-2-imidazolidon-1-yl-, 3,4,5,6-Tetrahydro-2-pyrimidon-1-yl-, 3-Methyl-3,4,5,6-tetrahydro-2-pyrimidon-1-yl-, 3-Ethyl-3,4,5,6-tetrahydro-2-pyrimidon-1-yl-, 3-n-Propyl-3,4,5,6-tetrahydro-2-pyrimidon-1-yl-, 3-Isopropyl-3,4,5,6-tetrahydro-2-pyrimidon-1-yl-, 3-Cyclopropyl-3,4,5,6-tetrahydro-2-pyrimidon-1-yl-, 3-Cyclobutyl-3,4,5,6-tetrahydro-2-pyrimidon-1-yl-, 3-Cyclopentyl-3,4,5,6-tetrahydro-2-pyrimidon-1-yl-, 3-Cyclohexyl-3,4,5,6-tetrahydro-2-pyrimidon-1-yl-, 3-Cycloheptyl-3,4,5,6-tetrahydro-2-pyrimidon-1-yl-, 3-Cyclopropylmethyl-3,4,5,6-tetrahydro-2-pyrimidon-1-yl-, 3-Cyclobutylmethyl-3,4,5,6-tetrahydro-2-pyrimidon-1-yl-, 3-Cyclopentylmethyl-3,4,5,6-tetrahydro-2-pyrimidon-1-yl-, 3-Cyclohexylmethyl-3,4,5,6-tetrahydro-2-pyrimidon-1-yl-, 3-Cycloheptylmethyl-3,4,5,6-tetrahydro-2-pyrimidon-1-yl-, 3-(2-Cyclopropylethyl)-3,4,5,6-tetrahydro-2-pyrimidon-1-yl-, 3-(2-Cyclobutylethyl)-3,4,5,6-tetrahydro-2-pyrimidon-1-yl-, 3-(2-Cyclopentylethyl)-3,4,5,6-tetrahydro-2-pyrimidon-1-yl-, 3-(2-Cyclohexylethyl)-3,4,5,6-tetrahydro-2-pyrimidon-1-yl-, 3-(2-Cycloheptylethyl)-3,4,5,6-tetrahydro-2-pyrimidon-1-yl-, 3-(3-Cyclopropylpropyl)-3,4,5,6-tetrahydro-2-pyrimidon-1-yl-, 3-(3-Cyclobutylpropyl)-3,4,5,6-tetrahydro-2-pyrimidon-1-yl-, 3-(3-Cyclopentylpropyl)-3,4,5,6-tetrahydro-2-pyrimidon-1-yl-, 3-(3-Cyclohexylpropyl)-3,4,5,6-tetrahydro-2-pyrimidon-1-yl-, 3-(3-Cycloheptylpropyl)-3,4,5,6-tetrahydro-2-pyrimidon-1-yl-, 3-Benzyl-3,4,5,6-tetrahydro-2-pyrimidon-1-yl-, 3-(2-Phenylethyl)-3,4,5,6-tetrahydro-2-pyrimidon-1-yl- oder 3-(3-Phenylpropyl)-3,4,5,6-tetrahydro-2-pyrimidon-1-yl-gruppe,

für $R_3$ die des Wasserstoff-, Fluor-, Chlor- oder Bromatoms, der Methyl-, Ethyl-, n-Propyl-, Isopropyl-, n-Butyl-, Isobutyl-, tert.Butyl-, n-Pentyl-, n-Hexyl-, 1-Methylpropyl-, 1-Methylbutyl-, 2-Methylbutyl-, 3-Methylbutyl-, 1-Methylpentyl-, 2-Methylpentyl-, 3-Methylpentyl-, 4-Methylpentyl-, 1-Ethylpropyl-, 1,1-Diethylethyl-, Methoxymethyl-, Ethoxymethyl-, (2-Hydroxyethoxy)-methyl-, 2-Methoxy-ethyl-, 2-Ethoxy-ethyl-, 3-Methoxy-propyl-, Methylmercaptomethyl-, 2-Methylmercapto-ethyl-, 3-Methylmercapto-propyl-, 4-Methylmercaptobutyl-, Methylsulfinylmethyl-, 2-Methylsulfinyl-ethyl-, 3-Methylsulfinyl-propyl-, 4-Methylsulfinyl-butyl-, Methylsulfonylmethyl-, 2-Methylsulfonyl-ethyl-, 3-Methylsulfonyl-propyl-, 4-Methylsulfonyl-butyl-, 2-Methylamino-ethyl-, 3-Methylamino-propyl-, 4-Methylamino-butyl-, 2-Dimethylamino-ethyl-, 3-Dimethylamino-propyl-, 4-Dimethylamino-butyl-, 5-Dimethylamino-pentyl-, 2-Diethylamino-ethyl-, 2-Di-n-propylamino-ethyl-, 2-(2-Hydroxyethoxy)-ethyl-, 3-(2-Hydroxyethoxy)-propyl-, 2-(2-Methoxyethoxy)-ethyl-, 2-(2-Methoxyethoxy)-isopropyl-, 3-(2-Methoxyethoxy)-propyl-, 2-(2-Ethoxyethoxy)-ethyl-, 2-(2-Ethoxyethoxy)-isopropyl-, 3-(2-Ethoxyethoxy)-propyl-, 2-(2-Isopropoxyethoxy)-ethyl-, Methoxy-, Ethoxy-, n-Propoxy-, Isopropoxy-, n-Butoxy-, 2-Hydroxyethoxy-, 3-Hydroxypropoxy-, 2-Methoxyethoxy-, 2-Ethoxyethoxy-, 2-Isopropoxyethoxy-, 3-Methoxypropoxy-, 3-Isopropoxypropoxy-, Mercapto-, Methylmercapto-, Ethylmercapto-, n-Propylmercapto-, Isopropylmercapto-, n-Butylmercapto-, Benzyl-, 2-Phenylethyl-, 3-Phenylpropyl-, Allyl-, n-But-2-enyl-, n-Pent-2-enyl-, n-Prop-1-enyl-, n-But-1-enyl-, n-Pent-1-enyl-, n-But-3-enyl-, n-Pent-3-enyl-, n-Pent-4-enyl-, Propargyl-, n-But-3-inyl-, n-Pent-3-inyl-, n-Pent-4-inyl-, Cyclopentyl-, Cyclohexyl-, Cycloheptyl-, Cyclopentylmethyl-, Cyclohexylmethyl-, Cycloheptylmethyl-, 2-Cyclopentylethyl-, 2-Cyclohexylethyl-, 2-Cycloheptylethyl-, 3-Cyclopentylpropyl-, 3-Cyclohexylpropyl-, Phenyl-, Hydroxyphenyl-, Fluorphenyl-, Chlorphenyl-, Bromphenyl-, Methylphenyl-, Ethylphenyl-, Isopropylphenyl-, Methoxyphenyl-, Ethoxyphenyl-, n-Propoxyphenyl-, n-Butoxyphenyl-, Hydroxy-, (Imidazol-4-yl)methylamino-, 2-(Imidazol-4-yl)ethylamino-, 3-(Imidazol-4-yl)propylamino-, Methylamino-, Ethylamino-, n-Propylamino-, Isopropylamino-, n-Butylamino-, Isobutylamino-, Aminocarbonyl-, Methylaminocarbonyl-, Ethylaminocarbonyl-, Isopropylaminocarbonyl-, n-Butylaminocarbonyl-, Cyclopentylaminocarbonyl-, Cyclohexylaminocarbonyl-, Cycloheptylaminocarbonyl-, Naphthyl-, Pyridyl-, Pyrimidyl-, Pyridazinyl-, Pyrazinyl-, Pyrazolyl-, 1,3-Dimethyl-pyrazolyl-, Pyrrolyl-, Imidazolyl-, Oxazolyl-, Thiazolyl-, Triazolyl- oder Tetrazolylgruppe und

für $R_4$ die der Carboxy-, Methoxycarbonyl-, Ethoxycarbonyl-, n-Propoxycarbonyl-, Isopropoxycarbonyl-, n-Butoxycarbonyl-, Isobutoxycarbonyl-, tert.Butoxycarbonyl-, n-Pentoxycarbonyl-, n-Hexoxycarbonyl-, Benzyloxycarbonyl-, 1-Phenylethoxycarbonyl-, 2-Phenyl-ethoxycarbonyl-, 3-Phenyl-propoxycarbonyl-, Pivaloyloxymethoxycarbonyl-, Phthalidyloxycarbonyl-, Ethoxycarbonyloxyethoxycarbonyl-, Methoxymethoxycarbonyl-, Cyclohexyloxycarbonylmethoxycarbonyl-, (1,3-Dioxa-2-oxo-4-methyl-cyclopenten-5-yl)-methoxycarbonyl-, Amino-, Phthalimido-, Aminoacetylamino-, Methoxycarbonylaminoacetylamino-, Ethoxycarbonylaminoacetylamino-, Isopropoxycarbonylaminoacetylamino-, n-Butoxycarbonylaminoacetylamino-, Methylcarbonylamino-, Trifluormethylcarbonylamino-, Ethylcarbonylamino-, n-Propylcarbonylamino-, Isopropylcarbonylamino-, Cyclopentylcarbonylamino-, Cyclohexylcarbonylamino-, Cycloheptylcarbonylamino-, Phenylcarbonylamino-, Fluorphenylcarbonylamino-, Chlorphenylcarbonylamino-, Bromphenylcarbonylamino-, Methylphenylcarbonylamino-, Ethylphenylcarbonylamino-, Isopropylphenylcarbonylamino-, Methoxyphenylcarbonylamino-, Ethoxyphenylcarbonylamino-, n-Propoxyphenylcarbonylamino-, Benzylcarbonylamino-, 2-Phenylethylcarbonylamino-, Methylsulfonylamino-, Trifluormethylsulfonylamino-, Ethylsulfonylamino-, n-Propylsulfonylamino-, Isopropylsulfonylamino-, Phenylsulfonylamino-, Fluorphenylsulfonylamino-, Chlorphenylsulfonylamino-, Bromphenylsulfonylamino-, Methylphenylsulfonylamino-, Ethylphenylsulfonylamino-, Isopropylphenylsulfonylamino-, Methoxyphenylsulfonylamino-, Ethoxyphenylsulfonylamino-, Benzylsulfonylamino-, Cyano-, Aminomethyl-, Methylsulfonylaminomethyl-, Ethylsulfonylaminoethyl-, n-Propylsulfonylaminomethyl-, Phenylsulfonylaminomethyl-, Methylphenylsulfonylaminomethyl-, Trifluormethylsulfonylaminomethyl-, Methylsulfonylaminocarbonyl-, Ethylsulfonylaminocarbonyl-, n-Propylsulfonylaminocarbonyl-, Isopropylsulfonylaminocarbonyl-, n-Butylsulfonylaminocarbonyl-, Trifluormethylsulfonylaminocarbonyl-, Perfluor-n-butylsulfonylaminocarbonyl-, Phenylsulfonylaminocarbonyl-, Benzylsulfonylaminocarbonyl-, 4-Methylphenylsulfonylaminocarbonyl-, 4-Chlorphenylsulfonylaminocarbonyl-, Trifluoracetylaminomethyl-, 1H-Tetrazolyl- oder 1-(Triphenylmethyl)-tetrazolylgruppe in Betracht.

Bevorzugte Verbindungen der allgemeinen Formel I sind diejenigen, in denen

$R_1$ ein Wasserstoff-, Fluor- oder Chloratom, eine Trifluormethyl-, Hydroxy-, Benzyloxy-, Carboxy-, Cyano-, Amino-, Nitro-, Aminosulfonyl-, Methylaminosulfonyl-, Dimethylaminosulfonyl-, Trifluormethylsulfonyloxy- oder Tetrazolylgruppe, eine Alkylgruppe mit 1 bis 4 Kohlenstoffatomen, wobei die Methylgruppe zusätzlich durch eine Hydroxy- oder Alkylaminogruppe mit 1 bis 4 Kohlenstoffatomen substituiert sein kann,
eine Alkoxygruppe mit 1 bis 4 Kohlenstoffatomen, die in 2-, 3- oder 4-Stellung durch eine Hydroxy-, Alkoxy-, Alkylamino-, Dialkylamino- oder Imidazolylgruppe substituiert sein und der Alkylsubstituent jeweils 1 bis 3 Kohlenstoffatome enthalten kann,
eine Alkanoyloxygruppe mit 1 bis 4 Kohlenstoffatomen oder eine Cycloalkylaminocarbonyloxygruppe mit 5 bis 7 Kohlenstoffatomen im Cycloalkylteil,
eine Aminogruppe, die durch eine Benzyl-, Decalin-, Trifluormethylcarbonyl-, Benzylcarbonyl-, Benzyloxycarbonyl-, 2-Ethyl-5-methylcyclohexyloxy- oder tert.Butoxycarbonylaminoacetylgruppe, durch eine Alkylgruppe mit 1 bis 5 Kohlenstoffatomen, durch eine Cycloalkyl-, Cycloalkylalkyl-, Cycloalkoxycarbonyl-, Cycloalkylcarbonyl- oder Cycloalkylalkanoylgruppe, wobei der Cycloalkylteil jeweils 5 bis 7 Kohlenstoffatome, der Alkylteil 1 bis 3 Kohlenstoffatome und der Alkanoylteil 1 bis 3 Kohlenstoffatome enthalten kann, durch eine Alkanoylgruppe mit 1 bis 6 Kohlenstoffatomen, durch eine Aminoalkanoyl- oder Dialkylaminoalkanoylgruppe, in welcher jeweils der Alkylteil und der Alkanoylteil 1 bis 3 Kohlenstoffatome enthalten kann, durch eine Alkoxycarbonylgruppe mit insgesamt 2 bis 7 Kohlenstoffatomen, durch eine Alkylsulfonylgruppe mit 1 bis 4 Kohlenstoffatomen oder durch eine durch eine Alkoxycarbonylgruppe mit insgesamt 2 bis 4 Kohlenstoffatomen substituierte Thiazolidin-3-yl-carbonylgruppe substituiert ist,
eine Benzylamino- oder Alkylaminogruppe mit 1 bis 5 Kohlenstoffatomen, die am Stickstoffatom zusätzlich durch eine Benzyl- oder Cyclohexylgruppe, durch eine Alkylgruppe mit 1 bis 3 Kohlenstoffatomen, die in 2- oder 3-Stellung durch eine Alkoxygruppe mit 1 bis 3 Kohlenstoffatomen oder durch eine Phenylaminogruppe substituiert sein kann, durch eine Alkanoylgruppe mit 1 bis 5 Kohlenstoffatomen, welche durch eine Alkoxygruppe mit 1 bis 3 Kohlenstoffatomen substituiert sein kann, durch eine Cycloalkylcarbonylgruppe mit insgesamt 6 bis 8 Kohlenstoffatomen oder durch eine Alkoxycarbonylgruppe mit insgesamt 2 bis 4 Kohlenstoffatomen substituiert ist,
eine Carbonylgruppe, die durch eine Alkylgruppe, die in 2- oder 3-Stellung durch eine Hydroxy-, Alkoxycarbonyl- oder Alkylaminogruppe substituiert sein kann, durch eine Alkylaminogruppe mit 1 bis 5 Kohlenstoffatomen, die im Alkylteil durch eine Carboxygruppe substituiert sein kann, durch eine Alkylaminogruppe, die in 2- oder 3-Stellung durch eine Phenylaminogruppe substituiert ist, durch eine Phenyl-, Alkoxy-, Amino-, Benzylamino-, Phenylethylamino-, Cycloalkylamino- oder Cycloalkylalkylamino-, in welchen jeweils der Alkylteil 1 bis 3 Kohlenstoffatome und der Cycloalkylteil 5 bis 7 Kohlenstoffatome enthalten kann sowie zusätzlich eine Alkylaminogruppe mit 1 bis 5 Kohlenstoffatomen am Stickstoffatom durch eine Alkylgruppe mit 1 bis 4 Kohlenstoffatomen substituiert sein kann, substituiert ist,
eine Aminocarbonylamino- oder Aminothiocarbonylaminogruppe, welche durch eine Alkylgruppe mit 1 bis 12 Kohlenstoffatomen, durch eine Alkenyl- oder Alkinylgruppe mit jeweils 3 bis 5 Kohlenstoffatomen, durch eine bi- oder tricyclische Alkylgruppe mit 7 bis 11 Kohlenstoffatomen, durch eine Cycloalkyl-, Cycloalkylalkyl- oder Phenylalkylgruppe mono-, di- oder trisubstituiert sein kann, wobei die Substituenten gleich oder verschieden sein können sowie jeweils der Alkylteil 1 bis 3 Kohlenstoffatome und der Cycloalkylteil 5 bis 7 Kohlenstoffatome enthalten kann sowie eine Methylengruppe in einem Cycloalkylrest mit 5 bis 7 Kohlenstoffatomen durch ein Sauerstoffatom ersetzt und eine Alkylgruppe in 4-, 5- oder 6-Stellung durch eine Hydroxy- oder Trifluoracetylgruppe substituiert sein kann,
eine Cycloalkyleniminocarbonylaminogruppe mit 4 bis 6 Kohlenstoffatomen im Cycloalkyleniminoteil oder eine Morpholinocarbonylaminogruppe, die beide am Aminostickstoff durch eine Alkylgruppe mit 1 bis 12 Kohlenstoffatomen, durch eine Cycloalkyl-, Cycloalkylalkyl- oder Phenylalkylgruppe substituiert sein können, in denen jeweils der Alkylteil 1 bis 3 Kohlenstoffatome und der Cycloalkylteil 5 bis 7 Kohlenstoffatme enthalten kann,
eine Phthalimino-, Homophthalimino-, 2-Carboxyphenylcarbonylamino-, 2-Carboxyphenylmethylamino-, 2-Carboxyphenylmethylencarbonylamino- oder 2-Carboxymethylenphenylcarbonylaminogruppe, wobei eine Carbonylgruppe in einer Phthaliminogruppe durch eine Methylengruppe ersetzt sowie eine Methylengruppe in einer Homophthalimino-, 2-Carboxyphenylmethylencarbonylamino- oder 2-Carboxymethylenphenylcarbonylaminogruppe durch eine oder zwei Methylgruppen substituiert sein kann, und zusätzlich die vorstehend erwähnten Phenylkerne durch Methyl- oder Methoxygruppen mono- oder disubstituiert, wobei die Substituenten gleich oder verschieden sein können, und gleichzeitig ganz oder teilweise hydriert sein können, eine in 2-Stellung durch eine Carboxygruppe substituierte Bicycloalkan-3-carbonsäureamino- oder Bicycloalken-3-carbonsäureaminogruppe, eine Bicycloalkan-2,3-dicarbonsäureimino- oder Bicycloalken-2,3-dicarbonsäureiminogruppe, in denen der Bicycloalkan- und Bicycloalkenteil jeweils 9 oder 10 Kohlenstoffatome enthalten, durch 1, 2 oder 3 Methylgruppen substituiert und eine Endomethylengruppe durch ein Sauerstoffatom ersetzt sein kann, eine Glutarsäureimino- oder 3-Carboxy-n-propylencarbonylgruppe, in denen jeweils die n-Propylengruppe perfluoriert, durch ein oder zwei Alkylgruppen oder durch eine Tetramethylen- oder Pentamethylengruppe substituiert sein kann, oder
eine 5,7-Dioxa-1H,3H-imidazo[1,5-c]thiazolylgruppe, eine gegebenfalls in 3-Stellung durch eine Alkylgruppe mit 1 bis 3 Kohlenstoffatomen substituierte 2-Imidazolidon-1-yl-gruppe oder eine 3,4,5,6-Tetrahydro-2-pyrimidon-1-yl-gruppe,
$R_2$ ein Wasserstoff-, Fluor- oder Chloratom, eine Methyl-, Hydroxy- oder Methoxygruppe, oder
$R_1$ und $R_2$ zusammen mit den beiden ortho-ständigen Kohlenstoffatomen des benachbarten Phenylringes eine Phenyl- oder 1-Alkyl-3,3-dialkyl-2,3-dihydro-pyrrol-2-on-gruppe, in welcher der Alkylteil jeweils 1 bis 3 Kohlenstoffatme enthalten

kann,
$R_3$ ein Wasserstoff-, Fluor- oder Chloratom,
eine Hydroxy-, Benzyl- oder Aminocarbonylgruppe,
eine Alkylgruppe mit 1 bis 5 Kohlenstoffatomen, wobei die Methylgruppe zusätzlich durch eine Hydroxygruppe, durch eine Alkoxy-, Alkylsulfenyl-, Alkylsulfinyl- oder Alkylsulfonylgruppe mit jeweils 1 bis 3 Kohlenstoffatomen substituiert sein kann, eine Cycloalkyl- oder Cycloalkylalkylgruppe, in welcher der Cycloalkylteil jeweils 5 bis 7 Kohlenstoffatome und der Alkylteil 1 bis 3 Kohlenstoffatome enthalten kann, eine Alkenyl- oder Alkinylgruppe mit jeweils 3 bis 5 Kohlenstoffatomen, eine Phenylalkylgruppe mit 1 bis 3 Kohlenstoffatomen,
eine Alkylaminogruppe mit 1 bis 4 Kohlenstoffatomen,
eine Pyridyl-, Furyl-, Thiazolyl- oder Pyrazolylgruppe, welche durch eine oder zwei Methylgruppen substituiert sein können,
$R_4$ eine Alkoxycarbonyl mit insgesamt 1 bis 5 Kohlenstoffatomen, eine Amino-, Phthalimino-, Aminomethyl-, Carboxy-, Cyano-, Methylsulfonylaminocarbonyl-, Trifluormethylsulfonylaminocarbonyl-, Benzolsulfonylaminocarbonyl-, Trifluormethylcarbonylaminomethyl-, Trifluormethylaminomethyl-, Tetrazolyl- oder 1-(Triphenylmethyl)-tetrazolylgruppe,
$R_5$ und $R_6$ jeweils ein Wasserstoffatom oder
$R_5$ und $R_6$ zusammen mit den beiden ortho-ständigen Kohlenstoffatomen eine Phenylgruppe bedeuten, deren 1-, 3-Isomerengemische und deren Salze, insbesondere für die pharmazeutische Anwendung deren physiologisch verträglichen Salze mit anorganischen oder organischen Säuren.

Besonders bevorzugte Verbindungen der allgemeinen Formel I sind jedoch diejenigen, in denen
$R_1$ eine Aminogruppe, die durch eine Benzyl-, Decalin-, Trifluormethylcarbonyl-, Benzylcarbonyl-, Benzyloxycarbonyl-, 2-Ethyl-5-methylcyclohexyloxy- oder tert.Butoxycarbonylaminoacetylgruppe, durch eine Alkylgruppe mit 1 bis 5 Kohlenstoffatomen, durch eine Cycloalkyl-, Cycloalkylalkyl-, Cycloalkoxycarbonyl-, Cycloalkylcarbonyl- oder Cycloalkylalkanoylgruppe, wobei der Cycloalkylteil jeweils 5 bis 7 Kohlenstoffatome, der Alkylteil 1 bis 3 Kohlenstoffatome und der Alkanoylteil 1 bis 3 Kohlenstoffatome enthalten kann, durch eine Alkanoylgruppe mit 1 bis 6 Kohlenstoffatomen, durch eine Aminoalkanoyl- oder Dialkylaminoalkanoylgruppe, in welcher jeweils der Alkylteil und der Alkanoylteil 1 bis 3 Kohlenstoffatome enthalten kann, durch eine Alkoxycarbonylgruppe mit insgesamt 2 bis 7 Kohlenstoffatomen, durch eine Alkylsulfonylgruppe mit 1 bis 4 Kohlenstoffatomen oder durch eine durch eine Alkoxycarbonylgruppe mit insgesamt 2 bis 4 Kohlenstoffatomen substituierte Thiazolidin-3-yl-carbonylgruppe substituiert ist,
eine Benzylamino- oder Alkylaminogruppe mit 1 bis 5 Kohlenstoffatomen, die am Stickstoffatom zusätzlich durch eine Benzyl- oder Cyclohexylgruppe, durch eine Alkylgruppe mit 1 bis 3 Kohlenstoffatomen, die in 2- oder 3-Stellung durch eine Alkoxygruppe mit 1 bis 3 Kohlenstoffatomen oder durch eine Phenylaminogruppe substituiert sein kann, durch eine Alkanoylgruppe mit 1 bis 5 Kohlenstoffatomen, welche durch eine Alkoxygruppe mit 1 bis 3 Kohlenstoffatomen substituiert sein kann, durch eine Cycloalkylcarbonylgruppe mit insgesamt 6 bis 8 Kohlenstoffatomen oder durch eine Alkoxycarbonylgruppe mit insgesamt 2 bis 4 Kohlenstoffatomen substituiert ist,
eine Aminocarbonylamino- oder Aminothiocarbonylaminogruppe, welche durch eine Alkylgruppe mit 1 bis 12 Kohlenstoffatomen, durch eine Alkenyl- oder Alkinylgruppe mit jeweils 3 bis 5 Kohlenstoffatomen, durch eine bi- oder tricyclische Alkylgruppe mit 7 bis 11 Kohlenstoffatomen, durch eine Cycloalkyl-, Cycloalkylalkyl- oder Phenylalkylgruppe mono-, di- oder trisubstituiert sein kann, wobei die Substituenten gleich oder verschieden sein können sowie jeweils der Alkylteil 1 bis 3 Kohlenstoffatome und der Cycloalkylteil 5 bis 7 Kohlenstoffatome enthalten kann sowie eine Methylengruppe in einem Cycloalkylrest mit 5 bis 7 Kohlenstoffatomen durch ein Sauerstoffatom ersetzt und eine Alkylgruppe in 4-, 5- oder 6-Stellung durch eine Hydroxy- oder Trifluoracetylgruppe substituiert sein kann,
eine Cycloalkyleniminocarbonylaminogruppe mit 4 bis 6 Kohlenstoffatomen im Cycloalkyleniminoteil oder eine Morpholinocarbonylaminogruppe, die beide am Aminostickstoff durch eine Alkylgruppe mit 1 bis 12 Kohlenstoffatomen, durch eine Cycloalkyl-, Cycloalkylalkyl- oder Phenylalkylgruppe substituiert sein können, in denen jeweils der Alkylteil 1 bis 3 Kohlenstoffatome und der Cycloalkylteil 5 bis 7 Kohlenstoffatme enthalten kann,
eine Phthalimino-, Homophthalimino-, 2-Carboxyphenylcarbonylamino-, 2-Carboxyphenylmethylamino-, 2-Carboxyphenylmethylencarbonylamino- oder 2-Carboxymethylenphenylcarbonylaminogruppe, wobei eine Carbonylgruppe in einer Phthaliminogruppe durch eine Methylengruppe ersetzt sowie eine Methylengruppe in einer Homophthalimino-, 2-Carboxyphenylmethylencarbonylamino- oder 2-Carboxymethylenphenylcarbonylaminogruppe durch eine oder zwei Methylgruppen substituiert sein kann, und zusätzlich die vorstehend erwähnten Phenylkerne durch Methyl- oder Methoxygruppen mono- oder disubstituiert, wobei die Substituenten gleich oder verschieden sein können, und gleichzeitig ganz oder teilweise hydriert sein können, eine in 2-Stellung durch eine Carboxygruppe substituierte Bicycloalkan-3-carbonsäureamino- oder Bicycloalken-3-carbonsäureaminogruppe, eine Bicycloalkan-2,3-dicarbonsäureimino- oder Bicycloalken-2,3-dicarbonsäureiminogruppe, in denen der Bicycloalkan- und Bicycloalkenteil jeweils 9 oder 10 Kohlenstoffatome enthalten, durch 1, 2 oder 3 Methylgruppen substituiert und eine Endomethylengruppe durch ein Sauerstoffatom ersetzt sein kann, eine Glutarsäureimino- oder 3-Carboxy-n-propylencarbonylgruppe, in denen jeweils die n-Propylengruppe perfluoriert, durch ein oder zwei Alkylgruppen oder durch eine Tetramethylen- oder Pentamethylengruppe substituiert sein kann, oder
eine 5,7-Dioxa-1H,3H-imidazo[1,5-c]thiazolylgruppe,

$R_2$ ein Wasserstoffatom, eine Methyl- oder Hydroxygruppe oder
$R_1$ und $R_2$ zusammen mit den beiden ortho-ständigen Kohlenstoffatomen des benachbarten Phenylringes eine Phenylgruppe,
$R_3$ eine Alkylgruppe mit 3 bis 5 Kohlenstoffatomen, eine Alkenyl- oder Alkinylgruppe mit jeweils 3 bis 5 Kohlenstoffatomen,
$R_4$ eine Carboxy-, Methylsulfonylaminocarbonyl-, Trifluormethylsulfonylaminocarbonyl-, Benzolsulfonylaminocarbonyl-, Trifluormethylcarbonylaminomethyl-, Trifluormethylaminomethyl- oder Tetrazolylgruppe,
$R_5$ und $R_6$ jeweils ein Wasserstoffatom oder
$R_5$ und $R_6$ zusammen mit den beiden ortho-ständigen Kohlenstoffatomen eine Phenylgruppe bedeuten, insbesondere diejenigen Verbindungen der allgemeinen Formel I, in der
$R_1$ eine Aminogruppe, die durch eine Alkanoylgruppe mit 1 bis 6 Kohlenstoffatomen, durch eine Aminoalkanoyl- oder Dialkylaminoalkanoylgruppe, in welcher der Alkylteil und der Alkanoylteil jeweils 1 bis 3 Kohlenstoffatome enthalten kann, durch eine Alkoxycarbonylgruppe mit insgesamt 2 bis 7 Kohlenstoffatomen oder durch eine durch eine Alkoxycarbonylgruppe mit insgesamt 2 bis 4 Kohlenstoffatomen substituierte Thiazolidin-3-yl-carbonylgruppe substituiert ist,
eine Benzylamino- oder Alkylaminogruppe mit 1 bis 5 Kohlenstoffatomen, die am Stickstoffatom durch eine Alkanoylgruppe mit 1 bis 5 Kohlenstoffatomen, welche durch eine Alkoxygruppe mit 1 bis 3 Kohlenstoffatomen substituiert sein kann, durch eine Cycloalkylcarbonylgruppe mit insgesamt 6 bis 8 Kohlenstoffatomen oder durch eine Alkoxycarbonylgruppe mit insgesamt 2 bis 4 Kohlenstoffatomen substituiert ist,
eine Aminocarbonylamino- oder Aminothiocarbonylaminogruppe, welche durch eine Alkylgruppe mit 1 bis 8 Kohlenstoffatomen, durch eine Alkenyl- oder Alkinylgruppe mit jeweils 3 bis 5 Kohlenstoffatomen, durch eine bi- oder tricyclische Alkylgruppe mit 7 bis 11 Kohlenstoffatomen, durch eine Cycloalkyl-, Cycloalkylalkyl- oder Phenylalkylgruppe mono-, di- oder trisubstituiert sein kann, wobei die Substituenten gleich oder verschieden sein können sowie jeweils der Alkylteil 1 bis 3 Kohlenstoffatome und der Cycloalkylteil 5 bis 7 Kohlenstoffatome enthalten kann sowie eine Methylengruppe in einem Cycloalkylrest mit 5 bis 7 Kohlenstoffatomen durch ein Sauerstoffatom ersetzt und eine Alkylgruppe in 4-, 5- oder 6-Stellung durch eine Hydroxy- oder Trifluoracetylgruppe substituiert sein kann,
eine Cycloalkyleniminocarbonylaminogruppe mit 4 bis 6 Kohlenstoffatomen im Cycloalkyleniminoteil oder eine Morpholinocarbonylaminogruppe, die beide am Aminostickstoff durch eine Alkylgruppe mit 1 bis 8 Kohlenstoffatomen, durch eine Cycloalkyl-, Cycloalkylalkyl- oder Phenylalkylgruppe substituiert sein können, in denen jeweils der Alkylteil 1 bis 3 Kohlenstoffatome und der Cycloalkylteil 5 bis 7 Kohlenstoffatme enthalten kann,
eine Phthalimino-, Homophthalimino-, 2-Carboxyphenylcarbonylamino-, 2-Carboxyphenylmethylamino-, 2-Carboxyphenylmethylencarbonylamino- oder 2-Carboxymethylenphenylcarbonylaminogruppe, wobei eine Carbonylgruppe in einer Phthaliminogruppe durch eine Methylengruppe ersetzt sowie eine Methylengruppe in einer Homophthalimino-, 2-Carboxyphenylmethylencarbonylamino- oder 2-Carboxymethylenphenylcarbonylaminogruppe durch eine oder zwei Methylgruppen substituiert sein kann, und zusätzlich die vorstehend erwähnten Phenylkerne durch Methyl- oder Methoxygruppen mono- oder disubstituiert, wobei die Substituenten gleich oder verschieden sein können, und gleichzeitig ganz oder teilweise hydriert sein können, eine in 2-Stellung durch eine Carboxygruppe substituierte Bicycloalkan-3-carbonsäureamino- oder Bicycloalken-3-carbonsäureaminogruppe, eine Bicycloalkan-2,3-dicarbonsäureimino- oder Bicycloalken-2,3-dicarbonsäureiminogruppe, in denen der Bicycloalkan- und Bicycloalkenteil jeweils 9 oder 10 Kohlenstoffatome enthalten, durch 1, 2 oder 3 Methylgruppen substituiert und eine Endomethylengruppe durch ein Sauerstoffatom ersetzt sein kann, eine Glutarsäureimino- oder 3-Carboxy-n-propylencarbonylgruppe, in denen jeweils die n-Propylengruppe perfluoriert, durch ein oder zwei Alkylgruppen oder durch eine Tetramethylen- oder Pentamethylengruppe substituiert sein kann, oder
eine 5,7-Dioxa-1H,3H-imidazo[1,5-c]thiazolylgruppe,
$R_2$ ein Wasserstoffatom,
$R_1$ und $R_2$ zusammen mit den beiden ortho-ständigen Kohlenstoffatomen des benachbarten Phenylringes eine Phenylgruppe,
$R_3$ eine Alkylgruppe mit 3 bis 5 Kohlenstoffatomen, eine Alkenyl- oder Alkinylgruppe mit jeweils 3 bis 5 Kohlenstoffatomen,
$R_4$ eine Carboxy- oder Tetrazolylgruppe,
$R_5$ und $R_6$ jeweils ein Wasserstoffatom oder
$R_5$ und $R_6$ zusammen mit den beiden ortho-ständigen Kohlenstoffatomen eine Phenylgruppe bedeuten, deren 1-, 3-Isomerengemische und deren Salze, insbesondere für die pharmazeutische Anwendung deren physiologisch verträglichen Salze mit anorganischen oder organischen Säuren.

Erfindungsgemäß erhält man die neuen Verbindungen der Formel I nach folgenden Verfahren:

a) Cyclisierung einer Verbindung der Formel

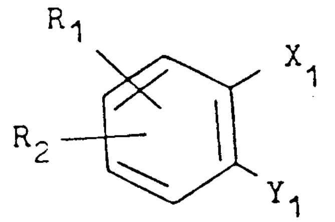

, (II)

in der
$R_1$ und $R_2$ wie eingangs definiert sind,
einer der Reste $X_1$ oder $Y_1$ eine Gruppe der Formel

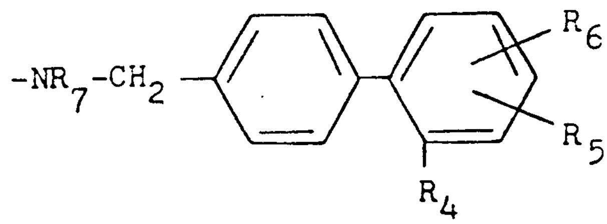

und der andere der Reste $X_1$ oder $Y_1$ eine Gruppe der Formel

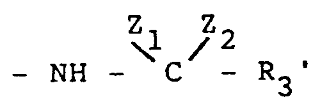

darstellen, wobei
$R_3$' mit Ausnahme des Fluor-, Chlor- oder Bromatoms, der Hydroxy-, Mercapto- oder Aminocarbonylgruppe, wobei die letztere durch eine Alkylgruppe mit 1 bis 3 Kohlenstoffatomen oder durch eine Cycloalkylgruppe mit 5 bis 7 Kohlenstoffatomen substituiert sein kann, die für $R_3$ eingangs erwähnten Bedeutungen besitzt,
$R_4$ bis $R_6$ wie eingangs definiert sind,
$R_7$ ein Wasserstoffatom, eine Hydroxygruppe oder eine $R_3$'CO-Gruppe, wobei $R_3$' wie vorstehend erwähnt definiert ist,
$Z_1$ und $Z_2$, die gleich oder verschieden sein können, gegebenenfalls substituierte Aminogruppen oder gegebenenfalls durch niedere Alkylgruppen substituierte Hydroxy- oder Mercaptogruppen oder
$Z_1$ und $Z_2$, zusammen ein Sauerstoff- oder Schwefelatom, eine gegebenenfalls durch eine Alkylgruppe mit 1 bis 3 Kohlenstoffatomen substituierte Iminogruppe, eine Alkylendioxy- oder Alkylendithiogruppe mit jeweils 2 oder 3 Kohlenstoffatomen bedeuten, wobei jedoch einer der Reste $X_1$ oder $Y_1$ eine Gruppe der Formel

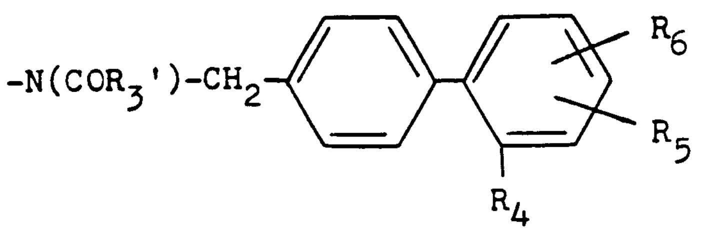

oder

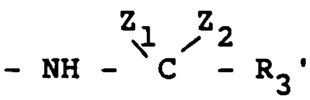

darstellen muß.

Die Cyclisierung wird zweckmäßigerweise in einem Lösungsmittel oder Lösungsmittelgemisch wie Ethanol, Isopropanol, Eisessig, Benzol, Chlorbenzol, Toluol, Xylol, Glycol, Glycolmonomethylether, Diethylenglycoldimethylether, Sulfolan, Dimethylformamid, Tetralin oder in einem Überschuß des zur Herstellung der Verbindung der allgemeinen Formel II verwendeten Acylierungsmittel, z.B. in dem entsprechenden Nitril, Anhydrid, Säurehalogenid, Ester oder Amid, beispielsweise bei Temperaturen zwischen 0 und 250°C, vorzugsweise jedoch bei der Siedetemperatur des Reaktionsgemisches, gegebenenfalls in Gegenwart eines Kondensationsmittels wie Phosphoroxychlorid, Thionylchlorid, Sulfurylchlorid, Schwefelsäure, p-Toluolsulfonsäure, Methansulfonsäure, Salzsäure, Phosphorsäure, Polyphosphorsäure, Essigsäureanhydrid oder gegebenenfalls auch in Gegenwart einer Base wie Kaliumäthylat oder Kaliumtert.butylat durchgeführt. Die Cyclisierung kann jedoch auch ohne Lösungsmittel und/oder Kondensationsmittel durchgeführt werden.

Besonders vorteilhaft wird die Umsetzung jedoch in der Weise durchgeführt, daß eine Verbindung der Formel II im Reaktionsgemisch durch Reduktion einer entsprechenden o-Nitro-aminoverbindung gegebenenfalls in Gegenwart einer Carbonsäure der Formel $R_3'COOH$ oder durch Acylierung einer entsprechenden o-Diaminoverbindung hergestellt wird. Bei Abbruch der Reduktion der Nitrogruppe auf der Hydroxylaminstufe erhält man bei der anschließenden Cyclisierung das N-Oxid einer Verbindung der Formel I. Das so erhaltene N-Oxid wird anschließend mittels Reduktion in eine entsprechende Verbindung der Formel I übergeführt.

Die anschließende Reduktion des erhaltenen N-Oxids der Formel I wird vorzugsweise in einem Lösungsmittel wie Wasser, Wasser/Äthanol, Methanol, Eisessig, Essigsäureäthylester oder Dimethylformamid mit Wasserstoff in Gegenwart eines Hydrierungskatalysators wie Raney-Nickel, Platin oder Palladium/Kohle, mit Metallen wie Eisen, Zinn oder Zink in Gegenwart einer Säure wie Essigsäure, Salzsäure oder Schwefelsäure, mit Salzen wie Eisen(II)sulfat, Zinn(II)chlorid oder Natriumdithionit, oder mit Hydrazin in Gegenwart von Raney-Nickel bei Temperaturen zwischen 0 und 50°C, vorzugsweise jedoch bei Raumtemperatur durchgeführt.

Eine gegebenenfalls so erhaltene 2-Alkoxyverbindung der Formel I kann anschließend gewünschtenfalls mittels Hydrolyse, vorzugsweise in Gegenwart einer Säure wie Salzsäure, Bromwasserstoffsäure, Schwefelsäure, Phosphorsäure oder Trichloressigsäure, in einem geeigneten Lösungsmittel wie Wasser, Wasser/Methanol, Ethanol, Wasser/Ethanol, Wasser/Isopropanol oder Wasser/Dioxan, bei Temperaturen zwischen -10 und 120°C, z.B. bei Temperaturen zwischen 20°C und der Siedetemperatur des Reaktionsgemisches, in eine entsprechende 2-Hydroxyverbindung der Formel I übergeführt werden.

b) Zur Herstellung der Verbindungen der Formel I, in der $R_3$ die für $R_3$ eingangs erwähnten aromatischen oder heteroaromatischen Reste darstellt:

Umsetzung eines Phenylendiamins der Formel

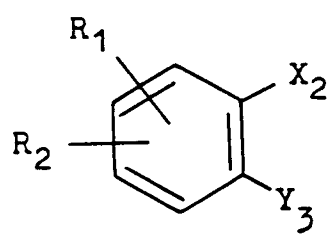

,(III)

in der
$R_1$ und $R_2$ wie eingangs definiert sind,
einer der Reste $X_2$ oder $Y_2$ eine Gruppe der Formel

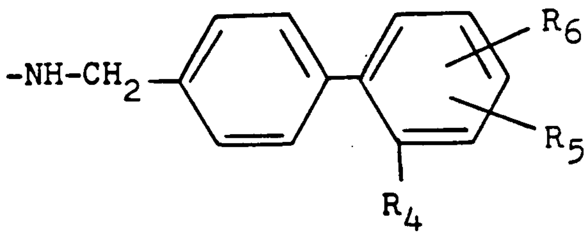

in der
$R_4$ bis $R_6$ wie eingangs definiert sind,
und der andere der Reste $X_2$ oder $Y_2$ eine Aminogruppe, bedeuten, mit einem Aldehyd der Formel

$$OCH - R_3'' ,(IV)$$

in der
$R_3''$ die für $R_3$ eingangs erwähnten aromatischen oder heteroaromatischen Reste bedeutet.

Die Umsetzung wird vorzugsweise in einem geeigneten Lösungsmittel wie Benzol, Toluol, Xylol, Mesitylen oder Dimethylacetamid in Gegenwart eines Oxidationsmittels wie Schwefel oder Luftsauerstoff bei Temperaturen zwischen 80 und 250°C, vorzugsweise bei der Siedetemperatur des Reaktionsgemisches, durchgeführt.

Eine gegebenenfalls so erhaltene 2-Alkoxyverbindung der Formel I kann anschließend gewünschtenfalls mittels Hydrolyse, vorzugsweise in Gegenwart einer Säure wie Salzsäure, Bromwasserstoffsäure, Schwefelsäure, Phosphorsäure oder Trichloressigsäure, in einem geeigneten Lösungsmittel wie Wasser, Wasser/Methanol, Ethanol, Wasser/Ethanol, Wasser/Isopropanol oder Wasser/Dioxan, bei Temperaturen zwischen -10 und 120°C, z.B. bei Temperaturen zwischen 20°C und der Siedetemperatur des Reaktionsgemisches, in eine entsprechende 2-Hydroxyverbindung der Formel I übergeführt werden.

c) Zur Herstellung von Verbindungen der Formel I, in der mindestens einer der Reste $R_1$, $R_2$ und/oder $R_3$ einen der für $R_1$, $R_2$ und/oder $R_3$ eingangs erwähnten eine Sulfinyl- oder Sulfonylgruppe enthaltenden Reste darstellt und die übrigen der Reste $R_1$, $R_2$ und/oder $R_3$ wie eingangs definiert sind:
Oxidation einer Verbindung der Formel

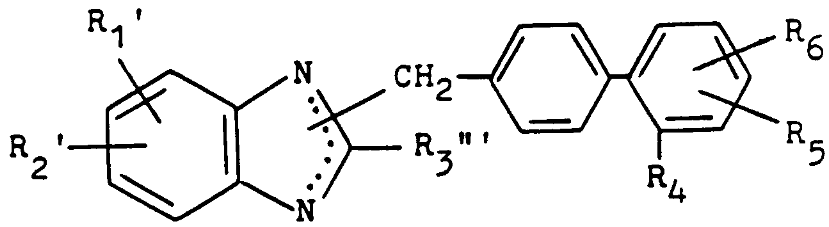
,(V)

in der
$R_4$ bis $R_6$ wie eingangs definiert sind,
mindestens einer der Reste $R_1'$, $R_2'$ und/oder $R_3'''$ einen der für $R_1$, $R_2$ und/oder $R_3$ eingangs erwähnten ein Schwefelatom oder eine Sulfinylgruppe enthaltenden Reste darstellt und die übrigen der Reste $R_1'$, $R_2'$ und/oder $R_3'''$ die für $R_1$, $R_2$ und/oder $R_3$ eingangs erwähnten Bedeutungen besitzen.

Die Oxidation wird vorzugsweise in einem Lösungsmittel oder Lösungsmittelgemisch, z.B. in Wasser, Wasser/Pyridin, Aceton, Eisessig, verdünnter Schwefelsäure oder Trifluoressigsäure, je nach dem verwendeten Oxidationsmittel zweckmäßigerweise bei Temperaturen zwischen -80 und 100°C durchgeführt.

Zur Herstellung einer Sulfinylverbindung der Formel I wird die Oxidation zweckmäßigerweise mit einem Äquivalent des verwendeten Oxidationsmittels durchgeführt, z.B. mit Wasserstoffperoxid in Eisessig, Trifluoressigsäure oder Ameisensäure bei 0 bis 20°C oder in Aceton bei 0 bis 60°C, mit einer Persäure wie Perameisensäure in Eisessig oder Trifluoressigsäure bei 0 bis 50°C oder mit m-Chlorperbenzoesäure in Methylenchlorid oder Chloroform bei -20 bis 60°C, mit Natriummetaperjodat in wässrigem Methanol oder Äthanol bei -15 bis 25°C, mit Brom in Eisessig

oder wässriger Essigsäure, mit N-Brom-succinimid in Äthanol, mit tert.Butyl-hypochlorit in Methanol bei -80 bis -30°C, mit Jodbenzodichlorid in wässrigem Pyridin bei 0 bis 50°C, mit Salpetersäure in Eisessig bei 0 bis 20°C, mit Chromsäure in Eisessig oder in Aceton bei 0 bis 20°C und mit Sulfurylchlorid in Methylenchlorid bei -70°C, der hierbei erhaltene Thioäther-Chlor-Komplex wird zweckmäßigerweise mit wäßrigem Äthanol hydrolysiert.

Zur Herstellung einer Sulfonylverbindung der Formel I wird die Oxidation zweckmäßigerweise mit einem bzw. mit zwei oder mehr Äquivalenten des verwendeten Oxidationsmittels durchgeführt, z.B. mit Wasserstoffperoxid in Eisessig, Trifluoressigsäure oder in Ameisensäure bei 20 bis 100°C oder in Aceton bei 0 bis 60°C, mit einer Persäure wie Perameisensäure oder m-Chlorperbenzoesäure in Eisessig, Trifluoressigsäure, Methylenchlorid oder Chloroform bei Temperaturen zwischen 0 und 60°C, mit Salpetersäure in Eisessig bei 0 bis 20°C, mit Chromsäure oder Kaliumpermanganat in Eisessig, Wasser/Schwefelsäure oder in Aceton bei 0 bis 20°C.

d) Zur Herstellung einer Verbindung der Formel I, in der $R_4$ eine Carboxy- oder Aminogruppe darstellt: Überführung einer Verbindung der Formel

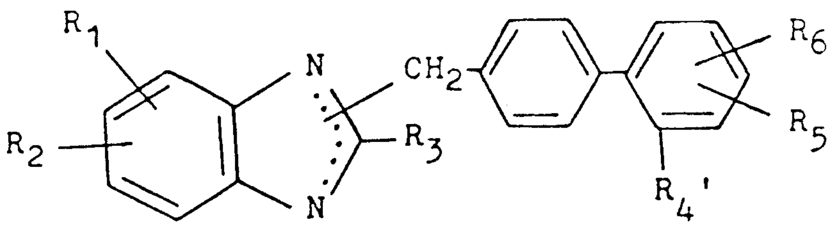 ,(VI)

in der

$R_1$ bis $R_3$, $R_5$ und $R_6$ wie eingangs definiert sind und

$R_4'$ eine mittels Hydrolyse, Thermolyse oder Hydrogenolyse in eine Carboxygruppe überführbare Gruppe oder eine mittels Hydrolyse, Hydrogenolyse oder Umamidierung in eine Aminogruppe überführbare Gruppe darstellt.

Beispielsweise können funktionelle Derivate der Carboxygruppe wie deren unsubstituierte oder substituierte Amide, Ester, Thiolester, Orthoester, Iminoäther, Amidine oder Anhydride, die Nitrilgruppe oder die Tetrazolylgruppe mittels Hydrolyse in eine Carboxygruppe,

Ester mit tertiären Alkoholen, z.B. der tert. Butylester, mittels Thermolyse in eine Carboxygruppe,

Ester mit Aralkanolen, z.B. der Benzylester, mittels Hydrogenolyse in eine Carboxygruppe,

Acylaminogruppen, z.B. die Benzoylamino- oder Phthalimidogruppe, mittels Hydrolyse in eine Aminogruppe Iminogruppen und

Iminogruppen, z.B. die Phthaliminogruppe, mittels Umamidierung in eine Aminogruppe übergeführt werden.

Die Hydrolyse wird zweckmäßigerweise entweder in Gegenwart einer Säure wie Salzsäure, Schwefelsäure, Phosphorsäure, Trichloressigsäure oder Trifluoressigsäure in Gegenwart einer Base wie Natriumhydroxid oder Kaliumhydroxid in einem geeigneten Lösungsmittel wie Wasser, Wasser/Methanol, Äthanol, Wasser/Äthanol, Wasser/Isopropanol oder Wasser/Dioxan bei Temperaturen zwischen -10°C und 120°C, z.B. bei Temperaturen zwischen Raumtemperatur und der Siedetemperatur des Reaktionsgemisches, durchgeführt. Bei der Hydrolyse in Gegenwart einer organischen Säuren wie Trichloressigsäure oder Trifluoressigsäure können gegebenenfalls vorhandene alkoholische Hydroxygruppen gleichzeitig in eine entsprechende Acyloxygruppe wie die Trifluoracetoxygruppe übergeführt werden.

Bedeutet $R_4'$ in einer Verbindung der Formel VI eine Cyano- oder Aminocarbonylgruppe, so können diese Gruppen auch mit einem Nitrit, z.B. Natriumnitrit, in Gegenwart einer Säure wie Schwefelsäure, wobei diese zweckmäßigerweise gleichzeitig als Lösungsmittel verwendet wird, bei Temperaturen zwischen O und 50°C in die Carboxygruppe übergeführt werden.

Bedeutet $R_4'$ in einer Verbindung der Formel VI beispielsweise die tert. Butyloxycarbonylgruppe, so kann die tert. Butylgruppe auch thermisch gegebenenfalls in einem inerten Lösungsmittel wie Methylenchlorid, Chloroform, Benzol, Toluol, Tetrahydrofuran oder Dioxan und vorzugsweise in Gegenwart einer katalytischen Menge einer Säure wie p-Toluolsulfonsäure, Schwefelsäure, Phosphorsäure oder Polyphosphorsäure vorzugsweise bei der Siedetemperatur des verwendeten Lösungsmittels, z.B. bei Temperaturen zwischen 40°C und 100°C, abgespalten werden.

Bedeutet $R_4'$ in einer Verbindung der Formel VI beispielsweise die Benzyloxycarbonylgruppe, so kann die Benzylgruppe auch hydrogenolytisch in Gegenwart eines Hydrierungskatalysators wie Palladium/Kohle in einem geeigneten Lösungsmittel wie Methanol, Äthanol, Äthanol/Wasser, Eisessig, Essigsäureäthylester, Dioxan oder Dimethylformamid vorzugsweise bei Temperaturen zwischen 0 und 50°C, z.B. bei Raumtemperatur, und einem Wasserstoffdruck von 1 bis 5 bar abgespalten werden. Bei der Hydrogenolyse können gleichzeitig andere Reste, z.B. eine Nitrogruppe zur Aminogruppe, eine Benzyloxygruppe zur Hydroxygruppe, eine Vinylidengruppe zur

entsprechenden Alkylidengruppe oder eine Zimtsäuregruppe zur entsprechenden Phenyl-propionsäuregruppe, mitreduziert oder durch Wasserstoffatome, z.B. ein Halogenatom durch ein Wasserstoffatom, ersetzt werden.

Bedeutet $R_4'$ eine Phthaliminogruppe, so kann diese Gruppe in Gegenwart einer primären organischen Base wie Methylamin, Ethylamin oder Propylamin in einem geeigneten Lösungsmittel wie Methanol, Ethanol, Isopropanol, Dimethylformamid, Methanol/Dimethylformamid oder Methanol/Wasser durch Umamidierung bei Temperaturen zwischen 0 und 50°C, vorzugsweise jedoch bei Raumtemperatur, besonders vorteilhaft in eine Aminogruppe übergeführt werden. Bedeutet hierbei $R_1$ und/oder $R_2$ einen der eingangs erwähnten Iminoreste, so werden diese gleichzeitig in eine entsprechende Aminogruppe übergeführt.

Bedeutet $R_1$ und/oder $R_2$ in einer Verbindung der Formel VI einen der eingangs erwähnten Iminoreste, so kann dieser desweiteren während der Umsetzung partiell hydrolysiert werden, also beispielsweise in eine 2-Carboxyphenylcarbonylamino-, 2-Carboxyphenylmethylamino-, 2-Carboxyphenylmethylencarbonylamino- oder 2-Carboxymethylenphenylcarbonylaminogruppe, wobei eine Methylengruppe in einer 2-Carboxyphenylmethylencarbonylamino- oder 2-Carboxymethylenphenylcarbonylaminogruppe durch eine oder zwei Alkylgruppen substituiert sein kann, und zusätzlich die vorstehend erwähnten Phenylkerne durch Alkyl- oder Alkoxygruppen mono- oder disubstituiert, wobei die Substituenten gleich oder verschieden sein können, und gleichzeitig ganz oder teilweise hydriert sein können, eine in 2-Stellung durch eine Carboxygruppe substituierte Bicycloalkan-3-carbonsäureamino- oder Bicycloalken-3-carbonsäureaminogruppe, in denen der Bicycloalkan- und Bicycloalkenteil jeweils 9 oder 10 Kohlenstoffatome enthalten, durch 1, 2 oder 3 Methylgruppen substituiert und eine Endomethylengruppe durch ein Sauerstoffatom ersetzt sein kann, eine 3-Carboxy-n-propylencarbonylgruppe, in der die n-Propylengruppe perfluoriert, durch ein oder zwei Alkylgruppen oder durch eine Tetramethylen- oder Pentamethylengruppe substituiert sein kann, übergeführt werden.

e) Umsetzung eines Benzimidazols der Formel

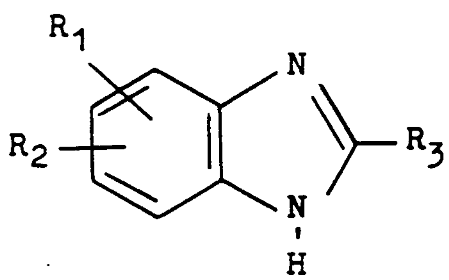
,(VII)

in der
$R_1$ bis $R_3$ wie eingangs definiert sind, mit einer Biphenylverbindung der Formel

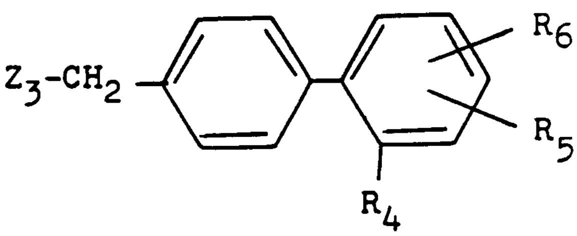
,(VIII)

in der
$R_4$ bis $R_6$ wie eingangs definiert sind und
$Z_3$ eine nukleophile Austrittsgruppe wie ein Halogenatom, z.B. ein Chlor-, Brom- oder Jodatom, oder eine substituierte Sulfonyloxygruppe, z.B. eine Methansulfonyloxy-, Phenylsulfonyloxy- oder p-Toluolsulfonyloxygruppe darstellt.

Die Umsetzung wird zweckmäßigerweise in einem Lösungsmittel oder Lösungsmittelgemisch wie Methylenchlorid, Diethylether, Tetrahydrofuran, Dioxan, Dimethylsulfoxid oder Benzol gegebenenfalls in Gegenwart eines säurebindenden Mittels, wie Natriumcarbonat, Kaliumcarbonat, Natriumhydroxid, Kalium-tert.butylat, Triethylamin oder Pyridin, wobei die beiden letzteren gleichzeitig auch als Lösungsmittel verwendet werden können, vorzugsweise bei Temperaturen zwischen 0 und 100°C, z.B. bei Temperaturen zwischen Raumtemperatur und 50°C, durchgeführt.

Bei der Umsetzung erhält man vorzugsweise ein Gemisch der 1- und 3-Isomeren.

f) Zur Herstellung einer Verbindung der Formel I, in der $R_4$ eine 1H-Tetrazolylgruppe darstellt:
Abspaltung eines Schutzrestes von einer Verbindung der Formel

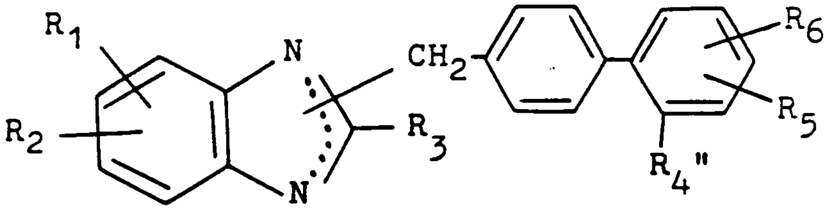
, (IX)

in der
$R_1$ bis $R_3$, $R_5$ und $R_6$ wie eingangs definiert sind und
$R_4''$ eine in 1-Stellung durch einen Schutzrest geschützte 1H-Tetrazolylgruppe darstellt.

Als Schutzrest kommt beispielsweise die Triphenylmethyl-, Tributylzinn- oder Triphenylzinngruppe in Betracht.

Die Abspaltung eines verwendeten Schutzrestes erfolgt vorzugsweise in Gegenwart eines Halogenwasserstoffes, vorzugsweise in Gegenwart von Chlorwasserstoff, in Gegenwart einer Base wie Natriumhydroxid oder alkoholischem Ammoniak in einem geeigneten Lösungsmittel wie Methylenchlorid, Methanol, Methanol/Ammoniak, Ethanol oder Isopropanol bei Temperaturen zwischen 0 und 100°C, vorzugsweise jedoch bei Raumtemperatur, oder auch, falls die Umsetzung in Gegenwart von alkoholischem Ammoniak durchgeführt wird, bei erhöhten Temperaturen, z.B. bei Temperaturen zwischen 100 und 150°C, vorzugsweise bei Temperaturen zwischen 120 und 140°C.

g) Zur Herstellung einer Verbindung der Formel I, in der $R_4$ eine 1H-Tetrazolylgruppe darstellt:
Umsetzung einer Verbindung der Formel

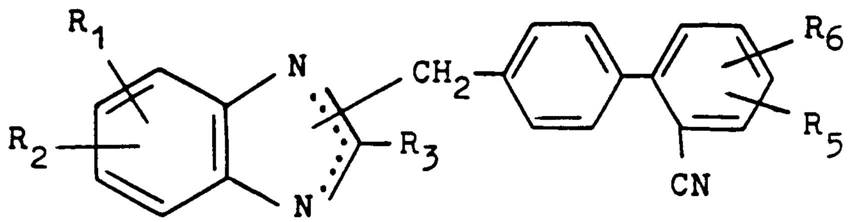
, (X)

in der
$R_1$ bis $R_3$, $R_5$ und $R_6$ wie eingangs definiert sind, mit Stickstoffwasserstoffsäure.

Die Umsetzung wird vorzugsweise in einem Lösungsmittel wie Benzol, Toluol oder Dimethylformamid bei Temperaturen zwischen 80 und 130°C, vorzugsweise bei 125°C, durchgeführt. Hierbei wird die Stickstoffwasserstoffsäure besonders vorteilhaft während der Umsetzung aus einem Alkaliazid, z.B. aus Natriumazid, in Gegenwart einer schwachen Säure wie Ammoniumchlorid freigesetzt.

h) Zur Herstellung einer Verbindung der Formel I, in der $R_1$ und/oder $R_2$ eine Aminocarbonylaminogruppe darstellt, welche durch eine bi- oder tricyclische Alkylgruppe oder durch eine Alkyl-, Alkenyl-, Alkinyl-, Cycloalkyl-, Cycloalkylalkyl-, Aralkyl- oder Arylgruppe mono-, di- oder tribsubstituiert sein kann, wobei eine Methylengruppe in einem Cycloalkylrest mit 4 bis 7 Kohlenstoffatomen durch ein Sauerstoffatom ersetzt sein kann:
Umsetzung einer Verbindung der Formel

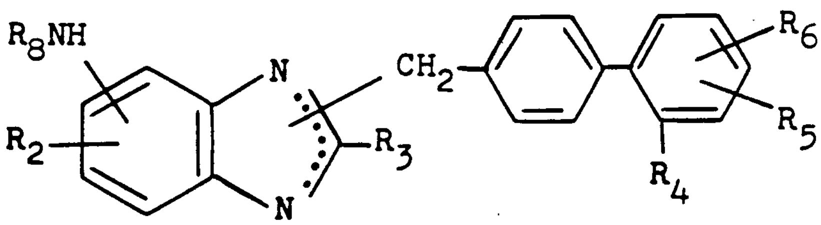
, (XI)

in der
$R_2$ bis $R_6$ wie eingangs definiert sind und
$R_8$ ein Wasserstoffatom, eine Alkylgruppe mit 1 bis 20 Kohlenstoffatomen, eine Alkenyl- oder Alkinylgruppe mit jeweils 3 bis 5 Kohlenstoffatomen, eine Cycloalkylgruppe mit 3 bis 7 Kohlenstoffatomen, eine Cycloalkyl-alkylgruppe,

in welcher der Cycloalkylteil 3 bis 7 Kohlenstoffatome, der Alkylteil 1 bis 3 Kohlenstoffatome enthalten und in einem Cycloalkylteil mit 4 bis 7 Kohlenstoffatomen eine Methylengruppe durch ein Sauerstoffatom ersetzt sein kann, eine bi- oder tricyclische Alkylgruppe mit 7 bis 10 Kohlenstoffatomen, eine Aryl- oder Aralkylgruppe mit 1 bis 3 Kohlenstoffatomen im Alkylteil, wobei die Arylgruppe eine gegebenenfalls durch eine Fluor-, Chlor- oder Bromatom, eine Hydroxy-, Alkyl- oder Alkoxygruppe mit jeweils 1 bis 4 Kohlenstoffatomen substituierte Phenylgruppe darstellen kann, bedeutet, mit einer Verbindung der Formel

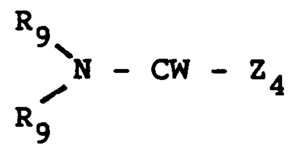

,(XII)

in der
$R_9$, die gleich oder verschieden sein können, die vorstehend für $R_8$ erwähnten Bedeutungen aufweisen,
W ein Sauerstoff- oder Schwefelatom,
$Z_4$ eine nukleophile Austrittsgruppe wie ein Chlor- oder Bromatom oder auch, wenn mindestens einer der Reste $R_9$ ein Wasserstoffatom darstellt,
$Z_4$ und $R_9$ zusammen eine Stickstoff-Kohlenstoff-Bindung oder eine Cycloalkyleniminogruppe mit 4 bis 6 Kohlenstoffatomen oder eine Morpholinogruppe darstellen.

Die Umsetzung wird vorzugsweise in einem Lösungsmittel wie Tetrahydrofuran, Dioxan, Ethylenchlorid oder Benzol gegebenenfalls in Gegewart eines säurebindenden Mittels wie Triethylamin oder Pyridin zweckmäßigerweise bei Temperaturen zwischen 0 und 100°C, vorzugsweise bei Temperaturen zwischen 20 und 80°C, durchgeführt.

i) Zur Herstellung einer Verbindung der Formel I, in der $R_4$ eine Trifluormethylcarbonylamino-, Trifluormethylcarbonylaminomethyl-, Trifluormethylsulfonylamino-, Trifluormethylsulfonylaminomethyl- oder 1H-Tetrazolylgruppe, eine Alkylcarbonylamino-, Alkylcarbonylaminomethyl-, Arylcarbonylamino-, Arylcarbonylaminomethyl-, Aralkylcarbonylamino-, Aralkylcarbonylaminomethyl-, Alkylsulfonylamino-, Alkylsulfonylaminomethyl-, Arylsulfonylamino-, Arylsulfonylaminomethyl-, Aralkylsulfonylamino- oder Aralkylsulfonylaminomethylgruppe, in denen der Alkylteil jeweils 1 bis 3 Kohlenstoffatome enthalten kann, darstellt:
Acylierung einer Verbindung der Formel

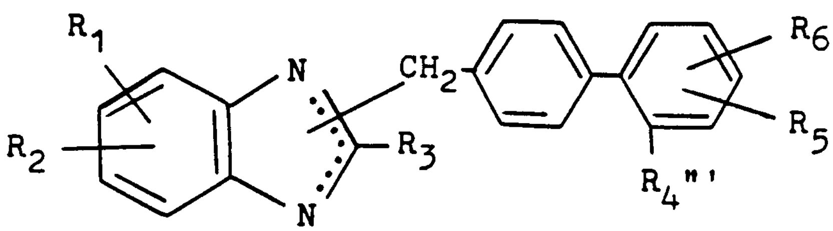

,(XIII)

in der
$R_1$ bis $R_3$, $R_5$ und $R_6$ wie eingangs definiert sind und
$R_4'''$ eine Amino- oder Aminomethylgruppe darstellt, mit einer Verbindung der Formel

$$HO - U - R_{10} ,(XIV)$$

in der
$R_{10}$ eine Trifluormethylgruppe, eine Alkyl-, Aryl- oder Aralkylgruppe, wobei der Alkylteil jeweils 1 bis 3 Kohlenstoffatome enthalten kann, und
U eine Carbonyl- oder Sulfonylgruppe bedeuten, oder mit deren reaktionsfähigen Derivaten wie deren Säurehalogeniden, Säureestern oder Säureanhydriden.

Als reaktionsfähige Derivate einer Verbindung der Formel XIV kommen beispielsweise deren Ester wie der Methyl-, Ethyl- oder Benzylester, deren Thioester wie der Methylthio- oder Ethylthioester, deren Halogenide wie das Säurechlorid, deren Anhydride oder Imidazolide in Betracht.

Die Umsetzung wird zweckmäßigerweise in einem Lösungsmittel oder Lösungsmittelgemisch wie Wasser, Methylenchlorid, Chloroform, Äther, Tetrahydrofuran, Dioxan oder Dimethylformamid mit einer entsprechenden Carbonsäure in Gegenwart eines die Säure aktivierenden oder wasserentziehenden Mittels wie Thionylchlorid, mit deren Anhydriden wie Essigsäureanhydrid, mit deren Estern wie Essigsäureäthylester, mit deren Halogeniden wie Acetylchlorid oder Methansulfonylchlorid gegebenenfalls in Gegenwart einer anorganischen oder tertiären organischen Base, wie Natriumhydroxid, Kaliumcarbonat, Triäthylamin oder Pyridin, wobei die beiden letzteren gleichzeitig auch als Lösungsmittel dienen können, bei Temperaturen zwischen -25 und 100°C, vorzugsweise jedoch bei Temperaturen zwischen -10 und 80°C, durchgeführt.

k) Zur Herstellung einer Verbindung der Formel I, in der $R_4$ eine Alkylsulfonylaminocarbonyl- oder Perfluoralkylsulfonylaminogruppe mit jeweils 1 bis 4 Kohlenstoffatomen im Alkylteil oder eine Benzylsulfonylaminocarbonylgruppe darstellt:

Umsetzung eines reaktionsfähigen Derivates einer Carbonsäure der Formel

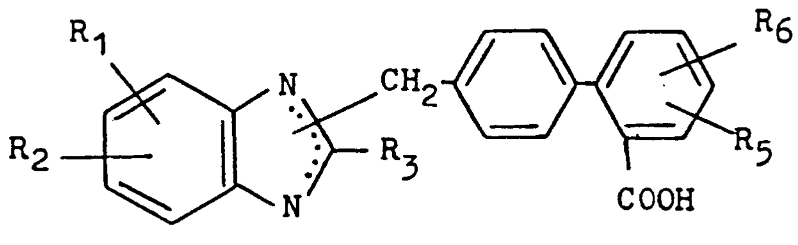

, (XV)

in der

$R_1$ bis $R_3$, $R_5$ und $R_6$ wie eingangs definiert sind,

mit einem Sulfonamid der Formel

$$H_2N - SO_2 - R_{11} ,(XVI)$$

in der

$R_{11}$ eine Alkyl- oder Perfluoralkylgruppe mit jeweils 1 bis 4 Kohlenstoffatomen oder eine Benzylgruppe darstellt, oder mit dessen Alkalisalz.

Als reaktionsfähige Derivate einer Carbonsäure der Formel XV, welche im Reaktionsgemisch hergestellt werden können, kommen deren Halogenide wie das Chlorid oder Bromid oder deren Kohlensäureesterderivate wie das Imidazolcarbonyloxy- oder Diphenylcarbamoyloxyderivat in Betracht.

Die Umsetzung wird zweckmäßigerweise in einem geeigneten Lösungsmittel wie Methylenchlorid, Tetrahydrofuran, Dioxan oder Dimethylsulfoxid bei Temperaturen zwischen 0 und 180°C, vorzugsweise zwischen Raumtemperatur und 150°C, durchgeführt. Die Umsetzung kann jedoch auch in der Schmelze durchgeführt werden.

l) Zur Herstellung einer Verbindung der Formel I, in der $R_2$ eine gegebenfalls in 3-Stellung durch eine Alkyl-, Cycloalkyl-, Cycloalkylalkyl- oder Aralkylgruppe substituierte 2-Imidazolidon-1-yl- oder 3,4,5,6-Tetrahydro-2-pyrimidon-1-yl-gruppe darstellt:

Cyclisierung einer Verbindung der Formel

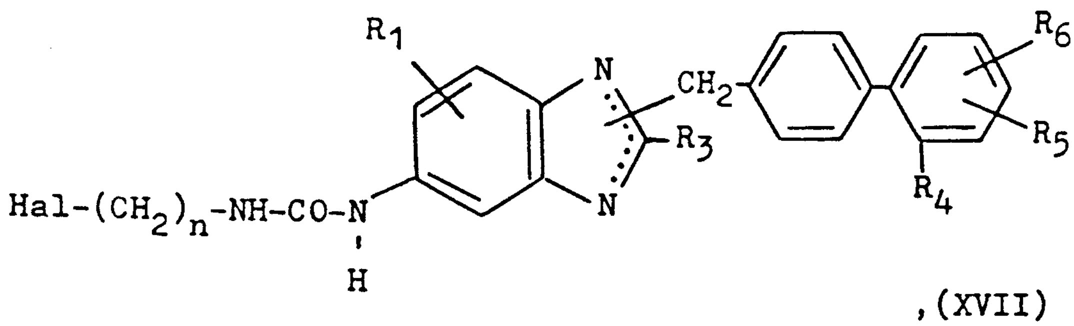

, (XVII)

in der

$R_1$, $R_3$ und $R_4$ bis $R_6$ wie eingangs definiert sind,

Hal ein Chlor-, Brom- oder Jodatom und

n die Zahl 2 oder 3 darstellen, und erforderlichenfalls anschließende Umsetzung mit einer Verbindung der Formel

$$R_{12} - Hal ,(XVIII)$$

in der

$R_{12}$ eine Alkyl-, Cycloalkyl-, Cycloalkylalkyl- oder Aralkylgruppe und
Hal ein Chlor-, Brom- oder Jodatom darstellt.

Die Cyclisierung und erforderlichenfalls die anschließende Alkylierung wird zweckmäßigerweise in einem Lösungsmittel wie Methanol, Ethanol, Benzol oder Dimethylsulfoxid gegebenenfalls in Gegenwart eines Phasentransferkatalysators wie Benzyltriethylammoniumbromid in Gegenwart eines säurebindenden Mittels wie Natriumhydroxid, Natriummethylat, Natriumethylat, Natriumhydrid oder Kalium-tert.butylat bei Temperaturen zwischen 20 und 100°C vorzugsweise bei Temperaturen zwischen 30 und 70°C, durchgeführt.

m) Zur Herstellung von Verbindungen der allgemeinen Formel I, in der

$R_1$ eine Aminogruppe, die durch eine Dialkylaminoalkanoyl-, Acyl-, Cycloalkoxycarbonyl-, Cycloalkylalkoxycarbonyl, Aryloxycarbonyl-, Aralkoxycarbonyl- oder Trifluoracetylgruppe, durch eine Alkylsulfonylgruppe mit 1 bis 4 Kohlenstoffatomen, durch eine Alkoxycarbonylgruppe mit insgesamt 2 bis 7 Kohlenstoffatomen oder durch eine durch eine Alkoxycarbonylgruppe substituierte Thiazolidin-3-yl-carbonylgruppe monosubstituiert ist,

eine Alkylaminogruppe mit 1 bis 6 Kohlenstoffatomen, die am Stickstoffatom durch eine Alkylsulfonyl- oder Acylgruppe substituiert sein kann, wobei, falls die Acylgruppe eine Alkanoylgruppe darstellt, diese zusätzlich durch eine Alkoxygruppe substituiert sein kann, und der Alkylsubstituent ab Position 2 durch eine Hydroxy-, Alkoxy- oder Arylaminogruppe substituiert sein kann,

eine N-Alkoxycarbonyl-alkylamino-, N-Cycloalkoxycarbonyl-alkylamino-, N-Cycloalkylalkoxycarbonyl-alkylamino-, N-Aryloxycarbonyl-alkylamino- oder N-Aralkoxycarbonyl-alkylaminogruppe, in der die Alkylgruppe jeweils 1 bis 6 Kohlenstoffatome enthalten kann, eine am Stickstoffatom durch eine Cycloalkyl-, Cycloalkylalkyl- oder Aralkylgruppe substituierte Alkoxycarbonylamino-, Cycloalkoxycarbonylamino-, Cycloalkylalkoxycarbonylamino-, Aryloxycarbonylamino-, Aralkoxycarbonylamino-, Acylamino- oder Alkylsulfonylaminogruppe, oder

eine Phthalimino-, Homophthalimino-, 2-Carboxyphenylcarbonylamino-, 2-Carboxyphenylmethylamino-, 2-Carboxyphenylmethylencarbonylamino- oder 2-Carboxymethylenphenylcarbonylaminogruppe, wobei eine Carbonylgruppe in einer Phthaliminogruppe durch eine Methylengruppe ersetzt sowie eine Methylengruppe in einer Homophthalimino-, 2-Carboxyphenylmethylencarbonylamino- oder 2-Carboxymethylenphenylcarbonylaminogruppe durch eine oder zwei Alkylgruppen substituiert sein kann, und zusätzlich die vorstehend erwähnten Phenylkerne durch Alkyl- oder Alkoxygruppen mono- oder disubstituiert, wobei die Substituenten gleich oder verschieden sein können, und gleichzeitig ganz oder teilweise hydriert sein können, eine in 2-Stellung durch eine Carboxygruppe substituierte Bicycloalkan-3-carbonsäureamino- oder Bicycloalken-3-carbonsäureaminogruppe, eine Bicycloalkan-2,3-dicarbonsäureimino- oder Bicycloalken-2,3-dicarbonsäureiminogruppe in denen der Bicycloalkan- und Bicycloalkenteil jeweils 9 oder 10 Kohlenstoffatome enthalten, durch 1, 2 oder 3 Methylgruppen substituiert und eine Endomethylengruppe durch ein Sauerstoffatom ersetzt sein kann, eine Glutarsäureimino- oder 3-Carboxy-n-propylencarbonylgruppe, in denen jeweils die n-Propylengruppe perfluoriert, durch ein oder zwei Alkylgruppen oder durch eine Tetramethylen- oder Pentamethylengruppe substituiert sein kann, bedeutet und

$R_4$ mit Ausnahme der Aminogruppe die für $R_4$ eingangs erwähnten Bedeutungen besitzt:

Acylierung einer Verbindung der allgemeinen Formel

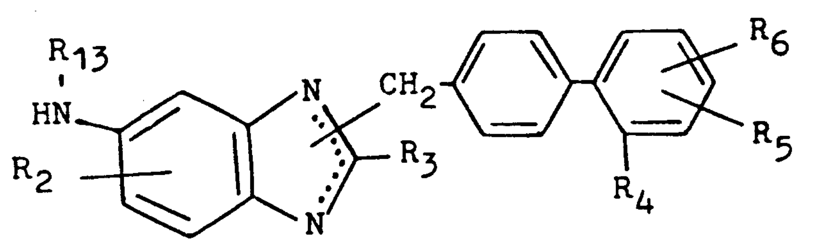

,(XIX)

in der

$R_2$, $R_3$, $R_5$ und $R_6$ wie eingangs definiert sind,
$R_4$ mit Ausnahme der Aminogruppe wie eingangs definiert ist und
$R_{13}$ ein Wasserstoffatom, eine Alkylgruppe mit 1 bis 6 Kohlenstoffatomen, welche ab Position 2 durch eine Hydroxy-, Alkoxy- oder Arylaminogruppe substituiert sein kann, eine Cycloalkyl-, Cycloalkylalkyl- oder Aralkylgruppe bedeutet, mit einer Verbindung der Formel

$$R_{14} - Z_5 ,(XX)$$

in der

$R_{14}$ eine Dialkylaminoalkanoyl-, Cycloalkoxycarbonyl-, Cycloalkylalkoxycarbonyl-, Aryloxycarbonyl-, Aralkoxycarbonyl- oder oder Trifluoracetylgruppe, eine Alkylsulfonylgruppe mit 1 bis 4 Kohlenstoffatomen, eine Alkoxycarbonylgruppe mit insgesamt 2 bis 7 Kohlenstoffatomen, eine durch eine Alkoxycarbonylgruppe substituierte Thiazolidin-3-yl-carbonylgruppe oder eine Acylgruppe, in welcher, falls die Acylgruppe eine Alkanoylgruppe darstellt, diese zusätzlich durch eine Alkoxygruppe substituiert sein kann, oder eine 2-Carboxyphenylcarbonyl-, 2-Carboxyphenylmethyl-, 2-Carboxyphenylmethylencarbonyl- oder 2-Carboxymethylenphenylcarbonylgruppe, wobei eine Methylengruppe in einer 2-Carboxyphenylmethylencarbonyl- oder 2-Carboxymethylenphenylcarbonylgruppe durch eine oder zwei Alkylgruppen substituiert sein kann, und zusätzlich die vorstehend erwähnten Phenylkerne durch Alkyl- oder Alkoxygruppen mono- oder disubstituiert, wobei die Substituenten gleich oder verschieden sein können, und gleichzeitig ganz oder teilweise hydriert sein können, eine in 2-Stellung durch eine Carboxygruppe substituierte Bicycloalkan-3-carbonyl- oder Bicycloalken-3-carbonylgruppe, in denen der Bicycloalkan- und Bicycloalkenteil jeweils 9 oder 10 Kohlenstoffatome enthalten, durch 1, 2 oder 3 Methylgruppen substituiert und eine Endomethylengruppe durch ein Sauerstoffatom ersetzt sein kann, eine 3-Carboxy-n-propylencarbonylgruppe, in der die n-Propylengruppe perfluoriert, durch ein oder zwei Alkylgruppen oder durch eine Tetramethylen- oder Pentamethylengruppe substituiert sein kann, und

$Z_5$ eine nukleophile Austrittsgruppe bedeuten, oder mit deren reaktionsfähigen Derivaten wie deren Säurehalogeniden, Säureestern oder Säureanhydriden.

Als reaktionsfähige Derivate einer Verbindung der Formel XIX kommen beispielsweise deren Ester wie der Methyl-, Ethyl-oder Benzylester, deren Thioester wie der Methylthio- oder Ethylthioester, deren Halogenide wie das Säurechlorid, deren Anhydride oder Imidazolide in Betracht.

Die Umsetzung wird zweckmäßigerweise in einem Lösungsmittel oder Lösungsmittelgemisch wie Wasser, Methylenchlorid, Chloroform, Äther, Tetrahydrofuran, Dioxan oder Dimethylformamid mit einer entsprechenden Carbonsäure in Gegenwart eines die Säure aktivierenden oder wasserentziehenden Mittels wie Thionylchlorid, mit deren Anhydriden wie Essigsäureanhydrid, mit deren Estern wie Essigsäureäthylester, mit deren Halogeniden wie Acetylchlorid oder Methansulfonylchlorid gegebenenfalls in Gegenwart einer anorganischen oder tertiären organischen Base, wie Natriumhydroxid, Kaliumcarbonat, Triäthylamin oder Pyridin, wobei die beiden letzteren gleichzeitig auch als Lösungsmittel dienen können, bei Temperaturen zwischen -25 und 100°C, vorzugsweise jedoch bei Temperaturen zwischen -10 und 80°C, durchgeführt.

n) Zur Herstellung von Verbindungen der allgemeinen Formel I, in der mindestens einer der Reste $R_1$ oder $R_2$ eine 2-Carboxyphenylcarbonylamino-, 2-Carboxyphenylmethylamino-, 2-Carboxyphenylmethylencarbonylamino- oder 2-Carboxymethylenphenylcarbonylaminogruppe, wobei eine Methylengruppe in einer 2-Carboxyphenylmethylencarbonylamino- oder 2-Carboxymethylenphenylcarbonylaminogruppe durch eine oder zwei Alkylgruppen substituiert sein kann, und zusätzlich die vorstehend erwähnten Phenylkerne durch Alkyl- oder Alkoxygruppen mono- oder disubstituiert, wobei die Substituenten gleich oder verschieden sein können, und gleichzeitig ganz oder teilweise hydriert sein können, eine in 2-Stellung durch eine Carboxygruppe substituierte Bicycloalkan-3-carbonsäureamino- oder Bicycloalken-3-carbonsäureaminogruppe, in denen der Bicycloalkan- und Bicycloalkenteil jeweils 9 oder 10 Kohlenstoffatome enthalten, durch 1, 2 oder 3 Methylgruppen substituiert und eine Endomethylengruppe durch ein Sauerstoffatom ersetzt sein kann, eine 3-Carboxy-n-propylencarbonylgruppe, in der die n-Propylengruppe perfluoriert, durch ein oder zwei Alkylgruppen oder durch eine Tetramethylen- oder Pentamethylengruppe substituiert sein kann, bedeutet:

Partielle Hydrolyse einer Verbindung der allgemeinen Formel

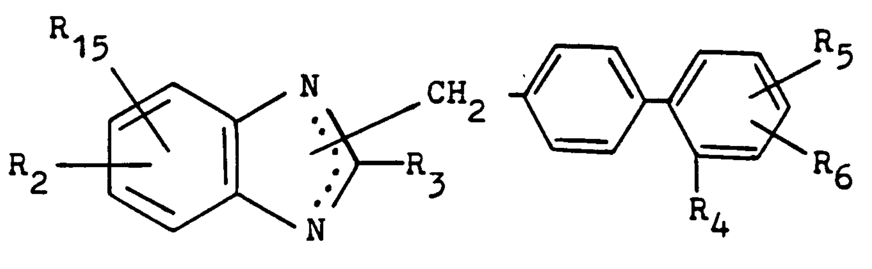

, (XXI)

in der

$R_2$ bis $R_6$ wie eingangs definiert sind und

$R_{15}$ eine Phthalimino- oder Homophthaliminogruppe, wobei eine Carbonylgruppe in einer Phthaliminogruppe durch eine Methylengruppe ersetzt sowie eine Methylengruppe in einer Homophthaliminogruppe durch eine oder zwei Alkylgruppen substituiert sein kann, und zusätzlich die vorstehend erwähnten Phenylkerne durch Alkyl- oder Alkoxygruppen mono- oder disubstituiert, wobei die Substituenten gleich oder verschieden sein können, und gleichzeitig ganz oder teilweise hydriert sein können, eine Bicycloalkan-2,3-dicarbonsäureimino- oder Bicycloalken-2,3-dicarbonsäureiminogruppe, in denen der Bicycloalkan- und Bicycloalkenteil jeweils 9 oder 10 Kohlenstoffatome enthalten, durch 1, 2 oder 3 Methylgruppen substituiert und eine Endomethylengruppe durch ein Sauerstoffatom ersetzt sein kann, eine Glutarsäureiminogruppe, in der die n-Propylengruppe perfluoriert, durch ein oder zwei Alkylgruppen oder durch eine Tetramethylen- oder Pentamethylengruppe substituiert sein kann, bedeutet.

Die partielle Hydrolyse wird zweckmäßigerweise entweder in Gegenwart einer Säure wie Salzsäure, Schwefelsäure, Phosphorsäure, Trichloressigsäure oder Trifluoressigsäure oder in Gegenwart einer Base wie Natriumhydroxid oder Kaliumhydroxid in einem geeigneten Lösungsmittel wie Wasser, Wasser/Methanol, Äthanol, Wasser/Äthanol, Wasser/Isopropanol oder Wasser/Dioxan bei Temperaturen zwischen -10°C und 120°C, z.B. bei Temperaturen zwischen Raumtemperatur und der Siedetemperatur des Reaktionsgemisches, durchgeführt. Besonders vorteilhaft wird die partielle Hydrolyse jedoch in Gegenwart eines Äquivalents einer anorganischen Base bei Raumtemperatur durchgeführt.

Bei den vorstehend beschriebenen Umsetzungen können gegebenenfalls vorhandene reaktive Gruppen wie Hydroxy-, Amino-oder Alkylaminogruppen während der Umsetzung durch übliche Schutzgruppen geschützt werden, welche nach der Umsetzung wieder abgespalten werden.

Beispielsweise kommt als Schutzrest für eine Hydroxygruppe die Trimethylsilyl-, Acetyl-, Benzoyl-, Methyl-, Ethyl-, tert.Butyl-, Benzyl- oder Tetrahydropyranylgruppe und als Schutzrest für eine Amino-, Alkylamino- oder Iminogruppe die Acetyl-, Benzoyl-, Ethoxycarbonyl- oder Benzylgruppe in Betracht.

Die gegebenenfalls anschließende Abspaltung eines verwendeten Schutzrestes erfolgt vorzugsweise hydrolytisch in einem wässrigen Lösungsmittel, z.B. in Wasser, Isopropanol/Wasser, Tetrahydrofuran/Wasser oder Dioxan/Wasser, in Gegenwart einer Säure wie Salzsäure oder Schwefelsäure oder in Gegenwart einer Alkalibase wie Natriumhydroxid oder Kaliumhydroxid bei Temperaturen zwischen 0 und 100°C, vorzugsweise bei der Siedetemperatur des Reaktionsgemisches. Die Abspaltung eines Benzylrestes erfolgt jedoch vorzugsweise hydrogenolytisch, z.B. mit Wasserstoff in Gegenwart eines Katalysators wie Palladium/Kohle in einem Lösungsmittel wie Methanol, Ethanol, Essigsäureethylester oder Eisessig gegebenenfalls unter Zusatz einer Säure wie Salzsäure bei Temperaturen zwischen 0 und 50°C, vorzugsweise jedoch bei Raumtemperatur, und einem Wasserstoffdruck von 1 bis 7 bar, vorzugsweise jedoch von 3 bis 5 bar.

Erhält man erfindungsgemäß eine Verbindung der Formel I, in der $R_1$ und/oder $R_2$ eine Nitrogruppe darstellt, so kann diese mittels Reduktion in eine entsprechende Aminoverbindung der Formel I übergeführt werden oder
eine Verbindung der Formel I, in der $R_1$ und/oder $R_2$ eine Hydroxy-, Amino-, Alkylamino-, Phenylalkylamino-, Alkylsulfonylamino- oder Acylaminogruppe darstellt, so kann diese mittels Alkylierung in eine entsprechende alkylierte Verbindung der Formel I übergeführt werden oder
eine Verbindung der Formel I, in der $R_1$ und/oder $R_2$ eine Benzyloxygruppe darstellt, so kann diese mittels Entbenzylierung in eine entsprechende Verbindung der Formel I, in der $R_1$ und/oder $R_2$ eine Hydroxygruppe darstellt, übergeführt werden oder
eine Verbindung der Formel I, in der $R_1$ und/oder $R_2$ eine Carboxygruppe darstellt, so kann diese mittels Amidierung in eine entsprechende Verbindung der Formel I, in der $R_1$ und/oder $R_2$ eine gegebenenfalls durch eine oder zwei Alkylgruppen mit jeweils 1 bis 3 Kohlenstoffatomen substituierte Aminocarbonylgruppe darstellt, übergeführt werden oder
eine Verbindung der Formel I, in der $R_1$ und/oder $R_2$ eine Carboxygruppe und/oder $R_4$ eine Cyanogruppe darstellt, so kann diese mittels Reduktion in eine entsprechende Verbindung der Formel I, in der $R_1$ und/oder $R_2$ eine Hydroxymethylgruppe und/oder $R_4$ eine Aminomethylgruppe darstellt, übergeführt werden oder
eine Verbindung der Formel I, in der $R_1$ und/oder $R_2$ eine Alkoxycarbonylgruppe darstellt, so kann diese mittels Hydrolyse in eine entsprechende Verbindung der Formel I, in der $R_1$ und/oder $R_2$ eine Carboxygruppe darstellt, übergeführt werden oder
eine Verbindung der Formel I, in der $R_1$ und/oder $R_2$ eine Alkoxy- oder Phenylalkoxygruppe darstellt, so kann diese mittels Etherspaltung in eine entsprechende Verbindung der Formel I, in der $R_1$ und/oder $R_2$ eine Hydroxygruppe darstellt, übergeführt werden oder
eine Verbindung der Formel I, in der $R_1$ und/oder $R_2$ eine Aminocarbonylamino- oder Aminothiocarbonylgruppe darstellt, die durch eine Cycloalkylgruppe mit 5 bis 10 Kohlenstoffatomen, in der eine Methylengruppe in 2-Stellung durch ein Sauerstoffatom ersetzt ist, substituiert ist und zusätzlich durch eine Alkylgruppe mit 1 bis 20 Kohlenstoffatomen, durch eine Alkenyl- oder Alkinylgruppe mit jeweils 3 bis 5 Kohlenstoffatomen, durch eine bi- oder tricyclische Alkylgruppe mit 7 bis 11 Kohlenstoffatomen, durch eine Cycloalkyl-, Cycloalkylalkyl-, Aralkyl- oder Arylgruppe mono- oder disubstituiert sein kann, so kann diese mittels Reduktion in eine entsprechende Verbindung der Formel I, in der $R_1$ und/oder $R_2$ eine Aminocarbonylamino- oder Aminothiocarbonylgruppe darstellt, die durch eine 4-Hydroxy-n-butyl-, 5-Hydroxy-n-pentyl- oder 6-Hydroxy-n-hexylgruppe substituiert ist und zusätzlich durch eine Alkylgruppe mit 1 bis 20 Kohlenstoffatomen, durch eine Alkenyl- oder Alkinylgruppe mit jeweils 3 bis 5 Kohlenstoffatomen, durch eine bi- oder tricyclische Alkylgruppe mit 7 bis 11 Kohlenstoffatomen, durch eine Cycloalkyl-, Cycloalkylalkyl-, Aralkyl- oder Arylgruppe mono- oder disubstituiert sein kann, darstellt, übergeführt werden oder
eine Verbindung der Formel I, in der $R_1$ und/oder $R_2$ eine Phthaliminogruppe darstellt, welche durch eine Alkyl- oder Alkoxygruppe mono- oder disubstituiert sein kann, wobei die Substituenten gleich oder verschieden sein können, so kann diese mittels Reduktion in eine entsprechende Verbindung der Formel I in der $R_1$ und/oder $R_2$ eine 1-Oxo-isoindolin-2-yl-gruppe, welche durch eine Alkyl- oder Alkoxygruppe mono- oder disubstituiert sein kann, darstellt, wobei die Substituenten gleich oder verschieden sein können, übergeführt werden oder
ein erhaltenes 1-, 3-Isomerengemisch einer Verbindung der Formel I mittels Isomerentrennung in ihr 1- und 3-Isomer aufgetrennt werden.

Die nachträgliche Reduktion der Nitrogruppe wird vorzugsweise in einem Lösungsmittel wie Wasser, Wasser/Ethanol, Methanol, Eisessig, Essigsäurethylester oder Dimethylformamid zweckmäßigerweise mit Wasserstoff in Gegenwart eines Hydrierungskatalysators wie Raney-Nickel, Platin oder Palladium/Kohle, mit Metallen wie Eisen, Zinn oder Zink in Gegenwart einer Säure, mit Salzen wie Eisen(II)sulfat, Zinn(II)chlorid, Natriumsulfid, Natriumhydrogensulfit oder Natriumdithionit, oder mit Hydrazin in Gegenwart von Raney-Nickel bei Temperaturen zwischen 0 und 80°C, vorzugsweise jedoch bei Temperaturen zwischen 20 und 40°C, durchgeführt.

Die nachträgliche Alkylierung wird vorzugsweise in einem Lösungsmittel oder Lösungsmittelgemisch wie Methylformamid, Dimethylformamid, Dimethylsulfoxid, Benzol, Chlorbenzol, Tetrahydrofuran, Benzol/Tetrahydrofuran oder Dioxan in Gegenwart von einem Alkylierungsmittel wie Methyljodid, Methylbromid, Ethylbromid, Dimethylsulfat, Benzylchlorid oder Diazomethan gegebenenfalls vorzugsweise in Gegenwart eines säurebindenden Mittels, z.B. eines Alkoholats wie Kalium-tert.butylat, eines Alkalihydroxids wie Natrium- oder Kaliumhydroxid, eines Alkalicarbonats wie Kaliumcarbonat, eines Alkaliamids wie Natriumamid oder eines Alkalihydrids wie Natriumhydrid zweckmäßigerweise bei Temperaturen zwischen 0 und 150°C, vorzugsweise bei Temperaturen zwischen 0 und 50°C, durchgeführt.

Die nachträgliche reduktive Aminierung einer Aminogruppe wird in einem geeigneten Lösungsmittel wie Methanol, Ethanol, Tetrahydrofuran, Dioxan oder Acetonitril in Gegenwart eines geeigneten Reduktionsmittels wie einem geeigneten komplexen Metallhydrid, vorzugsweise jedoch in Gegenwart von Natriumcyanborhydrid bei einem pH-Wert von 5 bis 7, bei Temperaturen zwischen 0 und 50°C, vorzugsweise jedoch bei Raumtemperatur, durchgeführt.

Die nachträgliche Abspaltung eines Benzylrestes erfolgt vorzugsweise hydrogenolytisch, z.B. mit Wasserstoff in Gegenwart eines Katalysators wie Palladium/Kohle in einem Lösungsmittel wie Methanol, Ethanol, Essigsäureethylester oder Eisessig gegebenenfalls unter Zusatz einer Säure wie Salzsäure bei Temperaturen zwischen 0 und 50°C, vorzugsweise jedoch bei Raumtemperatur, und einem Wasserstoffdruck von 1 bis 7 bar, vorzugsweise jedoch von 3 bis 5 bar.

Die nachträgliche Amidierung wird zweckmäßigerweise in einem Lösungsmittel wie Methylenchlorid, Chloroform, Tetrachlorkohlenstoff, Ether, Tetrahydrofuran, Dioxan, Benzol, Toluol, Acetonitril oder Dimethylformamid, besonders vorteilhaft jedoch in einem Überschuß des eingesetzten Amins, z.B. in Methanol, Ethanol, n-Propanol, Isopropanol, Ammoniak, Methylamin, Ethylamin, Dimethylamin oder Diethylamin, gegebenenfalls in Gegenwart eines die Säure aktivierenden Mittels oder eines wasserentziehenden Mittels, z.B. in Gegenwart von Chlorameisensäureäthylester, Thionylchlorid, Phosphortrichlorid, Phosphorpentoxid, N,N′-Dicyclohexylcarbodiimid, N,N′-Dicyclohexylcarbodiimid/N-Hydroxy-succinimid, N,N′-Carbonyldiimidazol oder N,N′-Thionyldiimidazol oder Triphenylphosphin/Tetrachlorkohlenstoff, oder eines die Aminogruppe aktivierenden Mittels, z.B. Phosphortrichlorid, und gegebenenfalls in Gegenwart einer anorganischen Base wie Natriumcarbonat oder einer tertiären organischen Base wie Triethylamin oder Pyridin, welche gleichzeitig als Lösungsmittel dienen können, bei Temperaturen zwischen -25°C und 250°C, vorzugsweise jedoch bei Temperaturen zwischen -10°C und der Siedetemperatur des verwendeten Lösungsmittels, durchgeführt.

Die nachträgliche Reduktion der Carboxygruppe wird in einem geeigneten Lösungsmittel wie Methanol, Äthanol, Äther, Tetrahydrofuran, Dioxan oder Eisessig in Gegenwart von katalytisch angeregtem Wasserstoff, z.B. von Wasserstoff in Gegenwart von Platin oder Palladium/Kohle, und gegebenenfalls in Gegenwart einer Säure wie Salzsäure oder Perchlorsäure oder in Gegenwart eines Metallhydrids wie Natriumborhydrid, Lithium-borhydrid oder Lithiumaluminiumhydrid bei Temperaturen zwischen 0 und 100°C, vorzugsweise bei Temperaturen zwischen 20 und 80°C, durchgeführt.

Die nachträgliche Hydrolye erfolgt vorzugsweise hydrolytisch in einem wässrigen Lösungsmittel, z.B. in Wasser, Isopropanol/Wasser, Tetrahydrofuran/Wasser oder Dioxan/Wasser, in Gegenwart einer Säure wie Salzsäure oder Schwefelsäure oder in Gegenwart einer Alkalibase wie Natriumhydroxid oder Kaliumhydroxid bei Temperaturen zwischen 0 und 100°C, vorzugsweise bei der Siedetemperatur des Reaktionsgemisches.

Die nachträgliche Etherspaltung wird in Gegenwart einer Säure wie Chlorwasserstoff, Bromwasserstoff, Schwefelsäure, Bortrichlorid, Bortribromid oder Aluminiumchlorid in einem geeigneten Lösungsmittel wie Methanol, Ethanol, Wasser/Isopropanol, Methylenchlorid, Chloroform oder Tetrachlorkohlenstoff bei Temperaturen zwischen -30°C und der Siedetemperatur des Reaktionsgemisches durchgeführt.

Die nachträgliche Reduktion wird vorzugsweise in einem Lösungsmittel wie Wasser, Wasser/Äthanol, Methanol, Eisessig, Essigsäureäthylester oder Dimethylformamid zweckmäßigerweise mit Wasserstoff in Gegenwart eines Hydrierungskatalysators wie Raney-Nickel, Platin oder Palladium/Kohle, oder mit Hydrazin in Gegenwart von Raney-Nickel bei Temperaturen zwischen 0 und 50°C, vorzugsweise jedoch bei Raumtemperatur durchgeführt.

Die anschließende Reduktion einer Phthaliminogruppe wird vorzugsweise in einem Lösungsmittel wie Eisessig mit nascierendem Wasserstoff, z.B. mit Zink/Eisessig, bei Temperaturen zwischen 80 und 120°C, vorzugsweise bei der Siedetemperatur des Reaktionsgemisches, durchgeführt.

Die nachträgliche Isomerentrennung erfolgt vorzugsweise chromatographisch unter Verwendung eines Trägers wie Kieselgel oder Aluminiumoxid.

Desweiteren können die erhaltenen Verbindungen der Formel I in ihre Säureadditionssalze, insbesondere für die pharmazeutische Anwendung in ihre physiologisch verträglichen Salze mit anorganischen oder organischen Säuren, übergeführt werden. Als Säuren kommen hierfür beispielsweise Salzsäure, Bromwasserstoffsäure, Schwefelsäure, Phosphorsäure, Fumarsäure, Bernsteinsäure, Milchsäure, Zitronensäure, Weinsäure oder Maleinsäure in Betracht.

Außerdem lassen sich die so erhaltenen neuen Verbindungen der Formel I, falls diese eine Carboxygruppe enthalten, gewünschtenfalls anschließend in ihre Salze mit anorganischen oder organischen Basen, insbesondere für die pharmazeutische Anwendung in ihre physiologisch verträglichen Salze, überführen. Als Basen kommen hierbei beispielsweise Natriumhydroxid, Kaliumhydroxid, Cyclohexylamin, Äthanolamin, Diäthanolamin und Triäthanolamin in Betracht.

Die als Ausgangsstoffe verwendeten Verbindungen der Formeln II bis XXI sind teilweise literaturbekannt oder man erhält diese nach literaturbekannten Verfahren.

So erhält man beispielsweise eine Verbindung der Formel II oder III durch Alkylierung einer entsprechenden o-Amino-nitroverbindung und anschließende Reduktion der Nitrogruppe.

Eine als Ausgangsstoff verwendete Verbindung der Formeln V, VI, VII, IX, X, XI, XIII, XV, XVII, XIX oder XXI erhält man durch Alkylierung eines entsprechenden o-Phenylendiamins oder einer entsprechenden o-Amino-nitroverbindung und anschließender Reduktion der Nitrogruppe und anschließender Cyclisierung einer so erhaltenen o-Diaminophenylverbindung oder durch NH-Alkylierung eines entsprechenden 1H-Benzimidazols, wobei das so erhaltene Isomerengemisch anschließend mittels üblicher Methoden, z.B. mittels Chromatographie, aufgetrennt werden kann.

Die neuen Verbindungen der Formel I, in denen $R_4$ eine Brönsted-Säure enthaltende Gruppe oder einen Rest, der in vivo in eine Brönsted-Säure enthaltende Gruppe übergeführt werden kann, darstellt, und deren physiologisch verträgliche Salze weisen wertvolle pharmakologische Eigenschaften auf. Sie stellen insbesondere Angiotensin-II-Antagonisten dar.

Beispielsweise wurden die Verbindungen

A = 4'-[(2-n-Butyl-benzimidazol-1-yl)-methyl]biphenyl-2-carbonsäure,
B = 4'-[(2-Methoxymethyl-benzimidazol-1-yl)-methyl]biphenyl-2-carbonsäure,
C = 4'-[(2-Methyl-5- und 6-butanoylamino-benzimidazol-1-yl)-methyl]biphenyl-2-carbonsäure,
D = 4'-[(2-n-Propyl-benzimidazol-1-yl)-methyl]biphenyl-2-carbonsäure,
E = 4'-[(2-Ethyl-benzimidazol-1-yl)-methyl]biphenyl-2-carbonsäure,
F = 4'-[(2-Methylthiomethyl-benzimidazol-1-yl)-methyl]biphenyl-2-carbonsäure-semitrifluoracetat,
G = 4'-[(2-Ethylthio-benzimidazol-1-yl)-methyl]biphenyl-2-carbonsäure,
H = 4'-[(2-n-Butyl-5-methoxy-benzimidazol-1-yl)-methyl]biphenyl-2-carbonsäure,
I = 4'-[(2-n-Butyl-6-methoxy-benzimidazol-1-yl)-methyl]biphenyl-2-carbonsäure,
J = 4'-[(2-n-Butyl-5,6-dimethyl-benzimidazol-1-yl)-methyl]biphenyl-2-carbonsäure,
K = 4'-[(2-n-Propyl-naphtho[2,3-d]imidazol-1-yl)-methyl]biphenyl-2-carbonsäure,
L = 4'-[(2-n-Butyl-5,6-dimethoxy-benzimidazol-1-yl)-methyl]biphenyl-2-carbonsäure,
M = 4'-[(2-(1-Butin-4-yl)-benzimidazol-1-yl]-methyl]biphenyl-2-carbonsäure,
N = 4'-[(2-n-Butyl-5-acetyl-benzimidazol-1-yl)-methyl]biphenyl-2-carbonsäure,
O = 4'-[(2-Cyclohexylmethyl-5,6-dihydroxy-benzimidazol-1-yl)methyl]biphenyl-2-carbonsäure,
P = 4'-[(6-Aminothiocarbonylamino-2-n-butyl-benzimidazol-1-yl)-methyl]biphenyl-2-carbonsäure-hemihydrat,
Q = 4'-[(6-Aminocarbonylamino-2-n-butyl-benzimidazol-1-yl)-methyl]biphenyl-2-carbonsäure-trifluoracetat und
R = 4'-[(2-n-Butyl-5-(n-butylaminocarbonylamino)-benzimidazol-1-yl)-methyl]biphenyl-2-carbonsäure-semitrifluoracetat

auf ihre biologischen Wirkungen wie folgt untersucht:

a) Ratten (männlich, 180-220 g) werden mit Hexobarbital-Natrium (150 mg/kg i.p.) narkotisiert. Nach Eintreten der Narkose wird eine Trachealkanüle gelegt, die Tiere werden despinalisiert und dann sofort mit einer Atempumpe künstlich beatmet. Der arterielle Blutdruck wird über eine Kanüle in der Arteria Carotis mittels eines Bell & Howell Druckaufnehmers registriert. Substanzen werden über eine Kanüle in die Vena Jugularis appliziert.

Test-Substanzen werden mit drei Dosierungen (10, 20 und 30 mg/kg i.v.) appliziert, wobei pro Tier eine Substanzdosis getestet wird. Drei Minuten nach der intravenösen Applikation der Testsubstanz wird Angiotensin-II in steigender Dosierung intravenös appliziert und so eine kumulative Dosis-Wirkungsbeziehung für Angiotensin-II in Gegenwart der Testsubstanzen erreicht. Der Meßparameter ist die Steigerung des arteriellen Blutdruckes.

Diese Dosis-Wirkungskurven werden mit Standardkurven für Angiotensin-II ohne Testsubstanzen verglichen. Mittels eines Computerprogramms werden die Rechtsverschiebungen der Dosis-Wirkungskurven von Angiotensin-II durch Testsubstanzen ermittelt und entsprechende $pA_2$-Werte für die Testsubstanzen berechnet.

Die nachfolgende Tabelle zeigt die durchschnittlichen $pA_2$-Werte der untersuchten Substanzen:

| Substanzen | $pA_2$-Werte |
|---|---|
| A | 4,40 |
| B | 5,10 |
| C | 5,30 |
| D | 5,49 |
| E | 5,25 |
| F | 5,25 |
| G | 5,10 |
| H | 5,00 |
| I | 5,17 |
| J | 5,41 |
| K | 4,90 |
| L | 5,27 |
| M | 4,97 |
| N | 4,88 |
| O | - |
| P | 5,22 |
| Q | 5,43 |
| R | 5,47 |

b) Die Hemmwirkung der neuen Verbindungen auf die Bindung von Angiotensin II an Rindernebennieren-Rezeptorpräparationen wurde analog der Methode von Glossmann et al. (J. Biol. Chem. 249, 825-834 (1974)) geprüft. Der Inkubationsansatz enthielt Aliquots einer Membranpräparation aus Nebennieren-Cortex des Rindes in Tris-Puffer, steigende Konzentrationen des möglichen Antagonisten sowie 50 pM $^{125}I$-Angiotensin II. Nach 45 Minuten wurde die Inkubation durch rasche Filtration durch Glasfiber-Filter beendet. Die Filter-gebundene Radioaktivität wurde in einem $\gamma$-Counter bei einer Zählausbeute von 80 % bestimmt.

Die nachfolgende Tabelle gibt die Inhibitorkonzentration der Antagonisten wieder, die 50 % Hemmung der spezifischen $^{125}$I-Angiotensin II-Bindung bewirkten:

| Substanzen | $IC_{50}$ [µM/l] |
|---|---|
| A | 1,2 |
| B | 5,9 |
| C | 1,9 |
| D | 0,6 |
| E | 1,0 |
| F | 1,5 |
| G | 2,1 |
| H | 0,9 |
| I | 2,4 |
| J | 9,5 |
| K | 2,3 |
| L | 4,0 |
| M | 6,4 |
| N | 2,4 |
| O | 29,0 |
| P | 0,8 |
| Q | 0,9 |
| R | 1,0 |

Desweiteren konnten bei der Applikation der vorstehenden Verbindungen bis zu einer Dosis von 30 mg/kg i.v. keine toxischen Nebenwirkungen, z.B. keine negativ inotrope Wirkung und keine Herzrhythmusstörungen, beobachtet werden. Die Verbindungen sind demnach gut verträglich.

Aufgrund ihrer pharmakologischen Eigenschaften eignen sich die neuen Verbindungen der Formel I, in denen $R_4$ eine Brönsted-Säure enthaltende Gruppe oder einen Rest, der in vivo in eine Brönsted-Säure enthaltende Gruppe übergeführt werden kann, darstellt, und deren physiologisch verträgliche Salze zur Behandlung der Hypertonie und Herzinsuffizienz, ferner zur Behandlung ischämischer peripherer Durchblutungsstörungen, der myokardialen Ischämie (Angina), zur Prävention der Herzinsuffizienzprogression nach Myokard-Infarkt, zur Behandlung der diabetischen Nephropathie, des Glaukoms, von gastrointestinalen Erkrankungen und Blasenerkrankungen.

Die zur Erzielung einer entsprechenden Wirkung erforderliche Dosierung beträgt zweckmäßigerweise bei intravenöser Gabe 20 bis 100 mg, vorzugsweise 30 bis 70 mg, und bei oraler Gabe 50 bis 200 mg, vorzugsweise 75 bis 150 mg, jeweils 1 bis 3 x täglich. Hierzu lassen sich die erfindungsgemäß hergestellten Verbindungen der Formel I, in denen $R_4$ eine Brönsted-Säure enthaltende Gruppe oder einen Rest, der in vivo in eine Brönsted-Säure enthaltende Gruppe übergeführt werden kann, darstellt, gegebenenfalls in Kombination mit anderen Wirksubstanzen, zusammen mit einem oder mehreren inerten üblichen Trägerstoffen und/oder Verdünnungsmitteln, z.B. mit Maisstärke, Milchzucker, Rohrzucker, mikrokristalliner Zellulose, Magnesiumstearat, Polyvinylpyrrolidon, Zitronensäure, Weinsäure, Wasser, Wasser/Äthanol, Wasser/Glycerin, Wasser/Sorbit, Wasser/Polyäthylenglykol, Propylenglykol, Cetylstearylalkohol, Carboxymethylcellulose oder fetthaltigen Substanzen wie Hartfett oder deren geeigneten Gemischen, in übliche galenische Zubereitungen wie Tabletten, Dragées, Kapseln, Pulver, Suspensionen oder Zäpfchen einarbeiten.

Die nachfolgenden Beispiele sollen die Erfindung näher erläutern:

Beispiel A

4′-[N-(5-Benzylamino-2-nitro-phenyl)-pentanoylaminomethyl]biphenyl-2-carbonsäure-tert.butylester

3,9 g (7,5 mMol) 4′-[N-(5-Chlor-2-nitro-phenyl)-pentanoylaminomethyl]biphenyl-2-carbonsäure-tert.butylester werden zusammen mit 3,9 ml Benzylamin 3 Stunden in einem 150-160°C heißem Ölbad gerührt. Nach Abkühlen des Reaktionsgemisches löst man den Rückstand in ca. 25 ml Methylenchlorid und reinigt die Substanz über eine Kieselgel-Säule (Korngröße: 0,063-0,2 mm, Elutionsmittel: Cyclohexan/5-10 % Essigester). Die entsprechenden Fraktionen werden am Rotationsverdampfer eingeengt.
Ausbeute: 2,9 g (65,5 % der Theorie),
Öl, $R_f$-Wert: 0,55 (Kieselgel: Cyclohexan/Essigester = 2:1)
Analog werden folgende Verbindungen erhalten:
4′-[N-(5-(N-Benzyl-methylamino)-2-nitro-phenyl)-pentanoylaminomethyl]biphenyl-2-carbonsäure-tert.butylester
Öl, $R_f$-Wert: 0,40 (Kieselgel: Cyclohexan/Essigester = 4:1)
4′-[N-(5-Dimethylamino-2-nitro-phenyl)-pentanoylaminomethyl]biphenyl-2-carbonsäure-tert.butylester
Öl, $R_f$-Wert: 0,35 (Kieselgel: Hexan/Essigester = 4:1)

Beispiel 1

4′-[(Benzimidazol-1-yl)-methyl]biphenyl-2-carbonsäure-tert.-butylester

0,95 g (8 mMol) Benzimidazol werden in 50 ml Dimethylsulfoxid gelöst und mit 1,0 g (9 mMol) Kalium-tert.butylat versetzt. Zur Salzbildung rührt man nun 30 Minuten bei Raumtemperatur nach und setzt dann 2,8 g (8 mMol) 4′-Brommethyl-biphenyl-2-carbonsäure-tert.butylester hinzu. Nach 2 Stunden Rühren bei Raumtemperatur ist die Reaktion beendet. Man verdünnt mit Wasser auf 500 ml und extrahiert 3 mal mit ca. 100 ml Essigester. Die vereinigten organischen Phasen werden mit Magnesiumsulfat getrocknet und zur Trockene eingeengt. Der ölige Rückstand wird über eine Kieselgel-Säule (Korngröße: 0,063-0,2 mm) gereinigt, wobei als Eluationsmittel Methylenchlorid mit 1 % Ethanol verwendet wird. Die einheitlichen Fraktionen werden zur Trockene eingeengt. Der Rückstand ist ein Öl.
Ausbeute: 2,8 g (90,8 % der Theorie),
$R_f$-Wert: 0,35 (Kieselgel: Methylenchlorid/Ethanol = 19:1)

| | | | | | | |
|---|---|---|---|---|---|---|
| Ber.: | C | 78,10 | H | 6,29 | N | 7,29 |
| Gef.: | | 78,18 | | 6,34 | | 7,19 |

Analog werden folgende Verbindungen erhalten:
4′-[(2-Hydroxy-benzimidazol-1-yl)-methyl]biphenyl-2-carbonsäure-tert.butylester
Öl, $R_f$-Wert: 0,15 (Kieselgel: Methylenchlorid/Ethanol = 49:1)
4′-[(2-n-Butyl-7-(2-diethylamino-ethoxy)-4-methyl-benzimidazol-1-yl)-methyl]biphenyl-2-carbonsäure-tert.butylester
Öl, $R_f$-Wert: 0,55 (Kieselgel: Methylenchlorid/Ethanol = 9:1)
4′-[(2-Ethylthio-benzimidazol-1-yl)-methyl]biphenyl-2-carbonsäure-tert.butylester
Öl, $R_f$-Wert: 0,45 (Kieselgel: Methylenchlorid/Ethanol = 49:1)
4′-[(2-n-Propylthiomethyl-benzimidazol-1-yl)-methyl]biphenyl-2-carbonsäure-tert.butylester
Öl, $R_f$-Wert: 0,55 (Kieselgel: Methylenchlorid/Ethanol = 19:1)
4′-[(2-Methyl-benzimidazol-1-yl)-methyl]biphenyl-2-carbonsäure-tert.butylester
Öl, $R_f$-Wert: 0,60 (Kieselgel: Methylenchlorid/Ethanol = 19:1)
4′-[(2-Methylmercapto-benzimidazol-1-yl)-methyl]biphenyl-2-carbonsäure-tert.butylester
Öl, $R_f$-Wert: 0,55 (Kieselgel: Methylenchlorid/Ethanol = 19:1)
4′-[(2-Methyl-5- und 6-nitro-benzimidazol-1-yl)-methyl]biphenyl-2-carbonsäure-tert.butylester
Öl, $R_f$-Wert: 0,50 (Kieseigel: Methylenchlorid/Ethanol = 19:1)
4′-[(2-Methyl-5- und 6-butanoylamino-benzimidazol-1-yl)-methyl]biphenyl-2-carbonsäure-tert.butylester
Öl, $R_f$-Wert: 0,60 (Kieselgel: Methylenchlorid/Ethanol = 9:1)
4′-[(2-(2-Methyl-propyl)-benzimidazol-1-yl)-methyl]biphenyl-2-carbonsäure-tert.butylester
Öl, $R_f$-Wert: 0,50 (Kieselgel: Methyl-ethylketon/Xylol = 1:1)
4′-[(2-Methoxymethyl-benzimidazol-1-yl)-methyl]biphenyl-2-carbonsäure-tert.butylester
Öl, $R_f$-Wert: 0,50 (Kieselgel: Methyl-ethylketon/Xylol = 1:1)
4′-[(2-(Pyridyl-(2))-benzimidazol-1-yl)-methyl]biphenyl-2-carbonsäure-tert.butylester

Öl, $R_f$-Wert: 0,40 (Kieselgel: Methylenchlorid/Ethanol = 19:1)
4′-[(5- und 6-Methyl-benzimidazol-1-yl)-methyl]biphenyl-2-carbonsäure-tert.butylester
Öl, $R_f$-Wert: 0,25 (Kieselgel: Methylenchlorid/Ethanol = 19:1)
4′-[(2-Phenyl-benzimidazol-1-yl)-methyl]biphenyl-2-carbonsäure-tert.butylester
Öl, $R_f$-Wert: 0,45 (Kieselgel: Methylenchlorid/Ethanol = 19:1)
4′-[(2-(Thiazol-4-yl)-benzimidazol-1-yl)-methyl]biphenyl-2-carbonsäure-tert.butylester
Öl, $R_f$-Wert: 0,30 (Kieselgel: Methylenchlorid/Ethanol = 49:1)
4′-[(2-(3,5-Dimethyl-pyrazol-1-yl)-benzimidazol-1-yl)-methyl]biphenyl-2-carbonsäure-tert.butylester
Öl, $R_f$-Wert: 0,35 (Kieselgel: Methylenchlorid/Ethanol = 19:1)
4′-[(2-(Furyl-(2))-benzimidazol-1-yl)-methyl]biphenyl-2-carbonsäure-tert.butylester
Öl, $R_f$-Wert: 0,40 (Kieselgel: Methylenchlorid/Ethanol = 19:1)
4′-[(2-Aminocarbonylamino-benzimidazol-1-yl)-methyl]biphenyl-2-carbonsäure-tert.butylester
Öl, $R_f$-Wert: 0,50 (Kieselgel: Methylenchlorid/Ethanol = 19:1)
4′-[(2-Isopropyl-benzimidazol-1-yl)-methyl]biphenyl-2-carbonsäure-tert.butylester
Öl, $R_f$-Wert: 0,50 (Kieselgel: Methyl-ethylketon/Xylol = 1:1)
4′-[(2-Hydroxymethyl-benzimidazol-1-yl)-methyl]biphenyl-2-carbonsäure-tert.butylester
Öl, $R_f$-Wert: 0,35 (Kieselgel: Methylenchlorid/Ethanol = 19:1)
4′-[(2-(3-Hydroxypropyl)-benzimidazol-1-yl)-methyl]biphenyl-2-carbonsäure-tert.butylester
Öl, $R_f$-Wert: 0,40 (Kieselgel: Methylenchlorid/Ethanol = 19:1)
4′-[(2-Methyl-5- und 6-(N-(methoxy-acetyl)-n-butylamino)-benzimidazol-1-yl)-methyl]biphenyl-2-carbonsäure-tert.-butylester
Öl, $R_f$-Wert: 0,20 (Kieselgel: Methylenchlorid/Ethanol = 19:1)
4′-[(2-(1-Methylpropyl)-benzimidazol-1-yl)-methyl]biphenyl-2-carbonsäure-tert.butylester
Öl, $R_f$-Wert: 0,80 (Kieselgel: Methyl-ethylketon/Xylol = 1:1)
4′-[(2-(2-Methylbutyl)-benzimidazol-1-yl)-methyl]biphenyl-2-carbonsäure-tert.butylester
Öl, $R_f$-Wert: 0,60 (Kieselgel: Methyl-ethylketon/Xylol = 1:1)
4′-[(2-Methyl-5- und 6-(N-(2-methoxy-ethyl)-n-butylamino)-benzimidazol-1-yl)-methyl]biphenyl-2-carbonsäure-tert.butylester
Öl, $R_f$-Wert: 0,25 (Kieselgel: Methylenchlorid/Ethanol = 19:1)
4′-[(2-n-Pentyl-benzimidazol-1-yl)-methyl]biphenyl-2-carbonsäure-tert.butylester
Öl, $R_f$-Wert: 0,50 (Kieselgel: Methyl-ethylketon/Xylol = 1:1)
4′-[(2-n-Butyl-7-methoxy-4-methyl-benzimidazol-1-yl)-methyl]biphenyl-2-carbonsäure-tert.butylester
Öl, $R_f$-Wert: 0,50 (Kieselgel: Methylenchlorid/Ethanol = 19:1)
4′-[(2-n-Propyl-benzimidazol-1-yl)-methyl]biphenyl-2-carbonsäure-tert.butylester
Schmelzpunkt: 140-141°C
4′-[(2-Ethyl-benzimidazol-1-yl)-methyl]biphenyl-2-carbonsäure-tert.butylester
Schmelzpunkt: 129-130°C
4′-[(2-Ethylthiomethyl-benzimidazol-1-yl)-methyl]biphenyl-2-carbonsäure-tert.butylester
Öl, $R_f$-Wert: 0,50 (Kieselgel: Methylenchlorid/Ethanol = 19:1)
4′-[(2-Methylthiomethyl-benzimidazol-1-yl)-methyl]biphenyl-2-carbonsäure-tert.butylester
Öl, $R_f$-Wert: 0,45 (Kieselgel: Methylenchlorid/Ethanol = 19:1)
4′-[(2-Chlor-benzimidazol-1-yl)-methyl]biphenyl-2-carbonsäure-tert.butylester
Öl, $R_f$-Wert: 0,55 (Kieselgel: Methylenchlorid/Ethanol = 19:1)
4′-(2-n-Butylthio-benzimidazol-1-yl)-methyl]biphenyl-2-carbonsäure-tert.butylester
Öl, $R_f$-Wert: 0,55 (Kieselgel: Methylenchlorid/Ethanol = 49:1)
4′-[(2-(4-Methoxyphenyl)-benzimidazol-1-yl)-methyl]biphenyl-2-carbonsäure-tert.butylester
Öl, $R_f$-Wert: 0,80 (Kieselgel: Methylethylketon/Xylol = 1:1)
4′-[(2-n-Propylthio-benzimidazol-1-yl)-methyl]biphenyl-2-carbonsäure-tert.butylester
Öl, $R_f$-Wert: 0,50 (Kieselgel: Methylenchlorid/Ethanol = 49:1)
4′-[(2-n-Butylamino-benzimidazol-1-yl)-methyl]biphenyl-2-carbonsäure-tert.butylester
Öl, $R_f$-Wert: 0,45 (Kieselgel: Methylenchlorid/Ethanol = 19:1)
4′-[(2-(4-Methoxy-phenyl)-5- und 6-chlor-benzimidazol-1-yl)-methyl]biphenyl-2-carbonsäure-tert.butylester
Öl, $R_f$-Wert: 0,50 (Kieselgel: Methyl-ethylketon/Xylol = 1:1)
4′-[(2-n-Butyl-5- und 6-acetamino-benzimidazol-1-yl)-methyl]biphenyl-2-carbonsäure-tert.butylester
Öl, $R_f$-Wert: 0,60 (Kieselgel: Essigester/Ethanol/Ammoniak = 90:10:1)
4′-[(2-n-Butyl-5- und 6-butanoylamino-benzimidazol-1-yl)-methyl]biphenyl-2-carbonsäure-tert.butylester
Öl, $R_f$-Wert: 0,80 (Kieselgel: Essigester/Ethanol/Ammoniak = 90:10:1)
4′-[(2-n-Butyl-5- und 6-methoxy-benzimidazol-1-yl)-methyl]biphenyl-2-carbonsäure-tert.butylester
Öl, $R_f$-Wert: 0,70 (Kieselgel: Methyl-ethylketon/Xylol = 1:1)

4'-[(7-n-Butoxy-2-n-butyl-4-methyl-benzimidazol-1-yl)-methyl]-biphenyl-2-carbonsäure-tert.butylester
Öl, $R_f$-Wert: 0,55 (Kieselgel: Methylenchlorid/Ethanol = 19:1)
4'-[(2-(4-Hydroxyphenyl)-benzimidazol-1-yl)-methyl]biphenyl-2-carbonsäure-tert.butylester
Schmelzpunkt: 258-260°C
4'-[(2-(4-n-Butoxyphenyl)-benzimidazol-1-yl)-methyl]biphenyl-2-carbonsäure-tert.butylester
Öl, $R_f$-Wert: 0,70 (Kieselgel: Methyl-ethylketon/Xylol = 1:1)
4'-[(2-n-Butyl-4-nitro-benzimidazol-1-yl)-methyl]biphenyl-2-carbonsäure-tert.butylester
Öl, $R_f$-Wert: 0,45 (Kieselgel: Methylenchlorid/Ethanol = 19:1)
4'-[(2-(3-Pyridyl)-benzimidazol-1-yl)-methyl]biphenyl-2-carbonsäure-tert.butylester
Öl, $R_f$-Wert: 0,40 (Kieselgel: Methyl-ethylketon/Xylol = 5:2)
4'-[(2-(4-Benzyloxyphenyl)-benzimidazol-1-yl)-methyl]biphenyl-2-carbonsäure-tert.butylester
Öl, $R_f$-Wert: 0,75 (Kieselgel: Methyl-ethylketon/Xylol = 1:1)
4'-[(2-n-Butyl-5,6-dimethyl-benzimidazol-1-yl)-methyl]biphenyl-2-carbonsäure-tert.butylester
Öl, $R_f$-Wert: 0,50 (Kieselgel: Methylenchlorid/Ethanol = 19:1)
4'-[(2-(2,2-Dimethylpropyl)-5,6-dimethyl-benzimidazol-1-yl)-methyl]biphenyl-2-carbonsäure-tert.butylester
Öl, $R_f$-Wert: 0,48 (Kieselgel: Methylenchlorid/Ethanol = 19:1)
4'-[(2-Benzyl-5,6-dimethyl-benzimidazol-1-yl)-methyl]biphenyl-2-carbonsäure-tert.butylester
Öl, $R_f$-Wert: 0,52 (Kieselgel: Methylenchlorid/Ethanol = 19:1)
4'-[(2-(2-Methylbutyl)-5,6-dimethyl-benzimidazol-1-yl)-methyl]biphenyl-2-carbonsäure-tert.butylester
Öl, $R_f$-Wert: 0,50 (Kieselgel: Methylenchlorid/Ethanol = 19:1)
4'-[(2--Cyclohexylmethyl)-5,6-dimethyl-benzimidazol-1-yl)-methyl]biphenyl-2-carbonsäure-tert.butylester
Öl, $R_f$-Wert: 0,52 (Kieselgel: Methylenchlorid/Ethanol = 19:1)
4'-[(2-Cyclohexylmethyl-5,6-dichlor-benzimidazol-1-yl)-methyl]biphenyl-2-carbonsäure-tert.butylester
Öl, $R_f$-Wert: 0,46 (Kieselgel: Methylenchlorid/Ethanol = 19:1)
4'-[(2-(2-Methylbutyl)-naphtho[2,3-d]imidazol-1-yl)-methyl]biphenyl-2-carbonsäure-tert.butylester
Öl, $R_f$-Wert: 0,57 (Kieselgel: Methylenchlorid/Ethanol = 19:1)
4'-[(2-n-Propyl-naphtho[2,3-d]imidazol-1-yl)-methyl]biphenyl-2-carbonsäure-tert.butylester
Öl, $R_f$-Wert: 0,57 (Kieselgel: Methylenchlorid/Ethanol = 19:1)
4'-[(2-n-Butyl-5,6-dichlor-benzimidazol-1-yl)-methyl]biphenyl-2-carbonsäure-tert.butylester
Öl, $R_f$-Wert: 0,45 (Kieselgel: Methylenchlorid/Ethanol = 19:1)
4'-[(2-Cyclohexylmethyl-5,6-dimethoxy-benzimidazol-1-yl)-methyl]biphenyl-2-carbonsäure-tert.butylester
Öl, $R_f$Wert: 0,47 (Kieselgel: Methylenchlorid/Ethanol = 19:1)
4'-[(2-n-Butyl-5,6-dimethoxy-benzimidazol-1-yl)-methyl]biphenyl-2-carbonsäure-tert.butylester
Öl, $R_f$-Wert: 0,46 (Kieselgel: Methylenchlorid/Ethanol = 19:1)
4'-[(2-Cyclopentylmethyl-5,6-dimethoxy-benzimidazol-1-yl)-methyl]biphenyl-2-carbonsäure-tert.butylester
Öl, $R_f$-Wert: 0,47 (Kieselgel: Methylenchlorid/Ethanol = 19:1)
4'-[(2-(3-Methylbutyl)-5,6-dimethoxy-benzimidazol-1-yl)-methyl]biphenyl-2-carbonsäure-tert.butylester
Öl, $R_f$-Wert: 0,47 (Kieselgel: Methylenchlorid/Ethanol = 19:1)
4'-[(2-Cyclohexyl-5,6-dimethyl-benzimidazol-1-yl)-methyl]biphenyl-2-carbonsäure-tert.butylester
Öl, $R_f$-Wert: 0,50 (Kieselgel: Methylenchlorid/Ethanol = 19:1)
4'-[(2-(1-Butin-4-yl)-benzimidazol-1-yl)-methyl]biphenyl-2-carbonsäure-tert.butylester
Öl, $R_f$-Wert: 0,49 (Kieselgel: Methylenchlorid/Ethanol = 19:1)
4'-[(2-n-Butyl-6-ethoxycarbonyl-benzimidazol-1-yl)-methyl]biphenyl-2-carbonsäure-tert.butylester
Öl, $R_f$-Wert: 0,60 (Kieselgel: Petrolether/Essigester = 1:1 + 1 % Eisessig)
4'-[(2-Cyclopentyl-5,6-dimethyl-benzimidazol-1-yl)-methyl]biphenyl-2-carbonsäure-tert.butylester
Öl, $R_f$-Wert: 0,50 (Kieselgel: Methylenchlorid/Ethanol = 19:1)
4'-[(2-n-Butyl-5- und 6-fluor-benzimidazol-1-yl)-methyl]biphenyl-2-carbonsäure-tert.butylester
Öl, $R_f$-Wert: 0,25 (Kieselgel: Methylenchlorid/Ethanol = 50:1)
4'-[(2-n-Butyl-5- und 6-benzoyl-benzimidazol-1-yl)-methyl]biphenyl-2-carbonsäure-tert.butylester
Öl, $R_f$-Wert: 0,28 (Kieselgel: Methylenchlorid/Ethanol = 50:1)
4'-[(2-n-Butyl-5- und 6-trifluoromethyl-benzimidazol-1-yl)-methyl]biphenyl-2-carbonsäure-tert.butylester
Öl, $R_f$-Wert: 0,33 (Kieselgel: Methylenchlorid/Ethanol = 50:1)
4'-[(2-n-Butyl-4-cyano-benzimidazol-1-yl)-methyl]biphenyl-2-carbonsäure-tert.butylester
Öl, $R_f$-Wert: 0,50 (Kieselgel: Methylenchlorid/Ethanol = 19:1)
4'-[(2-n-Butyl-5- und 6-n-butylaminocarbonyl-benzimidazol-1-yl)-methyl]biphenyl-2-carbonsäure-tert.butylester
Öl, $R_f$-Wert: 0,30 (Kieselgel: Methylenchlorid/Methanol/Essigsäure = 19:0,8:0,2)
4'-[(2-n-Butyl-6-carboxy-benzimidazol-1-yl)-methyl]biphenyl-2-carbonsäure-tert.butylester
Öl, $R_f$-Wert: 0,90 (Kieselgel: Methylenchlorid/Methanol/Essigsäure = 9:0,8:0,2)
4'-[(2-n-Butyl-5-carboxy-benzimidazol-1-yl)-methyl]biphenyl-2-carbonsäure-tert.butylester

Schmelzpunkt: 224-225°C
4′-[(2-n-Butyl-6-aminocarbonyl-benzimidazol-1-yl)-methyl]biphenyl-2-carbonsäure-tert.butylester
Schmelzpunkt: 159-160°C
4′-[(2-n-Butyl-5-aminocarbonyl-benzimidazol-1-yl)-methyl]biphenyl-2-carbonsäure-tert.butylester
Schmelzpunkt: 135-138°C (Zers.)
4′-[(2-n-Butyl-5- und 6-cyano-benzimidazol-1-yl)-methyl]biphenyl-2-carbonsäure-tert.butylester
Öl, $R_f$-Wert: 0,50 (Kieselgel: Methylenchlorid/Ethanol = 100:1)
4′-[(2-n-Butyl-6-(1H-tetrazol-5-yl)-benzimidazol-1-yl)-methyl]biphenyl-2-carbonsäure-tert.butylester
Schmelzpunkt: ab 115°C (Zers.)
4'-[(2-n-Butyl-5- und 6-(1H-tetrazol-5-yl)-benzimidazol-1-yl)-methyl]biphenyl-2-carbonsäure-tert.butylester
Schmelzpunkt: ab 92°C (Zers.)
4'-[(2-n-Butyl-6-(cis-hexahydro-phthalimino)-benzimidazol-1-yl)-methyl]-1-cyano-2-phenyl-naphthalin
4'-[(2-n-Butyl-6-(cis-hexahydro-phthalimino)-benzimidazol-1-yl)-methyl]-2-phenylnaphthalin-1-carbonsäure-tert.butylester

Beispiel 2

4′-[(2-n-Butyl-5- und 6-nitro-benzimidazol-1-yl)-methyl]biphenyl-2-carbonsäure-tert.butylester

a) 2-n-Butyl-6-nitro-benzimidazol
In eine Mischung aus 8,66 g (0,085 Mol) Valeriansäure und 120 ml Phosphoroxichlorid werden portionsweise bei Raumtemperatur 11,5 g (0,075 Mol) 4-Nitro-o-phenylendiamin eingetragen. Anschließend kocht man 3 Stunden am Rückfluß. Das Reaktionsgemisch wird in 1,5 kg Eiswasser zersetzt, mit konzentriertem Ammoniak alkalisch gestellt und dreimal mit 500 ml Essigester extrahiert. Die organische Phase wird abgetrennt, mit Magnesiumsulfat getrocknet und einrotiert. Den öligen Rückstand reinigt man über eine Kieselgel-Säule (Korngroße: 0,063-0,2 mm, Elutionsmittel: Methylenchlorid/0-2% Ethanol). Die einheitlichen Fraktionen werden zur Trockne eingeengt.
Ausbeute: 10,2 g (62,2 % der Theorie),
Schmelzpunkt: 139-141°C

| | | | | | | |
|---|---|---|---|---|---|---|
| Ber.: | C | 60,27 | H | 5,98 | N | 19,17 |
| Gef.: | | 60,30 | | 6,00 | | 19,42 |

b) 9,5 g (43 mMol) 2-n-Butyl-6-nitro-benzimidazol werden in 100 ml Dimethylsulfoxid gelöst und mit 5,3 g (47,6 mMol) Kalium-tert.butylat versetzt. Man rührt 30 Minuten nach und gibt 16,6 g (47,6 mMol) 4′-Brommethyl-biphenyl-2-carbonsäure-tert.butylester hinzu. Nach 2 Stunden wird das Reaktionsgemisch mit ca. 600 ml Wasser versetzt und 3 x mit ca. 200 ml Essigester extrahiert. Die organische Phase wird mit Magnesiumsulfat getrocknet und einrotiert. Das so erhaltene Rohprodukt wird über eine Kieselgel-Säule gereinigt (Korngrößе: 0,063-0,2 mm, Elutionsmittel: Methylenchlorid/0-1 % Ethanol). Die einheitlichen Fraktionen werden zur Trockne eingeengt.
Ausbeute: 19,9 g (95,2 % der Theorie),
Öl, $R_f$-Wert: 0,50 (Kieselgel: Methylenchlorid + 5 % Ethanol)

| | | | | | | |
|---|---|---|---|---|---|---|
| Ber.: | C | 71,73 | H | 6,43 | N | 8,65 |
| Gef.: | | 72,00 | | 6,66 | | 8,80 |

Analog werden folgende Verbindungen erhalten: 4′-[(2-n-Butyl-4- und 7-nitro-benzimidazol-1-yl)-methyl]biphenyl-2-carbonsäure-tert.butylester
Öl, $R_f$-Wert: 0,45 und 0,47 (Kieselgel: Methylenchlorid/Ethanol = 19:1)
4′-[(2-n-Butyl-5- und 6-methoxy-benzimidazol-1-yl)-methyl]biphenyl-2-carbonsäure-tert.butylester
Öl, $R_f$-Wert: 0,55 (Kieselgel: Methyl-ethylketon/Xylol = 1:1)
4′-[(2-n-Butyl-5- und 6-nitro-benzimidazol-1-yl)-methyl]biphenyl-2-carbonsäure-tert.butylester
Öl, $R_f$-Wert: 0,60 (Kieselgel: Methyl-ethylketon/Xylol = 1:1)
4′-[(2-n-Butyl-5- und 6-chlor-benzimidazol-1-yl)-methyl]biphenyl-2-carbonsäure-tert.butylester
Öl, $R_f$-Wert: 0,55 (Kieselgel: Methyl-ethylketon/Xylol = 1:1)
4′-[(2-n-Butyl-5- und 6-acetamino-benzimidazol-1-yl)-methyl]biphenyl-2-carbonsäure-tert.butylester

Öl, $R_f$-Wert: 0,20 (Kieselgel: Methyl-ethylketon/Xylol = 1:1)
4′-[(2-n-Butyl-5- und 6-butanoylamino-benzimidazol-1-yl)-methyl]biphenyl-2-carbonsäure-tert.butylester
Öl, $R_f$-Wert: 0,80 (Kieselgel: Essigester/Ethanol/Ammoniak = 90:10:1)

Beispiel 3

4′-[(5- und 6-Amino-2-n-butyl-benzimidazol-1-yl)-methyl]biphenyl-2-carbonsäure-tert.butylester

18,3 g (37,7 mMol) 4′-[(5- und 6-Nitro-2-n-butyl-benzimidazol-1-yl)-methyl]biphenyl-2-carbonsäure-tert.butylester werden in 200 ml Ethanol gelöst, mit 10 g Raney-Nickel versetzt und 3 Stunden bei 50°C mit 5 bar Wasserstoffdruck hydriert. Nach beendigter Wasserstoffaufnahme wird vom Katalysator abgesaugt und einrotiert.
Ausbeute: 17,1 g (100 % der Theorie),
Öl, $R_f$-Wert: 0,1 und 0,2 (Kieselgel: Methylenchlorid/Ethanol = 95:5)
Analog wird folgende Verbindung hergestellt:
4′-[(4- und 7-Amino-2-n-butyl-benzimidazol-1-yl)-methyl]biphenyl-2-carbonsäure-tert-butylester
Öl, $R_f$-Wert: 0,20 und 0,22 (Kieselgel: Methylenchlorid/Ethanol = 50:1)

Beispiel 4

4′-[(6-Amino-2-n-butyl-benzimidazol-1-yl)-methyl]biphenyl-2-carbonsäure-tert.butylester (I)
und
4′-[(5-Amino-2-n-butyl-benzimidazol-1-yl)-methyl]biphenyl-2-carbonsäure-tert.butylester (II)

Das in Beispiel 3 erhaltene Isomerengemisch von I und II (17,1 g) wird über eine mit 1700 ml Kieselgel (Korngröße: 0,063-0,2 mm) gefüllte Chromatographiesäule aufgetrennt, wobei zunächst durch Elution mit Methylenchlorid/Ethanol = 98:2 I isoliert wird.
Ausbeute: 7,32 g (42,8 % der Theorie),
Öl, $R_f$-Wert: 0,2 (Kieselgel: Methylenchlorid/Ethanol = 95:5)
Mit Methylenchlorid/Ethanol = 96:4 wird anschließend II eluiert.
Ausbeute: 9,28 g (54,3 % der Theorie),
Öl, $R_f$-Wert: 0,1 (Kieselgel: Methylenchlorid/Ethanol = 95:5)
Analog wurden aus den entsprechenden Isomerengemischen folgende Verbindungen rein isoliert:
4′-[(4-Amino-2-n-butyl-benzimidazol-1-yl)-methyl]biphenyl-2-carbonsäure-tert.butylester
Ausbeute: 86,1 % der Theorie,
Öl, $R_f$-Wert: 0,25 (Kieselgel: Methylenchlorid/Ethanol = 99:1)
und
4′-[(7-Amino-2-n-butyl-benzimidazol-1-yl)-methyl]biphenyl-2-carbonsäure-tert.butylester
Ausbeute: 5,0 % der Theorie,
Öl, $R_f$-Wert: 0,30 (Kieselgel: Methylenchlorid/Ethanol = 99:1)
4′-[(7-Benzyloxy-2-n-butyl-4-methyl-benzimidazol-1-yl)-methyl]biphenyl-2-carbonsäure-tert.butylester
Ausbeute: 90,5 % der Theorie,
Schmelzpunkt: 100-102°C
und
4′-[(4-Benzyloxy-2-n-butyl-7-methyl-benzimidazol-1-yl)-methyl]biphenyl-2-carbonsäure-tert.butylester
Ausbeute: 1,9 % der Theorie,
Öl, $R_f$-Wert: 0,20 (Aluminiumoxid: Cyclohexan/Essigester = 4:1)

Beispiel 5

4′-[(2-n-Butyl-6-methyl-benzimidazol-1-yl)-methyl]biphenyl-2-carbonsäure-tert.butylester

a) 2-Pentanoylamino-5-methyl-nitrobenzol

6 g (0,04 Mol) 2-Nitro-6-methyl-anilin werden in 50 ml Pyridin gelöst, auf 0°C abgekühlt und unter Rühren mit 5,3 g (0,04 Mol + 10 %) Valerylchlorid versetzt. Nach 1-stündigem Rühren bei Raumtemperatur wird in Eiswasser gegossen und das Kristallisat abgesaugt und getrocknet.
Ausbeute: 9 g (96,7 % der Theorie),

Schmelzpunkt: 69-71°C

| | | | | | | |
|---|---|---|---|---|---|---|
| Ber.: | C | 61,00 | H | 6,83 | N | 11,86 |
| Gef.: | | 61,21 | | 6,79 | | 11,72 |

b) 2-Pentanoylamino-5-methyl-anilin

8,5 g (0,035 Mol) 2-Pentanoylamino-5-methyl-nitrobenzol werden in 100 ml Methylalkohol gelöst und bei Raumtemperatur und 5 bar mit 2 g 10%iger Palladium/Kohle hydriert. Nach beendeter Reaktion wird vom Katalysator abgesaugt und im Vakuum eingedampft.
Ausbeute: 7,1 g (94,7 % der Theorie),
Schmelzpunkt: 132-134°C (Methanol)

| | | | | | | |
|---|---|---|---|---|---|---|
| Ber.: | C | 69,87 | H | 8,80 | N | 13,58 |
| Gef.: | | 69,28 | | 8,74 | | 13,31 |

c) N-(2-Pentanoylamino-5-methyl-phenyl)-2-tert.butoxycarbonyl-biphenyl-4′-yl-methylamin

2,1g (0,01 Mol) 2-Pentanoylamino-5-methyl-anilin werden in 10 ml Dimethylformamid und 5 ml N,N-Diisopropylethylamin gelöst, mit 3,5 g (0,01 Mol) 4′-Brommethyl-biphenyl-2-carbonsäure-tert.butylester versetzt und unter Rühren auf 120°C erhitzt. Nach 2 Stunden ist die Reaktion beendet und das Lösungsmittel wird im Vakuum abdestilliert. Der erhaltene ölige Rückstand wird in Essigester gelöst, mit Wasser gewaschen und nach Trocknen über Natriumsulfat im Vakuum eingedampft. Nach Säulenchromatographie an Kieselgel (Korngröße: 0,06-0,2 mm, Elutionsmittel: Methylenchlorid) erhält man ein gelbliches Öl.
Ausbeute: 3,8 g (80,0 % der Theorie),
Öl, $R_f$-Wert: 0,75 (Kieselgel: Methyl-ethylketon/Xylol = 1:1)

| | | | | | | |
|---|---|---|---|---|---|---|
| Ber.: | C | 76,24 | H | 7,68 | N | 5,93 |
| Gef.: | | 76,16 | | 7,85 | | 5,87 |

d) 4′-[(2-n-Butyl-6-methyl-benzimidazol-1-yl)-methyl]biphenyl-2-carbonsäure-tert.butylester

3,6 g (0,076 Mol) N-(2-Pentanoylamino-5-methyl-phenyl)-2-tert.butoxycarbonyl-biphenyl-4′-yl-methylamin werden in 5 ml Diglyme aufgenommen und zum Rückfluß erhitzt. Nach 2 Stunden ist die Reaktion beendet, das Reaktionsprodukt wird in Essigester gelöst, mit Wasser gewaschen und nach Trocknen über Natriumsulfat im Vakuum eingedampft. Nach Säulenchromatographie an Kieselgel (Korngröße: 0,06-0,2 mm, Elutionsmittel: Methylenchlorid + 1 % Ethanol) wird ein gelbliches Öl erhalten.
Ausbeute: 2,1 g (61,7 % der Theorie),
Öl, $R_f$-Wert: 0,6 (Kieselgel: Methyl-ethylketon/Xyclol = 1:2

| | | | | | | |
|---|---|---|---|---|---|---|
| Ber.: | C | 79,26 | H | 7,54 | N | 6,16 |
| Gef.: | | 79,12 | | 7,46 | | 6,09 |

Analog werden folgende Verbindungen erhalten:
4′-[(2-n-Butyl-6-chlor-benzimidazol-1-yl)-methyl]biphenyl-2-carbonsäure-tert.butylester
Öl, $R_f$-Wert: 0,6 (Kieselgel: Methyl-ethylketon/Xylol = 1:2)
4′-[(2-n-Butyl-6-methoxy-benzimidazol-1-yl)-methyl]biphenyl-2-carbonsäure-tert.butylester
Öl, $R_f$-Wert: 0,65 (Kieselgel: Methyl-ethylketon/Xylol = 1:2)

Beispiel 6

4′-[(2-n-Butyl-5-methoxy-benzimidazol-1-yl)-methyl]biphenyl-2-carbonsäure-tert.butylester

a) 2-Pentanoylamino-5-methoxy-nitrobenzol

4,2 g (0,025 Mol) 2-Nitro-4-methoxy-anilin werden in 30 ml Pyridin gelöst, auf 0°C abgekühlt und unter Rühren mit 3,3 g (0,0275 Mol) Valerylchlorid versetzt. Nach 1-stündigem Rühren bei Raumtemperatur wird in Eiswasser gegossen, das Kristallisat abgesaugt und getrocknet.
Ausbeute: 6 g (95,2 % der Theorie),
Schmelzpunkt: 74-75°C

| $C_{12}H_{16}N_2O_4$ (252,27) | | | | | | |
|---|---|---|---|---|---|---|
| Ber.: | C | 57,13 | H | 6,39 | N | 11,11 |
| Gef.: | | 57,43 | | 6,45 | | 10,97 |

b) N-(2-Nitro-4-methoxy-phenyl)-N-pentanoyl-(2-tert.butoxycarbonyl-biphenyl-4′-yl)-methylamin

2,5 g (0,01 Mol) 2-Pentanoylamino-5-methoxy-nitrobenzol werden in 30 ml Dimethylformamid gelöst, mit 550 mg (0,11 Mol) Natriumhydrid (50% in Öl) versetzt und 30 Minuten bei 80°C gerührt. Dann werden 3,5 g (0,01 Mol) 4′-Brommethyl-biphenyl-2-carbonsäure-tert.butylester zugegeben und noch 2 Stunden bei 80°C gerührt. Das Lösungsmittel wird im Vakuum abdestilliert, der erhaltene ölige Rückstand in Essigester gelöst, mit Wasser gewaschen und nach Trocknen über Natriumsulfat im Vakuum eingedampft. Nach Säulenchromatographie (Kieselgel: 0,06-0,2 mm, Elutionsmittel: Methylenchlorid) erhält man ein gelbliches Öl.
Ausbeute: 4,0 g (78,4 % der Theorie),
Öl, $R_f$-Wert: 0,8 (Kieselgel: Methyl-ethylketon/Xylol = 1:2)

| $C_{30}H_{34}N_2O_6$ (518,61) | | | | | | |
|---|---|---|---|---|---|---|
| Ber.: | C | 69,48 | H | 6,61 | N | 5,40 |
| Gef.: | | 69,31 | | 6,53 | | 5,39 |

c) N-(2-Amino-4-methoxy-phenyl)-N-pentanoyl-(2-tert.butoxycarbonyl-biphenyl-4′-yl)-methylamin

3,8 g (0,007 Mol) N-(2-Nitro-4-methoxy-phenyl)-N-pentanoyl-(2-tert.butoxy-carbonyl-biphenyl-4′-yl)-methylamin werden in 100 ml Methanol gelöst und bei Raumtemperatur und 5 bar in Gegenwart von 1 g 70%igem Palladium/Kohle hydriert. Nach beendeter Reaktion wird vom Katalysator abgesaugt und im Vakuum eingedampft.
Ausbeute: 3,2 g (93,6 % der Theorie),

Öl, $R_f$-Wert: 0,5 (Kieselgel: Methyl-ethylketon/Xylol = 1:2)

| $C_{30}H_{36}N_2O_4$ (488,63) | | | | | | |
|---|---|---|---|---|---|---|
| Ber.: | C | 73,74 | H | 7,43 | N | 5,73 |
| Gef.: | | 73,59 | | 7,40 | | 5,72 |

d) 4'-[(2-n-Butyl-5-methoxy-benzimidazol-1-yl)-methyl]biphenyl-2-carbonsäure-tert.butylester

3 g (0,006 Mol) N-(2-Amino-4-methoxy-phenyl)-N-pentanoyl-(2-tert.butoxy-carbonyl-biphenyl-4′-yl)-methylamin werden in 100 ml Eisessig gelöst und unter Rühren zum Rückfluß erhitzt. Nach einer Stunde wird das Lösungsmittel abdestilliert und das erhaltene Öl über Kieselgel (Korngröße: 0,06-0,2 mm, Elutionsmittel: Methylenchlorid/Ethanol = 19:1) zur weiteren Reinigung chromatographiert.
Ausbeute: 2,3 g (82,1 % der Theorie),
Öl, $R_f$-Wert: 0,7 (Kieselgel: Essigester/Ethanol/Ammoniak = 90:10:1)

| $C_{30}H_{34}N_2O_3$ (470,61) | | | | | | |
|---|---|---|---|---|---|---|
| Ber.: | C | 76,56 | H | 7,28 | N | 5,95 |
| Gef.: | | 76,36 | | 7,31 | | 5,79 |

Analog werden folgende Verbindungen erhalten:
4′-[(2-n-Butyl-5-chlor-benzimidazol-1-yl)-methyl]biphenyl-2-carbonsäure-tert.butylester
Öl, $R_f$-Wert: 0,7 (Kieselgel: Essigester/Ethanol/Ammoniak = 90:10:1)
4′-[(5-Amino-2-n-butyl-benzimidazol-1-yl)-methyl]biphenyl-2-carbonsäure-tert.butylester
Öl, $R_f$-Wert: 0,5 (Kieselgel: Essigester/Ethanol/Ammoniak = 90:10:1)
4′-[(2-n-Butyl-5-methyl-benzimidazol-1-yl)-methyl]biphenyl-2-carbonsäure-tert.butylester
Öl, $R_f$-Wert: 0,8 (Kieselgel: Essigester/Ethanol/Ammoniak = 90:10:1)
4′-[(2-n-Butyl-7-cyano-benzimidazol-1-yl)-methyl]biphenyl-2-carbonsäure-tert.butylester
Öl, $R_f$Wert: 0,49 (Kieselgel: Methylenchlorid/Ethanol = 19:1)
4′-[(2-n-Butyl-5,7-difluor-benzimidazol-1-yl)-methyl]biphenyl-2-carbonsäure-tert.butylester
Öl, $R_f$-Wert: 0,27 (Kieselgel: Methylenchlorid/Ethanol = 50:1)
4′-[(2-n-Butyl-5-acetyl-benzimidazol-1-yl)-methyl]biphenyl-2-carbonsäure-tert.butylester
Öl, $R_f$-Wert: 0,30 (Kieselgel: Methylenchlorid/Ethanol = 50:1)
4′-[(2-n-Butyl-6-dimethylamino-benzimidazol-1-yl)-methyl]biphenyl-2-carbonsäure-tert.butylester
Öl, $R_f$-Wert: 0,60 (Kieselgel: Methylenchlorid/Ethanol = 19:1)
4′-[(7-Amino-2-n-butyl-benzimidazol-1-yl)-methyl]biphenyl-2-carbonsäure-tert.butylester
Öl, $R_f$-Wert: 0,30 (Aluminiumoxid: Methylenchlorid/Ethanol = 99:1)
4′-[(6-(N-Benzyl-methylamino)-2-n-butyl-benzimidazol-1-yl)-methyl]biphenyl-2-carbonsäure-tert.butylester
Öl, $R_f$-Wert: 0,40 (Kieselgel: Methylenchlorid/Ethanol = 19:1)
4′-[(2-n-Butyl-5-dimethylaminosulfonyl-3-N-oxido-benzimidazol-1-yl)-methyl]biphenyl-2-carbonsäure-tert.butylester
(hergestellt durch unvollständige Reduktion der Nitrogruppe und anschließende Cyclisierung)
Schmelzpunkt: 59-62°C
4′-[(7-Benzyloxy-2-n-butyl-benzimidazol-1-yl)-methyl]biphenyl-2-carbonsäure-tert.butylester
Öl, $R_f$-Wert: 0,50 (Kieselgel: Methyl-ethylketon/Xylol = 1:1)
4′-[(4-Benzyloxy-2-n-butyl-benzimidazol-1-yl)-methyl]biphenyl-2-carbonsäure-tert.butylester
Öl, $R_f$-Wert: 0,65 (Kieselgel: Methyl-ethylketon/Xylol = 1:4)
4′-[(2-n-Butyl-7-methoxy-benzimidazol-1-yl)-methyl]biphenyl-2-carbonsäure-tert.butylester
Öl, $R_f$-Wert: 0,80 (Kieselgel: Methyl-ethylketon/Xylol = 1:1)
4′-[(2-n-Butyl-4-methoxy-benzimidazol-1-yl)-methyl]biphenyl-2-carbonsäure-tert.butylester
Öl, $R_f$-Wert: 0,70 (Kieselgel: Methyl-ethylketon/Xylol = 1:2)
4'-[(2,5-Di-n-butyl-benzimidazol-1-yl)-methyl]biphenyl-2-carbonsäure-tert.butylester
4'-[(2,6-Di-n-butyl-benzimidazol-1-yl)-methyl]biphenyl-2-carbonsäure-tert.butylester
4'-[(6-Benzyloxy-2-n-butyl-benzimidazol-1-yl)-methyl]biphenyl-2-carbonsäure-tert.butylester

Öl, $R_f$-Wert: 0,40 (Kieselgel: Methyl-ethylketon/Xylol = 1:2)
4'-[(2-n-Butyl-6-methylamino-benzimidazol-1-yl)-methyl]biphenyl-2-carbonsäure-tert.butylester
Öl, $R_f$-Wert: 0,10 (Kieselgel: Methylenchlorid/Ethanol = 50:1)
4'-[(2-n-Butyl-6-cyclohexylamino-benzimidazol-1-yl)-methyl]biphenyl-2-carbonsäure-tert.butylester
Öl, $R_f$-Wert: 0,20 (Kieselgel: Essigester/Petrolether = 60:40)
4'-[(2-n-Butyl-6-(3-cyclohexyl-piperidino)-benzimidazol-1-yl)-methyl]biphenyl-2-carbonsäure-tert.butylester
Öl, $R_f$-Wert: 0,65 (Kieselgel: Methylenchlorid/Ethanol = 9:1)
4'-[(2-n-Butyl-6-phthalimino-benzimidazol-1-yl)-methyl]biphenyl-2-carbonsäure-tert.butylester
Schmelzpunkt: 182-184°C
4'-[(2-n-Butyl-6-(n-pentylamino)-benzimidazol-1-yl)-methyl]biphenyl-2-carbonsäure-tert.butylester
Öl, $R_f$-Wert: 0,70 (Kieselgel: Methyl-ethylketon/Xylol = 1:1)

Beispiel 7

4'-[(2-n-Butyl-5-(n-butylaminocarbonylamino)-benzimidazol-1-yl)-methyl]biphenyl-2-carbonsäure-tert.butylester

2,0 g (4,4 mMol) 4'-[(5-Amino-2-n-butyl-benzimidazol-1-yl)-methyl]biphenyl-2-carbonsäure-tert.butylester werden in 50 ml Tetrahydrofuran gelöst und mit 2,0 ml Triethylamin sowie 2,0 ml n-Butylisocyanat versetzt. Das Reaktionsgemisch wird 4 Stunden am Rückfluß gekocht, einrotiert, und der Rückstand über eine Kieselgel-Säule (Korngröße: 0,063 mm-0,2 mm, Elutionsmittel: Methylenchlorid/0-2 % Ethanol) gereinigt. Die einheitlichen Fraktionen werden zur Trockene eingeengt.
Ausbeute: 2,1 g (86,4 % der Theorie),
Öl, $R_f$-Wert: 0,45 (Kieselgel: Methylenchlorid/Ethanol = 19:1)
Analog werden folgende Verbindungen erhalten:
4'-[(2-n-Butyl-6-phenylaminocarbonylamino-benzimidazol-1-yl)-methyl]biphenyl-2-carbonsäure-tert.butylester
Amorphe Substanz, $R_f$-Wert: 0,30 (Kieselgel: Methylenchlorid/Ethanol = 19:1)
4'-[(2-n-Butyl-6-cyclohexylaminocarbonylamino-benzimidazol-1-yl)-methyl]biphenyl-2-carbonsäure-tert.butylester
Amorphe Substanz, $R_f$-Wert: 0,25 (Kieselgel: Methylenchlorid/Ethanol = 19:1)
4′-[(2-n-Butyl-4-ethylaminocarbonylamino-benzimidazol-1-yl)-methyl]biphenyl-2-carbonsäure-tert.butylester
Amorphe Substanz, $R_f$-Wert: 0,50 (Kieselgel: Methylenchlorid/Ethanol = 19:1)
4′-[(6-Aminocarbonylamino-2-n-butyl-benzimidazol-1-yl)-methyl]biphenyl-2-carbonsäure-tert.butylester
Schmelzpunkt: 210-211°C
4′-[(2-n-Butyl-6-(n-hexylaminocarbonylamino)-benzimidazol-1-yl)-methyl]biphenyl-2-carbonsäure-tert.butylester
Schmelzpunkt: 142-143°C
4′-[(2-n-Butyl-4-cyclohexylaminocarbonylamino-benzimidazol-1-yl)-methyl]biphenyl-2-carbonsäure-tert.butylester
Schmelzpunkt: 104-106°C (amorph)
4′-[(2-n-Butyl-5-cyclohexylaminocarbonylamino-benzimidazol-1-yl)-methyl]biphenyl-2-carbonsäure-tert.butylester
Schmelzpunkt: 118-120°C (amorph)
4′-[(2-n-Butyl-7-cyclohexylaminocarbonylamino-benzimidazol-1-yl)-methyl]biphenyl-2-carbonsäure-ethylester
Schmelzpunkt: 152-154°C
4′-[(6-Aminothiocarbonylamino-2-n-butyl-benzimidazol-1-yl)-methyl]biphenyl-2-carbonsäure-tert.butylester
Schmelzpunkt: 175-176°C
4′-[(2-n-Butyl-6-cyclohexylaminothiocarbonylamino-benzimidazol-1-yl)-methyl]biphenyl-2-carbonsäure-tert.butylester
Schmelzpunkt: 91-93°C (amorph)
4′-[(5-Aminothiocarbonylamino-2-n-butyl-benzimidazol-1-yl)-methyl]biphenyl-2-carbonsäure-tert.butylester
Schmelzpunkt: 196-197°C
4′-[(2-n-Butyl-6-benzylaminocarbonylamino-benzimidazol-1-yl)-methyl]biphenyl-2-carbonsäure-tert.butylester
Schmelzpunkt: 163-165°C
4′-[(6-Allylaminocarbonylamino-2-n-butyl-benzimidazol-1-yl)-methyl]biphenyl-2-carbonsäure-tert.butylester
Öl, $R_f$-Wert: 0,10 (Kieselgel: Methylenchlorid/Ethanol = 19:1)
4′-[(2-n-Butyl-6-(tetrahydropyran-2-yl-aminocarbonylamino)-benzimidazol-1-yl)-methyl]biphenyl-2-carbonsäure-ethylester
Schmelzpunkt: 86-88°C (amorph)
4′-[(2-n-Butyl-6-(tetrahydropyran-2-yl-aminocarbonylamino)-benzimidazol-1-yl)-methyl]biphenyl-2-carbonsäure-tert.butylester
Öl, $R_f$-Wert: 0,20 (Kieselgel: Methylenchlorid/Ethanol = 19:1)
4′-[(2-n-Butyl-6-(N-methylaminocarbonyl-n-pentylamino)-benzimidazol-1-yl)-methyl]biphenyl-2-carbonsäure-tert.butylester
Öl, $R_f$-Wert: 0,30 (Kieselgel: Methyl-ethylketon/Xylol = 5:2)

4′-[(2-n-Butyl-6-cyclohexylaminocarbonylamino-benzimidazol-1-yl)-methyl]-2-phthalimino-biphenyl
Öl, $R_f$-Wert: 0,45 (Kieselgel: Methylenchlorid/Ethanol = 9:1)
4′-[(6-(Adamant-1-yl-aminocarbonylamino)-2-n-butyl-benzimidazol-1-yl)-methyl]biphenyl-2-carbonsäure
Schmelzpunkt: 251-253°C
4′-[(6-(Adamant-1-yl-aminocarbonylamino)-2-n-butyl-benzimidazol-1-yl)-methyl]biphenyl-2-carbonsäure-tert.butylester
Schmelzpunkt: 196-198°C
4′-[(2-n-Butyl-6-(N-methylaminocarbonyl-methylamino)-benzimidazol-1-yl)-methyl]biphenyl-2-carbonsäure-tert.butylester
Öl, $R_f$-Wert: 0,30 (Kieselgel: Methyl-ethylketon/Xylol = 5:2)
4′-[(2-n-Butyl-6-(N-(n-butylaminocarbonyl)-methylamino)-benzimidazol-1-yl)-methyl]biphenyl-2-carbonsäure-tert.butylester
Öl, $R_f$-Wert: 0,70 (Kieselgel: Methyl-ethylketon/Xylol = 5:2)
4′-[(2-n-Butyl-5-(N-methylaminocarbonyl-n-pentylamino)-benzimidazol-1-yl)-methyl]biphenyl-2-carbonsäure-tert.butylester
Öl, $R_f$-Wert: 0,45 (Kieselgel: Methyl-ethylketon/Xylol = 1:2)
4′-[(6-(n-Butylaminocarbonylamino)-2-n-butyl-benzimidazol-1-yl)-methyl]biphenyl-2-carbonsäure-tert.butylester
Öl, $R_f$-Wert: 0,7 (Kieselgel: Essigester/Ethanol/Ammoniak = 90:10:1)
4′-[(2-n-Butyl-6-(N-cyclohexylaminocarbonyl-methylamino)benzimidazol-1-yl)-methyl]biphenyl-2-carbonsäure-tert.butylester
Öl, $R_f$-Wert: 0,7 (Kieselgel: Methyl-ethylketon/Xylol = 5:2)
4′-[(2-n-Butyl-6-(N-(n-butylaminocarbonyl)-methylamino-benzimidazol-1-yl)-methyl]biphenyl-2-carbonsäure-tert.butylester
Öl, $R_f$-Wert: 0,55 (Kieselgel: Methyl-ethylketon = 5:2)
4′-[(2-n-Butyl-6-(N-cyclohexylaminocarbonyl-n-pentylamino)-benzimidazol-1-yl)-methyl]biphenyl-2-carbonsäure-tert.butylester
Öl, $R_f$-Wert: 0,8 (Kieselgel: Methyl-ethylketon = 5:2)
4′-[(2-n-Butyl-6-(N-(n-hexylaminocarbonyl)-cyclohexylamino)-benzimidazol-1-yl)-methyl]biphenyl-2-carbonsäure-tert.butylester
Öl, $R_f$-Wert: 0,40 (Aluminiumoxid-Platte: Essigester/Petrolether = 3:7)
4′-[(2-n-Butyl-6-(N-cyclohexylaminocarbonyl-cyclohexylamino)-benzimidazol-1-yl)-methyl]biphenyl-2-carbonsäure-tert.butylester
Öl, $R_f$-Wert: 0,40 (Aluminiumoxid: Essigester/Petrolether = 4:1)
4′-[(2-n-Butyl-6-(N-(n-butylaminocarbonyl)-cyclohexylamino)-benzimidazol-1-yl)-methyl]-biphenyl-2-carbonsäure-tert.butylester
Öl, $R_f$-Wert: 0,35 (Kieselgel: Essigester/Petrolether = 4:1)
4′-[(2-n-Butyl-6-(N-methylaminocarbonyl-cyclohexylamino)-benzimidazol-1-yl)-methyl]biphenyl-2-carbonsäure-tert.butylester
Öl, $R_f$-Wert: 0,45 (Aluminiumoxid: Essigester/Petrolether = 7:3)
4′-[(2-n-Butyl-6-(N-methylaminocarbonyl-benzylamino)-benzimidazol-1-yl)-methyl]biphenyl-2-carbonsäure-tert.butylester
Öl, $R_f$-Wert: 0,45 (Aluminiumoxid: Essigester/Petrolether = 6:4)
4′-[(2-n-Butyl-6-(N-(n-hexylaminocarbonyl)-benzylamino)-benzimidazol-1-yl)-methyl]biphenyl-2-carbonsäure-tert.butylester
Öl, $R_f$-Wert: 0,50 (Aluminiumoxid: Essigester/Petrolether = 4:6)
4′-[(2-n-Butyl-6-(N-cyclohexylaminocarbonyl-n-butylamino)-benzimidazol-1-yl)-methyl]biphenyl-2-carbonsäure-tert.butylester
Schmelzpunkt: 106-108°C (amorph)
4′-[(2-n-Butyl-6-(N-cyclohexylaminocarbonyl-benzylamino)-benzimidazol-1-yl)-methyl]biphenyl-2-carbonsäure-tert.butylester
Öl, $R_f$-Wert: 0,60 (Kieselgel: Essigester/Petrolether = 6:4)
4′-[(2-n-Butyl-6-(N-(2-trifluormethylphenylaminocarbonyl)-methylamino)-benzimidazol-1-yl)-methyl]biphenyl-2-carbonsäure-tert.butylester
Öl, $R_f$-Wert: 0,90 (Kieselgel: Methyl-ethylketon/Xylol = 5:2)
4′-[(2-n-Butyl-6-(N-cyclohexylaminocarbonyl-n-hexylamino)-benzimidazol-1-yl)-methyl]biphenyl-2-carbonsäure-tert.butylester
Öl, $R_f$-Wert: 0,70 (Kieselgel: Essigester/Petrolether = 6:4)
4′-[(2-n-Butyl-6-(N-cyclohexylaminocarbonyl-n-propylamino)-benzimidazol-1-yl)-methyl]biphenyl-2-carbonsäure-tert.butylester
Öl, $R_f$-Wert: 0,50 (Kieselgel: Essigester/Petrolether = 6:4)

4′-[(2-n-Butyl-6-(N-cyclohexylaminocarbonyl-ethylamino)-benzimidazol-1-yl)-methyl]biphenyl-2-carbonsäure-tert.butylester
Öl, $R_f$-Wert: 0,20 (Kieselgel: Essigester/Petrolether = 6:4)
4′-[(2-n-Butyl-6-(N-cyclohexylaminocarbonyl-N-(2-phenylethyl)-amino)-benzimidazol-1-yl)-methyl]biphenyl-2-carbonsäure-tert.butylester
Öl, $R_f$-Wert: 0,55 (Kieselgel: Essigester/Petrolether = 6:4)
4′-[(2-n-Butyl-6-(N-(n-hexylaminocarbonyl)-N-(2-phenylethyl)-amino)-benzimidazol-1-yl)-methyl]biphenyl-2-carbonsäure-tert.butylester
Öl, $R_f$-Wert: 0,20 (Kieselgel: Essigester/Petrolether = 7:3)
4′-[(2-(1-trans-Butenyl)-6-cyclohexylaminocarbonylamino-benzimidazol-1-yl)-methyl]biphenyl-2-carbonsäure-tert.butylester
Öl, $R_f$-Wert: 0,20 (Aluminiumoxid: Methylenchlorid/Ethanol = 50:1)
4′-[(2-n-Butyl-6-(N-(dimethylaminocarbonyl)-n-pentylamino)-benzimidazol-1-yl)-methyl]biphenyl-2-carbonsäure-tert.butylester
Öl, $R_f$-Wert: 0,50 (Kieselgel: Methyl-ethylketon/Xylol = 1:1)
4'-[(2-n-Butyl-6-cyclohexylaminocarbonylamino-benzimidazol-1-yl)-methyl]-2-(1-triphenylmethyl-tetrazol-5-yl)-biphenyl
Schmelzpunkt: 183-187°C
4'-[(2-n-Butyl-6-(N-cyclohexylaminocarbonyl-methylamino)-benzimidazol-1-yl)-methyl]-2-(1-triphenylmethyl-tetrazol-5-yl)-biphenyl
Schmelzpunkt: 185-186°C
4'-[(2-n-Butyl-6-cyclohexylaminocarbonyloxy-benzimidazol-1-yl)-methyl]biphenyl-2-carbonsäure-tert.butylester
Schmelzpunkt: 76-78°C (amorph)
4'-[(2-n-Butyl-6-(N-methylaminocarbonyl-N-(3-cyclohexyl-n-propyl)-amino)-benzimidazol-1-yl)-methyl]biphenyl-2-carbonsäure-tert.butylester
Öl, $R_f$-Wert: 0,15 (Aluminiumoxid: Petrolether/Essigester = 2:3)
4'-[(2-n-Butyl-6-(N-cyclohexylaminocarbonyl-methylamino)-benzimidazol-1-yl)-methyl]-1-cyano-2-phenylnaphthalin
4'-[(2-n-Propyl-6-(N-cyclohexylaminocarbonyl-methylamino)-benz-imidazol-1-yl)-methyl]-2-phenylnaphthalin-1-carbonsäure-tert.-butylester
4′-[(2-n-Butyl-6-(N-(n-dodecylaminocarbonyl)-methylamino)-benzimidazol-1-yl)-methyl]biphenyl-2-carbonsäure-tert.butylester
Öl, $R_f$-Wert: 0,40 (Aluminiumoxid: Petrolether/Essigester = 3:7)
4′-[(2-n-Butyl-6-(N-(cyclohexylaminocarbonyl)-n-octylamino)-benzimidazol-1-yl)-methyl]biphenyl-2-carbonsäure-tert.butylester
Öl, $R_f$-Wert: 0,35 (Aluminiumoxid: Petrolether/Essigester = 3:2)
4′-[(2-n-Butyl-6-(N-(n-octylaminocarbonyl)-methylamino)-benzimidazol-1-yl)-methyl]biphenyl-2-carbonsäure-tert.butylester
Öl, $R_f$-Wert: 0,35 (Aluminiumoxid: Petrolether/Essigester = 2:3)

Beispiel 8

4′-[(2-n-Butyl-4-butanoylamino-benzimidazol-1-yl)-methyl]biphenyl-2-carbonsäure-tert.butylester

1,6 g (3,5 mMol) 4′-[(4-Amino-2-n-butyl-benzimidazol-1-yl)-methyl]biphenyl-2-carbonsäure-tert.butylester werden in 30 ml Pyridin gelöst und unter Rühren bei Raumtemperatur 0,52 ml (5,0 mMol) Butyrylchlorid zugetropft. Nach einer Stunde wird vom Lösungsmittel abdestilliert und der Rückstand mit ca. 20 ml Diethylether verrieben. Man saugt vom Niederschlag ab und trocknet bei 50°C im Vakuum.
Ausbeute: 1,75 g (94,6 % der Theorie),
Schmelzpunkt: 167-168°C
Analog werden folgende Verbindungen erhalten:
4′-[(2-n-Butyl-5-acetamino-benzimidazol-1-yl)-methyl]biphenyl-2-carbonsäure-tert.butylester
Öl, $R_f$-Wert: 0,70 (Kieselgel: Methylenchlorid/Ethanol = 9:1)
4′-[(2-n-Butyl-5-butanoylamino-benzimidazol-1-yl)-methyl]biphenyl-2-carbonsäure-tert.butylester
Öl, $R_f$-Wert: 0,80 (Kieselgel: Essigester/Ethanol/Ammoniak = 90:10:1)
4′-[(2-n-Butyl-5-methansulfonamino-benzimidazol-1-yl)-methyl]biphenyl-2-carbonsäure-tert.butylester
Öl, $R_f$-Wert: 0,75 (Kieselgel: Essigester/Ethanol/Ammoniak = 90:10:1)
4′-[(2-n-Butyl-6-isopropylsulfonamino-benzimidazol-1-yl)-methyl]biphenyl-2-carbonsäure-tert.butylester
Amorphe Substanz, $R_f$-Wert: 0,55 (Kieselgel: Methylenchlorid/Ethanol = 19:1)
4′-[(2-n-Butyl-6-ethoxycarbonylamino-benzimidazol-1-yl)-methyl]biphenyl-2-carbonsäure-tert.butylester
Amorphe Substanz, $R_f$-Wert: 0,30 (Kieselgel: Methylenchlorid/Ethanol = 19:1)

4′-[(2-n-Butyl-5-(tert.butoxycarbonylamino-acetamino)-benzimidazol-1-yl)-methyl]biphenyl-2-carbonsäure-tert.butylester
Schmelzpunkt: 101-103°C (amorph)
4′-[(2-n-Butyl-6-(N-butanoyl-methylamino)-benzimidazol-1-yl)-methyl]biphenyl-2-carbonsäure-tert.butylester
Öl, $R_f$-Wert: 0,70 (Kieselgel: Methylenchlorid/Ethanol = 19:1)
4′-[(2-n-Butyl-7-butansulfonylamino-benzimidazol-1-yl)-methyl]biphenyl-2-carbonsäure-ethylester
Öl, Rf-Wert: 0,50 (Kieselgel: Methylenchlorid/Ethanol = 19:1)
4′-[(2-n-Butyl-5-propanoylamino-benzimidazol-1-yl)-methyl]biphenyl-2-carbonsäure-ethylester
Schmelzpunkt: 221-223°C
4′-[(6-Acetamino-2-n-butyl-benzimidazol-1-yl)-methyl]biphenyl-2-carbonsäure-tert.butylester
Schmelzpunkt: 192-194°C
4′-[(2-n-Butyl-6-propanoylamino-benzimidazol-1-yl)-methyl]biphenyl-2-carbonsäure-tert.butylester
Schmelzpunkt: 133-135°C
4′-[(6-Butanoylamino-2-n-butyl-benzimidazol-1-yl)-methyl]biphenyl-2-carbonsäure-tert.butylester
Schmelzpunkt: 127-129°C
4′-[(2-n-Butyl-6-n-pentanoylamino-benzimidazol-1-yl)-methyl]biphenyl-2-carbonsäure-tert.butylester
Öl, $R_f$-Wert: 0,6 (Kieselgel: Methylenchlorid/Ethanol = 19:1)
4′-[(2-n-Butyl-6-dimethylaminocarbonylamino-benzimidazol-1-yl)-methyl]biphenyl-2-carbonsäure-tert.butylester
Öl, $R_f$-Wert: 0,4 (Kieselgel: Methyl-ethylketon/Xylol = 5:2)
4′-[(2-n-Butyl-6-(N-(dimethylaminocarbonyl)-methylamino)-benzimidazol-1-yl)-methyl]biphenyl-2-carbonsäure-tert.butylester
Öl, $R_f$-Wert: 0,45 (Kieselgel: Methylenchlorid/Ethanol = 19:1)
4′-[(2-n-Butyl-6-phenylacetamino-benzimidazol-1-yl)-methyl]biphenyl-2-carbonsäure-tert.butylester
$R_f$-Wert: 0,30 (Kieselgel: Methylenchlorid/Ethanol = 50:1)
Schmelzpunkt: 178-180°C
4′-[(2-n-Butyl-6-cyclohexylacetamino-benzimidazol-1-yl)-methyl]biphenyl-2-carbonsäure-tert.butylester
$R_f$-Wert: 0,30 (Kieselgel: Essigester/Petrolether = 4:1)
Schmelzpunkt: 64-66°C (amorph)
4′-[(2-n-Butyl-6-cyclohexylcarbonylamino-benzimidazol-1-yl)-methyl]biphenyl-2-carbonsäure-tert.butylester
$R_f$-Wert: 0,70 (Kieselgel: Essigester/Petrolether = 9:1)
Schmelzpunkt: 72-76°C (amorph)
4′-[(6-Benzyloxycarbonylamino-2-n-butyl-benzimidazol-1-yl)-methyl]biphenyl-2-carbonsäure-tert.butylester
$R_f$-Wert: 0,80 (Kieselgel: Essigester/Petrolether = 9:1)
Schmelzpunkt: 84-86°C
4′-[(2-n-Butyl-6-cyclohexylmethylaminocarbonyl-benzimidazol-1-yl)-methyl]biphenyl-2-carbonsäure-tert.butylester
Öl, $R_f$-Wert: 0,45 (Kieselgel: Petrolether/Essigester/Ethanol = 2,5:2,5:0,5)
4′-[(2-n-Butyl-6-cyclohexylmethylaminocarbonyl-benzimidazol-1-yl)-methyl]biphenyl-2-carbonsäure-tert.butylester
Öl, $R_f$-Wert: 0,50 (Kieselgel: Petrolether/Essigester/Ethanol = 2,5:2,5:0,5)
4′-[(2-n-Butyl-6-(N-methyl-n-butylaminocarbonyl)-benzimidaol-1-yl)-methyl]biphenyl-2-carbonsäure-tert.butylester
Öl, $R_f$-Wert: 0,40 (Kieselgel: Petrolether/Essigester/Ethanol = 6:3:1 + 1 % Eisessig)
4′-[(2-n-Butyl-5-(N-butylaminocarbonyl)-benzimidaol-1-yl)-methyl]biphenyl-2-carbonsäure-tert.butylester
Öl, $R_f$-Wert: 0,75 (Kieselgel: Petrolether/Essigester = 1:2 + 1 % Eisessig)
4′-[(2-n-Butyl-6-(2-isopropyl-5-methyl-cyclohexyloxycarbonylamino)-benzimidazol-1-yl)-methyl]biphenyl-2-carbonsäure-tert.butylester
Öl, $R_f$-Wert: 0,45 (Kieselgel: Essigester/Petrolether = 60:40)
4′-[(2-n-Butyl-6-(N-ethoxycarbonyl-cyclohexylamino)-benzimidazol-1-yl)-methyl]biphenyl-2-carbonsäure-tert.butylester
Öl, $R_f$-Wert: 0,65 (Kieselgel: Essigester/Petrolether = 6:4)
4′-[(2-n-Butyl-6-cyclohexylacetamino-benzimidazol-1-yl)-methyl]biphenyl-2-carbonsäure-tert.butylester
Schmelzpunkt: 65-66°C (amorph)
4′-[(2-n-Butyl-6-(N-ethoxycarbonyl-benzylamino)-benzimidazol-1-yl)-methyl]biphenyl-2-carbonsäure-tert.butylester
Öl, $R_f$-Wert: 0,60 (Kieselgel: Essigester/Petrolether = 7:3)
4′-[(2-n-Butyl-6-(n-hexyloxycarbonylamino)-benzimidazol-1-yl)-methyl]biphenyl-2-carbonsäure-tert.butylester
Öl, $R_f$-Wert: 0,55 (Kieselgel: Essigester/Petrolether = 6:4)
4′-[(2-n-Butyl-6-(5,7-dioxo-1H,3H-imidazo[1,5-c]thiazol-6-yl)-benzimidazol-1-yl)-methyl]biphenyl-2-carbonsäure-tert.butylester
Öl, $R_f$-Wert: 0,55 (Aluminiumoxid: Essigester/Petrolether = 7:3)
4′-[(2-n-Butyl-6-(N-(n-butanoyl)-n-butylamino)-benzimidazol-1-yl)-methyl]biphenyl-2-carbonsäure-tert.butylester
Öl, $R_f$-Wert: 0,60 (Kieselgel: Essigester/Petrolether = 6:4)
4′-[(2-n-Butyl-6-(N-(4-methoxycarbonyl-thiazolidin-3-yl-carbonyl)-n-butylamino)-benzimidazol-1-yl)-methyl]biphenyl-2-carbonsäure-tert.butylester
Öl, $R_f$-Wert: 0,55 (Kieselgel: Essigester/Petrolether = 6:4)
4′-[(2-n-Butyl-6-(N-cyclohexylcarbonyl-n-hexylamino)-benzimidazol-1-yl)-methyl]biphenyl-2-carbonsäure-tert.butylester
Öl, $R_f$-Wert: 0,55 (Kieselgel: Essigester/Petrolether = 6:4)
4′-[(2-n-Butyl-6-(N-cyclohexylcarbonyl-methylamino)-benzimidazol-1-yl)-methyl]biphenyl-2-carbonsäure-tert.butylester
Öl, $R_f$-Wert: 0,70 (Kieselgel: Essigester/Petrolether = 6:4)
4′-[(2-n-Butyl-6-(N-(n-butanoyl)-n-pentylamino)-benzimidazol-1-yl)-methyl]biphenyl-2-carbonsäure-tert.butylester
Öl, $R_f$-Wert: 0,60 (Kieselgel: Methyl-ethylketon/Xylol = 1:1)
4′-[(2-n-Butyl-6-isopropylcarbonylamino-benzimidazol-1-yl)-methyl]biphenyl-2-carbonsäure-tert.butylester
Öl, $R_f$-Wert: 0,15 (Kieselgel: Essigester/Petrolether = 7:3)
4′-[(2-n-Butyl-6-(N-ethoxycarbonyl-methylamino)-benzimidazol-1-yl)-methyl]biphenyl-2-carbonsäure-tert.butylester
Öl, $R_f$-Wert: 0,35 (Kieselgel: Essigester/Petrolether = 6:4)
4′-[(2-n-Butyl-6-(N-ethoxycarbonyl-n-pentylamino)-benzimidazol-1-yl)-methyl]biphenyl-2-carbonsäure-tert.butylester
Öl, $R_f$-Wert: 0,70 (Kieselgel: Methyl-ethylketon/Xylol = 1:1)
4′-[(2-n-Butyl-6-(N-(dimethylaminocarbonyl)-benzylamino)-benzimidazol-1-yl)-methyl]biphenyl-2-carbonsäure-tert.butylester
Öl, $R_f$-Wert: 0,45 (Kieselgel: Essigester/Petrolether = 4:1)
4′-[(2-n-Butyl-6-diethylaminocarbonylamino-benzimidazol-1-yl)-methyl]biphenyl-2-carbonsäure-tert.butylester
Öl, $R_f$-Wert: 0,20 (Kieselgel: Essigester/Petrolether = 9:1)
4′-[(2-n-Butyl-6-(dimethylaminoacetamino)-benzimidazol-1-yl)-methyl]biphenyl-2-carbonsäure-tert.butylester
Öl, $R_f$-Wert: 0,40 (Kieselgel: Methyl-ethylketon/Xylol = 5:2)
4′-[(2-n-Butyl-6-(2,2-dimethyl-propionylamino)-benzimidazol-1-yl)-methyl]biphenyl-2-carbonsäure-tert.butylester
Öl, $R_f$-Wert: 0,60 (Kieselgel: Essigester/Petrolether = 9:1)
4′-[(2-n-Butyl-6-cyclopentylcarbonylamino-benzimidazol-1-yl)-methyl]biphenyl-2-carbonsäure-tert.butylester
Öl, $R_f$-Wert: 0,35 (Kieselgel: Essigester/Petrolether = 7:3)
4′-[(2-n-Butyl-6-cyclopropylcarbonylamino-benzimidazol-1-yl)-methyl]biphenyl-2-carbonsäure-tert.butylester
Schmelzpunkt: 175-177°C
4′-[(2-n-Butyl-6-cyclohexyloxycarbonylamino-benzimidazol-1-yl)-methyl]biphenyl-2-carbonsäure-tert.butylester
Schmelzpunkt: 68-70°C (amorph)
4′-[(2-n-Butyl-6-dimethylaminocarbonylamino-benzimidazol-1-yl)-methyl]-2-(1-triphenylmethyl-tetrazol-5-yl)-biphenyl
Öl, $R_f$-Wert: 0,75 (Kieselgel: Essigester/Ethanol/Ammoniak = 90:10:1)
4′-[(2-n-Butyl-6-morpholinocarbonylamino-benzimidazol-1-yl)-methyl]biphenyl-2-carbonsäure-tert.butylester
Schmelzpunkt: 74-76°C
4′-[(2-n-Butyl-6-pyrrolidinocarbonylamino-benzimidazol-1-yl)-methyl]biphenyl-2-carbonsäure-tert.butylester
4′-[(2-n-Butyl-6-dimethylaminocarbonyloxy-benzimidazol-1-yl)-methyl]biphenyl-2-carbonsäure-tert.butylester
Schmelzpunkt: 137-138°C
4′-[(2-n-Butyl-6-(N-acetyl-n-octylamino)-benzimidazol-1-yl)-methyl]biphenyl-2-carbonsäure-tert.butylester
Öl, $R_f$-Wert: 0,25 (Kieselgel: Essigester/Petrolether = 7:3)

## Beispiel 9

### 4′-[(2-Hydroxy-benzimidazol-1-yl)-methyl]biphenyl-2-carbonsäure

0,9 g (2,25 mMol) 4′-[(2-Hydroxy-benzimidazol-1-yl)-methyl]biphenyl-2-carbonsäure-tert.butylester werden in 10 ml Methylenchlorid gelöst und mit 10 ml Trifluoressigsäure versetzt. Die Lösung wird 2 Stunden bei Raumtemperatur gerührt und anschließend am Rotationsverdampfer zur Trockene eingeengt. Der ölige Rückstand wird in 50 ml Methylenchlorid gelöst und zweimal mit Wasser ausgeschüttelt. Die organische Phase wird mit Magnesiumsulfat getrocknet und zur Trockene eingeengt. Der so erhaltene kristalline Rückstand wird mit wenig Diethylether verrieben, abgesaugt und im Vakuum bei 50°C getrocknet.
Ausbeute: 0,75 g (97,4 % der Theorie),

Schmelzpunkt: 303-304°C

| $C_{21}H_{16}N_2O_3$ (344,37) | | | | | | |
|---|---|---|---|---|---|---|
| Ber.: | C | 73,24 | H | 4,68 | N | 8,13 |
| Gef.: | | 73,07 | | 4,81 | | 7,95 |

Analog werden folgende Verbindungen erhalten:
4′-[(2,5-Di-n-butyl-benzimidazol-1-yl)-methyl]biphenyl-2-carbonsäure
Schmelzpunkt: 199-201°C
4′-[(2,6-Di-n-butyl-benzimidazol-1-yl)-methyl]biphenyl-2-carbonsäure
Schmelzpunkt: 188-190°C

Beispiel 10

4′-[(2-n-Butyl-6-methoxy-benzimidazol-1-yl)-methyl]biphenyl-2-carbonsäure

Hergestellt analog Beispiel 9 aus 4′-[(2-n-Butyl-6-methoxy-benzimidazol-1-yl)-methyl]biphenyl-2-carbonsäure-tert.butylester und Trifluoressigsäure.
Ausbeute: 73,9 % der Theorie,
Öl, $R_f$-Wert: 0,6 (Kieselgel: Essigester/Ethanol/Ammoniak = 50:45:5)

| $C_{26}H_{26}N_2O_3$ (414,51) | | | | | | |
|---|---|---|---|---|---|---|
| Ber.: | C | 75,34 | H | 6,23 | N | 6,76 |
| Gef.: | | 75,27 | | 6,03 | | 6,52 |

Beispiel 11

4′-[(2-n-Butyl-4-methyl-7-(2-diethylamino-ethoxy)-benzimidazol-1-yl)-methyl]biphenyl-2-carbonsäure-dihydrat

Hergestellt analog Beispiel 9 aus 4′-[(2-n-Butyl-4-methyl-7-(2-diethylamino-ethoxy)-benzimidazol-1-yl)-methyl]biphenyl-2-carbonsäure-tert.butylester und Trifluoressigsäure.
Ausbeute: 94,2 % der Theorie,
Schmelzpunkt: 94-96°C

| $C_{32}H_{39}N_3O_3$ x 2 $H_2O$ (549,72) | | | | | | |
|---|---|---|---|---|---|---|
| Ber.: | C | 69,92 | H | 7,88 | N | 7,64 |
| Gef.: | | 69,62 | | 7,60 | | 7,40 |

Beispiel 12

4′-[(2-Ethylthio-benzimidazol-1-yl)-methyl]biphenyl-2-carbonsäure

Hergestellt analog Beispiel 9 aus 4′-[(2-Ethylthio-benzimidazol-1-yl)-methyl]biphenyl-2-carbonsäure-tert.butylester und Trifluoressigsäure.
Ausbeute: 92,9 % der Theorie,

Schmelzpunkt: 197-198°C

| $C_{23}H_{20}N_2O_2S$ (388,48) | | | | | | | | |
|---|---|---|---|---|---|---|---|---|
| Ber.: | C | 71,11 | H | 5,19 | N | 7,21 | S | 8,25 |
| Gef.: | | 71,12 | | 5,13 | | 7,23 | | 8,31 |

Beispiel 13

4′-[(2-n-Propylthiomethyl-benzimidazol-1-yl)-methyl]biphenyl-2-carbonsäure

Hergestellt analog Beispiel 9 aus 4′-[(2-n-Propylthiomethyl-benzimidazol-1-yl)-methyl]biphenyl-2-carbonsäure-tert.butylester und Trifluoressigsäure.
Ausbeute: 96,8 % der Theorie,
Schmelzpunkt: 139-141°C

| $C_{25}H_{24}N_2O_2S$ (416,54) | | | | | | | | |
|---|---|---|---|---|---|---|---|---|
| Ber.: | C | 72,09 | H | 5,81 | N | 6,73 | S | 7,70 |
| Gef.: | | 71,82 | | 5,83 | | 6,57 | | 7,43 |

Beispiel 14

4′-[(2-Methyl-benzimidazol-1-yl)-methyl]biphenyl-2-carbonsäure x 0,33 HCl

Hergestellt analog Beispiel 9 aus 4′-[(2-Methyl-benzimidazol-1-yl)-methyl]biphenyl-2-carbonsäure-tert.butylester und Trifluoressigsäure.
Ausbeute: 93,2 % der Theorie,
Schmelzpunkt: 255-256°C

| $C_{22}H_{18}N_2O_2$ x 0,33 HCl (354,54) | | | | | | | | |
|---|---|---|---|---|---|---|---|---|
| Ber.: | C | 74,53 | H | 5,21 | N | 7,90 | Cl | 3,32 |
| Gef.: | | 74,60 | | 5,14 | | 8,16 | | 3,40 |

Beispiel 15

4′-[(2-Methylmercapto-benzimidazol-1-yl)-methyl]biphenyl-2-carbonsäure

Hergestellt analog Beispiel 9 aus 4′-[(2-Methylmercapto-benzimidazol-1-yl)-methyl]biphenyl-2-carbonsäure-tert.butyl-ester und Trifluoressigsäure.
Ausbeute: 88,2 % der Theorie,

Schmelzpunkt: 197-199°C

| $C_{22}H_{18}N_2O_2S$ (374,46) | | | | | | | | |
|---|---|---|---|---|---|---|---|---|
| Ber.: | C | 70,57 | H | 4,84 | N | 7,48 | S | 8,56 |
| Gef.: | | 70,30 | | 4,87 | | 7,25 | | 8,25 |

Beispiel 16

4'-[(2-Methyl-5- und 6-nitro-benzimidazol-1-yl)-methyl]biphenyl-2-carbonsäure

Hergestellt analog Beispiel 9 aus 4'-[(2-Methyl-5- und 6-nitro-benzimidazol-1-yl)-methyl]biphenyl-2-carbonsäure-tert.butylester und Trifluoressigsäure.
Ausbeute: 71,1 % der Theorie,
Schmelzpunkt: 285-288°C

| $C_{22}H_{17}N_3O_4$ (387,39) | | | | | | |
|---|---|---|---|---|---|---|
| Ber.: | C | 68,21 | H | 4,42 | N | 10,85 |
| Gef.: | | 67,96 | | 4,40 | | 10,83 |

Beispiel 17

4'-[(2-Methyl-5- und 6-butanoylamino-benzimidazol-1-yl)-methyl]biphenyl-2-carbonsäure

Hergestellt analog Beispiel 9 aus 4'-[(2-Methyl-5- und 6-butanoylamino-benzimidazol-1-yl)-methyl]biphenyl-2-carbonsäure-tert.butylester und Trifluoressigsäure.
Ausbeute: 79,5 % der Theorie,
Schmelzpunkt: 261-262°C

| $C_{26}H_{25}N_3O_3$ (427,50) | | | | | | |
|---|---|---|---|---|---|---|
| Ber.: | C | 73,05 | H | 5,89 | N | 9,83 |
| Gef.: | | 72,85 | | 5,90 | | 9,80 |

Beispiel 18

4'-[(2-(2-Methylpropyl)-benzimidazol-1-yl)-methyl]biphenyl-2-carbonsäure

Hergestellt analog Beispiel 9 aus 4'-[(2-(2-Methylpropyl)-benzimidazol-1-yl)-methyl]biphenyl-2-carbonsäure-tert.butylester und Trifluoressigsäure.
Ausbeute: 71,9 % der Theorie,

Schmelzpunkt: 211-212°C

| $C_{25}H_{24}N_2O_2$ (384,48) | | | | | | |
|---|---|---|---|---|---|---|
| Ber.: | C | 78,10 | H | 6,29 | N | 7,29 |
| Gef.: | | 77,95 | | 6,22 | | 7,15 |

Beispiel 19

4'-[(2-Methoxymethyl-benzimidazol-1-yl)-methyl]biphenyl-2-carbonsäure

Hergestellt analog Beispiel 9 aus 4'-[(2-Methoxymethyl-benzimidazol-1-yl)-methyl]biphenyl-2-carbonsäure-tert.butylester und Trifluoressigsäure.
Ausbeute: 80,3 % der Theorie,
Schmelzpunkt: 195-197°C

| $C_{23}H_{20}N_2O_3$ (372,43) | | | | | | |
|---|---|---|---|---|---|---|
| Ber.: | C | 74,18 | H | 5,41 | N | 7,52 |
| Gef.: | | 73,99 | | 5,39 | | 7,43 |

Beispiel 20

4'-[(2-(2-Pyridyl)-benzimidazol-1-yl)-methyl]biphenyl-2-carbonsäure

Hergestellt analog Beispiel 9 aus 4'-[(2-(2-Pyridyl)-benzimidazol-1-yl)-methyl]biphenyl-2-carbonsäure-tert.butylester und Trifluoressigsäure.
Ausbeute: 50,0 % der Theorie,
Schmelzpunkt: 262-264°C

| $C_{26}H_{19}N_3O_2$ (405,45) | | | | | | |
|---|---|---|---|---|---|---|
| Ber.: | C | 77,02 | H | 4,72 | N | 10,36 |
| Gef.: | | 77,21 | | 4,58 | | 10,20 |

Beispiel 21

4'-[(5- und 6-Methyl-benzimidazol-1-yl)-methyl]biphenyl-2-carbonsäure

Hergestellt analog Beispiel 9 aus 4'-[(5- und 6-Methyl-benzimidazol-1-yl)-methyl]biphenyl-2-carbonsäure-tert.butylester und Trifluoressigsäure.
Ausbeute: 92,0 % der Theorie,

Schmelzpunkt: 228-230°C

| $C_{22}H_{18}N_2O_2$ (342,396) | | | | | | |
|---|---|---|---|---|---|---|
| Ber.: | C | 77,17 | H | 5,30 | N | 8,18 |
| Gef.: | | 76,94 | | 5,23 | | 7,93 |

Beispiel 22

4′-[(2-Phenyl-benzimidazol-1-yl)-methyl]biphenyl-2-carbonsäure

Hergestellt analog Beispiel 9 aus 4′-[(2-Phenyl-benzimidazol-1-yl)-methyl]biphenyl-2-carbonsäure-tert.butylester und Trifluoressigsäure.
Ausbeute: 56,8 % der Theorie,
Schmelzpunkt: 275-277°C

| $C_{27}H_{20}N_2O_2$ (404,47) | | | | | | |
|---|---|---|---|---|---|---|
| Ber.: | C | 80,18 | H | 4,98 | N | 6,93 |
| Gef.: | | 79,90 | | 5,05 | | 6,92 |

Beispiel 23

4′-[(2-(Thiazol-4-yl)-benzimidazol-1-yl)-methyl]biphenyl-2-carbonsäure

Hergestellt analog Beispiel 9 aus 4′-[(2-(Thiazol-4-yl)-benzimidazol-1-yl)-methyl]biphenyl-2-carbonsäure-tert.butylester und Trifluoressigsäure.
Ausbeute: 94,6 % der Theorie,
Schmelzpunkt: 284-286°C

| $C_{24}H_{17}N_3O_2S$ (411,48) | | | | | | | | |
|---|---|---|---|---|---|---|---|---|
| Ber.: | C | 70,06 | H | 4,16 | N | 10,21 | S | 7,79 |
| Gef.: | | 69,90 | | 4,29 | | 9,97 | | 7,59 |

Beispiel 24

4′-[(2-(3,5-Dimethyl-pyrazol-1-yl)-benzimidazol-1-yl)-methyl]biphenyl-2-carbonsäure

Hergestellt analog Beispiel 9 aus 4′-[(2-(3,5-Dimethyl-pyrazol-1-yl)-benzimidazol-1-yl)-methyl]biphenyl-2-carbonsäure-tert.-butylester und Trifluoressigsäure.
Ausbeute: 87,2 % der Theorie,

Schmelzpunkt: 185-187°C

| $C_{26}H_{22}N_4O_2$ (422,49) | | | | | | |
|---|---|---|---|---|---|---|
| Ber.: | C | 73,92 | H | 5,25 | N | 13,26 |
| Gef.: | | 73,86 | | 5,37 | | 13,27 |

Beispiel 25

4′-[(2-(Furyl-(2))-benzimidazol-1-yl)-methyl]biphenyl-2-carbonsäure

Hergestellt analog Beispiel 9 aus 4′-[(2-(Furyl-(2))-benzimidazol-1-yl)-methyl]biphenyl-2-carbonsäure-tert.butylester und Trifluoressigsäure.
Ausbeute: 78,4 % der Theorie,
Schmelzpunkt: 263-265°C

| $C_{25}H_{18}N_2O_3$ (394,43) | | | | | | |
|---|---|---|---|---|---|---|
| Ber.: | C | 76,13 | H | 4,60 | N | 7,10 |
| Gef.: | | 75,94 | | 4,64 | | 6,83 |

Beispiel 26

4′-[(2-Aminocarbonylamino-benzimidazol-1-yl)-methyl]biphenyl-2-carbonsäure-monohydrat

Hergestellt analog Beispiel 9 aus 4′-[(2-Aminocarbonylamino-benzimidazol-1-yl)-methyl]biphenyl-2-carbonsäure-tert.butylester und Trifluoressigsäure.
Ausbeute: 53,3 % der Theorie,
Schmelzpunkt: 214-216°C

| $C_{22}H_{18}N_4O_3$ x $H_2O$ (404,42) | | | | | | |
|---|---|---|---|---|---|---|
| Ber.: | C | 65,34 | H | 4,98 | N | 13,85 |
| Gef.: | | 65,18 | | 5,05 | | 13,61 |

Beispiel 27

4′-[(2-Isopropyl-benzimidazol-1-yl)-methyl]biphenyl-2-carbonsäure

Hergestellt analog Beispiel 9 aus 4′-[(2-Isopropyl-benzimidazol-1-yl)-methyl]biphenyl-2-carbonsäure-tert.butylester und Trifluoressigsäure.
Ausbeute: 80 % der Theorie,

Schmelzpunkt: 206-208°C

| $C_{24}H_{22}N_2O_2$ (370,46) | | | | | | |
|---|---|---|---|---|---|---|
| Ber.: | C | 77,81 | H | 5,99 | N | 7,56 |
| Gef.: | | 77,55 | | 5,97 | | 7,43 |

Beispiel 28

4′-[(7-Benzyloxy-2-n-butyl-4-methyl-benzimidazol-1-yl)-methyl]biphenyl-2-carbonsäure-trifluoracetat

Hergestellt analog Beispiel 9 aus 4′-[(7-Benzyloxy-2-n-butyl-4-methyl-benzimidazol-1-yl)-methyl]biphenyl-2-carbonsäure-tert.butylester und Trifluoressigsäure.
Ausbeute: 85,5 % der Theorie,
Schmelzpunkt: 217-219°C

| $C_{33}H_{32}N_2O_3$ x $CF_3COOH$ (618,66) | | | | | | |
|---|---|---|---|---|---|---|
| Ber.: | C | 67,95 | H | 5,37 | N | 4,52 |
| Gef.: | | 68,19 | | 5,56 | | 4,74 |

Beispiel 29

4′-[(2-Hydroxymethyl-benzimidazol-1-yl)-methyl]biphenyl-2-carbonsäure-trifluoracetat

Hergestellt analog Beispiel 9 aus 4′-[(2-Hydroxymethyl-benzimidazol-1-yl)-methyl]biphenyl-2-carbonsäure-tert.butylester und Trifluoressigsäure.
Ausbeute: 81,9 % der Theorie,
Schmelzpunkt: 188-190°C

| $C_{22}H_{18}N_2O_3$ x $CF_3COOH$ (472,42) | | | | | | |
|---|---|---|---|---|---|---|
| Ber.: | C | 61,02 | H | 4,05 | N | 5,93 |
| Gef.: | | 61,23 | | 4,08 | | 5,91 |

Beispiel 30

4′-[(2-(3-Hydroxypropyl-benzimidazol-1-yl)-methyl]biphenyl-2-carbonsäure-trifluoracetat

Hergestellt analog Beispiel 9 aus 4′-[(2-(3-Hydroxypropyl-benzimidazol-1-yl)-methyl]biphenyl-2-carbonsäure-tert.butylester und Trifluoressigsäure.
Ausbeute: 58,8 % der Theorie,

Schmelzpunkt: 150-152°C

| $C_{24}H_{22}N_2O_3$ x $CF_3COOH$ (500,47) | | | | | | |
|---|---|---|---|---|---|---|
| Ber.: | C | 62,40 | H | 4,63 | N | 5,60 |
| Gef.: | | 62,13 | | 4,70 | | 5,83 |

Beispiel 31

4′-[(2-Methyl-5- und 6-(N-(2-methoxy-acetyl)-n-butylamino)-benzimidazol-1-yl)-methyl]biphenyl-2-carbonsäure

Hergestellt analog Beispiel 9 aus 4′-[(2-Methyl-5- und 6-(N-(2-methoxy-acetyl)-n-butylamino)-benzimidazol-1-yl)-methyl]biphenyl-2-carbonsäure-tert.butylester und Trifluoressigsäure.
Ausbeute: 84,8 % der Theorie,
Schmelzpunkt: 186-188°C

| $C_{29}H_{31}N_3O_4$ (485,58) | | | | | | |
|---|---|---|---|---|---|---|
| Ber.: | C | 71,73 | H | 6,43 | N | 8,65 |
| Gef.: | | 72,67 | | 6,68 | | 8,74 |

Beispiel 32

4′-[(2-(1-Methylpropyl)-benzimidazol-1-yl)-methyl]biphenyl-2-carbonsäure

Hergestellt analog Beispiel 9 aus 4′-[(2-(1-Methylpropyl)-benzimidazol-1-yl)-methyl]biphenyl-2-tert.butylester und Trifluoressigsäure.
Ausbeute: 80 % der Theorie,
Schmelzpunkt: 147-148°C

| $C_{25}H_{24}N_2O_2$ (384,48) | | | | | | |
|---|---|---|---|---|---|---|
| Ber.: | C | 78,10 | H | 6,29 | N | 7,29 |
| Gef.: | | 77,91 | | 6,23 | | 7,37 |

Beispiel 33

4′-[(2-(2-Methylbutyl)-benzimidazol-1-yl)-methyl]biphenyl-2-carbonsäure

Hergestellt analog Beispiel 9 aus 4′-[(2-(2-Methylbutyl)-benzimidazol-1-yl)-methyl]biphenyl-2-carbonsäure-tert.butylester und Trifluoressigsäure.
Ausbeute: 60 % der Theorie,

Schmelzpunkt: 209-210°C

| $C_{26}H_{28}N_2O_2$ (398,51) | | | | | | |
|---|---|---|---|---|---|---|
| Ber.: | C | 78,36 | H | 6,58 | N | 7,03 |
| Gef.: | | 78,27 | | 6,51 | | 6,99 |

Beispiel 34

4′-[(2-Methyl-5- und 6-(N-(2-methoxy-ethyl)-n-butylamino)-benzimidazol-1-yl)-methyl]biphenyl-2-carbonsäure

Hergestellt analog Beispiel 9 aus 4′-[(2-Methyl-5- und 6-(N-(2-methoxy-ethyl)-n-butylamino)-benzimidazol-1-yl)-methyl]biphenyl-2-carbonsäure-tert.butylester und Trifluoressigsäure.
Ausbeute: 46,5% der Theorie,
Schmelzpunkt: 102-106°C

| $C_{29}H_{33}N_3O_3$ (471,598) | | | | | | |
|---|---|---|---|---|---|---|
| Ber.: | C | 73,86 | H | 7,05 | N | 8,91 |
| Gef.: | | 73,60 | | 7,13 | | 8,85 |

Beispiel 35

4′-[(2-n-Pentyl-benzimidazol-1-yl)-methyl]biphenyl-2-carbonsäure

Hergestellt analog Beispiel 9 aus 4′-[(2-n-Pentyl-benzimidazol-1-yl)-methyl]biphenyl-2-carbonsäure-tert.butylester und Trifluoressigsäure.
Ausbeute: 87 % der Theorie,
Schmelzpunkt: 181-183°C

| $C_{26}H_{26}N_2O_2$ (398,51) | | | | | | |
|---|---|---|---|---|---|---|
| Ber.: | C | 78,36 | H | 6,58 | N | 7,03 |
| Gef.: | | 78,12 | | 6,42 | | 7,09 |

Beispiel 36

4′-[(Benzimidazol-1-yl)-methyl]biphenyl-2-carbonsäure

Hergestellt analog Beispiel 9 aus 4′-[(Benzimidazol-1-yl)-methyl]biphenyl-2-carbonsäure-tert.butylester und Trifluoressigsäure.
Ausbeute: 90,9 % der Theorie,

Schmelzpunkt: 217-219°C

| $C_{21}H_{10}N_2O_2$ (328,37) | | | | | | |
|---|---|---|---|---|---|---|
| Ber.: | C | 76,81 | H | 4,91 | N | 8,53 |
| Gef.: | | 77,03 | | 5,00 | | 8,42 |

Beispiel 37

4′-[(2-n-Butyl-4-methyl-7-methoxy-benzimidazol-1-yl)-methyl]biphenyl-2-carbonsäure

Hergestellt analog Beispiel 9 aus 4′-[(2-n-Butyl-4-methyl-7-methoxy-benzimidazol-1-yl)-methyl]biphenyl-2-carbonsäure-tert.butylester und Trifluoressigsäure in Methylenchlorid.
Ausbeute: 86,6 % der Theorie,
Schmelzpunkt: 216-218°C

| $C_{27}H_{28}N_2O_3$ (428,54) | | | | | | |
|---|---|---|---|---|---|---|
| Ber.: | C | 75,68 | H | 6,59 | N | 6,54 |
| Gef.: | | 75,48 | | 6,59 | | 6,45 |

Beispiel 38

4′-[(2-n-Propyl-benzimidazol-1-yl)-methyl]biphenyl-2-carbonsäure

Hergestellt analog Beispiel 9 aus 4′-[(2-n-Propyl-benzimidazol-1-yl)-methyl]biphenyl-2-carbonsäure-tert.butylester und Trifluoressigsäure.
Ausbeute: 85,2 % der Theorie,
Schmelzpunkt: 237-238°C

| $C_{24}H_{22}N_2O_2$ (370,45) | | | | | | |
|---|---|---|---|---|---|---|
| Ber.: | C | 77,81 | H | 5,99 | N | 7,59 |
| Gef.: | | 78,08 | | 5,74 | | 7,37 |

Beispiel 39

4′-[(2-Ethyl-benzimidazol-1-yl)-methyl]biphenyl-2-carbonsäure

Hergestellt analog Beispiel 9 aus 4′-[(2-Ethyl-benzimidazol-1-yl)-methyl]biphenyl-2-carbonsäure-tert.butylester und Trifluoressigsäure.
Ausbeute: 81,6 % der Theorie,

Schmelzpunkt: 251-253°C

| $C_{23}H_{20}N_2O_2$ (356,42) | | | | | | |
|---|---|---|---|---|---|---|
| Ber.: | C | 77,51 | H | 5,66 | N | 7,86 |
| Gef.: | | 77,72 | | 5,64 | | 7,59 |

Beispiel 40

4'-[(2-Ethylthiomethyl-benzimidazol-1-yl)-methyl]biphenyl-2-carbonsäure-semitrifluoracetat

Hergestellt analog Beispiel 9 aus 4'-[(2-Ethylthiomethyl-benzimidazol-1-yl)-methyl]biphenyl-2-carbonsäure-tert.butylester und Trifluoressigsäure.
Ausbeute: 96,1 % der Theorie,
Schmelzpunkt: 139-141°C

| $C_{24}H_{22}N_2O_2S$ x 1/2 $CF_3COOH$ (459,52) | | | | | | | | |
|---|---|---|---|---|---|---|---|---|
| Ber.: | C | 65,35 | H | 4,94 | N | 6,10 | S | 6,98 |
| Gef.: | | 65,24 | | 5,00 | | 6,18 | | 6,98 |

Beispiel 41

4'-[(2-Methylthiomethyl-benzimidazol-1-yl)-methyl]biphenyl-2-carbonsäure-semitrifluoracetat

Hergestellt analog Beispiel 9 aus 4'-[(2-Methylthiomethyl-benzimidazol-1-yl)-methyl]biphenyl-2-carbonsäure-tert.butylester und Trifluoressigsäure.
Ausbeute: 98,6 % der Theorie,
Schmelzpunkt: 147-149°C

| $C_{23}H_{20}N_2O_2S$ x 1/2 $CF_3COOH$ (445,495) | | | | | | | | |
|---|---|---|---|---|---|---|---|---|
| Ber.: | C | 64,71 | H | 4,64 | N | 6,29 | S | 7,20 |
| Gef.: | | 64,70 | | 5,04 | | 6,51 | | 6,91 |

Beispiel 42

4'-[(2-Chlor-benzimidazol-1-yl)-methyl]biphenyl-2-carbonsäure

Hergestellt analog Beispiel 9 aus 4'-[(2-Chlor-benzimidazol-yl-1)-methyl]biphenyl-2-carbonsäure-tert.butylester und Trifluoressigsäure.
Ausbeute: 84,0 % der Theorie,

Schmelzpunkt: 169-171°C

| $C_{21}H_{15}ClN_2O_2$ (362,815) | | | | | | | | |
|---|---|---|---|---|---|---|---|---|
| Ber.: | C | 69,52 | H | 4,17 | N | 7,72 | Cl | 9,77 |
| Gef.: | | 69,39 | | 4,13 | | 7,66 | | 9,72 |

Beispiel 43

4′-[(2-n-Butylthio-benzimidazol-1-yl)-methyl]biphenyl-2-carbonsäure

Hergestellt analog Beispiel 9 aus 4′-[(2-n-Butylthio-benzimidazol-1-yl)-methyl]biphenyl-2-carbonsäure-tert.butylester und Trifluoressigsäure.
Ausbeute: 88,0 % der Theorie,
Schmelzpunkt: 160-162°C

| $C_{25}H_{24}N_2O_2S$ (416,54) | | | | | | | | |
|---|---|---|---|---|---|---|---|---|
| Ber.: | C | 72,09 | H | 5,81 | N | 6,73 | S | 7,70 |
| Gef.: | | 71,93 | | 5,75 | | 6,74 | | 7,71 |

Beispiel 44

4′-[(2-n-Butyl-5-acetamino-benzimidazol-1-yl)-methyl]biphenyl-2-carbonsäure-hydrat

Hergestellt analog Beispiel 9 aus 4′-[(2-n-Butyl-5-acetamino-benzimidazol-1-yl)-methyl]biphenyl-2-carbonsäure-tert.butylester und Trifluoressigsäure.
Ausbeute: 71,3 % der Theorie,
Schmelzpunkt: 187-189°C

| $C_{27}H_{27}N_3O_3$ x $H_2O$ (459,54) | | | | | | |
|---|---|---|---|---|---|---|
| Ber.: | C | 70,56 | H | 6,36 | N | 9,14 |
| Gef.: | | 70,40 | | 6,22 | | 9,08 |

Beispiel 45

4′-[(2-(4-Methoxyphenyl)-benzimidazol-1-yl)-methyl]biphenyl-2-carbonsäure

Hergestellt analog Beispiel 9 aus 4′-[(2-(4-Methoxyphenyl)-benzimidazol-1-yl)-methyl]biphenyl-2-carbonsäure-tert.butylester und Trifluoressigsäure.
Ausbeute: 87,5 % der Theorie,

Schmelzpunkt: 283-286°C

| $C_{28}H_{22}N_2O_3$ (434,50) | | | | | | |
|---|---|---|---|---|---|---|
| Ber.: | C | 77,40 | H | 5,10 | N | 6,45 |
| Gef.: | | 77,45 | | 5,20 | | 6,44 |

Beispiel 46

4′-[(2-n-Propylthio-benzimidazol-1-yl)-methyl]biphenyl-2-carbonsäure

Hergestellt analog Beispiel 9 aus 4′-[(2-n-Propylthio-benzimidazol-1-yl)-methyl]biphenyl-2-carbonsäure-tert.butylester und Trifluoressigsäure.
Ausbeute: 90,5 % der Theorie,
Schmelzpunkt: 219-220°C

| $C_{24}H_{22}N_2O_2S$ (402,51) | | | | | | | | |
|---|---|---|---|---|---|---|---|---|
| Ber.: | C | 71,62 | H | 5,51 | N | 6,96 | S | 7,97 |
| Gef.: | | 71,47 | | 5,51 | | 6,75 | | 8,09 |

Beispiel 47

4′-[(2-n-Butylamino-benzimidazol-1-yl)-methyl]biphenyl-2-carbonsäure-semitrifluoracetat

Hergestellt analog Beispiel 9 aus 4′-[(2-n-Butylamino-benzimidazol-1-yl)-methyl]biphenyl-2-carbonsäure-tert.butylester und Trifluoressigsäure.
Ausbeute: 80,0 % der Theorie,
Schmelzpunkt: 247-249°C

| $C_{25}H_{25}N_3O_2$ x 1/2 $CF_3COOH$ (456,50) | | | | | | |
|---|---|---|---|---|---|---|
| Ber.: | C | 68,41 | H | 5,63 | N | 9,20 |
| Gef.: | | 68,56 | | 5,84 | | 9,07 |

Beispiel 48

4′-[(2-(4-Methoxy-phenyl)-5- und 6-chlor-benzimidazol-1-yl)-methyl]biphenyl-2-carbonsäure

Hergestellt analog Beispiel 9 aus 4′-[(2-(4-Methoxy-phenyl)-5- und 6-chlor-benzimidazol-1-yl)-methyl]biphenyl-2-carbonsäure-2-tert.butylester und Trifluoressigsäure.
Ausbeute: 76,3 % der Theorie,

Schmelzpunkt: 234-236°C

| $C_{28}H_{21}ClN_2O_3$ (468,95) | | | | | | | | |
|---|---|---|---|---|---|---|---|---|
| Ber.: | C | 71,71 | H | 4,51 | N | 5,97 | Cl | 7,56 |
| Gef.: | | 71,57 | | 4,39 | | 5,85 | | 7,79 |

Beispiel 49

4'-[(2-n-Butyl-5-methoxy-benzimidazol-1-yl)-methyl]biphenyl-2-carbonsäure

Hergestellt analog Beispiel 9 aus 4'-[(2-n-Butyl-5-methoxy-benzimidazol-1-yl)-methyl]biphenyl-2-carbonsäure-tert.butylester und Trifluoressigsäure.
Ausbeute: 66,2 % der Theorie,
Schmelzpunkt: 203-205°C

| $C_{26}H_{26}N_2O_3$ (414,51) | | | | | | |
|---|---|---|---|---|---|---|
| Ber.: | C | 75,34 | H | 6,32 | N | 6,76 |
| Gef.: | | 75,19 | | 6,31 | | 6,61 |

Beispiel 50

4'-[(2-n-Butyl-5- und 6-acetamino-benzimidazol-1-yl)-methyl]biphenyl-2-carbonsäure

Hergestellt analog Beispiel 9 aus 4'-[(2-n-Butyl-5- und 6-acetamino-benzimidazol-1-yl)-methyl]biphenyl-2-carbonsäure-tert.butylester und Trifluoressigsäure.
Ausbeute: 83,7 % der Theorie,
Schmelzpunkt: 117-119°C

| $C_{27}H_{27}N_3O_3$ (441,54) | | | | | | |
|---|---|---|---|---|---|---|
| Ber.: | C | 73,45 | H | 6,16 | N | 9,52 |
| Gef.: | | 73,25 | | 6,23 | | 9,47 |

Beispiel 51

4'-[(2-n-Butyl-5- und 6-butanoylamino-benzimidazol-1-yl)-methyl]biphenyl-2-carbonsäure

Hergestellt analog Beispiel 9 aus 4'-[(2-n-Butyl-5- und 6-butanoylamino-benzimidazol-1-yl)-methyl]biphenyl-2-carbonsäure-tert.butylester und Trifluoressigsäure.
Ausbeute: 80,0 % der Theorie,

Schmelzpunkt: 123-127°C

| $C_{29}H_{31}N_3O_3$ (469,59) | | | | | | |
|---|---|---|---|---|---|---|
| Ber.: | C | 74,18 | H | 6,65 | N | 8,95 |
| Gef.: | | 73,96 | | 6,19 | | 8,99 |

Beispiel 52

4′-[(6-(N-Benzyl-methylamino)-2-n-butyl-benzimidazol-1-yl)-methyl]biphenyl-2-carbonsäure

Hergestellt analog Beispiel 9 aus 4′-[(6-(N-Benzyl-methylamino)-2-n-butyl-benzimidazol-1-yl)-methyl]biphenyl-2-carbonsäure-tert.butylester und Trifluoressigsäure.
Ausbeute: 82,0 % der Theorie,
Schmelzpunkt: 237-238°C

| $C_{33}H_{33}N_3O_2$ (503,64) | | | | | | |
|---|---|---|---|---|---|---|
| Ber.: | C | 78,70 | H | 6,60 | N | 8,34 |
| Gef.: | | 78,68 | | 6,71 | | 8,44 |

Beispiel 53

4′-[(2-n-Butyl-5-chlor-benzimidazol-1-yl)-methyl]biphenyl-2-carbonsäure

Hergestellt analog Beispiel 9 aus 4′-[(2-n-Butyl-5-chlor-benzimidazol-1-yl)-methyl]biphenyl-2-carbonsäure-tert.butylester und Trifluoressigsäure.
Ausbeute: 70,6 % der Theorie,
Schmelzpunkt: 191-193°C

| $C_{25}H_{23}ClN_2O_2$ (418,92) | | | | | | | | |
|---|---|---|---|---|---|---|---|---|
| Ber.: | C | 71,68 | H | 5,53 | N | 6,69 | Cl | 8,46 |
| Gef.: | | 71,48 | | 5,40 | | 6,53 | | 8,43 |

Beispiel 54

4′-[(2-n-Butyl-5- und 6-methoxy-benzimidazol-1-yl)-methyl]biphenyl-2-carbonsäure

Hergestellt analog Beispiel 9 aus 4′-[(2-n-Butyl-5- und 6-methoxy-benzimidazol-1-yl)-methyl]biphenyl-2-carbonsäure-tert.butylester und Trifluoressigsäure.
Ausbeute: 84,1 % der Theorie,

Schmelzpunkt: 128-133°C

| $C_{26}H_{26}N_2O_3$ (414,51) | | | | | | |
|---|---|---|---|---|---|---|
| Ber.: | C | 75,34 | H | 6,32 | N | 6,76 |
| Gef.: | | 75,32 | | 6,14 | | 6,75 |

Beispiel 55

4'-[(2-n-Butyl-7-n-butoxy-4-methyl-benzimidazol-1-yl)-methyl]biphenyl-2-carbonsäure-trifluoracetat

Hergestellt analog Beispiel 9 aus 4'-[(2-n-Butyl-7-n-butoxy-4-methyl-benzimidazol-1-yl)-methyl]biphenyl-2-carbonsäure-tert.butylester in Methylenchlorid.
Ausbeute: 63,3 % der Theorie,
Schmelzpunkt: 172-173°C

| $C_{30}H_{34}N_2O_3$ x $CF_3COOH$ (584,65) | | | | | | |
|---|---|---|---|---|---|---|
| Ber.: | C | 65,74 | H | 6,03 | N | 4,73 |
| Gef.: | | 66,52 | | 6,15 | | 4,95 |

Beispiel 56

2-n-Butyl-1-[(2-carboxy-biphenyl-4'-yl)methyl]-6,7-dihydro-7,7-dimethyl-5-ethyl-5H-pyrrolo[2,3-f]benzimidazol-6-on-semihydrat

5 g (10,3 mMol) 6-Amino-5-[(2-tert.butoxycarbonyl-biphenyl-4'-yl)-methyl]-amino-3,4-dimethyl-1-ethyl-indol-2-on und 5 ml Valeriansäure werden 4 Stunden am Rückfluß gekocht. Nach dem Abkühlen wird in 50 ml gesättigte wässrige Natriumcarbonatlösung eingerührt. Es wird 3 mal mit je 30 ml Methylenchlorid ausgeschüttelt. Die Methylenchloridphase wird mit Natriumsulfat getrocknet und im Vakuum eingeengt. Das Rohprodukt wird mit einer Kieselgelsäule (Elutionsmittel: Essigester/Ethanol/Ammoniak = 90:10:1) gereinigt.
Ausbeute: 1,55 g (30,3 % der Theorie),
Schmelzpunkt: 185-187°C

| $C_{31}H_{33}N_3O_3$ x 1/2 $H_2O$ (504,64) | | | | | | |
|---|---|---|---|---|---|---|
| Ber.: | C | 73,81 | H | 6,79 | N | 8,33 |
| Gef.: | | 73,91 | | 6,86 | | 8,36 |

Beispiel 57

4'-[(2-n-Butyl-7-hydroxy-4-methyl-benzimidazol-1-yl)-methyl]-2-(1H-tetrazol-5-yl)-biphenyl-hydrat

0,9 g (1,7 mMol) 4'-[(2-n-Butyl-7-benzyloxy-4-methyl-benzimidazol-1-yl)-methyl]-2-(1H-tetrazol-5-yl)-biphenyl, gelöst in 100 ml Methanol, werden unter 5 bar Wasserstoffdruck in Gegenwart von 0,9 g 20%igem Palladiumhydroxid auf Kohle bei Zimmertemperatur hydriert. Anschließend wird der Katalysator abgesaugt und das Filtrat im Vakuum zur Trockene eingeengt. Das Rohprodukt wird aus Aceton/Ether umkristallisiert und im Vakuum bei 50°C getrocknet.
Ausbeute: 0,72 g (97,3 % der Theorie),

Schmelzpunkt: 231-233°C

| $C_{26}H_{26}N_6O$ x $H_2O$ (456,56) | | | | | | |
|---|---|---|---|---|---|---|
| Ber.: | C | 68,40 | H | 6,18 | N | 18,40 |
| Gef.: | | 68,64 | | 6,40 | | 18,55 |

Analog werden folgende Verbindungen erhalten:
4′-[(2-n-Butyl-7-hydroxy-4-methyl-benzimidazol-1-yl)-methyl]-2-trifluoracetamino-biphenyl
Schmelzpunkt: 243-245°C
4′-[(2-n-Butyl-7-hydroxy-4-methyl-benzimidazol-1-yl)-methyl]-2-trifluormethansulfonamino-biphenyl
Schmelzpunkt: 160-162°C
4′-[(2-n-Butyl-7-hydroxy-benzimidazol-1-yl)-methyl]biphenyl-2-carbonsäure-tert.butylester
Öl, $R_f$-Wert: 0,60 (Kieselgel: Methyl-ethylketon/Xylol = 1:1)
4′-[(2-n-Butyl-6-hydroxy-benzimidazol-1-yl)-methyl]biphenyl-2-carbonsäure-tert.butylester
Öl, $R_f$-Wert: 0,55 (Kieselgel: Methyl-ethylketon/Xylol = 1:1)
4′-[(2-n-Butyl-4-hydroxy-benzimidazol-1-yl)-methyl]biphenyl-2-carbonsäure-tert.butylester
Schmelzpunkt: 91-93°C
4′-[(2-n-Butyl-5-hydroxy-benzimidazol-1-yl)-methyl]biphenyl-2-carbonsäure-tert.butylester
Öl, $R_f$-Wert: 0,60 (Kieselgel: Methyl-ethylketon/Xylol = 1:2)
4′-[(2-n-Butyl-7-hydroxy-4-methyl-benzimidazol-1-yl)-methyl]-2-phthalimino-biphenyl
Schmelzpunkt: 224-226°C

## Beispiel 58

### 4′-[(2-n-Butyl-benzimidazol-1-yl)-methyl]-2-(1H-tetrazol-5-yl)-biphenyl

a) 4′-[(2-n-Butyl-benzimidazol-1-yl)-methyl]-2-(1-triphenylmethyl-tetrazol-5-yl)-biphenyl

0,87 g (5 mMol) 2-n-Butyl-benzimidazol werden in 20 ml Dimethylsulfoxid gelöst und unter Rühren mit 0,61 g (5,5 mMol) Kalium-tert.butylat versetzt. Nach 1/2 Stunde versetzt man mit 4′-Brommethyl-2-(1-triphenylmethyl-1H-tetrazol-5-yl)-biphenyl und rührt 3 Stunden bei Zimmertemperatur. Es wird in ca. 50 ml Eiswasser gegossen und 3 mal mit je 30 ml Essigester ausgeschüttelt. Die Essigesterphase wird mit 20 ml Wasser ausgeschüttelt, über Natriumsulfat getrocknet und zur Trockene eingeengt. Das Rohprodukt wird über eine Kieselgelsäule (Korngröße: 0,063-0,2 mm, Elutionsmittel: Methylenchlorid/Ethanol = 100:1) gereinigt.
Ausbeute: 2,1 g (64,6 % der Theorie),
Schmelzpunkt: 85-87°C

| $C_{44}H_{38}N_6$ (650,84) | | | | | | |
|---|---|---|---|---|---|---|
| Ber.: | C | 81,20 | H | 5,88 | N | 12,91 |
| Gef.: | | 80,97 | | 5,90 | | 12,66 |

b) 4′-[(2-n-Butyl-benzimidazol-1-yl)-methyl]-2-(1H-tetrazol-5-yl)-biphenyl

2 g (3 mMol) 4′-[(2-n-Butyl-benzimidazol-1-yl)-methyl]-2-(1-triphenylmethyl-tetrazol-5-yl)-biphenyl werden in einem Gemisch aus 10 ml Methylenchlorid und 10 ml Methanol gelöst, mit 10 ml etherischer Salzsäure versetzt und 3 Stunden bei Zimmertemperatur gerührt. Es wird im Vakuum zur Trockne einrotiert. Der Rückstand wird in Methanol gelöst, mit Ammoniak alkalisch gestellt und erneut einrotiert. Das Rohprodukt wird über eine Kieselgelsäule (Korngröße: 0,063-0,2 mm, Elutionsmittel: Methylenchlorid/Ethanol/Ammoniak = 19:1:0,1 gereinigt. Man kristallisiert aus Ether und trocknet bei 50°C im Vakuum.
Ausbeute: 1,02 g (81,6 % der Theorie),

Schmelzpunkt: 241-243°C

| $C_{25}H_{24}N_6$ (408,52) | | | | | | |
|---|---|---|---|---|---|---|
| Ber.: | C | 73,50 | H | 5,92 | N | 20,57 |
| Gef.: | | 73,34 | | 5,92 | | 20,47 |

Beispiel 59

4'-[(2-n-Butyl-7-benzyloxy-4-methyl-benzimidazol-1-yl)-methyl]-2-(1H-tetrazol-5-yl)-biphenyl-semihydrat

Ein Gemisch aus 3,8 g (7,1 mMol) Ammoniumchlorid, 4,5 g (69 mMol) Natriumazid, 20 ml Dimethylformamid und 2,2 g (4,53 mMol) 4'-[(2-n-Butyl-7-benzyloxy-4-methyl-benzimidazol-1-yl)-methyl]-2-cyano-biphenyl wird 36 Stunden unter Rühren auf 120°C Innentemperatur erhitzt. Man filtriert vom gebildeten Natriumchlorid ab und rotiert das Filtrat im Vakuum ein. Den öligen Rückstand versetzt man mit 50 ml Wasser und stellt unter Kühlung mit konzentrierter Salzsäure auf pH 2. Man filtriert vom öligen Rohprodukt ab, nimmt dieses in 50 ml Methylenchlorid auf und trocknet über Natriumsulfat. Danach reinigt man über eine Kieselgelsäule (Korngröße: 0,063-0,2 mm, Elutionsmittel: Methylenchlorid/Ethanol = 19:1). Die einheitlichen Fraktionen werden im Vakuum zur Trockne eingeengt und der Rückstand bei 50°C getrocknet.
Ausbeute: 1,6 g (68 % der Theorie),
Schmelzpunkt: 112-116°C

| $C_{33}H_{32}N_6O$ x 1/2 $H_2O$ (537,66) | | | | | | |
|---|---|---|---|---|---|---|
| Ber.: | C | 73,73 | H | 6,18 | N | 15,63 |
| Gef.: | | 73,55 | | 6,33 | | 15,91 |

Beispiel 60

4'-[(7-Acetoxy-2-n-butyl-4-methyl-benzimidazol-1-yl)-methyl]biphenyl-2-carbonsäure

0,55 g (1 mMol) 4'-[(2-n-Butyl-7-hydroxy-4-methyl-benzimidazol-1-yl)-methyl]biphenyl-2-carbonsäure-trifluoracetat werden in 20 ml Pyridin gelöst. Dazu werden unter Rühren bei 5°C 0,5 ml (7 mMol) Acetylchlorid zugetropft. Man rührt 1 Stunde bei 5°C und anschließend 2 Stunden bei Zimmertemperatur. Das Pyridin wird am Rotationsverdampfer im Vakuum abdestilliert. Der Rückstand wird mit Wasser verrieben und abgesaugt. Nach dem Waschen mit Wasser wird im Vakuum bei 50°C getrocknet.
Ausbeute: 0,43 g (94 % der Theorie),
Schmelzpunkt: 242-244°C

| $C_{28}H_{28}N_2O_4$ (456,55) | | | | | | |
|---|---|---|---|---|---|---|
| Ber.: | C | 73,66 | H | 6,18 | N | 6,14 |
| Gef.: | | 73,50 | | 6,20 | | 6,36 |

Beispiel 61

4′-[(7-n-Butoxy-2-n-butyl-4-methyl-benzimidazol-1-yl)-methyl]biphenyl-2-carbonsäure-trifluoracetat

a) 4′-[(7-n-Butoxy-2-n-butyl-4-methyl-benzimidazol-1-yl)-methyl]-biphenyl-2-carbonsäure-tert.butylester

Ein Gemisch aus 0,94 g (2 mMol) 4′-[(2-n-Butyl-7-hydroxy-4-methyl-benzimidazol-1-yl)-methyl]biphenyl-2-carbonsäure-tert.butylester, 36 ml Dimethylformamid, 4 ml Wasser, 1,4 g (10 mMol) Kaliumcarbonat und 0,9 g (6,6 mMol) n-Butylbromid wird 16 Stunden bei Zimmertemperatur gerührt. Man gießt auf 200 ml Eiswasser und nimmt den ausgefallenen öligen Niederschlag nach dem Abdekantieren in Methylenchlorid auf. Die Methylenchloridlösung wird über Natriumsulfat getrocknet und im Vakuum eingeengt. Das Rohprodukt reinigt man über eine Kieselgelsäule (Korngröße: 0,065-0,2 mm, Elutionsmittel: Methylenchlorid/Ethanol = 50:1).
Ausbeute: 0,8 g (76,2 % der Theorie),
Öl, $R_f$-Wert: 0,6 (Kieselgel: Methylenchlorid/Äthanol = 19:1)

| $C_{34}H_{42}N_2O_3$ (526,7) | | | | | | |
|---|---|---|---|---|---|---|
| Ber.: | C | 77,53 | H | 8,04 | N | 5,32 |
| Gef.: | | 77,53 | | 7,87 | | 5,31 |

Analog werden folgende Verbindungen erhalten:
4′-[(2-n-Butyl-7-methoxy-benzimidazol-1-yl)-methyl]biphenyl-2-carbonsäure-tert.butylester
Öl, $R_f$-Wert: 0,70 (Kieselgel: Methyl-ethylketon/Xylol = 1:1)
4′-[(2-n-Butyl-7-(2-methoxy-ethoxy)-4-methyl-benzimidazol-1-yl)-methyl]biphenyl-2-carbonsäure-tert.butylester
Öl, $R_f$-Wert: 0,35 (Aluminiumoxid-Platte: Methylenchlorid/Ethanol = 99:1)

b) 4′-[(7-n-Butoxy-2-n-butyl-4-methyl-benzimidazol-1-yl)-methyl]biphenyl-2-carbonsäure-trifluoracetat

0,8 g (1,5 mMol) 4′-[(7-n-Butoxy-2-n-butyl-4-methyl-benzimidazol-1-yl)-methyl]biphenyl-2-carbonsäure-tert.butylester werden in 15 ml Methylenchlorid gelöst, mit 5 ml Trifluoressigsäure versetzt und 2 Stunden bei Raumtemperatur gerührt. Man rotiert im Vakuum zur Trockene ein und kristallisiert den Rückstand aus Aceton um. Das Kristallisat wird im Vakuum bei 50°C getrocknet.
Ausbeute: 0,45 g (51,3 % der Theorie),
Schmelzpunkt: 172-174°C

| $C_{30}H_{34}N_2O_3$ x $CF_3COOH$ (584,65) | | | | | | |
|---|---|---|---|---|---|---|
| Ber.: | C | 65,74 | H | 6,03 | N | 4,73 |
| Gef.: | | 65,52 | | 6,15 | | 4,95 |

Analog werden folgende Verbindungen erhalten:
4′-[(2-n-Butyl-7-(2-(1-imidazolyl)-ethoxy)-4-methyl-benzimidazol-1-yl)-methyl]biphenyl-2-carbonsäure-bis-trifluoracetat
Schmelzpunkt: 229-231°C
4′-[(2-n-Butyl-7-(2-hydroxy-ethoxy)-4-methyl-benzimidazol-1-yl)-methyl]biphenyl-2-carbonsäure
Schmelzpunkt: 216°C

Beispiel 62

4′-[(2-n-Butyl-4-hydroxy-7-methyl-benzimidazol-1-yl)-methyl]biphenyl-2-carbonsäure

Hergestellt analog Beispiel 57 aus 4′-[(4-Benzyloxy-2-n-butyl-7-methyl-benzimidazol-1-yl)-methyl]biphenyl-2-carbonsäure und Wasserstoff in Methanol/Dimethylformamid in Gegenwart von 20%igem Palladiumhydroxid auf Kohle.
Ausbeute: 64,4 % der Theorie,

Schmelzpunkt: 291-294°C

| $C_{26}H_{26}N_2O_3$ (414,51) | | | | | | |
|---|---|---|---|---|---|---|
| Ber.: | C | 75,34 | H | 6,32 | N | 6,76 |
| Gef.: | | 75,22 | | 6,40 | | 6,64 |

Beispiel 63

4′-[(2-Ethylsulfinylmethyl-benzimidazol-1-yl)-methyl]biphenyl-2-carbonsäure

2,01 g (5,0 mMol) 4′-[(2-Ethylthiomethyl-benzimidazol-1-yl)-methyl]biphenyl-2-carbonsäure werden in 25 ml Eisessig gelöst und mit 0,51 ml 30%igem Wasserstoffperoxid versetzt. Die Lösung wird 24 Stunden bei Raumtemperatur stehengelassen und anschließend zur Trockene eingeengt. Den Rückstand reinigt man über eine Kieselgelsäule (Korngröße: 40-63 μ, Elutionsmittel: Methylenchlorid/Ethanol/Eisessig = 50/1/0,15). Die einheitlichen Fraktionen werden vereinigt, die Lösungsmittel abdestilliert, der Rückstand in Methylenchlorid gelöst und dreimal mit Wasser gewaschen. Die organische Phase wird mit Magnesiumsulfat getrocknet und zur Trockene eingeengt. Den kristallinen Rückstand verreibt man mit Diethylether, saugt ab und trocknet die Kristalle im Vakuum bei 75°C.
Ausbeute: 1,90 g (90,9 % der Theorie),
Schmelzpunkt: 161-163°C

| $C_{24}H_{22}N_2O_3S$ (418,51) | | | | | | | | |
|---|---|---|---|---|---|---|---|---|
| Ber.: | C | 68,88 | H | 5,30 | N | 6,69 | S | 7,66 |
| Gef.: | | 68,65 | | 5,40 | | 6,72 | | 7,64 |

Beispiel 64

4′-[(2-n-Propylsulfinylmethyl-benzimidazol-1-yl)-methyl]biphenyl-2-carbonsäure

Hergestellt analog Beispiel 63 aus 4′-[(2-n-Propylthiomethyl-benzimidazol-1-yl)-methyl]biphenyl-2-carbonsäure und Wasserstoffperoxid in Eisessig.
Ausbeute: 78,7 % der Theorie,
Schmelzpunkt: 128-130°C

| $C_{25}H_{24}N_2O_3S$ (432,54) | | | | | | | | |
|---|---|---|---|---|---|---|---|---|
| Ber.: | C | 69,42 | H | 5,59 | N | 6,48 | S | 7,41 |
| Gef.: | | 69,57 | | 5,46 | | 6,04 | | 7,28 |

Beispiel 65

4′-[(2-Hydroxy-benzimidazol-1-yl)-methyl]biphenyl-2-carbonsäure-3-N-oxid

Hergestellt analog Beispiel 63 aus 4′-[(2-Methylmercapto-benzimidazol-1-yl)-methyl]biphenyl-2-carbonsäure und Wasserstoffperoxid in Eisessig.
Ausbeute: 35,9 % der Theorie,

Schmelzpunkt: 272-274°C

| $C_{21}H_{16}N_2O_4$ (360,37) | | | | | | |
|---|---|---|---|---|---|---|
| Ber.: | C | 69,99 | H | 4,47 | N | 7,77 |
| Gef.: | | 70,00 | | 4,85 | | 7,51 |

Beispiel 66

4′-[(2-Methylsulfinylmethyl-benzimidazol-1-yl)-methyl]biphenyl-2-carbonsäure

Hergestellt analog Beispiel 63 aus 4′-[(2-Methylthiomethyl-benzimidazol-1-yl)-methyl]biphenyl-2-carbonsäure und Wasserstoffperoxid/Essigsäure.
Ausbeute: 79,2 % der Theorie,
Schmelzpunkt: 232-234°C

| $C_{23}H_{20}N_2O_3S$ (404,48) | | | | | | | | |
|---|---|---|---|---|---|---|---|---|
| Ber.: | C | 68,30 | H | 4,98 | N | 6,93 | S | 7,93 |
| Gef.: | | 68,17 | | 4,86 | | 7,04 | | 7,89 |

Beispiel 67

4′-[(2-Ethylsulfonylmethyl-benzimidazol-1-yl)-methyl]biphenyl-2-carbonsäure

2,01 g (5,0 mMol) 4′-[(2-Ethylthiomethyl-benzimidazol-1-yl)-methyl]biphenyl-2-carbonsäure werden in 25 ml Ameisensäure gelöst und mit 1,02 ml (10 mMol) 30%igem Wasserstoffperoxid versetzt. Die Lösung wird 24 Stunden bei Raumtemperatur stehen gelassen und anschließend zur Trockene eingeengt. Den Rückstand reinigt man über eine Kieselgelsäule (Korngroße: 40-63 μ, Elutionsmittel: Methylenchlorid/Ethanol/Eisessig = 100/1/0,15 bis 50/1/0,15). Die einheitlichen Fraktionen werden vereinigt und zur Trockene eingeengt. Der Rückstand wird in Methylenchlorid gelöst, dreimal mit Wasser gewaschen, die organische Phase mit Magnesiumsulfat getrocknet und zur Trockene eingeengt. Der kristalline Rückstand wird mit Diethylether verrieben, abgesaugt und im Vakuum bei 75°C getrocknet.
Ausbeute: 1,80 g (82,9 % der Theorie),
Schmelzpunkt: 158-160°C

| $C_{24}H_{22}N_2O_4S$ (434,51) | | | | | | | | |
|---|---|---|---|---|---|---|---|---|
| Ber.: | C | 66,34 | H | 5,10 | N | 6,45 | S | 7,38 |
| Gef.: | | 66,32 | | 5,05 | | 6,54 | | 7,27 |

Analog werden folgende Verbindungen erhalten:
4′-[(2-n-Butyl-6-methylsulfonyl-benzimidazol-1-yl)-methyl]biphenyl-2-carbonsäure
4′-[(2-n-Butyl-6-ethylsulfonyl-benzimidazol-1-yl)-methyl]biphenyl-2-carbonsäure
4′-[(2-n-Butyl-6-n-butylsulfonyl-benzimidazol-1-yl)-methyl]biphenyl-2-carbonsäure

Beispiel 68

4′-[(2-n-Propylsulfonylmethyl-benzimidazol-1-yl)-methyl]biphenyl-2-carbonsäure

Hergestellt analog Beispiel 67 aus 4′-[(2-n-Propylthiomethyl-benzimidazol-1-yl)-methyl]biphenyl-2-carbonsäure und Wasserstoffperoxid in Ameisensäure.
Ausbeute: 78,1 % der Theorie,
Schmelzpunkt: 135-137°C

| $C_{25}H_{24}N_2O_4S$ (448,54) | | | | | | | | |
|---|---|---|---|---|---|---|---|---|
| Ber.: | C | 66,95 | H | 5,39 | N | 6,25 | S | 7,15 |
| Gef.: | | 66,83 | | 5,46 | | 6,03 | | 7,06 |

Beispiel 69

4′-[(2-Methylsulfonylmethyl-benzimidazol-1-yl)-methyl]biphenyl-2-carbonsäure

Hergestellt analog Beispiel 67 aus 4′-[(2-Methylthiomethyl-benzimidazol-1-yl)-methyl]biphenyl-2-carbonsäure und Wasserstoffperoxid.
Ausbeute: 80,0 % der Theorie,
Schmelzpunkt: 290-292°C

| $C_{23}H_{20}N_2O_4S$ (420,48) | | | | | | | | |
|---|---|---|---|---|---|---|---|---|
| Ber.: | C | 65,70 | H | 4,79 | N | 6,66 | S | 7,62 |
| Gef.: | | 65,67 | | 4,64 | | 6,88 | | 7,72 |

Beispiel 70

4′-[(2-n-Butyl-benzimidazol-1-yl)-methyl]biphenyl-2-carbonsäure-ethylester

1,2 g (7 mMol) 2-n-Butyl-benzimidazol werden in 25 ml Dimethylsulfoxid gelöst, mit 0,8 g (7 mMol) Kalium-tert.butylat versetzt und 20 Minuten bei Raumtemperatur gerührt. Dann werden 2,25 g (7 mMol) 2-Carbethoxy-biphenyl-4′-yl-methylbromid zugegeben und bis zur quantitativen Umsetzung (ca. 1 Stunde) bei Raumtemperatur gerührt. Nach dem Eingießen in 100 ml Eiswasser wird 2 x mit Essigester extrahiert, die vereinigten organischten Extrakte über Natriumsulfat getrocknet und eingedampft. Das erhaltene Rohprodukt wird über eine Kieselgelsäule (Korngröße: 0,06-0,2 mm, Elutionsmittel: Methylenchlorid/Ethanol = 24:1) gereinigt.
Ausbeute: 2,3 g (79,3 % der Theorie),
Öl, $R_f$-Wert: 0,6 (Kieselgel: Methyl-ethylketon/Xylol = 1:1)

| $C_{27}H_{28}N_2O_2$ (412,53) | | | | | | |
|---|---|---|---|---|---|---|
| Ber.: | C | 78,61 | H | 6,84 | N | 6,79 |
| Gef.: | | 78,43 | | 6,76 | | 7,01 |

Analog wird folgende Verbindung erhalten:
4′-[(2-n-Butyl-7-n-butylsulfonamino-benzimidazol-1-yl)-methyl]biphenyl-2-carbonsäure-ethylester
Öl, $R_f$-Wert 0,50 (Kieselgel: Methylenchlorid/Ethanol = 10:1)

Beispiel 71

4'-[(2-n-Butyl-benzimidazol-1-yl)-methyl]biphenyl-2-carbonsäure

Hergestellt analog Beispiel 9 aus 4'-[(2-n-Butyl-benzimidazol-1-yl)-methyl]biphenyl-2-carbonsäure-tert.butylester und Trifluoressigsäure in Methylenchlorid.
Ausbeute: 62,3 % der Theorie,
Schmelzpunkt: 214-215°C

| $C_{25}H_{24}N_2O_2$ (384,48) | | | | | | |
|---|---|---|---|---|---|---|
| Ber.: | C | 78,10 | H | 6,29 | N | 7,29 |
| Gef.: | | 77,93 | | 6,21 | | 7,39 |

Beispiel 72

4'-[(6-Benzylamino-2-n-butyl-benzimidazol-1-yl)-methyl]biphenyl-2-carbonsäure

1,30 g (2,5 mMol) 4'-[(6-Benzylamino-2-n-butyl-benzimidazol-1-yl)-methyl]biphenyl-2-carbonsäure-ethylester werden in 20 ml Ethanol gelöst, mit 20 ml 2n Natronlauge versetzt und 2 Stunden am Rückfluß gekocht. Nach dem Abkühlen auf Raumtemperatur verdünnt man die Lösung mit Wasser auf 500 ml und säuert mit Eisessig auf pH 5 an. Der dabei ausfallende Niederschlag wird abgesaugt, in Aceton suspendiert, nochmals abgesaugt und im Vakuum bei 90°C getrocknet.
Ausbeute: 1,10 g (89,4 % der Theorie),
Schmelzpunkt: 250-251°C

| $C_{32}H_{31}N_3O_2$ (489,62) | | | | | | |
|---|---|---|---|---|---|---|
| Ber.: | C | 78,50 | H | 6,38 | N | 8,58 |
| Gef.: | | 78,30 | | 6,49 | | 8,71 |

Analog wird folgende Verbindung erhalten:
4'-[(2-n-Butyl-7-cyclohexylaminocarbonylamino-benzimidazol-1-yl)-methyl]biphenyl-2-carbonsäure
Schmelzpunkt: 278-279°C (Zers.)

Beispiel 73

4'-[(2-n-Butyl-4-methyl-7-hydroxy-benzimidazol-1-yl)-methyl]biphenyl-2-carbonsäure-hydrat

Hergestellt analog Beispiel 72 aus 4'-[(2-n-Butyl-4-methyl-7-hydroxy-benzimidazol-1-yl)-methyl]biphenyl-2-carbonsäure-methylester und 2n Natronlauge in Ethanol.
Ausbeute: 55,1 % der Theorie,
Schmelzpunkt: 313-315°C

| $C_{26}H_{26}N_2O_3$ x $H_20$ (432,52) | | | | | | |
|---|---|---|---|---|---|---|
| Ber.: | C | 72,20 | H | 6,53 | N | 6,43 |
| Gef.: | | 72,43 | | 6,30 | | 6,34 |

Beispiel 74

4′-[(2-n-Butyl-4-methyl-7-hydroxy-benzimidazol-1-yl)-methyl]biphenyl-2-carbonsäure-tert.butylester

Hergestellt analog Beispiel 57 aus 4′-[(2-n-Butyl-4-methyl-7-benzyloxy-benzimidazol-1-yl)-methyl]biphenyl-2-carbonsäure-tert.butylester und Wasserstoff in Gegenwart von Palladiumhydroxid auf Kohle in Methanol.
Ausbeute: 80,0 % der Theorie,
Schmelzpunkt: 214-216°C

| $C_{30}H_{34}N_2O_3$ (470,62) | | | | | | |
|---|---|---|---|---|---|---|
| Ber.: | C | 76,57 | H | 7,28 | N | 5,95 |
| Gef.: | | 76,83 | | 7,12 | | 6,00 |

Beispiel 75

4′-[(2-n-Butyl-7-methyl-benzimidazol-1-yl)-methyl]biphenyl-2-carbonsäure-hydrat

Hergestellt analog Beispiel 56 aus 3-Amino-2-[(2-tert.butoxycarbonyl-biphenyl-4′-yl)-methyl]aminotoluol und Valeriansäure.
Ausbeute: 28,6 % der Theorie,
Schmelzpunkt: 231-233°C

| $C_{26}H_{26}N_2O_2$ x $H_2O$ (416,52) | | | | | | |
|---|---|---|---|---|---|---|
| Ber.: | C | 74,98 | H | 6,78 | N | 6,73 |
| Gef.: | | 74,89 | | 6,52 | | 6,85 |

Beispiel 76

4′-[(6-Amino-2-n-butyl-benzimidazol-1-yl)-methyl]biphenyl-2-carbonsäure-ditrifluoracetat

Hergestellt analog Beispiel 9 aus 4′-[(6-Amino-2-n-butyl-benzimidazol-1-yl)-methyl]biphenyl-2-carbonsäure-tert.butylester und Trifluoressigsäure.
Ausbeute: 72,5 % der Theorie,
Schmelzpunkt: 72-74°C

| $C_{25}H_{25}N_3O_2$ x 2 $CF_3COOH$ (627,54) | | | | | | |
|---|---|---|---|---|---|---|
| Ber.: | C | 55,51 | H | 4,34 | N | 6,70 |
| Gef.: | | 55,70 | | 4,61 | | 6,55 |

Beispiel 77

4′-[(5-Amino-2-n-butyl-benzimidazol-1-yl)-methyl]biphenyl-2-carbonsäure-ditrifluoracetat

Hergestellt analog Beispiel 9 aus 4′-[(5-Amino-2-n-butyl-benzimidazol-1-yl)-methyl]biphenyl-2-carbonsäure-tert.butylester und Trifluoressigsäure.
Ausbeute: 45,9 % der Theorie,

Schmelzpunkt: 64-66°C

| $C_{25}H_{25}N_3O_2$ x 2 $CF_3COOH$ (627,54) | | | | | | |
|---|---|---|---|---|---|---|
| Ber.: | C | 55,51 | H | 4,34 | N | 6,70 |
| Gef.: | | 55,66 | | 4,42 | | 6,54 |

Beispiel 78

4′-[(2-n-Butyl-5-(n-butylaminocarbonylamino)-benzimidazol-1-yl)-methyl]biphenyl-2-carbonsäure-semitrifluoracetat

Hergestellt analog Beispiel 9 aus 4′-[(2-n-Butyl-5-(n-butyl-aminocarbonylamino)-benzimidazol-1-yl)-methyl]biphenyl-2-carbonsäure-tert.butylester und Trifluoressigsäure.
Ausbeute: 84,2 % der Theorie,
Schmelzpunkt: 165-167°C

| $C_{30}H_{34}N_4O_3$ x 1/2 $CF_3COOH$ (555,64) | | | | | | |
|---|---|---|---|---|---|---|
| Ber.: | C | 67,01 | H | 6,26 | N | 10,08 |
| Gef.: | | 66,88 | | 6,51 | | 9,89 |

Beispiel 79

4′-[(2-n-Butyl-6-phenylaminocarbonylamino-benzimidazol-1-yl)-methyl]biphenyl-2-carbonsäure

Hergestellt analog Beispiel 9 aus 4′-[(2-n-Butyl-6-phenylaminocarbonylamino-benzimidazol-1-yl)-methyl]biphenyl-2-carbonsäure-tert.butylester und Trifluoressigsäure.
Ausbeute: 92,6 % der Theorie,
Schmelzpunkt: 281-283°C

| $C_{32}H_{30}N_4O_3$ (518,61) | | | | | | |
|---|---|---|---|---|---|---|
| Ber.: | C | 74,11 | H | 5,83 | N | 10,80 |
| Gef.: | | 73,93 | | 5,83 | | 10,58 |

Beispiel 80

4′-[(2-n-Butyl-6-cyclohexylaminocarbonylamino-benzimidazol-1-yl)-methyl]biphenyl-2-carbonsäure-trifluoracetat

Hergestellt analog Beispiel 9 aus 4′-[(2-n-Butyl-6-cyclohexylaminocarbonylamino-benzimidazol-1-yl)-methyl]biphenyl-2-carbonsäure-tert.butylester und Trifluoressigsäure.
Ausbeute: 86,5 % der Theorie,

Schmelzpunkt: 199-200°C

| $C_{32}H_{36}N_4O_3$ x $CF_3COOH$ (638,68) | | | | | | |
|---|---|---|---|---|---|---|
| Ber.: | C | 63,94 | H | 5,84 | N | 8,77 |
| Gef.: | | 64,12 | | 6,15 | | 9,01 |

Beispiel 81

4′-[(2-n-Butyl-5-butanoylamino-benzimidazol-1-yl)-methyl]biphenyl-2-carbonsäure

Hergestellt analog Beispiel 9 aus 4′-[(2-n-Butyl-5-butanoylamino-benzimidazol-1-yl)-methyl]biphenyl-2-carbonsäure-tert.butylester und Trifluoressigsäure/Methylenchlorid.
Ausbeute: 93 % der Theorie,
Schmelzpunkt: 163-165°C

| $C_{29}H_{31}N_3O_3$ (469,59) | | | | | | |
|---|---|---|---|---|---|---|
| Ber.: | C | 74,18 | H | 6,65 | N | 8,95 |
| Gef.: | | 74,13 | | 6,67 | | 8,74 |

Beispiel 82

4′-[(2-(4-Hydroxyphenyl)-benzimidazol-1-yl)-methyl]biphenyl-2-carbonsäure

Hergestellt analog Beispiel 9 aus 4′-[(2-(4-Hydroxyphenyl)-benzimidazol-1-yl)-methyl]biphenyl-2-carbonsäure-tert.butylester und Trifluoressigsäure/Methylenchlorid.
Ausbeute: 87,3 % der Theorie,
Schmelzpunkt: 251-253°C

| $C_{27}H_{20}N_2O_3$ (420,47) | | | | | | |
|---|---|---|---|---|---|---|
| Ber.: | C | 77,13 | H | 4,79 | N | 6,66 |
| Gef.: | | 76,98 | | 4,83 | | 6,62 |

Beispiel 83

4′-[(2-(4-n-Butoxyphenyl)-benzimidazol-1-yl)methyl]biphenyl-2-carbonsäure

Hergestellt analog Beispiel 9 aus 4′-[(2-(4-n-Butoxyphenyl)-benzimidazol-1-yl)-methyl]biphenyl-2-carbonsäure-tert.butylester und Trifluoressigsäure/Methylenchlorid.
Ausbeute: 85,2 % der Theorie,

Schmelzpunkt: 246-248°C

| $C_{31}H_{28}N_2O_3$ (476,58) | | | | | | |
|---|---|---|---|---|---|---|
| Ber.: | C | 78,13 | H | 5,92 | N | 5,88 |
| Gef.: | | 78,33 | | 5,76 | | 5,67 |

Beispiel 84

4'-[(2-n-Butyl-6-chlor-benzimidazol-1-yl)-methyl]biphenyl-2-carbonsäure

Hergestellt analog Beispiel 9 aus 4'-[(2-n-Butyl-6-chlor-benzimidazol-1-yl)-methyl]biphenyl-2-carbonsäure-tert.butylester und Trifluoressigsäure/Methylenchlorid.
Ausbeute: 63,6 % der Theorie,
Schmelzpunkt: 220-222°C

| $C_{25}H_{23}ClN_2O_2$ (418,92) | | | | | | | | |
|---|---|---|---|---|---|---|---|---|
| Ber.: | C | 71,68 | H | 5,53 | N | 6,69 | Cl | 8,46 |
| Gef.: | | 71,81 | | 5,64 | | 6,69 | | 8,39 |

Beispiel 85

4'-[(2-n-Butyl-6-methyl-benzimidazol-1-yl)-methyl]biphenyl-2-carbonsäure

Hergestellt analog Beispiel 9 aus 4'-[(2-n-Butyl-6-methyl-benzimidazol-1-yl)-methyl]biphenyl-2-carbonsäure-tert.butylester und Trifluoressigsäure/Methylenchlorid.
Ausbeute: 70,6 % der Theorie,
Schmelzpunkt: 219-221°C

| $C_{26}H_{26}N_2O_2$ (398,51) | | | | | | |
|---|---|---|---|---|---|---|
| Ber.: | C | 78,36 | H | 6,58 | N | 7,03 |
| Gef.: | | 78,49 | | 6,53 | | 6,98 |

Beispiel 86

4'-[(2-n-Butyl-5-methansulfonamino-benzimidazol-1-yl)-methyl]biphenyl-2-carbonsäure

Hergestellt analog Beispiel 9 aus 4'-[(2-n-Butyl-5-methansulfonamino-benzimidazol-1-yl)-methyl]biphenyl-2-carbonsäure-tert.butylester und Trifluoressigsäure/Methylenchlorid.
Ausbeute: 75,7 % der Theorie,

Schmelzpunkt: 242-244°C

| $C_{26}H_{27}N_3O_4S$ (477,59) | | | | | | | | |
|---|---|---|---|---|---|---|---|---|
| Ber.: | C | 65,39 | H | 5,70 | N | 8,80 | S | 6,71 |
| Gef.: | | 65,52 | | 5,65 | | 8,55 | S | 6,51 |

Beispiel 87

4′-[(7-n-Butanoyloxy-2-n-butyl-4-methyl-benzimidazol-1-yl)-methyl]biphenyl-2-carbonsäure

Hergestellt analog Beispiel 60 aus 4′-[(2-n-Butyl-7-hydroxy-4-methyl-benzimidazol-1-yl)-methyl]biphenyl-2-carbonsäure und Buttersäurechlorid in Pyridin.
Ausbeute: 29,1 % der Theorie,
Schmelzpunkt: 240-242°C

| $C_{30}H_{32}N_2O_4$ (484,60) | | | | | | |
|---|---|---|---|---|---|---|
| Ber.: | C | 74,63 | H | 6,66 | N | 5,78 |
| Gef.: | | 74,20 | | 6,76 | | 5,87 |

Beispiel 88

4′-[(2-n-Butyl-6-ethoxycarbonylamino-benzimidazol-1-yl)-methyl]biphenyl-2-carbonsäure

Hergestellt analog Beispiel 9 aus 4′-[(2-n-Butyl-6-ethoxy-carbonylamino-benzimidazol-1-yl)-methyl]biphenyl-2-carbonsäure-tert.butylester und Trifluoressigsäure/Methylenchlorid.
Ausbeute: 88,2 % der Theorie,
Schmelzpunkt: 240-242°C

| $C_{28}H_{29}N_3O_3$ (471,55) | | | | | | |
|---|---|---|---|---|---|---|
| Ber.: | C | 71,32 | H | 6,20 | N | 8,91 |
| Gef.: | | 71,06 | | 6,36 | | 9,04 |

Beispiel 89

4′-[(2-n-Butyl-6-isopropylsulfonamino-benzimidazol-1-yl)-methyl]biphenyl-2-carbonsäure

Hergestellt analog Beispiel 9 aus 4′-[(2-n-Butyl-6-isopropylsulfonamino-benzimidazol-1-yl)-methyl]biphenyl-2-carbonsäure-tert.butylester und Trifluoressigsäure/Methylenchlorid.
Ausbeute: 91,5 % der Theorie,

Schmelzpunkt: 155-157°C

| $C_{28}H_{31}N_3O_4S$ (505,63) | | | | | | | | |
|---|---|---|---|---|---|---|---|---|
| Ber.: | C | 66,51 | H | 6,18 | N | 8,31 | S | 6,34 |
| Gef.: | | 66,26 | | 6,33 | | 8,34 | | 6,43 |

Beispiel 90

4′-[(2-n-Butyl-4-nitro-benzimidazol-1-yl)-methyl]biphenyl-2-carbonsäure

Hergestellt analog Beispiel 9 aus 4′-[(2-n-Butyl-4-nitro-benzimidazol-1-yl)-methyl]biphenyl-2-carbonsäure-tert.butylester und Trifluoressigsäure/Methylenchlorid.
Ausbeute: 84,6 % der Theorie,
Schmelzpunkt: 238-240°C

| $C_{25}H_{23}N_3O_4$ (429,48) | | | | | | |
|---|---|---|---|---|---|---|
| Ber.: | C | 69,92 | H | 5,40 | N | 9,78 |
| Gef.: | | 69,85 | | 5,43 | | 9,67 |

Beispiel 91

4′-[(2-n-Butyl-4-butanoylamino-benzimidazol-1-yl)-methyl]biphenyl-2-carbonsäure

Hergestellt analog Beispiel 9 aus 4′-[(2-n-Butyl-4-butanoylamino-benzimidazol-1-yl)-methyl]biphenyl-2-carbonsäure-tert.butylester und Trifluoressigsäure/Methylenchlorid.
Ausbeute: 96,1 % der Theorie,
Schmelzpunkt: 270-271°C

| $C_{29}H_{31}N_3O_3$ (469,58) | | | | | | |
|---|---|---|---|---|---|---|
| Ber.: | C | 74,18 | H | 6,65 | N | 8,95 |
| Gef.: | | 74,02 | | 6,74 | | 8,99 |

Beispiel 92

4′-[(2-n-Butyl-4-ethylaminocarbonylamino-benzimidazol-1-yl)-methyl]biphenyl-2-carbonsäure

Hergestellt analog Beispiel 9 aus 4′-[(2-n-Butyl-4-ethylaminocarbonylamino-benzimidazol-1-yl)-methyl]biphenyl-2-carbonsäure-tert.butylester und Trifluoressigsäure/Methylenchlorid.
Ausbeute: 87,5 % der Theorie,

Schmelzpunkt: 344-346°C (Zers.)

| $C_{28}H_{30}N_4O_3$ (470,57) | | | | | | |
|---|---|---|---|---|---|---|
| Ber.: | C | 71,47 | H | 6,43 | N | 11,91 |
| Gef.: | | 71,51 | | 6,29 | | 11,48 |

Beispiel 93

4′-[(2-(3-Pyridyl)-benzimidazol-1-yl)-methyl]biphenyl-2-carbonsäure

Hergestellt analog Beispiel 9 aus 4′-[(2-(3-Pyridyl)-benzimidazol-1-yl)-methyl]biphenyl-2-carbonsäure-tert.butylester und Trifluoressigsäure/Methylenchlorid.
Ausbeute: 73,3 % der Theorie,
Schmelzpunkt: 249-251°C

| $C_{26}H_{19}N_3O_2$ (405,46) | | | | | | |
|---|---|---|---|---|---|---|
| Ber.: | C | 77,02 | H | 4,72 | N | 10,36 |
| Gef.: | | 76,85 | | 4,72 | | 10,15 |

Beispiel 94

4′-[(2-n-Butyl-5-methyl-benzimidazol-1-yl)-methyl]biphenyl-2-carbonsäure

Hergestellt analog Beispiel 9 aus 4′-[(2-n-Butyl-5-methyl-benzimidazol-1-yl)-methyl]biphenyl-2-carbonsäure-tert.butylester und Trifluoressigsäure/Methylenchlorid.
Ausbeute: 58,8 % der Theorie,
Schmelzpunkt: 188-190°C (Zers.)

| $C_{26}H_{26}N_2O_2$ (398,51) | | | | | | |
|---|---|---|---|---|---|---|
| Ber.: | C | 78,36 | H | 6,58 | N | 7,03 |
| Gef.: | | 78,21 | | 6,62 | | 6,98 |

Beispiel 95

4′-[(2-n-Butyl-6-dimethylamino-benzimidazol-1-yl)-methyl]biphenyl-2-carbonsäure-trifluoracetat

Hergestellt analog Beispiel 9 aus 4′-[(2-n-Butyl-6-dimethylamino-benzimidazol-1-yl)-methyl]biphenyl-2-carbonsäure-tert.butylester und Trifluoressigsäure in Methylenchlorid.
Ausbeute: 65,4 % der Theorie,

| $C_{27}H_{29}N_3O_2$ x $CF_3COOH$ (541,58) | | | | | | |
|---|---|---|---|---|---|---|
| Ber.: | C | 64,31 | H | 5,58 | N | 7,76 |
| Gef.: | | 64,53 | | 5,66 | | 7,89 |

Beispiel 96

4′-[(2-n-Butyl-5-(tert.butoxycarbonylamino-acetamino)-benzimidazol-1-yl)-methyl]biphenyl-2-carbonsäure

Hergestellt analog Beispiel 72 aus 4′-[(2-n-Butyl-5-(tert.butoxycarbonylamino-acetamino)-benzimidazol-1-yl)-methyl]biphenyl-2-carbonsäure-ethylester und 2 N Natronlauge.
Ausbeute: 16,7 % der Theorie,
Schmelzpunkt: 149-151°C

| $C_{32}H_{36}N_4O_5$ (556,66) | | | | | | |
|---|---|---|---|---|---|---|
| Ber.: | C | 69,05 | H | 6,52 | N | 10,06 |
| Gef.: | | 69,12 | | 6,32 | | 9,87 |

Beispiel 97

4′-[(2-n-Butyl-5,6-dimethyl-benzimidazol-1-yl)-methyl]biphenyl-2-carbonsäure

Hergestellt analog Beispiel 9 aus 4′-[(2-n-Butyl-5,6-dimethyl-benzimidazol-1-yl)-methyl]biphenyl-2-carbonsäure-tert. butylester und Trifluoressigsäure in Methylenchlorid.
Ausbeute: 17,5 % der Theorie,
Schmelzpunkt: 222-225°C

| $C_{27}H_{28}N_2O_2$ (412,50) | | | | | | |
|---|---|---|---|---|---|---|
| Ber.: | C | 78,62 | H | 6,84 | N | 6,79 |
| Gef.: | | 78,36 | | 6,90 | | 6,83 |

Beispiel 98

4′-[2-(2,2-Dimethylpropyl)-5,6-dimethyl-benzimidazol-1-yl)-methyl]biphenyl-2-carbonsäure

Hergestellt analog Beispiel 9 aus 4′-[2-(2,2-Dimethylpropyl)-5,6-dimethyl-benzimidazol-1-yl)-methyl]biphenyl-2-carbonsäure-tert.butylester und Trifluoressigsäure in Methylenchlorid.
Ausbeute: 45 % der Theorie,
Schmelzpunkt: ab 115°C (amorph)

| $C_{28}H_{30}N_2O_2$ (426,60) | | | | | | |
|---|---|---|---|---|---|---|
| Ber.: | C | 78,83 | H | 7,09 | N | 6,57 |
| Gef.: | | 78,64 | | 7,11 | | 6,89 |

Beispiel 99

4′-[(2-Benzyl-5,6-dimethyl-benzimidazol-1-yl)-methyl]biphenyl-2-carbonsäure

Hergestellt analog Beispiel 9 aus 4′-[(2-Benzyl-5,6-dimethyl-benzimidazol-1-yl)-methyl]biphenyl-2-carbonsäure-tert.butylester und Trifluoressigsäure in Methylenchlorid.
Ausbeute: 68 % der Theorie,

Schmelzpunkt: 252-255°C

| $C_{30}H_{26}N_2O_2$ (446,60) | | | | | | |
|---|---|---|---|---|---|---|
| Ber.: | C | 80,69 | H | 5,87 | N | 6,27 |
| Gef.: | | 80,94 | | 5,76 | | 5,97 |

Beispiel 100

4'-[(2-(2-Methylbutyl)-5,6-dimethyl-benzimidazol-1-yl)-methyl]biphenyl-2-carbonsäure

Hergestellt analog Beispiel 9 aus 4'-[(2-(2-Methylbutyl)-5,6-dimethyl-benzimidazol-1-yl)-methyl]biphenyl-2-carbonsäure-tert.butylester und Trifluoressigsäure in Methylenchlorid.
Ausbeute: 57 % der Theorie,
Schmelzpunkt: 211-215°C

| $C_{28}H_{30}N_2O_2$ (426,60) | | | | | | |
|---|---|---|---|---|---|---|
| Ber.: | C | 78,84 | H | 7,09 | N | 6,57 |
| Gef.: | | 78,67 | | 7,24 | | 6,43 |

Beispiel 101

4'-[(2-Cyclohexylmethyl)-5,6-dimethyl-benzimidazol-1-yl)-methyl]biphenyl-2-carbonsäure

Hergestellt analog Beispiel 9 aus 4'-[(2-Cyclohexylmethyl)-5,6-dimethyl-benzimidazol-1-yl)-methyl]biphenyl-2-carbonsäure-tert.butylester und Trifluoressigsäure in Methylenchlorid.
Ausbeute: 31 % der Theorie,
Schmelzpunkt: 199-201°C

| $C_{30}H_{32}N_2O_2$ (452,60) | | | | | | |
|---|---|---|---|---|---|---|
| Ber.: | C | 79,61 | H | 7,13 | N | 6,19 |
| Gef.: | | 79,45 | | 7,17 | | 6,06 |

Beispiel 102

4'-[(2-Cyclohexylmethyl-5,6-dichlor-benzimidazol-1-yl)-methyl]biphenyl-2-carbonsäure

Hergestellt analog Beispiel 9 aus 4'-[(2-Cyclohexylmethyl-5,6-dichlor-benzimidazol-1-yl)-methyl]biphenyl-2-carbonsäure-tert.butylester und Trifluoressigsäure in Methylenchlorid.
Ausbeute: 39 % der Theorie,

Schmelzpunkt: 219-222°C

| $C_{28}H_{26}Cl_2N_2O_2$ (493,40) | | | | | | | | |
|---|---|---|---|---|---|---|---|---|
| Ber.: | C | 68,16 | H | 5,31 | N | 5,68 | Cl | 14,37 |
| Gef.: | | 67,97 | | 5,29 | | 5,52 | | 14,12 |

Beispiel 103

4′-[(2-(2-Methylbutyl)-naphtho[2,3-d]imidazol-1-yl)-methyl]biphenyl-2-carbonsäure

Hergestellt analog Beispiel 9 aus 4′-[(2-(2-Methylbutyl)-naphtho[2,3-d]imidazol-1-yl)-methyl]biphenyl-2-carbonsäure-tert.butylester und Trifluoressigsäure in Methylenchlorid.
Ausbeute: 64 % der Theorie,
Schmelzpunkt: 206-208°C

| $C_{30}H_{28}N_2O_2$ (448,60) | | | | | | |
|---|---|---|---|---|---|---|
| Ber.: | C | 80,33 | H | 6,29 | N | 6,25 |
| Gef.: | | 80,20 | | 6,36 | | 6,24 |

Beispiel 104

4′-[(2-n-Propyl-naphtho[2,3-d]imidazol-1-yl)-methyl]biphenyl-2-carbonsäure

Hergestellt analog Beispiel 9 aus 4′-[(2-n-Propyl-naphtho[2,3-d]imidazol-1-yl)-methyl]biphenyl-2-carbonsäure-tert.butylester und Trifluoressigsäure in Methylenchlorid.
Ausbeute: 85 % der Theorie,
Schmelzpunkt: 269-272°C

| $C_{28}H_{24}N_2O_2$ (420,50) | | | | | | |
|---|---|---|---|---|---|---|
| Ber.: | C | 79,98 | H | 5,75 | N | 6,66 |
| Gef.: | | 79,87 | | 5,68 | | 6,48 |

Beispiel 105

4′-[(2-n-Butyl-5,6-dichlor-benzimidazol-1-yl)-methyl]biphenyl-2-carbonsäure

Hergestellt analog Beispiel 9 aus 4′-[(2-n-Butyl-5,6-dichlor-benzimidazol-1-yl)-methyl]biphenyl-2-carbonsäure-tert.butylester und Trifluoressigsäure in Methylenchlorid.
Ausbeute: 50 % der Theorie,

Schmelzpunkt: 237-239°C

| $C_{25}H_{22}Cl_2N_2O_2$ (453,40) | | | | | | | | |
|---|---|---|---|---|---|---|---|---|
| Ber.: | C | 66,23 | H | 4,89 | N | 6,18 | Cl | 15,64 |
| Gef.: | | 66,10 | | 4,82 | | 6,05 | | 15,42 |

Beispiel 106

4'-[(2-Cyclohexylmethyl-5,6-dimethoxy-benzimidazol-1-yl)-methyl]biphenyl-2-carbonsäure

Hergestellt analog Beispiel 9 aus 4'-[(2-Cyclohexylmethyl-5,6-dimethoxy-benzimidazol-1-yl)-methyl]biphenyl-2-carbonsäure-tert.butylester und Trifluoressigsäure in Methylenchlorid.
Ausbeute: 45 % der Theorie,
Schmelzpunkt: 245-247°C

| $C_{30}H_{32}N_2O_4$ (484,60) | | | | | | |
|---|---|---|---|---|---|---|
| Ber.: | C | 74,36 | H | 6,66 | N | 5,78 |
| Gef.: | | 74,11 | | 6,58 | | 6,02 |

Beispiel 107

4'-[(2-n-Butyl-5,6-dimethoxy-benzimidazol-1-yl)-methyl]biphenyl-2-carbonsäure

Hergestellt analog Beispiel 9 aus 4'-[(2-n-Butyl-5,6-dimethoxy-benzimidazol-1-yl)-methyl]biphenyl-2-carbonsäure-tert.butylester und Trifluoressigsäure in Methylenchlorid.
Ausbeute: 52 % der Theorie,
Schmelzpunkt: 257-259°C

| $C_{27}H_{28}N_2O_4$ (444,50) | | | | | | |
|---|---|---|---|---|---|---|
| Ber.: | C | 72,95 | H | 6,35 | N | 6,30 |
| Gef.: | | 72,77 | | 6,26 | | 6,49 |

Beispiel 108

4'-[(2-Cyclopentylmethyl-5,6-dimethoxy-benzimidazol-1-yl)-methyl]biphenyl-2-carbonsäure

Hergestellt analog Beispiel 9 aus 4'-[(2-Cyclopentylmethyl-5,6-dimethoxy-benzimidazol-1-yl)-methyl]biphenyl-2-carbonsäure-tert.butylester und Trifluoressigsäure in Methylenchlorid.
Ausbeute: 47 % der Theorie,

Schmelzpunkt: 233-234°C

| $C_{29}H_{30}N_2O_4$ (470,60) | | | | | | |
|---|---|---|---|---|---|---|
| Ber.: | C | 74,02 | H | 6,43 | N | 5,95 |
| Gef.: | | 73,96 | | 6,56 | | 6,18 |

Beispiel 109

4′-[(2-(3-Methylbutyl)-5,6-dimethoxy-benzimidazol-1-yl)-methyl]biphenyl-2-carbonsäure

Hergestellt analog Beispiel 9 aus 4′-[(2-(3-Methylbutyl)-5,6-dimethoxy-benzimidazol-1-yl)-methyl]biphenyl-2-carbonsäure-tert.butylester und Trifluoressigsäure in Methylenchlorid.
Ausbeute: 39 % der Theorie,
Schmelzpunkt: 237-239°C

| $C_{28}H_{30}N_2O_4$ (485,60) | | | | | | |
|---|---|---|---|---|---|---|
| Ber.: | C | 73,34 | H | 6,59 | N | 6,11 |
| Gef.: | | 73,50 | | 6,48 | | 6,02 |

Beispiel 110

4′-[(2-Cyclohexyl-5,6-dimethyl-benzimidazol-1-yl)-methyl]biphenyl-2-carbonsäure

Hergestellt analog Beispiel 9 aus 4′-[(2-Cyclohexyl-5,6-dimethyl-benzimidazol-1-yl)-methyl]biphenyl-2-carbonsäure-tert.butylester und Trifluoressigsäure in Methylenchlorid.
Ausbeute: 27 % der Theorie,
Schmelzpunkt: 240-242°C

| $C_{29}H_{30}N_2O_2$ (438,60) | | | | | | |
|---|---|---|---|---|---|---|
| Ber.: | C | 79,42 | H | 6,89 | N | 6,39 |
| Gef.: | | 79,30 | | 7,02 | | 6,39 |

Beispiel 111

4′-[(2-(1-Butin-4-yl)-benzimidazol-1-yl]-methyl]biphenyl-2-carbonsäure

Hergestellt analog Beispiel 9 aus 4′-[(2-(1-Butin-4-yl)-benzimidazol-1-yl)-methyl]biphenyl-2-carbonsäure-tert.butylester und Trifluoressigsäure in Methylenchlorid.
Ausbeute: 55 % der Theorie,

Schmelzpunkt: 218-221°C

| $C_{25}H_{20}N_2O_2$ (380,50) | | | | | | |
|---|---|---|---|---|---|---|
| Ber.: | C | 78,93 | H | 5,30 | N | 7,36 |
| Gef.: | | 78,97 | | 5,24 | | 7,31 |

Beispiel 112

4′-[(2-Cyclopentyl-5,6-dimethyl-benzimidazol-1-yl)-methyl]biphenyl-2-carbonsäure

Hergestellt analog Beispiel 9 aus 4′-[(2-Cyclopentyl-5,6-dimethyl-benzimidazol-1-yl)-methyl]biphenyl-2-carbonsäure-tert.butylester und Trifluoressigsäure in Methylenchlorid.
Ausbeute: 75 % der Theorie,
Schmelzpunkt: 262-265°C

| $C_{28}H_{28}N_2O_2$ (424,50) | | | | | | |
|---|---|---|---|---|---|---|
| Ber.: | C | 79,22 | H | 6,65 | N | 6,60 |
| Gef.: | | 78,99 | | 6,54 | | 6,67 |

Beispiel 113

4′-[(2-n-Butyl-5- und 6-fluor-benzimidazol-1-yl)-methyl]biphenyl-2-carbonsäure

Hergestellt analog Beispiel 9 aus 4′-[(2-n-Butyl-5- und 6-fluor-benzimidazol-1-yl)-methyl]biphenyl-2-carbonsäure-tert.butylester und Trifluoressigsäure in Methylenchlorid.
Ausbeute: 80 % der Theorie,
Schmelzpunkt: 167-169°C

| $C_{25}H_{23}FN_2O_2$ (402,50) | | | | | | |
|---|---|---|---|---|---|---|
| Ber.: | C | 74,61 | H | 5,76 | N | 6,96 |
| Gef.: | | 74,57 | | 5,77 | | 7,05 |

Beispiel 114

4′-[(2-n-Butyl-5- und 6-benzoyl-benzimidazol-1-yl)-methyl]biphenyl-2-carbonsäure

Hergestellt analog Beispiel 9 aus 4′-[(2-n-Butyl-5-benzoyl- und 6-benzoyl-benzimidazol-1-yl)-methyl]biphenyl-2-carbonsäure-tert.butylester und Trifluoressigsäure in Methylenchlorid.
Ausbeute: 58,5 % der Theorie,

Schmelzpunkt: 180-182°C

| $C_{32}H_{28}N_2O_3$ (488,60) | | | | | | |
|---|---|---|---|---|---|---|
| Ber.: | C | 78,67 | H | 5,78 | N | 5,73 |
| Gef.: | | 78,75 | | 5,74 | | 5,63 |

Beispiel 115

4′-[(2-n-Butyl-5- und 6-trifluormethyl-benzimidazol-1-yl)-methyl]biphenyl-2-carbonsäure

Hergestellt analog Beispiel 9 aus 4′-[(2-n-Butyl-5- und 6-trifluormethyl-benzimidazol-1-yl)-methyl]biphenyl-2-carbonsäure-tert.butylester und Trifluoressigsäure in Methylenchlorid.
Ausbeute: 85 % der Theorie,
Schmelzpunkt: 172-174°C

| $C_{26}H_{23}F_3N_2O_3$ (452,50) | | | | | | |
|---|---|---|---|---|---|---|
| Ber.: | C | 69,01 | H | 5,12 | N | 6,19 |
| Gef.: | | 69,10 | | 5,24 | | 6,11 |

Beispiel 116

4′-[(2-n-Butyl-4-cyano-benzimidazol-1-yl)-methyl]biphenyl-2-carbonsäure

Hergestellt analog Beispiel 9 aus 4′-[(2-n-Butyl-4-cyano-benzimidazol-1-yl)-methyl]biphenyl-2-carbonsäure-tert.butylester und Trifluoressigsäure in Methylenchlorid.
Ausbeute: 72 % der Theorie,
Schmelzpunkt: 190-192°C

| $C_{26}H_{23}N_3O_2$ (409,49) | | | | | | |
|---|---|---|---|---|---|---|
| Ber.: | C | 76,26 | H | 5,66 | N | 10,62 |
| Gef.: | | 76,01 | | 5,72 | | 10,51 |

Beispiel 117

4′-[(2-n-Butyl-5- und 6-n-butylaminocarbonyl-benzimidazol-1-yl)-methyl]biphenyl-2-carbonsäure

Hergestellt analog Beispiel 9 aus 4′-[(2-n-Butyl-5- und 6-n-butylaminocarbonyl-benzimidazol-1-yl)-methyl]biphenyl-2-carbonsäure-tert.butylester und Trifluoressigsäure in Methylenchlorid.
Ausbeute: 69 % der Theorie,

Schmelzpunkt: ab 88°C (Zers.)

| $C_{30}H_{33}N_3O_3$ x 1/2 $CF_3COOH$ (483,60) | | | | | | |
|---|---|---|---|---|---|---|
| Ber.: | C | 68,88 | H | 6,25 | N | 7,77 |
| Gef.: | | 68,65 | | 6,32 | | 7,71 |

Beispiel 118

4'-[(2-n-Butyl-6-carboxy-benzimidazol-1-yl)-methyl]biphenyl-2-carbonsäure

Hergestellt analog Beispiel 9 aus 4'-[(2-n-Butyl-6-carboxy-benzimidazol-1-yl)-methyl]biphenyl-2-carbonsäure-tert.butylester und Trifluoressigsäure in Methylenchlorid.
Ausbeute: 91 % der Theorie,
Schmelzpunkt: 315-320°C (Zers.)

| $C_{26}H_{24}N_2O_4$ (428,50) | | | | | | |
|---|---|---|---|---|---|---|
| Ber.: | C | 72,88 | H | 5,65 | N | 6,54 |
| Gef.: | | 72,74 | | 5,66 | | 6,55 |

Beispiel 119

4'-[(2-n-Butyl-5-carboxy-benzimidazol-1-yl)-methyl]biphenyl-2-carbonsäure

Hergestellt analog Beispiel 9 aus 4'-[(2-n-Butyl-5-carboxy-benzimidazol-1-yl)-methyl]biphenyl-2-carbonsäure-tert.butylester und Trifluoressigsäure in Methylenchlorid.
Ausbeute: 63 % der Theorie,
Schmelzpunkt: 247-248°C

| $C_{26}H_{24}N_2O_4$ (428,50) | | | | | | |
|---|---|---|---|---|---|---|
| Ber.: | C | 72,88 | H | 5,65 | N | 6,54 |
| Gef.: | | 72,76 | | 5,52 | | 6,52 |

Beispiel 120

4'-[(2-n-Butyl-6-aminocarbonyl-benzimidazol-1-yl)-methyl]biphenyl-2-carbonsäure

Hergestellt analog Beispiel 9 aus 4'-[(2-n-Butyl-6-aminocarbonyl-benzimidazol-1-yl)-methyl]biphenyl-2-carbonsäure-tert.butylester und Trifluoressigsäure in Methylenchlorid.
Ausbeute: 45 % der Theorie,

Schmelzpunkt: 243-244°C

| $C_{26}H_{25}N_3O_3$ x 1/2 $H_2O$ (436,51) | | | | | | |
|---|---|---|---|---|---|---|
| Ber.: | C | 71,54 | H | 6,00 | N | 9,63 |
| Gef.: | | 71,34 | | 6,16 | | 9,45 |

Beispiel 121

4′-[(2-n-Butyl-5-aminocarbonyl-benzimidazol-1-yl)-methyl]biphenyl-2-carbonsäure

Hergestellt analog Beispiel 9 aus 4′-[(2-n-Butyl-5-aminocarbonyl-benzimidazol-1-yl)-methyl]biphenyl-2-carbonsäure-tert.butylester und Trifluoressigsäure in Methylenchlorid.
Ausbeute: 55 % der Theorie,
Schmelzpunkt: 227-228°C

| $C_{26}H_{25}N_3O_3$ x 1/2 $H_2O$ (436,51) | | | | | | |
|---|---|---|---|---|---|---|
| Ber.: | C | 71,54 | H | 6,00 | N | 9,63 |
| Gef.: | | 71,42 | | 5,94 | | 9,46 |

Beispiel 122

4′-[(2-n-Butyl-5- und 6-cyano-benzimidazol-1-yl)-methyl]biphenyl-2-carbonsäure

Hergestellt analog Beispiel 9 aus 4′-[(2-n-Butyl-5- und 6-cyano-benzimidazol-1-yl)-methyl]biphenyl-2-carbonsäure-tert.butylester und Trifluoressigsäure in Methylenchlorid.
Ausbeute: 70 % der Theorie,
Schmelzpunkt: 214-215°C

| $C_{26}H_{23}N_3O_2$ (409,50) | | | | | | |
|---|---|---|---|---|---|---|
| Ber.: | C | 76,26 | H | 5,66 | N | 10,26 |
| Gef.: | | 76,06 | | 5,44 | | 10,11 |

Beispiel 123

4′-[(2-n-Butyl-5-(1H-tetrazol-5-yl)-benzimidazol-1-yl)-methyl]biphenyl-2-carbonsäure

Hergestellt analog Beispiel 9 aus 4′-[(2-n-Butyl-5-(1H-tetrazol-5-yl)-benzimidazol-1-yl)-methyl]biphenyl-2-carbonsäure-tert.butylester und Trifluoressigsäure in Methylenchlorid.
Ausbeute: 66 % der Theorie,

Schmelzpunkt: 249-250°C

| $C_{26}H_{24}N_6O_2$ (452,50) | | | | | | |
|---|---|---|---|---|---|---|
| Ber.: | C | 69,01 | H | 5,35 | N | 18,57 |
| Gef.: | | 68,92 | | 5,48 | | 18,78 |

Beispiel 124

4'-[(2-n-Butyl-5- und 6-(1H-tetrazol-5-yl)-benzimidazol-1-yl)-methyl]biphenyl-2-carbonsäure

Hergestellt analog Beispiel 9 aus 4'-[(2-n-Butyl-5- und 6-(1H-tetrazol-5-yl)-benzimidazol-1-yl)-methyl]biphenyl-2-carbonsäure-tert.butylester und Trifluoressigsäure in Methylenchlorid.
Ausbeute: 55 % der Theorie,
Schmelzpunkt: ab 220°C (Zers.)

| $C_{26}H_{24}N_6O_2$ (452,50) | | | | | | |
|---|---|---|---|---|---|---|
| Ber.: | C | 69,01 | H | 5,35 | N | 18,57 |
| Gef.: | | 68,92 | | 5,41 | | 18,35 |

Beispiel 125

4'-[(2-n-Butyl-5-dimethylaminosulfonyl-benzimidazol-1-yl)-methyl]biphenyl-2-carbonsäure-tert.butylester

Hergestellt analog Beispiel 9 aus 4'-[(2-n-Butyl-5-dimethylaminosulfonyl-3-N-oxido-benzimidazol-1-yl)-methyl]biphenyl-2-carbonsäure-tert.butylester durch katalytische Hydrierung in Gegenwart von Raney-Nickel und anschließender Umsetzung mit Trifluoressigsäure in Methylenchlorid.
Ausbeute: 92 % der Theorie,
Schmelzpunkt: 240-242°C

| $C_{27}H_{29}N_3O_4S$ (491,60) | | | | | | | | |
|---|---|---|---|---|---|---|---|---|
| Ber.: | C | 65,97 | H | 5,95 | N | 8,55 | S | 6,52 |
| Gef.: | | 65,41 | | 6,09 | | 8,46 | | 6,60 |

Beispiel 126

4'-[(2-n-Butyl-5- und 6-methoxycarbonyl-benzimidazol-1-yl)-methyl]-2-(1H-tetrazol-5-yl)-biphenyl

0,71 g (1,0 mMol) 4'-[(2-n-Butyl-5- und 6-methoxycarbonyl-benzimidazol-1-yl)-methyl]-2-(1-triphenylmethyl-tetrazol-5-yl)-biphenyl werden mit 25 ml 5%igem methanolischem Ammoniak 16 Stunden in einem Druckgefäß auf 125-130°C erhitzt. Nach dem Abkühlen wird das Lösungsmittel abdestilliert, der Rückstand in verdünnter Essigsäure aufgenommen und dreimal mit Essigester extrahiert. Man wäscht die vereinigten Essigesterphasen mit Kochsalz-Lösung, trocknet über Natriumsulfat, engt ein und reinigt den Rückstand über eine Kieselgelsäule mit Methylenchlorid/Ethanol = 25:1 als Elutionsmittel.
Ausbeute: 0,34 g (73 % der Theorie),

Schmelzpunkt: 136-138°C

| $C_{27}H_{26}N_6O_2$ (466,56) | | | | | | |
|---|---|---|---|---|---|---|
| Ber.: | C | 69,50 | H | 5,57 | N | 17,98 |
| Gef.: | | 69,42 | | 5,33 | | 17,44 |

Beispiel 127

4′-[(2-n-Butyl-5- und 6-n-butylaminocarbonyl-benzimidazol-1-yl)-methyl]-2-(1H-tetrazol-5-yl)-biphenyl

216 mg (1,05 mMol) Dicyclohexylcarbodiimid und 141 mg (1,05 mMol) 1-Hydroxy-benzotriazolhydrat werden in 30 ml Acetonitril gelöst und mit 452 mg (1,00 mMol) 4′-[(2-n-Butyl-5- und 6-carboxy-benzimidazol-1-yl)-methyl]-2-(1H-tetrazol-5-yl)-biphenyl versetzt. Nach 30-minütigem Rühren bei Raumtemperatur werden 146 mg (2,00 mMol) n-Butylamin zugesetzt und das Reaktionsgemisch 4 Stunden bei Raumtemperatur gerührt. Der ausgefallene Dicyclohexylharnstoff wird abfiltriert und das Filtrat eingeengt. Der erhaltene Rückstand wird in Methylenchlorid aufgenommen und je einmal mit 5%iger Natriumbicarbonat-Lösung und Kochsalz-Lösung gewaschen. Nach erneutem Einengen erhält man das Endprodukt durch Chromatographie an einer Kieselgelsäule (Elutionsmittel: Methylenchlorid/Ethanol = 25:1).
Ausbeute: 95 mg (19 % der Theorie),
Schmelzpunkt: 245-247°C

| $C_{30}H_{33}N_7O$ (507,63) | | | | | | |
|---|---|---|---|---|---|---|
| Ber.: | C | 71,00 | H | 6,50 | N | 19,30 |
| Gef.: | | 70,77 | | 6,66 | | 19,36 |

Beispiel 128

4′-[(2-n-Butyl-5- und 6-aminocarbonyl-benzimidazol-1-yl)-methyl]-2-(1H-tetrazol-5-yl)-biphenyl

Hergestellt analog Beispiel 127 aus 4′-[(2-n-Butyl-5- und 6-carboxy-benzimidazol-1-yl)-methyl]-2-(1H-tetrazol-5-yl)-biphenyl.
Ausbeute: 17 % der Theorie,
Schmelzpunkt: 225-227°C

| $C_{26}H_{25}N_7O$ (451,62) | | | | | | |
|---|---|---|---|---|---|---|
| Ber.: | C | 69,98 | H | 5,54 | N | 21,62 |
| Gef.: | | 69,85 | | 5,30 | | 21,48 |

Beispiel 129

4′-[(2-n-Butyl-5- und 6-hydroxymethyl-benzimidazol-1-yl)-methyl]-2-(1H-tetrazol-5-yl)-biphenyl

452 mg (1,00 mMol) 4′-[(2-n-Butyl-5- und 6-carboxy-benzimidazol-1-yl)-methyl]-2-(1H-tetrazol-5-yl)-biphenyl werden in 20 ml Tetrahydrofuran gelöst und mit 67,0 mg (1,75 mMol) Lithiumaluminiumhydrid versetzt. Die Suspension wird 4 Stunden bei 40°C nachgerührt und anschließend mit 20 ml Wasser/Ethanol (1:1) zersetzt. Nach Filtration über Kieselgur wird eingeengt und an Kieselgel (Elutionsmittel: Methylenchlorid/Ethanol = 9:1 unter Zusatz von 1 % Ammoniak) chromatographiert.
Ausbeute: 90 mg (21 % der Theorie),

Schmelzpunkt: 173-175°C

| $C_{26}H_{26}N_6O$ (438,54) | | | | | | |
|---|---|---|---|---|---|---|
| Ber.: | C | 71,50 | H | 5,96 | N | 19,20 |
| Gef.: | | 71,32 | | 6,06 | | 19,02 |

Analog wird folgende Verbindung erhalten:
4′-[(2-n-Butyl-5- und 6-(n-butylaminomethyl)-benzimidazol-1-yl)-methyl]-2-(1H-tetrazol-5-yl)-biphenyl
Schmelzpunkt: 146-149°C

Beispiel 130

4′-[(2-n-Butyl-5- und 6-carboxy-benzimidazol-1-yl)-methyl]-2-(1H-tetrazol-5-yl)-biphenyl

2,1 g (3,0 mMol) 4′-[(2-n-Butyl-5- und 6-methoxycarbonyl-benzimidazol-1-yl)-methyl]-2-(1-triphenyl-methyl-tetrazol-5-yl)-biphenyl werden in 100 ml Ethanol unter Erwärmen gelöst, bei 40°C mit 25 ml 1 N Natronlauge versetzt und 60 Stunden bei Raumtemperatur gerührt. Man engt die Reaktionslösung ein, löst den erhaltenen Rückstand in Eiswasser und bringt mit 2 N Essigsäure auf pH 4-5. Das ausgefallene Rohprodukt wird abgesaugt, mit Wasser neutral gewaschen und über eine Kieselgelsäule (Elutionsmittel: Methylenchlorid/Ethanol = 9:1 mit Zusatz von 1 % Eisessig) gereinigt.
Ausbeute: 0,65 g (48 % der Theorie),
Schmelzpunkt: 188-190°C

| $C_{26}H_{24}N_6O_2$ (452,60) | | | | | | |
|---|---|---|---|---|---|---|
| Ber.: | C | 68,80 | H | 5,53 | N | 18,52 |
| Gef.: | | 68,63 | | 5,34 | | 18,65 |

Beispiel 131

4′-[(2-n-Butyl-7-cyano-benzimidazol-1-yl)-methyl]biphenyl-2-carbonsäure

Hergestellt analog Beispiel 9 aus 4′-[(2-n-Butyl-7-cyano-benzimidazol-1-yl)-methyl]biphenyl-2-carbonsäure-tert.butylester und Trifluoressigsäure in Methylenchlorid.
Ausbeute: 68 % der Theorie,
Schmelzpunkt: 190-192°C

| $C_{26}H_{23}N_3O_2$ (409,49) | | | | | | |
|---|---|---|---|---|---|---|
| Ber.: | C | 76,26 | H | 5,66 | N | 10,26 |
| Gef.: | | 75,99 | | 5,46 | | 10,25 |

Beispiel 132

4′-[(2-n-Butyl-5,7-difluor-benzimidazol-1-yl)-methyl]biphenyl-2-carbonsäure

Hergestellt analog Beispiel 9 aus 4′-[(2-n-Butyl-5,7-difluor-benzimidazol-1-yl)-methyl]biphenyl-2-carbonsäure-tert.butylester und Trifluoressigsäure in Methylenchlorid.
Ausbeute: 49 % der Theorie,

Schmelzpunkt: ab 155°C (amorph)

| $C_{25}H_{22}F_2N_2O_2$ (420,40) | | | | | | |
|---|---|---|---|---|---|---|
| Ber.: | C | 71,41 | H | 5,27 | N | 6,66 |
| Gef.: | | 71,38 | | 5,09 | | 6,39 |

Beispiel 133

4′-[(2-n-Butyl-5-acetyl-benzimidazol-1-yl)-methyl]biphenyl-2-carbonsäure

Hergestellt analog Beispiel 9 aus 4′-[(2-n-Butyl-5-acetyl-benzimidazol-1-yl)-methyl]biphenyl-2-carbonsäure-tert.butylester und Trifluoressigsäure in Methylenchlorid.
Ausbeute: 59,5 % der Theorie,
Schmelzpunkt: 182-184°C

| $C_{27}H_{26}N_2O_3$ (426,50) | | | | | | |
|---|---|---|---|---|---|---|
| Ber.: | C | 76,03 | H | 6,14 | N | 6,57 |
| Gef.: | | 75,89 | | 6,35 | | 6,33 |

Beispiel 134

4′-[(2-Cyclohexylmethyl-5,6-dihydroxy-benzimidazol-1-yl)-methyl]biphenyl-2-carbonsäure

Zu einer Suspension von 242 mg (0,5 mMol) 4′-[(2-Cyclohexylmethyl-5,6-dimethoxy-benzimidazol-1-yl)-methyl]biphenyl-2-carbonsäure in 25 ml Dichlormethan werden bei -5°C 0,29 ml (3 mMol) Bortribromid zugetropft. Nach beendeter Zugabe wird das Kühlbad entfernt und 5 Stunden lang bei Raumtemperatur gerührt. Unter Eiskühlung werden danach 20 ml Methanol hinzugegeben, anschließend das Reaktionsgemisch bis zur Trockne eingedampft und der Rückstand unter Rühren in 10 ml Wasser suspendiert. Das ausgefallene Rohprodukt wird abgesaugt, weiteren 10 ml Wasser gewaschen, getrocknet und über eine Kieselgelsäule (Elutionsmittel: Dichlormethan/Ethanol = 9:1) chromatographiert.
Ausbeute: 51 mg (22 % der Theorie),
Schmelzpunkt: amorph

| $C_{28}H_{28}N_2O_4$ (456,50) | | | | | | |
|---|---|---|---|---|---|---|
| Ber.: | C | 73,66 | H | 6,18 | N | 6,14 |
| Gef.: | | 73,79 | | 6,31 | | 6,22 |

Analog werden folgende Verbindungen erhalten:
4′-[(2-n-Butyl-5-hydroxy-benzimidazol-1-yl)-methyl]biphenyl-2-carbonsäure
Schmelzpunkt: 306-307°C
4′-[(2-n-Butyl-4-hydroxy-benzimidazol-1-yl)-methyl]biphenyl-2-carbonsäure
Schmelzpunkt: 292-293°C
4′-[(2-n-Butyl-7-hydroxy-benzimidazol-1-yl)-methyl]biphenyl-2-carbonsäure
Schmelzpunkt: 295-297°C

Beispiel 135

4′-[(2-(3-Methylbutyl)-5-methoxy-6-hydroxy-benzimidazol-1-yl)-methyl]biphenyl-2-carbonsäure und
4′-[(2-(3-Methylbutyl)-5-hydroxy-6-methoxy-benzimidazol-1-yl)-methyl]biphenyl-2-carbonsäure

Zu einer Suspension von 600 mg (1,3 mMol) 4′-[(2-(3-Methylbutyl)-5,6-dimethoxy-benzimidazol-1-yl)-methyl]biphenyl-2-carbonsäure in 50 ml getrocknetem Dichlormethan werden 3 g Aluminiumtrichlorid gegeben und das Gemisch 15 Minuten lang unter Rückfluß erhitzt. Anschließend wird das Lösungsmittel abdestilliert, der Rückstand abgesaugt, mit 30 ml Wasser gewaschen und getrocknet. Nach Chromatographie über Kieselgel (Elutionsmittel: Dichlormethan/Ethanol = 9:1) erhält man 360 mg (62 % der Theorie) eines Gemisches der isomeren Produkte.

Zur Trennung der Isomeren werden 230 mg des Gemisches erneut über Kieselgel chromatographiert (Elutionsmittel: Dichlormethan/Ethanol = 11:1). Man erhält so 80 mg 4′-[(2-(3-Methylbutyl)-5-methoxy-6-hydroxy-benzimidazol-1-yl)-methyl]biphenyl-2-carbonsäure (Schmelzpunkt: 189-192°C)

| $C_{27}H_{28}N_2O_4$ (444,50) | | | | | | |
|---|---|---|---|---|---|---|
| Ber.: | C | 72,95 | H | 6,35 | N | 6,30 |
| Gef.: | | 72,84 | | 6,32 | | 6,19 |

und 30 mg 4′-[(2-(3-Methylbutyl)-5-hydroxy-6-methoxy-benzimidazol-1-yl)-methyl]biphenyl-2-carbonsäure (Schmelzpunkt: 188-190°C)

| $C_{27}H_{28}N_2O_4$ (444,50) | | | | | | |
|---|---|---|---|---|---|---|
| Ber.: | C | 72,95 | H | 6,35 | N | 6,30 |
| Gef.: | | 73,13 | | 6,39 | | 6,41 |

Beispiel 136

4′-[(2-n-Butyl-7-n-propylamino-benzimidazol-1-yl)-methyl]biphenyl-2-carbonsäure-ethylester

2,0 g (4,7 mMol) 4′-[(7-Amino-2-n-butyl-benzimidazol-1-yl)-methyl]biphenyl-2-carbonsäure-ethylester werden in 50 ml Ethanol gelöst und mit 0,53 ml (7,5 mMol) Propionaldehyd sowie mit 0,5 g 10%igem Palladium auf Kohle versetzt und 2 Stunden bei Raumtemperatur und 5 bar Wasserstoffdruck hydriert. Der Katalysator wird anschließend abgesaugt und das Lösungsmittel im Vakuum entfernt. Den öligen Rückstand reinigt man über eine Aluminoxid-Säule (neutral, Aktivität II-III), wobei mit Cyclohexan/Methylenchlorid = 3:1 und 1:1 eluiert wird. Die entsprechenden Fraktionen werden vereinigt und einrotiert.
Ausbeute: 2,0 g (90,9 % der Theorie),
Öl, $R_f$-Wert: 0,50 (Kieselgel: Methylenchlorid/Ethanol) = 19:1)
Analog werden folgende Verbindungen erhalten:
4′-[(2-n-Butyl-5-n-pentylamino-benzimidazol-1-yl)-methyl]biphenyl-2-carbonsäure-tert.butylester
$R_f$-Wert: 0,45 (Aluminiumoxid, Methylenchlorid)
4′-[(2-n-Butyl-6-n-pentylamino-benzimidazol-1-yl)-methyl]biphenyl-2-carbonsäure-tert.butylester
Öl, $R_f$-Wert: 0,40 (Kieselgel: Methyl-ethylketon/Xylol = 1:1)
4′-[(2-n-Butyl-6-n-butylamino-benzimidazol-1-yl)-methyl]biphenyl-2-carbonsäure-tert.butylester
Öl, $R_f$-Wert: 0,50 (Kieselgel: Methyl-ethylketon/Xylol = 1:1)
4′-[(2-n-Butyl-6-(2-cyclohexyl-ethylamino)-benzimidazol-1-yl)-methyl]biphenyl-2-carbonsäure-tert.butylester
Öl, $R_f$-Wert: 0,40 (Kieselgel: Essigester/Petrolether = 60:40)
4′-[(2-n-Butyl-6-(cis- und trans-decahydro-naphth-2-yl-amino)-benzimidazol-1-yl)-methyl]biphenyl-2-carbonsäure-tert.butylester
Öl, $R_f$-Wert: 0,65 (Aluminiumoxid-Platte: Petrolether/Essigester = 9:1)

Beispiel 137

4'-[(2-n-Butyl-7-n-propylamino-benzimidazol-1-yl)-methyl]biphenyl-2-carbonsäure

Hergestellt analog Beispiel 72 aus 4'-[(2-n-Butyl-7-n-propylamino-benzimidazol-1-yl)-methyl]biphenyl-2-carbonsäure-ethylester und 2 N Natronlauge/Ethanol.
Ausbeute: 85,7 % der Theorie,
Schmelzpunkt: 262-263°C

| $C_{28}H_{31}N_3O_2$ (441,57) | | | | | | |
|---|---|---|---|---|---|---|
| Ber.: | C | 76,16 | H | 7,08 | N | 9,52 |
| Gef.: | | 76,35 | | 7,26 | | 9,60 |

Beispiel 138

4'-[(2-n-Butyl-5- und 6-nitro-benzimidazol-1-yl)-methyl]biphenyl-2-carbonsäure

Hergestellt analog Beispiel 9 aus 4'-[(2-n-Butyl-5- und 6-nitro-benzimidazol-1-yl)-methyl]biphenyl-2-carbonsäure-tert.butylester und Trifluoressigsäure in Methylenchlorid.
Ausbeute: 54,4 % der Theorie,
Schmelzpunkt: 223-224°C

| $C_{25}H_{23}N_3O_4$ (429,48) | | | | | | |
|---|---|---|---|---|---|---|
| Ber.: | C | 69,92 | H | 5,60 | N | 9,78 |
| Gef.: | | 69,81 | | 5,47 | | 9,72 |

Beispiel 139

4'-[(2-n-Butyl-7-n-butylsulfonamino-benzimidazol-1-yl)-methyl]biphenyl-2-carbonsäure

Hergestellt analog Beispiel 72 aus 4'-[(2-n-Butyl-7-n-butylsulfonamino-benzimidazol-1-yl)-methyl]biphenyl-2-carbonsäureethylester und Natronlauge in Ethanol.
Ausbeute: 94,7 % der Theorie,
Schmelzpunkt: 225-226°C

| $C_{29}H_{33}N_3O_4S$ (519,66) | | | | | | | | |
|---|---|---|---|---|---|---|---|---|
| Ber.: | C | 67,03 | H | 6,40 | N | 8,09 | S | 6,17 |
| Gef.: | | 66,92 | | 6,63 | | 8,09 | | 5,91 |

Beispiel 140

4'-[(2-n-Butyl-5-n-pentylamino-benzimidazol-1-yl)-methyl]biphenyl-2-carbonsäure-hemitrifluoracetat

Hergestellt analog Beispiel 9 aus 4'-[(2-n-Butyl-5-n-pentylamino-benzimidazol-1-yl)-methyl]biphenyl-2-carbonsäure-tert.butylester und Trifluoressigsäure in Methylenchlorid.
Ausbeute: 92,3 % der Theorie,

Schmelzpunkt: 155-157°C

| $C_{30}H_{35}N_3O_4$ x 1/2 $CF_3COOH$ (526,64) | | | | | | |
|---|---|---|---|---|---|---|
| Ber.: | C | 70,70 | H | 6,79 | N | 7,98 |
| Gef.: | | 70,84 | | 6,94 | | 8,05 |

Beispiel 141

4′-[(2-n-Butyl-6-cyclohexylaminocarbonylamino-benzimidazol-1-yl)-methyl]-2-amino-biphenyl

2 g (3,2 mMol) 4′-[(2-n-Butyl-6-cyclohexylaminocarbonylamino-benzimidazol-1-yl)-methyl]-2-phthalimino-biphenyl werden in 20 ml Methanol und 10 ml Dimethylformamid gelöst und nach Zugabe von 20 ml 40%iger Methylaminlösung 2 Stunden bei Zimmertemperatur gerührt. Es wird im Vakuum zur Trockene eingedampft. Der verbleibende Rückstand wird in Ether suspendiert und das unlösliche N-Methyl-phthalimid abfiltriert. Das Filtrat wird im Vakuum zur Trockene eingedampft und über Kieselgel (Korngröße: 0,063-0,2 mm, Elutionsmittel: Methylenchlorid mit 0,5-2 % Ethanol) gereinigt. Das erhaltene Produkt wird in Ether suspendiert, abgesaugt, mit Ether gewaschen und im Vakuum getrocknet.
Ausbeute: 1,37 g (85,6 % der Theorie),
Schmelzpunkt: 209-211°C

| $C_{31}H_{37}N_5O$ (495,68) | | | | | | |
|---|---|---|---|---|---|---|
| Ber.: | C | 75,12 | H | 7,52 | N | 14,13 |
| Gef.: | | 75,33 | | 7,57 | | 14,01 |

Analog wird folgende Verbindung erhalten:
4′-[(2-n-Butyl-7-hydroxy-4-methyl-benzimidazol-1-yl)-methyl]-2-amino-biphenyl
Schmelzpunkt: 249-250°C

Beispiel 142

4′-[(2-n-Butyl-7-hydroxy-4-methyl-benzimidazol-1-yl)-methyl]-2-aminomethyl-biphenyl

3,3 g (7,1 mMol) 4′-[(7-Benzyloxy-2-n-butyl-4-methyl-benzimidazol-1-yl)-methyl]-2-cyano-biphenyl werden in 100 ml Methanol gelöst und in Gegenwart von 0,5 g Palladium (10 % auf Kohle) mit 5 bar Wasserstoff bei Zimmertemperatur hydriert. Nach 3 Stunden wird vom Katalysator abfiltriert. Das Filtrat wird mit 20 ml 20%igem Ammoniak in Methanol versetzt und in Gegenwart von 0,5 g Raney-Nickel mit 5 bar Wasserstoff bei 70°C erneut hydriert. Nach 4 Stunden wird vom Katalysator abgesaugt und das Filtrat im Vakuum zur Trockene eingedampft. Das kristalline Rohprodukt wird in Ether suspendiert, abgesaugt, mit Ether gewaschen und im Vakuum getrocknet.
Ausbeute: 2,6 g (92,8 % der Theorie),
Schmelzpunkt: 207-212°C

| $C_{26}H_{29}N_3O$ (399,53) | | | | | | |
|---|---|---|---|---|---|---|
| Ber.: | C | 78,16 | H | 7,32 | N | 10,52 |
| Gef.: | | 77,97 | | 7,34 | | 10,51 |

Beispiel 143

4'-[(2-n-Butyl-6-cycohexylaminocarbonylamino-benzimidazol-1-yl)-methyl]-2-trifluoracetamino-biphenyl

0,35 g (0,7 mMol) 4'-[(2-n-Butyl-6-cycohexylaminocarbonylamino-benzimidazol-1-yl)-methyl]-2-amino-biphenyl, gelöst in 15 ml Methylenchlorid und 0,85 ml (6,1 mMol) Triethylamin, werden auf -60°C abgekühlt und unter Rühren tropfenweise mit 0,85 ml Trifluoressigsäureanhydrid in 2 ml Methylenchlorid versetzt. Man läßt über Nacht auf Raumtemperatur ansteigen und dampft zur Trockene ein. Der Rückstand wird über Kieselgel (Elutionsmittel: Methylenchlorid mit 0,5-2 % Ethanol) gereinigt. Die gesammelten Eluate werden eingedampft, aus Ether/Benzin 60-80°C kristallisiert und im Vakuum getrocknet.
Ausbeute: 0,21 g (50 % der Theorie),
Schmelzpunkt: 231-233°C

| $C_{33}H_{36}F_3N_5O_2$ (591,68) | | | | | | |
|---|---|---|---|---|---|---|
| Ber.: | C | 66,99 | H | 6,13 | N | 11,83 |
| Gef.: | | 66,83 | | 5,96 | | 11,71 |

Analog werden folgende Verbindungen erhalten:
4'-[(7-Benzyloxy-2-n-butyl-4-methyl-benzimidazol-1-yl)-methyl]-2-trifluoracetamino-biphenyl
Schmelzpunkt: 147-149°C
4'-[(2-n-Butyl-7-hydroxy-4-methyl-benzimidazol-1-yl)-methyl]-2-trifluoracetaminomethyl-biphenyl
Schmelzpunkt: 247-249°C
4'-[(7-Benzyloxy-2-n-butyl-4-methyl-benzimidazol-1-yl)-methyl]-2-trifluormethansulfonamino-biphenyl
Öl, $R_f$-Wert: 0,65 (Kieselgel: Petroläther/Essigester = 1:1)
4'-[(2-n-Butyl-7-trifluormethansulfonyloxy-4-methyl-benzimidazol-1-yl)-methyl]-2-trifluormethansulfonaminomethyl-biphenyl
Schmelzpunkt: 137-139°C

Beispiel 144

4'-[(2-n-Butyl-4-methoxy-benzimidazol-1-yl)-methyl]biphenyl-2-carbonsäure

Hergestellt analog Beispiel 9 aus 4'-[(2-n-Butyl-4-methoxy-benzimidazol-1-yl)-methyl]biphenyl-2-carbonsäure-tert.butylester und Trifluoressigsäure.
Ausbeute: 76,5 % der Theorie,
Schmelzpunkt: 179-181°C

| $C_{26}H_{26}N_2O_3$ (414,51) | | | | | | |
|---|---|---|---|---|---|---|
| Ber.: | C | 75,34 | H | 6,32 | N | 6,76 |
| Gef.: | | 75,07 | | 6,35 | | 6,71 |

Beispiel 145

4'-[(2-n-Butyl-7-methoxy-benzimidazol-1-yl)-methyl]biphenyl-2-carbonsäure

Hergestellt analog Beispiel 9 aus 4'-[(2-n-Butyl-7-methoxy-benzimidazol-1-yl)-methyl]biphenyl-2-carbonsäure-tert.butylester und Trifluoressigsäure.
Ausbeute: 80 % der Theorie,

Schmelzpunkt: 260-261°C

| $C_{26}H_{26}N_2O_3$ (414,51) | | | | | | |
|---|---|---|---|---|---|---|
| Ber.: | C | 75,34 | H | 6,32 | N | 6,76 |
| Gef.: | | 75,09 | | 6,37 | | 6,79 |

Beispiel 146

4′-[(5-Aminoacetamino-2-n-butyl-benzimidazol-1-yl)-methyl]biphenyl-2-carbonsäure

Hergestellt analog Beispiel 9 aus 4′-[(5-Aminoacetamino-2-n-butyl-benzimidazol-1-yl)-methyl]biphenyl-2-carbonsäure-tert.butylester und Trifluoressigsäure.
Ausbeute: 40 % der Theorie,
Schmelzpunkt: 230-232°C (Zers.)

| $C_{27}H_{28}N_4O_3$ (456,54) | | | | | | |
|---|---|---|---|---|---|---|
| Ber.: | C | 71,03 | H | 6,18 | N | 12,27 |
| Gef.: | | 70,83 | | 6,36 | | 11,98 |

Beispiel 147

4′-[(2-n-Butyl-5-n-pentylamino-benzimidazol-1-yl)-methyl]biphenyl-2-carbonsäure-hemitrifluoracetat

Hergestellt analog Beispiel 9 aus 4′-[(2-n-Butyl-5-n-pentylamino-benzimidazol-1-yl)-methyl]biphenyl-2-carbonsäure-tert.butylester und Trifluoressigsäure.
Ausbeute: 92,3 % der Theorie,
Schmelzpunkt: 155-157°C (Zers.)

| $C_{30}H_{35}N_3O_2$ x 1/2 $CF_3COOH$ (526,64) | | | | | | |
|---|---|---|---|---|---|---|
| Ber.: | C | 70,70 | H | 6,79 | N | 7,98 |
| Gef.: | | 71,04 | | 7,14 | | 8,05 |

Beispiel 148

4′-[(2-n-Butyl-6-methylamino-benzimidazol-1-yl)-methyl]biphenyl-2-carbonsäure-hemitrifluoracetat

Hergestellt analog Beispiel 9 aus 4′-[(2-n-Butyl-6-methylamino-benzimidazol-1-yl)-methyl]biphenyl-2-carbonsäure-tert.butylester und Trifluoressigsäure.
Ausbeute: 77,3 % der Theorie,

Schmelzpunkt: 197-199°C

| $C_{26}H_{27}N_3O_2$ x 1/2 $CF_3COOH$ (470,53) | | | | | | |
|---|---|---|---|---|---|---|
| Ber.: | C | 68,92 | H | 5,89 | N | 8,93 |
| Gef.: | | 68,82 | | 5,80 | | 8,62 |

Beispiel 149

4′-[(2-n-Butyl-6-(N-butanoyl-methylamino)-benzimidazol-1-yl)-methyl]biphenyl-2-carbonsäure

Hergestellt analog Beispiel 9 aus 4′-[(2-n-Butyl-6-(N-butanoyl-methylamino)-benzimidazol-1-yl)-methyl]biphenyl-2-carbonsäure-tert.butylester und Trifluoressigsäure.
Ausbeute: 70,3 % der Theorie,
Schmelzpunkt: 165-167°C

| $C_{30}H_{33}N_3O_3$ (483,62) | | | | | | |
|---|---|---|---|---|---|---|
| Ber.: | C | 74,51 | H | 6,87 | N | 8,68 |
| Gef.: | | 74,51 | | 6,89 | | 8,56 |

Beispiel 150

4′-[(6-Aminocarbonylamino-2-n-butyl-benzimidazol-1-yl)-methyl]biphenyl-2-carbonsäure-trifluoracetat

Hergestellt analog Beispiel 9 aus 4′-[(6-Aminocarbonylamino-2-n-butyl-benzimidazol-1-yl)-methyl]biphenyl-2-carbonsäure-tert.butylester und Trifluoressigsäure.
Ausbeute: 93,8 % der Theorie,
Schmelzpunkt: 134-136°C (amorph)

| $C_{26}H_{26}N_4O_3$ x $CF_3COOH$ (556,54) | | | | | | |
|---|---|---|---|---|---|---|
| Ber.: | C | 60,43 | H | 4,89 | N | 10,07 |
| Gef.: | | 60,22 | | 4,87 | | 9,80 |

Beispiel 151

4′-[(2-n-Butyl-6-(n-hexylaminocarbonylamino)-benzimidazol-1-yl)-methyl]biphenyl-2-carbonsäure-trifluoracetat

Hergestellt analog Beispiel 9 aus 4′-[(2-n-Butyl-6-(n-hexylaminocarbonylamino)-benzimidazol-1-yl)-methyl]biphenyl-2-carbonsäure-tert.butylester und Trifluoressigsäure.
Ausbeute: 93,3 % der Theorie,

Schmelzpunkt: 138-140°C

| $C_{32}H_{38}N_4O_3$ x $CF_3COOH$ (640,70) | | | | | | |
|---|---|---|---|---|---|---|
| Ber.: | C | 63,74 | H | 6,14 | N | 8,74 |
| Gef.: | | 63,66 | | 6,19 | | 8,51 |

Beispiel 152

4'-[(2-n-Butyl-4-hydroxy-benzimidazol-1-yl)-methyl]biphenyl-2-carbonsäure

Hergestellt analog Beispiel 9 aus 4'-[(2-n-Butyl-4-hydroxy-benzimidazol-1-yl)-methyl]biphenyl-2-carbonsäure-tert.butylester und Trifluoressigsäure.
Ausbeute: 72,5 % der Theorie,
Schmelzpunkt: 292-293°C

| $C_{25}H_{24}N_2O_3$ (400,48) | | | | | | |
|---|---|---|---|---|---|---|
| Ber.: | C | 74,98 | H | 6,04 | N | 7,00 |
| Gef.: | | 74, 85 | | 6,13 | | 6,91 |

Beispiel 153

4'-[(2-n-Butyl-5-cyclohexylaminocarbonylamino-benzimidazol-1-yl)-methyl]biphenyl-2-carbonsäure-trifluoracetat

Hergestellt analog Beispiel 9 aus 4'-[(2-n-Butyl-5-cyclohexylaminocarbonylamino-benzimidazol-1-yl)-methyl]biphenyl-2-carbonsäure-tert.butylester und Trifluoressigsäure.
Ausbeute: 81,1 % der Theorie,
Schmelzpunkt: 176-177°C (amorph)

| $C_{32}H_{36}N_4O_3$ x $CF_3COOH$ (638,69) | | | | | | |
|---|---|---|---|---|---|---|
| Ber.: | C | 63,94 | H | 5,84 | N | 8,77 |
| Gef.: | | 64,04 | | 6,00 | | 9,05 |

Beispiel 154

4'-[(2-n-Butyl-7-isopropylaminomethyl-benzimidazol-1-yl)-methyl]biphenyl-2-carbonsäure-hydrat

Hergestellt analog Beispiel 9 aus 4'-[(2-n-Butyl-7-isopropylaminomethyl-benzimidazol-1-yl)-methyl]biphenyl-2-carbonsäure-tert.butylester und Trifluoressigsäure.
Ausbeute: 53 % der Theorie,

Schmelzpunkt: 156-158°C

| $C_{29}H_{33}N_3O_2$ x $H_2O$ (473,61) | | | | | | |
|---|---|---|---|---|---|---|
| Ber.: | C | 73,54 | H | 7,45 | N | 8,87 |
| Gef.: | | 73,69 | | 7,37 | | 8,91 |

Beispiel 155

4′-[(6-Aminothiocarbonylamino-2-n-butyl-benzimidazol-1-yl)-methyl]biphenyl-2-carbonsäure-hemihydrat

Hergestellt analog Beispiel 9 aus 4′-[(6-Aminothiocarbonylamino-2-n-butyl-benzimidazol-1-yl)-methyl]biphenyl-2-carbonsäure-tert.butylester und Trifluoressigsäure.
Ausbeute: 80,4 % der Theorie,
Schmelzpunkt: 147-149°C

| $C_{26}H_{26}N_4O_2S$ x 1/2 $H_2O$ (467,58) | | | | | | |
|---|---|---|---|---|---|---|
| Ber.: | C | 66,80 | H | 5,81 | N | 6,85 |
| Gef.: | | 66,92 | | 5,91 | | 6,66 |

Beispiel 156

4′-[(2-n-Butyl-6-cyclohexylaminothiocarbonylamino-benzimidazol-1-yl)-methyl]biphenyl-2-carbonsäure

Hergestellt analog Beispiel 9 aus 4′-[(2-n-Butyl-6-cyclohexylaminothiocarbonylamino-benzimidazol-1-yl)-methyl]biphenyl-2-carbonsäure-tert.butylester und Trifluoressigsäure.
Ausbeute: 88,2 % der Theorie,
Schmelzpunkt: 223-225°C (Zers.)

| $C_{32}H_{36}N_4O_2S$ (540,72) | | | | | | | | |
|---|---|---|---|---|---|---|---|---|
| Ber.: | C | 71,08 | H | 6,71 | N | 10,36 | S | 5,93 |
| Gef.: | | 70,95 | | 6,77 | | 10,53 | | 6,23 |

Beispiel 157

4′-[(2-n-Butyl-6-hydroxy-benzimidazol-1-yl)-methyl]biphenyl-2-carbonsäure

Hergestellt analog Beispiel 9 aus 4′-[(2-n-Butyl-6-hydroxy-benzimidazol-1-yl)-methyl]biphenyl-2-carbonsäure-tert.butylester und Trifluoressigsäure.
Ausbeute: 76,5 % der Theorie,

Schmelzpunkt: 264-266°C

| $C_{25}H_{24}N_2O_3$ (400,48) | | | | | | |
|---|---|---|---|---|---|---|
| Ber.: | C | 74,98 | H | 6,04 | N | 7,00 |
| Gef.: | | 75,06 | | 5,95 | | 6,98 |

Beispiel 158

4'-[(2-n-Butyl-7-(2-methoxy-ethoxy)-4-methyl-benzimidazol-1-yl)-methyl]biphenyl-2-carbonsäure

Hergestellt analog Beispiel 9 aus 4'-[(2-n-Butyl-7-(2-methoxy-ethoxy)-4-methyl-benzimidazol-1-yl)-methyl]biphenyl-2-carbonsäure-tert.butylester und Trifluoressigsäure.
Ausbeute: 63,6 % der Theorie,
Schmelzpunkt: 205-207°C

| $C_{29}H_{32}N_2O_4$ (472,58) | | | | | | |
|---|---|---|---|---|---|---|
| Ber.: | C | 73,71 | H | 6,82 | N | 5,93 |
| Gef.: | | 73,48 | | 6,64 | | 6,15 |

Beispiel 159

4'-[(2-n-Butyl-6-trifluoracetylamino-benzimidazol-1-yl)-methyl]biphenyl-2-carbonsäure

Hergestellt analog Beispiel 9 aus 4'-[(2-n-Butyl-6-trifluoracetylamino-benzimidazol-1-yl)-methyl]biphenyl-2-carbonsäure-tert.butylester und Trifluoressigsäure.
Ausbeute: 69,6 % der Theorie,
Schmelzpunkt: 84-86°C

| $C_{27}H_{24}N_3O_3F_3$ (495,50) | | | | | | |
|---|---|---|---|---|---|---|
| Ber.: | C | 65,45 | H | 4,88 | N | 8,48 |
| Gef.: | | 65,20 | | 5,06 | | 8,64 |

Beispiel 160

4'-[(2-n-Butyl-4-cyclohexylaminocarbonylamino-benzimidazol-1-yl)-methyl]biphenyl-2-carbonsäure

Hergestellt analog Beispiel 9 aus 4'-[(2-n-Butyl-4-cyclohexylaminocarbonylamino-benzimidazol-1-yl)-methyl]biphenyl-2-carbonsäure-tert.butylester und Trifluoressigsäure.
Ausbeute: 94,4 % der Theorie,

Schmelzpunkt: 242-244°C (Zers.)

| $C_{32}H_{36}N_4O_3$ (524,66) | | | | | | |
|---|---|---|---|---|---|---|
| Ber.: | C | 73,26 | H | 6,92 | N | 10,68 |
| Gef.: | | 72,42 | | 6,93 | | 10,77 |

Beispiel 161

4′-[(6-Allylaminocarbonylamino-2-n-butyl-benzimidazol-1-yl)-methyl]biphenyl-2-carbonsäure-trifluoracetat

Hergestellt analog Beispiel 9 aus 4′-[(6-Allylaminocarbonylamino-2-n-butyl-benzimidazol-1-yl)-methyl]biphenyl-2-carbonsäure-tert.butylester und Trifluoressigsäure.
Ausbeute: 96 % der Theorie,
Schmelzpunkt: 90-92°C

| $C_{29}H_{30}N_4O_3$ x $CF_3COOH$ (596,61) | | | | | | |
|---|---|---|---|---|---|---|
| Ber.: | C | 62,41 | H | 5,24 | N | 9,39 |
| Gef.: | | 62,20 | | 5,17 | | 9,13 |

Beispiel 162

4′-[(6-Benzylaminocarbonylamino-2-n-butyl-benzimidazol-1-yl)-methyl]biphenyl-2-carbonsäure

Hergestellt analog Beispiel 9 aus 4′-[(6-Benzylaminocarbonylamino-2-n-butyl-benzimidazol-1-yl)-methyl]biphenyl-2-carbonsäure-tert.butylester und Trifluoressigsäure.
Ausbeute: 79,4 % der Theorie,
Schmelzpunkt: 244-245°C

| $C_{33}H_{32}N_4O_3$ (532,64) | | | | | | |
|---|---|---|---|---|---|---|
| Ber.: | C | 74,41 | H | 6,06 | N | 10,52 |
| Gef.: | | 74,32 | | 6,09 | | 10,31 |

Beispiel 163

4′-(2-n-Butyl-6-(N-methylaminocarbonyl-n-pentylamino)-benzimidazol-1-yl)-methyl]biphenyl-2-carbonsäure

Hergestellt analog Beispiel 9 aus 4′-[(2-n-Butyl-6-(N-methylaminocarbonyl-n-pentylamino)-benzimidazol-1-yl)-methyl]biphenyl-2-carbonsäure-tert.butylester und Trifluoressigsäure.
Ausbeute: 54 % der Theorie,

Schmelzpunkt: 149-152°C

| $C_{32}H_{38}N_4O_3$ (526,68) | | | | | | |
|---|---|---|---|---|---|---|
| Ber.: | C | 72,98 | H | 7,27 | N | 10,64 |
| Gef.: | | 72,95 | | 7,27 | | 10,73 |

Beispiel 164

4′-[(5-Aminothiocarbonylamino-2-n-butyl-benzimidazol-1-yl)-methyl]biphenyl-2-carbonsäure-hydrochlorid

Hergestellt analog Beispiel 9 aus 4′-[(5-Aminothiocarbonylamino-2-n-butyl-benzimidazol-1-yl)-methyl]biphenyl-2-carbonsäure-tert.butylester und Trifluoressigsäure.
Ausbeute: 66,6 % der Theorie,
Schmelzpunkt: 150-152°C

| $C_{26}H_{27}N_4O_2SCl$ (495,04) | | | | | | | | |
|---|---|---|---|---|---|---|---|---|
| Ber.: | C | 63,08 | H | 5,49 | N | 11,31 | S | 6,46 |
| Gef.: | | 62,83 | | 5,76 | | 11,15 | | 6,22 |

Beispiel 165

4′-[(2-n-Butyl-5-formylamino-benzimidazol-1-yl)-methyl]biphenyl-2-carbonsäure

Hergestellt analog Beispiel 9 aus 4′-[(2-n-Butyl-5-formylamino-benzimidazol-1-yl)-methyl]biphenyl-2-carbonsäure-tert.butylester und Trifluoressigsäure.
Ausbeute: 64,6 % der Theorie,
Schmelzpunkt: 229-230°C

| $C_{26}H_{25}N_3O_3$ (427,51) | | | | | | |
|---|---|---|---|---|---|---|
| Ber.: | C | 73,05 | H | 5,89 | N | 9,83 |
| Gef.: | | 73,31 | | 6,11 | | 9,58 |

Beispiel 166

4′-[(2-n-Butyl-5-(N-propanoyl-methylamino)-benzimidazol-1-yl)-methyl]biphenyl-2-carbonsäure

Hergestellt analog Beispiel 9 aus 4′-[(2-n-Butyl-5-(N-propanoyl-methylamino)-benzimidazol-1-yl)-methyl]biphenyl-2-carbonsäure-tert.butylester und Trifluoressigsäure.
Ausbeute: 77,8 % der Theorie,

Schmelzpunkt: 178-180°C

| $C_{29}H_{31}N_3O_3$ (469,59) | | | | | | |
|---|---|---|---|---|---|---|
| Ber.: | C | 74,18 | H | 6,65 | N | 8,95 |
| Gef.: | | 73,93 | | 6,70 | | 9,07 |

Beispiel 167

4′-[(2-n-Butyl-6-(N-methylaminocarbonyl-methylamino)-benzimidazol-1-yl)-methyl]biphenyl-2-carbonsäure

Hergestellt analog Beispiel 9 aus 4′-[(2-n-Butyl-6-(N-methylaminocarbonyl-methylamino)-benzimidazol-1-yl)-methyl]biphenyl-2-carbonsäure-tert.butylester und Trifluoressigsäure.
Ausbeute: 85,7 % der Theorie,
Schmelzpunkt: 163-165°C

| $C_{28}H_{30}N_4O_3$ (470,58) | | | | | | |
|---|---|---|---|---|---|---|
| Ber.: | C | 71,46 | H | 6,42 | N | 11,91 |
| Gef.: | | 71,33 | | 6,64 | | 11,74 |

Beispiel 168

4′-[(2-n-Butyl-6-(N-(n-butylaminocarbonyl)-methylamino)-benzimidazol-1-yl)-methyl]biphenyl-2-carbonsäure

Hergestellt analog Beispiel 9 aus 4′-[(2-n-Butyl-6-(N-(n-butylaminocarbonyl)-methylamino)-benzimidazol-1-yl)-methyl]biphenyl-2-carbonsäure-tert.butylester und Trifluoressigsäure.
Ausbeute: 90 % der Theorie,
Schmelzpunkt: 145-147°C

| $C_{31}H_{36}N_4O_3$ (512,65) | | | | | | |
|---|---|---|---|---|---|---|
| Ber.: | C | 72,64 | H | 7,08 | N | 10,93 |
| Gef.: | | 72,90 | | 7,16 | | 10,69 |

Beispiel 169

4′-(2-n-Butyl-5-(N-methylaminocarbonyl-n-pentylamino)-benzimidazol-1-yl)-methyl]biphenyl-2-carbonsäure

Hergestellt analog Beispiel 9 aus 4′-[(2-n-Butyl-5-(N-methylaminocarbonyl-n-pentylamino)-benzimidazol-1-yl)-methyl]biphenyl-2-carbonsäure-tert.butylester und Trifluoressigsäure.
Ausbeute: 75 % der Theorie,

Schmelzpunkt: 240-242°C

| $C_{32}H_{38}N_4O_3$ (526,69) | | | | | | |
|---|---|---|---|---|---|---|
| Ber.: | C | 72,97 | H | 7,27 | N | 10,64 |
| Gef.: | | 72,78 | | 7,23 | | 10,66 |

Beispiel 170

4′-[(2-n-Butyl-6-(N-propanoyl-methylamino)-benzimidazol-1-yl)-methyl]biphenyl-2-carbonsäure

Hergestellt analog Beispiel 9 aus 4′-[(2-n-Butyl-6-(N-propanoyl-methylamino)-benzimidazol-1-yl)-methyl]biphenyl-2-carbonsäure-tert.butylester und Trifluoressigsäure.
Ausbeute: 53,7 % der Theorie,
Schmelzpunkt: 152-154°C

| $C_{29}H_{31}N_3O_3$ (469,59) | | | | | | |
|---|---|---|---|---|---|---|
| Ber.: | C | 74,16 | H | 6,65 | N | 8,95 |
| Gef.: | | 73,96 | | 6,53 | | 8,97 |

Beispiel 171

4′-[(6-Acetamino-2-n-butyl-benzimidazol-1-yl)-methyl]biphenyl-2-carbonsäure

Hergestellt analog Beispiel 9 aus 4′-[(6-Acetamino-2-n-butyl-benzimidazol-1-yl)-methyl]biphenyl-2-carbonsäure-tert.butylester und Trifluoressigsäure.
Ausbeute: 87 % der Theorie,
Schmelzpunkt: 252-254°C

| $C_{27}H_{27}N_3O_3$ (441,53) | | | | | | |
|---|---|---|---|---|---|---|
| Ber.: | C | 73,45 | H | 6,16 | N | 9,52 |
| Gef.: | | 73,28 | | 5,95 | | 9,39 |

Beispiel 172

4′-[(2-n-Butyl-6-propionylamino-benzimidazol-1-yl)-methyl]biphenyl-2-carbonsäure

Hergestellt analog Beispiel 9 aus 4′-[(2-n-Butyl-6-propionylamino-benzimidazol-1-yl)-methyl]biphenyl-2-carbonsäure-tert.butylester und Trifluoressigsäure.
Ausbeute: 90 % der Theorie,

Schmelzpunkt: 269-271°C

| $C_{28}H_{29}N_3O_3$ (455,56) | | | | | | |
|---|---|---|---|---|---|---|
| Ber.: | C | 73,82 | H | 6,42 | N | 9,22 |
| Gef.: | | 73,99 | | 6,42 | | 9,18 |

Beispiel 173

4'-[(6-n-Butanoylamino-2-n-butyl-benzimidazol-1-yl)-methyl]biphenyl-2-carbonsäure

Hergestellt analog Beispiel 9 aus 4'-[(6-n-Butanoylamino-2-n-butyl-benzimidazol-1-yl)-methyl]biphenyl-2-carbonsäure-tert.butylester und Trifluoressigsäure.
Ausbeute: 79,1 % der Theorie,
Schmelzpunkt: 253-255°C

| $C_{29}H_{31}N_3O_3$ (469,58) | | | | | | |
|---|---|---|---|---|---|---|
| Ber.: | C | 74,18 | H | 6,66 | N | 8,96 |
| Gef.: | | 73,99 | | 6,65 | | 8,87 |

Beispiel 174

4'-[(2-n-Butyl-6-(n-butylaminocarbonylamino)-benzimidazol-1-yl)-methyl]biphenyl-2-carbonsäure

Hergestellt analog Beispiel 9 aus 4'-[(2-n-Butyl-6-(n-butylaminocarbonylamino)-benzimidazol-1-yl)-methyl]biphenyl-2-carbonsäure-tert.butylester und Trifluoressigsäure.
Ausbeute: 69,2 % der Theorie,
Schmelzpunkt: 239-242°C

| $C_{30}H_{34}N_4O_3$ (498,62) | | | | | | |
|---|---|---|---|---|---|---|
| Ber.: | C | 72,27 | H | 6,87 | N | 11,24 |
| Gef.: | | 71,92 | | 6,86 | | 10,93 |

Beispiel 175

4'-[(2-n-Butyl-6-(N-cyclohexylaminocarbonyl-methylamino)-benzimidazol-1-yl)-methyl]biphenyl-2-carbonsäure

Hergestellt analog Beispiel 9 aus 4'-[(2-n-Butyl-6-(N-cyclohexylaminocarbonyl-methylamino)-benzimidazol-1-yl)-methyl]biphenyl-2-carbonsäure-tert.butylester und Trifluoressigsäure.
Ausbeute: 80 % der Theorie,

Schmelzpunkt: 215-217°C

| $C_{33}H_{38}N_4O_3$ (538,69) | | | | | | |
|---|---|---|---|---|---|---|
| Ber.: | C | 73,58 | H | 7,11 | N | 10,40 |
| Gef.: | | 73,52 | | 7,19 | | 10,54 |

Beispiel 176

4'-(2-n-Butyl-6-(N-(dimethylaminocarbonyl)-methylamino)-benzimidazol-1-yl)-methyl]biphenyl-2-carbonsäure

Hergestellt analog Beispiel 9 aus 4'-[(2-n-Butyl-6-(N-(dimethylaminocarbonyl)-methylamino)-benzimidazol-1-yl)-methyl]biphenyl-2-carbonsäure-tert.butylester und Trifluoressigsäure.
Ausbeute: 66 % der Theorie,
Schmelzpunkt: 224-226°C

| $C_{29}H_{32}N_4O_3$ (484,60) | | | | | | |
|---|---|---|---|---|---|---|
| Ber.: | C | 71,88 | H | 6,66 | N | 11,56 |
| Gef.: | | 71,61 | | 6,92 | | 11,27 |

Beispiel 177

4'-(2-n-Butyl-6-n-pentanoylamino-benzimidazol-1-yl)-methyl]biphenyl-2-carbonsäure

Hergestellt analog Beispiel 9 aus 4'-[(2-n-Butyl-6-n-pentanoylamino-benzimidazol-1-yl)-methyl]biphenyl-2-carbonsäure-tert.butylester und Trifluoressigsäure.
Ausbeute: 74,5 % der Theorie,
Schmelzpunkt: 253-255°C

| $C_{30}H_{33}N_3O_3$ (483,61) | | | | | | |
|---|---|---|---|---|---|---|
| Ber.: | C | 74,57 | H | 6,88 | N | 8,70 |
| Gef.: | | 74,23 | | 7,08 | | 8,63 |

Beispiel 178

4'-[(2-n-Butyl-6-(N-(dimethylaminocarbonyl)-amino)-benzimidazol-1-yl-)-methyl]biphenyl-2-carbonsäure-trifluoracetat

Hergestellt analog Beispiel 9 aus 4'-[(2-n-Butyl-6-(N-(dimethylaminocarbonyl)-amino)-benzimidazol-1-yl)-methyl]biphenyl-2-carbonsäure-tert.butylester und Trifluoressigsäure.
Ausbeute: 83,1 % der Theorie,

Schmelzpunkt: 198-200°C

| $C_{28}H_{30}N_4O_3$ x $CF_3COOH$ (584,60) | | | | | | |
|---|---|---|---|---|---|---|
| Ber.: | C | 61,63 | H | 5,34 | N | 9,58 |
| Gef.: | | 61,62 | | 5,50 | | 9,68 |

Beispiel 179

4′-[(2-n-Butyl-6-(N-(n-butylaminocarbonyl)-methylamino)-benzimidazol-1-yl)-methyl]biphenyl-2-carbonsäure-trifluoracetat

Hergestellt analog Beispiel 9 aus 4′-[(2-n-Butyl-6-(N-(n-butylaminocarbonyl)-methylamino)-benzimidazol-1-yl)-methyl]biphenyl-2-carbonsäure-tert.butylester und Trifluoressigsäure.
Ausbeute: 70 % der Theorie,
Schmelzpunkt: 152-154°C

| $C_{31}H_{36}N_4O_3$ x $CF_3COOH$ (626,68) | | | | | | |
|---|---|---|---|---|---|---|
| Ber.: | C | 63,25 | H | 5,95 | N | 8,94 |
| Gef.: | | 63,18 | | 6,07 | | 9,03 |

Beispiel 180

4′-[(2-n-Butyl-6-(N-cyclohexylaminocarbonyl-n-pentylamino)-benzimidazol-1-yl)-methyl]biphenyl-2-carbonsäure

Hergestellt analog Beispiel 9 aus 4′-[(2-n-Butyl-6-(N-cyclohexylaminocarbonyl-n-pentylamino)-benzimidazol-1-yl)-methyl]biphenyl-2-carbonsäure-tert.butylester und Trifluoressigsäure.
Ausbeute: 46,7 % der Theorie,
Schmelzpunkt: 134-137°C

| $C_{37}H_{46}N_4O_3$ (594,80) | | | | | | |
|---|---|---|---|---|---|---|
| Ber.: | C | 74,72 | H | 7,79 | N | 9,42 |
| Gef.: | | 74,52 | | 7,85 | | 9,34 |

Beispiel 181

4′-[(2-n-Butyl-6-(N-acetyl-methylamino)-benzimidazol-1-yl)-methyl]biphenyl-2-carbonsäure-tert.butylester

4,5 g (9 mMol) 4′-[(2-n-Butyl-6-acetylamino-benzimidazol-1-yl)-methyl]biphenyl-2-carbonsäure-tert.butylester werden in 50 ml Dimethylformamid gelöst und mit 0,48 g (9 mMol + 10 %) Natriumhydrid-Suspension in Öl (50%ig) versetzt. Das Reaktionsgemisch wird 30 Minuten bei 80°C gerührt, auf Raumtemperatur abgekühlt und mit 1,5 g Methyljodid (9 mMol + 20 %) versetzt. Nach beendeter Reaktion wird im Vakuum eingedampft, in Essigester aufgenommen und mit Wasser gewaschen. Die organische Phase wird über Natriumsulfat getrocknet und im Vakuum eingedampft, wobei ein öliger Rückstand erhalten wird. Nach der Reinigung über eine Kieselgelsäule (Korngröße: 0,02-0,5 mm, Elutionsmittel: Methylenchlorid/Ethanol = 49:1, 24:1) erhält man ein gelbliches Öl.
Ausbeute: 3,7 g (80,4 % der Theorie),

Öl, $R_f$-Wert: 0,75 (Kieselgel: Methylenchlorid/Ethanol = 19:1)

| $C_{32}H_{37}N_3O_3$ (511,66) | | | | | | |
|---|---|---|---|---|---|---|
| Ber.: | C | 75,12 | H | 7,29 | N | 8,21 |
| Gef.: | | 74,99 | | 7,32 | | 8,22 |

Analog werden folgende Verbindungen hergestellt:
4'-[(2-n-Butyl-5-(N-propionyl-methylamino)-benzimidazol-1-yl)-methyl]biphenyl-2-carbonsäure-tert.butylester
Öl, $R_f$-Wert: 0,80 (Kieselgel: Essigester/Ethanol/Ammoniak = 90:10:1)
4'-[(2-n-Butyl-6-(N-propionyl-methylamino)-benzimidazol-1-yl)-methyl]biphenyl-2-carbonsäure-tert.butylester
Öl, $R_f$-Wert: 0,65 (Kieselgel: Methylenchlorid/Ethanol = 19:1)

Beispiel 182

4'-[(2-n-Butyl-6-(tetrahydropyran-2-yl-aminocarbonylamino)-benzimidazol-1-yl)-methyl]biphenyl-2-carbonsäure

Hergestellt analog Beispiel 7 aus 4'-[(6-Amino-2-n-butyl-benzimidazol-1-yl)-methyl]biphenyl-2-carbonsäure und Tetrahydropyran-2-yl-isocyanat.
Ausbeute: 35,7 % der Theorie,
Schmelzpunkt: 172-174°C

| $C_{31}H_{34}N_4O_4$ (562,64) | | | | | | |
|---|---|---|---|---|---|---|
| Ber.: | C | 70,70 | H | 6,51 | N | 10,64 |
| Gef.: | | 70,59 | | 6,77 | | 10,88 |

Beispiel 183

4'-[(2-n-Butyl-6-phenylacetamino-benzimidazol-1-yl)-methyl]biphenyl-2-carbonsäure-hemitrifluoracetat

Hergestellt analog Beispiel 9 aus 4'-[(2-n-Butyl-6-phenylacetamino-benzimidazol-1-yl)-methyl]biphenyl-2-carbonsäure-tert.butylester und Trifluoressigsäure.
Ausbeute: 29,2 % der Theorie,
Schmelzpunkt: 273-275°C (Zers.)

| $C_{33}H_{31}N_3O_3$ x 0,5 $CF_3COOH$ (574,64) | | | | | | |
|---|---|---|---|---|---|---|
| Ber.: | C | 71,07 | H | 5,53 | N | 7,31 |
| Gef.: | | 71,01 | | 5,60 | | 7,11 |

Beispiel 184

4'-[(2-n-Butyl-6-(N-(n-hexylaminocarbonyl)-cyclohexylamino)-benzimidazol-1-yl)-methyl]biphenyl-2-carbonsäure

Hergestellt analog Beispiel 9 aus 4'-[(2-n-Butyl-6-(N-(n-hexylaminocarbonyl)-cyclohexylamino)-benzimidazol-1-yl)-methyl]biphenyl-2-carbonsäure-tert.butylester und Trifluoressigsäure.
Ausbeute: 97,1 % der Theorie,

Schmelzpunkt: 196-197°C

| $C_{38}H_{48}N_4O_3$ (608,82) | | | | | | |
|---|---|---|---|---|---|---|
| Ber.: | C | 74,97 | H | 7,95 | N | 9,20 |
| Gef.: | | 74,75 | | 7,92 | | 9,19 |

Beispiel 185

4′-[(2-n-Butyl-6-cyclohexylcarbonylamino-benzimidazol-1-yl)-methyl]biphenyl-2-carbonsäure

Hergestellt analog Beispiel 9 aus 4′-[(2-n-Butyl-6-cyclohexyl-carbonylamino-benzimidazol-1-yl)-methyl]biphenyl-2-carbonsäure-tert.butylester und Trifluoressigsäure.
Ausbeute: 68,4 % der Theorie,
Schmelzpunkt: 298-300°C (Zers.)

| $C_{32}H_{35}N_3O_4$ (509,65) | | | | | | |
|---|---|---|---|---|---|---|
| Ber.: | C | 75,41 | H | 6,92 | N | 8,24 |
| Gef.: | | 75,38 | | 6,78 | | 8,11 |

Beispiel 186

4′-[(6-Benzyloxycarbonylamino-2-n-butyl-benzimidazol-1-yl)-methyl]biphenyl-2-carbonsäure

Hergestellt analog Beispiel 9 aus 4′-[(6-Benzyloxycarbonylamino-2-n-butyl-benzimidazol-1-yl)-methyl]biphenyl-2-carbonsäure-tert.butylester und Trifluoressigsäure.
Ausbeute: 64,9 % der Theorie,
Schmelzpunkt: 238-240°C (Zers.)

| $C_{33}H_{31}N_3O_4$ (533,63) | | | | | | |
|---|---|---|---|---|---|---|
| Ber.: | C | 74,28 | H | 5,86 | N | 7,87 |
| Gef.: | | 74,14 | | 5,97 | | 7,72 |

Beispiel 187

4′-[(2-n-Butyl-6-(2-cyclohexyl-ethylamino)-benzimidazol-1-yl)-methyl]biphenyl-2-carbonsäure

Hergestellt analog Beispiel 9 aus 4′-[(2-n-Butyl-6-(2-cyclohexyl-ethylamino)-benzimidazol-1-yl)-methyl]biphenyl-2-carbonsäure-tert.butylester und Trifluoressigsäure.
Ausbeute: 66,6 % der Theorie,

Schmelzpunkt: 217-219°C

| $C_{33}H_{39}N_3O_2$ (509,69) | | | | | | |
|---|---|---|---|---|---|---|
| Ber.: | C | 77,77 | H | 7,71 | N | 8,24 |
| Gef.: | | 77,57 | | 7,56 | | 8,23 |

Beispiel 188

4′-[(2-n-Butyl-6-cyclohexylmethylaminocarbonyl-benzimidazol-1-yl)-methyl]biphenyl-2-carbonsäure

Hergestellt analog Beispiel 9 aus 4′-[(2-n-Butyl-6-cyclohexylmethylaminocarbonyl-benzimidazol-1-yl)-methyl]biphenyl-2-carbonsäure-tert.butylester und Trifluoressigsäure.
Ausbeute: 80,4 % der Theorie,
Schmelzpunkt: 239-241°C

| $C_{33}H_{37}N_3O_3$ (523,67) | | | | | | |
|---|---|---|---|---|---|---|
| Ber.: | C | 75,69 | H | 7,12 | N | 8,02 |
| Gef.: | | 75,53 | | 6,94 | | 7,97 |

Beispiel 189

4′-[(2-n-Butyl-6-cyclohexylaminocarbonyl-benzimidazol-1-yl)-methyl]biphenyl-2-carbonsäure

Hergestellt analog Beispiel 9 aus 4′-(2-n-Butyl-6-cyclohexylaminocarbonyl-benzimidazol-1-yl)-methyl]biphenyl-2-carbonsäure-tert.butylester und Trifluoressigsäure.
Ausbeute: 89,2 % der Theorie,
Schmelzpunkt: 305-308°C (Zers.)

| $C_{32}H_{35}N_3O_3$ (509,65) | | | | | | |
|---|---|---|---|---|---|---|
| Ber.: | C | 75,42 | H | 6,92 | N | 8,24 |
| Gef.: | | 75,31 | | 7,03 | | 8,11 |

Beispiel 190

4′-[(2-n-Butyl-6-(N-methyl-n-butylaminocarbonyl)-benzimidazol-1-yl)-methyl]biphenyl-2-carbonsäure

Hergestellt analog Beispiel 9 aus 4′-[(2-n-Butyl-6-(N-methyl-n-butylaminocarbonyl)-benzimidazol-1-yl)-methyl]biphenyl-2-carbonsäure-tert.butylester und Trifluoressigsäure.
Ausbeute: 94,5 % der Theorie,

Schmelzpunkt: 176-178°C

| $C_{31}H_{35}N_3O_3$ (497,64) | | | | | | |
|---|---|---|---|---|---|---|
| Ber.: | C | 74,82 | H | 7,09 | N | 8,44 |
| Gef.: | | 74,98 | | 7,21 | | 8,50 |

Beispiel 191

4'-[(2-n-Butyl-6-ethoxycarbonyl-benzimidazol-1-yl)-methyl]biphenyl-2-carbonsäure

Hergestellt analog Beispiel 9 aus 4'-[(2-n-Butyl-6-ethoxycarbonyl-benzimidazol-1-yl)-methyl]biphenyl-2-carbonsäure-tert.butylester und Trifluoressigsäure.
Ausbeute: 59,7 % der Theorie,
Schmelzpunkt: 219-220°C

| $C_{28}H_{28}N_2O_4$ (456,54) | | | | | | |
|---|---|---|---|---|---|---|
| Ber.: | C | 73,66 | H | 6,18 | N | 6,14 |
| Gef.: | | 73,49 | | 6,13 | | 5,94 |

Beispiel 192

4'-[(2-n-Butyl-5-n-butylaminocarbonyl-benzimidazol-1-yl)-methyl]biphenyl-2-carbonsäure

Hergestellt analog Beispiel 9 aus 4'-(2-n-Butyl-5-n-butylaminocarbonyl-benzimidazol-1-yl)-methyl]biphenyl-2-carbonsäure-tert.butylester und Trifluoressigsäure.
Ausbeute: 48,9 % der Theorie,
Schmelzpunkt: 209-210°C

| $C_{30}H_{33}N_3O_3$ (483,61) | | | | | | |
|---|---|---|---|---|---|---|
| Ber.: | C | 74,51 | H | 6,88 | N | 8,69 |
| Gef.: | | 74,54 | | 6,79 | | 8,79 |

Beispiel 193

4'-[(2-n-Butyl-6-(3-cyclohexyl-piperidino)-benzimidazol-1-yl)-methyl]biphenyl-2-carbonsäure-sesquitrifluouracetat

Hergestellt analog Beispiel 9 aus 4'-[(2-n-Butyl-6-(3-cyclohexyl-piperidino)-benzimidazol-1-yl)-methyl]biphenyl-2-carbonsäure-tert.butylester und Trifluoressigsäure.
Ausbeute: 85,0 % der Theorie,

Schmelzpunkt: 155-157°C

| $C_{36}H_{43}N_3O_2$ x 1,5 $CF_3COOH$ (720,80) | | | | | | |
|---|---|---|---|---|---|---|
| Ber.: | C | 64,99 | H | 6,22 | N | 5,83 |
| Gef.: | | 64,88 | | 6,41 | | 5,95 |

Beispiel 194

4′-[(2-n-Butyl-6-(cis- und trans-decahydronaphth-2-yl-amino)-benzimidazol-1-yl)-methyl]biphenyl-2-carbonsäure-dihydrochlorid

Hergestellt analog Beispiel 9 aus 4′-[(2-n-Butyl-6-(cis- und trans-decahydronaphth-2-yl-amino)-benzimidazol-1-yl)-methyl]biphenyl-2-carbonsäure-tert.butylester und Trifluoressigsäure.
Ausbeute: 24,0 % der Theorie,
Schmelzpunkt: ab 132°C

| $C_{35}H_{41}N_3O_2$ x 2 HCl (608,65) | | | | | | |
|---|---|---|---|---|---|---|
| Ber.: | C | 69,07 | H | 7,12 | N | 6,90 |
| Gef.: | | 68,98 | | 7,23 | | 6,97 |

Beispiel 195

4′-[(2-n-Butyl-6-cyclohexylamino-benzimidazol-1-yl)-methyl]biphenyl-2-carbonsäure-sesquitrifluoracetat

Hergestellt analog Beispiel 9 aus 4′-[(2-n-Butyl-6-cyclohexylamino-benzimidazol-1-yl)-methyl]biphenyl-2-carbonsäure-tert.butylester und Trifluoressigsäure.
Ausbeute: 99,0 % der Theorie,
Schmelzpunkt: 64-66°C (amorph)

| $C_{31}H_{35}N_3O_2$ x 1,5 $CF_3COOH$ (652,68) | | | | | | |
|---|---|---|---|---|---|---|
| Ber.: | C | 62,57 | H | 5,64 | N | 6,44 |
| Gef.: | | 62,68 | | 5,81 | | 6,25 |

Beispiel 196

4′-[(2-n-Butyl-6-(2-isopropyl-5-methyl-cyclohexyloxycarbonylamino)-benzimidazol-1-yl)-methyl]biphenyl-2-carbonsäure-semitrifluoracetat

Hergestellt analog Beispiel 9 aus 4′-(2-n-Butyl-6-(2-isopropyl-5-methyl-cyclohexyloxycarbonylamino)-benzimidazol-1-yl)-methyl]biphenyl-2-carbonsäure-tert.butylester und Trifluoressigsäure.
Ausbeute: 94,1 % der Theorie,

Schmelzpunkt: 149-151°C

| $C_{36}H_{43}N_3O_4$ x 0,5 $CF_3COOH$ (638,77) | | | | | | |
|---|---|---|---|---|---|---|
| Ber.: | C | 69,57 | H | 6,86 | N | 6,58 |
| Gef.: | | 69,39 | | 6,91 | | 6,56 |

Beispiel 197

4′-[(2-n-Butyl-6-cyclohexylacetamino-benzimidazol-1-yl)-methyl]biphenyl-2-carbonsäure

Hergestellt analog Beispiel 9 aus 4′-[(2-n-Butyl-6-cyclohexylacetamino-benzimidazol-1-yl)-methyl]biphenyl-2-carbonsäure-tert.butylester und Trifluoressigsäure.

Beispiel 198

4′-[(2-n-Butyl-benzimidazol-1-yl)methyl]biphenyl-2-carbonsäure-(N-benzolsulfonyl)-amid

960 mg (2,5 mMol) 4′-[(2-n-Butyl-benzimidazol-1-yl)-methyl]biphenyl-2-carbonsäure werden in 25 ml Methylenchlorid gelöst, mit 2 ml Thionylchlorid versetzt und eine Stunde unter Rückfluß erhitzt. Anschließend destilliert man das Lösungsmittel ab und dampft 2 x mal nach Zugabe von Methylenchlorid ab. Der Rückstand wird mit 390 mg (2,5 mMol) Benzolsulfonamid versetzt und eine Stunde auf 140°C erhitzt. Nach dem Abkühlen wird das erhaltene Öl in Essigester/Natriumchlorid-Lösung aufgenommen und 3 x mal mit Essigester extrahiert. Die vereinigten Essigesterphasen werden über Natriumsulfat getrocknet, eingeengt und das erhaltene Rohprodukt über eine Kieselgelsäule mit Methylenchlorid/Ethanol als Eluens gereinigt.
Ausbeute: 0,15 g (11 % der Theorie),
Schmelzpunkt: 131-132°C

| $C_{31}H_{29}N_3O_3S$ (523,65) | | | | | | |
|---|---|---|---|---|---|---|
| Ber.: | C | 71,11 | H | 5,58 | N | 8,02 |
| Gef.: | | 70,96 | | 5,49 | | 8,21 |

Analog werden folgende Verbindungen erhalten:
4′-[(2-n-Butyl-6-methoxy-benzimidazol-1-yl)-methyl]biphenyl-2-carbonsäure-(N-methansulfonyl)-amid
4′-[(2-Methoxymethyl-benzimidazol-1-yl)-methyl]biphenyl-2-carbonsäure-(N-methansulfonyl)-amid
4′-[(2-n-Pentyl-benzimidazol-1-yl)-methyl]biphenyl-2-carbonsäure-(N-methansulfonyl)-amid
4′-[(2-n-Butyl-4-methyl-7-methoxy-benzimidazol-1-yl)-methyl]biphenyl-2-carbonsäure-(N-methansulfonyl)-amid
4′-[(2-n-Propyl-benzimidazol-1-yl)-methyl]biphenyl-2-carbonsäure-(N-methansulfonyl)-amid
4′-[(2-n-Butyl-5-acetamino-benzimidazol-1-yl)-methyl]biphenyl-2-carbonsäure-(N-methansulfonyl)-amid
4′-[(2-n-Butyl-5-chlor-benzimidazol-1-yl)-methyl]biphenyl-2-carbonsäure-(N-methansulfonyl)-amid
4′-[(2-n-Butyl-5-butylaminocarbonylamino-benzimidazol-1-yl)-methyl]biphenyl-2-carbonsäure-(N-methansulfonyl)-amid
4′-[(2-n-Butyl-6-phenylaminocarbonylamino-benzimidazol-1-yl)-methyl]biphenyl-2-carbonsäure-(N-methansulfonyl)-amid
4′-[(2-n-Butyl-6-cyclohexylaminocarbonylamino-benzimidazol-1-yl)-methyl]biphenyl-2-carbonsäure-(N-methansulfonyl)-amid
4′-[(2-n-Butyl-5-butanoylamino-benzimidazol-1-yl)-methyl]biphenyl-2-carbonsäure-(N-methansulfonyl)-amid
4′-[(2-n-Butyl-6-chlor-benzimidazol-1-yl)-methyl]biphenyl-2-carbonsäure-(N-methansulfonyl)-amid
4′-[(2-n-Butyl-5-methansulfonamino-benzimidazol-1-yl)-methyl]biphenyl-2-carbonsäure-(N-methansulfonyl)-amid
4′-[(2-n-Butyl-6-ethoxycarbonylamino-benzimidazol-1-yl)-methyl]biphenyl-2-carbonsäure-(N-methansulfonyl)-amid
4′-[(2-n-Butyl-6-isopropylsulfonamino-benzimidazol-1-yl)-methyl]biphenyl-2-carbonsäure-(N-methansulfonyl)-amid
4′-[(2-n-Butyl-4-butanoylamino-benzimidazol-1-yl)-methyl]biphenyl-2-carbonsäure-(N-methansulfonyl)-amid
4′-[(2-n-Butyl-6-dimethylamino-benzimidazol-1-yl)-methyl]biphenyl-2-carbonsäure-(N-methansulfonyl)-amid
4′-[(2-n-Butyl-5,6-dimethoxy-benzimidazol-1-yl)-methyl]biphenyl-2-carbonsäure-(N-methansulfonyl)-amid

4′-[(2-(1-Butin-4-yl)-benzimidazol-1-yl]-methyl]biphenyl-2-carbonsäure-(N-methansulfonyl)-amid
4′-[(2-n-Butyl-5- und 6-trifluormethyl-benzimidazol-1-yl)-methyl]biphenyl-2-carbonsäure-(N-methansulfonyl)-amid
4′-[(2-n-Butyl-5- und 6-n-butylaminocarbonyl-benzimidazol-1-yl)-methyl]biphenyl-2-carbonsäure-(N-methansulfonyl)-amid
4′-[(2-n-Butyl-5-dimethylaminosulfonyl-benzimidazol-1-yl)-methyl]biphenyl-2-carbonsäure-(N-methansulfonyl)-amid
4′-[(2-n-Butyl-7-cyano-benzimidazol-1-yl)-methyl]biphenyl-2-carbonsäure-(N-methansulfonyl)-amid
4′-[(2-n-Butyl-6-cyclohexylaminocarbonyl-benzimidazol-1-yl)-methyl]biphenyl-2-carbonsäure-(N-methansulfonyl)-amid
4′-[(2-n-Butyl-6-(N-methyl-n-butylaminocarbonyl)-benzimidazol-1-yl)-methyl]biphenyl-2-carbonsäure-(N-methansulfonyl)-amid
4′-[(2-n-Butyl-6-cyclohexylcarbonylamino-benzimidazol-1-yl)-methyl]biphenyl-2-carbonsäure-(N-methansulfonyl)-amid
4′-[(2-n-Butyl-6-(N-cyclohexylaminocarbonyl-n-pentylamino)-benzimidazol-1-yl)-methyl]biphenyl-2-carbonsäure-(N-methansulfonyl)-amid
4′-[(6-n-Butanoylamino-2-n-butyl-benzimidazol-1-yl)-methyl]biphenyl-2-carbonsäure-(N-methansulfonyl)-amid
4′-[(2-n-Butyl-6-propionylamino-benzimidazol-1-yl)-methyl]biphenyl-2-carbonsäure-(N-methansulfonyl)-amid
4′-[(2-n-Butyl-5-(N-methylaminocarbonyl-n-pentylamino)-benzimidazol-1-yl)-methyl]biphenyl-2-carbonsäure-(N-methansulfonyl)-amid
4′-[(2-n-Butyl-6-(N-(n-butylaminocarbonyl)-methylamino)-benzimidazol-1-yl)-methyl]biphenyl-2-carbonsäure-(N-methansulfonyl)-amid
4′-[(2-n-Butyl-5-(N-propanoyl-methylamino)-benzimidazol-1-yl)-methyl]biphenyl-2-carbonsäure-(N-methansulfonyl)-amid
4′-[(2-n-Butyl-5-formylamino-benzimidazol-1-yl)-methyl]biphenyl-2-carbonsäure-(N-methansulfonyl)-amid
4′-[(2-n-Butyl-6-(N-methylaminocarbonyl-n-pentylamino)-benzimidazol-1-yl)-methyl]biphenyl-2-carbonsäure-(N-methansulfonyl)-amid
4′-[(6-Aminothiocarbonylamino-2-n-butyl-benzimidazol-1-yl)-methyl]biphenyl-2-carbonsäure-(N-methansulfonyl)-amid
4′-[(2-n-Butyl-7-methoxy-benzimidazol-1-yl)-methyl]biphenyl-2-carbonsäure-(N-methansulfonyl)-amid
4′-[(2-n-Butyl-6-cyclohexylmethylaminocarbonyl-benzimidazol-1-yl)-methyl]biphenyl-2-carbonsäure-(N-methansulfonyl)-amid
4′-[(2-n-Butyl-6-methoxy-benzimidazol-1-yl)-methyl]biphenyl-2-carbonsäure-(N-trifluormethansulfonyl)-amid
4′-[(2-Methoxymethyl-benzimidazol-1-yl)-methyl]biphenyl-2-carbonsäure-(N-trifluormethansulfonyl)-amid
4′-[(2-n-Pentyl-benzimidazol-1-yl)-methyl]biphenyl-2-carbonsäure-(N-trifluormethansulfonyl)-amid
4′-[(2-n-Butyl-4-methyl-7-methoxy-benzimidazol-1-yl)-methyl]biphenyl-2-carbonsäure-(N-trifluormethansulfonyl)-amid
4′-[(2-n-Propyl-benzimidazol-1-yl)-methyl]biphenyl-2-carbonsäure-(N-trifluormethansulfonyl)-amid
4′-[(2-n-Butyl-5-acetamino-benzimidazol-1-yl)-methyl]biphenyl-2-carbonsäure-(N-trifluormethansulfonyl)-amid
4′-[(2-n-Butyl-5-chlor-benzimidazol-1-yl)-methyl]biphenyl-2-carbonsäure-(N-trifluormethansulfonyl)-amid
4′-[(2-n-Butyl-5-butylaminocarbonylamino-benzimidazol-1-yl)-methyl]biphenyl-2-carbonsäure-(N-trifluormethansulfonyl)-amid
4′-[(2-n-Butyl-6-phenylaminocarbonylamino-benzimidazol-1-yl)-methyl]biphenyl-2-carbonsäure-(N-trifluormethansulfonyl)-amid
4′-[(2-n-Butyl-6-cyclohexylaminocarbonylamino-benzimidazol-1-yl)-methyl]biphenyl-2-carbonsäure-(N-trifluormethansulfonyl)-amid
4′-[(2-n-Butyl-5-butanoylamino-benzimidazol-1-yl)-methyl]biphenyl-2-carbonsäure-(N-trifluormethansulfonyl)-amid
4′-[(2-n-Butyl-6-chlor-benzimidazol-1-yl)-methyl]biphenyl-2-carbonsäure-(N-trifluormethansulfonyl)-amid
4′-[(2-n-Butyl-5-methansulfonamino-benzimidazol-1-yl)-methyl]biphenyl-2-carbonsäure-(N-trifluormethansulfonyl)-amid
4′-[(2-n-Butyl-6-ethoxycarbonylamino-benzimidazol-1-yl)-methyl]biphenyl-2-carbonsäure-(N-trifluormethansulfonyl)-amid
4′-[(2-n-Butyl-6-isopropylsulfonamino-benzimidazol-1-yl)-methyl]biphenyl-2-carbonsäure-(N-trifluormethansulfonyl)-amid
4′-[(2-n-Butyl-4-butanoylamino-benzimidazol-1-yl)-methyl]biphenyl-2-carbonsäure-(N-trifluormethansulfonyl)-amid
4′-[(2-n-Butyl-6-dimethylamino-benzimidazol-1-yl)-methyl]biphenyl-2-carbonsäure-(N-trifluormethansulfonyl)-amid
4′-[(2-n-Butyl-5,6-dimethoxy-benzimidazol-1-yl)-methyl]biphenyl-2-carbonsäure-(N-trifluormethansulfonyl)-amid
4′-[(2-(1-Butin-4-yl)-benzimidazol-1-yl]-methyl]biphenyl-2-carbonsäure-(N-trifluormethansulfonyl)-amid
4′-[(2-n-Butyl-5- und 6-trifluormethyl-benzimidazol-1-yl)-methyl]biphenyl-2-carbonsäure-(N-trifluormethansulfonyl)-amid
4′-[(2-n-Butyl-5- und 6-n-butylaminocarbonyl-benzimidazol-1-yl)-methyl]biphenyl-2-carbonsäure-(N-trifluormethansulfonyl)-amid
4′-[(2-n-Butyl-5-dimethylaminosulfonyl-benzimidazol-1-yl)-methyl]biphenyl-2-carbonsäure-(N-trifluormethansulfonyl)-amid
4′-[(2-n-Butyl-7-cyano-benzimidazol-1-yl)-methyl]biphenyl-2-carbonsäure-(N-trifluormethansulfonyl)-amid

4′-[(2-n-Butyl-6-cyclohexylaminocarbonyl-benzimidazol-1-yl)-methyl]biphenyl-2-carbonsäure-(N-trifluormethansulfonyl)-amid
4′-[(2-n-Butyl-6-(N-methyl-n-butylaminocarbonyl)-benzimidazol-1-yl)-methyl]biphenyl-2-carbonsäure-(N-trifluormethansulfonyl)-amid
4′-[(2-n-Butyl-6-cyclohexylcarbonylamino-benzimidazol-1-yl)-methyl]biphenyl-2-carbonsäure-(N-trifluormethansulfonyl)-amid
4′-[(2-n-Butyl-6-(N-cyclohexylaminocarbonyl-n-pentylamino)-benzimidazol-1-yl)-methyl]biphenyl-2-carbonsäure-(N-trifluormethansulfonyl)-amid
4′-[(6-n-Butanoylamino-2-n-butyl-benzimidazol-1-yl)-methyl]biphenyl-2-carbonsäure-(N-trifluormethansulfonyl)-amid
4′-[(2-n-Butyl-6-propionylamino-benzimidazol-1-yl)-methyl]biphenyl-2-carbonsäure-(N-trifluormethansulfonyl)-amid
4′-[(2-n-Butyl-5-(N-methylaminocarbonyl-n-pentylamino)-benzimidazol-1-yl)-methyl]biphenyl-2-carbonsäure-(N-trifluormethansulfonyl)-amid
4′-[(2-n-Butyl-6-(N-(n-butylaminocarbonyl)-methylamino)-benzimidazol-1-yl)-methyl]biphenyl-2-carbonsäure-(N-trifluormethansulfonyl)-amid
4′-[(2-n-Butyl-5-(N-propanoyl-methylamino)-benzimidazol-1-yl)-methyl]biphenyl-2-carbonsäure-(N-trifluormethansulfonyl)-amid
4′-[(2-n-Butyl-5-formylamino-benzimidazol-1-yl)-methyl]biphenyl-2-carbonsäure-(N-trifluormethansulfonyl)-amid
4′-[(2-n-Butyl-6-(N-methylaminocarbonyl-n-pentylamino)-benzimidazol-1-yl)-methyl]biphenyl-2-carbonsäure-(N-trifluormethansulfonyl)-amid
4′-[(6-Aminothiocarbonylamino-2-n-butyl-benzimidazol-1-yl)-methyl]biphenyl-2-carbonsäure-(N-trifluormethansulfonyl)-amid
4′-[(2-n-Butyl-7-methoxy-benzimidazol-1-yl)-methyl]biphenyl-2-carbonsäure-(N-trifluormethansulfonyl)-amid
4′-[(2-n-Butyl-6-cyclohexylmethylaminocarbonyl-benzimidazol-1-yl)-methyl]biphenyl-2-carbonsäure-(N-trifluormethansulfonyl)-amid
4′-[(2-n-Butyl-6-methoxy-benzimidazol-1-yl)-methyl]biphenyl-2-carbonsäure-(N-benzolsulfonyl)-amid
4′-[(2-Methoxymethyl-benzimidazol-1-yl)-methyl]biphenyl-2-carbonsäure-(N-benzolsulfonyl)-amid
4′-[(2-n-Pentyl-benzimidazol-1-yl)-methyl]biphenyl-2-carbonsäure-(N-benzolsulfonyl)-amid
4′-[(2-n-Butyl-4-methyl-7-methoxy-benzimidazol-1-yl)-methyl]biphenyl-2-carbonsäure-(N-benzolsulfonyl)-amid
4′-[(2-n-Propyl-benzimidazol-1-yl)-methyl]biphenyl-2-carbonsäure-(N-benzolsulfonyl)-amid
4′-[(2-n-Butyl-5-acetamino-benzimidazol-1-yl)-methyl]biphenyl-2-carbonsäure-(N-benzolsulfonyl)-amid
4′-[(2-n-Butyl-5-chlor-benzimidazol-1-yl)-methyl]biphenyl-2-carbonsäure-(N-benzolsulfonyl)-amid
4′-[(2-n-Butyl-5-butylaminocarbonylamino-benzimidazol-1-yl)-methyl]biphenyl-2-carbonsäure-(N-benzolsulfonyl)-amid
4′-[(2-n-Butyl-6-phenylaminocarbonylamino-benzimidazol-1-yl)-methyl]biphenyl-2-carbonsäure-(N-benzolsulfonyl)-amid
4′-[(2-n-Butyl-6-cyclohexylaminocarbonylamino-benzimidazol-1-yl)-methyl]biphenyl-2-carbonsäure-(N-benzolsulfonyl)-amid
4′-[(2-n-Butyl-5-butanoylamino-benzimidazol-1-yl)-methyl]biphenyl-2-carbonsäure-(N-benzolsulfonyl)-amid
4′-[(2-n-Butyl-6-chlor-benzimidazol-1-yl)-methyl]biphenyl-2-carbonsäure-(N-benzolsulfonyl)-amid
4′-[(2-n-Butyl-5-methansulfonamino-benzimidazol-1-yl)-methyl]biphenyl-2-carbonsäure-(N-benzolsulfonyl)-amid
4′-[(2-n-Butyl-6-ethoxycarbonylamino-benzimidazol-1-yl)-methyl]biphenyl-2-carbonsäure-(N-benzolsulfonyl)-amid
4′-[(2-n-Butyl-6-isopropylsulfonamino-benzimidazol-1-yl)-methyl]biphenyl-2-carbonsäure-(N-benzolsulfonyl)-amid
4′-[(2-n-Butyl-4-butanoylamino-benzimidazol-1-yl)-methyl]biphenyl-2-carbonsäure-(N-benzolsulfonyl)-amid
4′-[(2-n-Butyl-6-dimethylamino-benzimidazol-1-yl)-methyl]biphenyl-2-carbonsäure-(N-benzolsulfonyl)-amid
4′-[(2-n-Butyl-5,6-dimethoxy-benzimidazol-1-yl)-methyl]biphenyl-2-carbonsäure-(N-benzolsulfonyl)-amid
4′-[(2-(1-Butin-4-yl)-benzimidazol-1-yl]-methyl]biphenyl-2-carbonsäure-(N-benzolsulfonyl)-amid
4′-[(2-n-Butyl-5- und 6-trifluormethyl-benzimidazol-1-yl)-methyl]biphenyl-2-carbonsäure-(N-benzolsulfonyl)-amid
4′-[(2-n-Butyl-5- und 6-n-butylaminocarbonyl-benzimidazol-1-yl)-methyl]biphenyl-2-carbonsäure-(N-benzolsulfonyl)-amid
4′-[(2-n-Butyl-5-dimethylaminosulfonyl-benzimidazol-1-yl)-methyl]biphenyl-2-carbonsäure-(N-benzolsulfonyl)-amid
4′-[(2-n-Butyl-7-cyano-benzimidazol-1-yl)-methyl]biphenyl-2-carbonsäure-(N-benzolsulfonyl)-amid
4′-[(2-n-Butyl-6-cyclohexylaminocarbonyl-benzimidazol-1-yl)-methyl]biphenyl-2-carbonsäure-(N-benzolsulfonyl)-amid
4′-[(2-n-Butyl-6-(N-methyl-n-butylaminocarbonyl)-benzimidazol-1-yl)-methyl]biphenyl-2-carbonsäure-(N-benzolsulfonyl)-amid
4′-[(2-n-Butyl-6-cyclohexylcarbonylamino-benzimidazol-1-yl)-methyl]biphenyl-2-carbonsäure-(N-benzolsulfonyl)-amid
4′-[(2-n-Butyl-6-(N-cyclohexylaminocarbonyl-n-pentylamino)-benzimidazol-1-yl)-methyl]biphenyl-2-carbonsäure-(N-benzolsulfonyl)-amid
4′-[(6-n-Butanoylamino-2-n-butyl-benzimidazol-1-yl)-methyl]biphenyl-2-carbonsäure-(N-benzolsulfonyl)-amid
4′-[(2-n-Butyl-6-propionylamino-benzimidazol-1-yl)-methyl]biphenyl-2-carbonsäure-(N-benzolsulfonyl)-amid
4′-[(2-n-Butyl-5-(N-methylaminocarbonyl-n-pentylamino)-benzimidazol-1-yl)-methyl]biphenyl-2-carbonsäure-(N-benzolsulfonyl)-amid
4′-[(2-n-Butyl-6-(N-(n-butylaminocarbonyl)-methylamino)-benzimidazol-1-yl)-methyl]biphenyl-2-carbonsäure-(N-benzolsulfonyl)-amid
4′-[(2-n-Butyl-5-(N-propanoyl-methylamino)-benzimidazol-1-yl)-methyl]biphenyl-2-carbonsäure-(N-benzolsulfonyl)-amid
4′-[(2-n-Butyl-5-formylamino-benzimidazol-1-yl)-methyl]biphenyl-2-carbonsäure-(N-benzolsulfonyl)-amid
4′-[(2-n-Butyl-6-(N-methylaminocarbonyl-n-pentylamino)-benzimidazol-1-yl)-methyl]biphenyl-2-carbonsäure-(N-benzolsulfonyl)-amid
4′-[(6-Aminothiocarbonylamino-2-n-butyl-benzimidazol-1-yl)-methyl]biphenyl-2-carbonsäure-(N-benzolsulfonyl)-amid
4′-[(2-n-Butyl-7-methoxy-benzimidazol-1-yl)-methyl]biphenyl-2-carbonsäure-(N-benzolsulfonyl)-amid
4′-[(2-n-Butyl-6-cyclohexylmethylaminocarbonyl-benzimidazol-1-yl)-methyl]biphenyl-2-carbonsäure-(N-benzolsulfonyl)-amid

Beispiel 199

4′-[(2-n-Butyl-6-formylamino-benzimidazol-1-yl)-methyl]biphenyl-2-carbonsäure x 0,75 Trifluoressigsäure

Hergestellt analog Beispiel 9 aus 4′-[(2-n-Butyl-6-formylamino-benzimidazol-1-yl)-methyl]biphenyl-2-carbonsäure-tert.butylester und Trifluoressigsäure.
Ausbeute: 84,0 % der Theorie,
Schmelzpunkt: 110-112°C (amorph)

| $C_{33}H_{37}N_3O_3$ x 0,75 $CF_3COOH$ (513,02) | | | | | | |
|---|---|---|---|---|---|---|
| Ber.: | C | 64,38 | H | 5,06 | N | 8,19 |
| Gef.: | | 64,70 | | 5,25 | | 7,91 |

Beispiel 200

4′-[(2-n-Butyl-6-(N-cyclohexylaminocarbonyl-cyclohexylamino)-benzimidazol-1-yl)-methyl]biphenyl-2-carbonsäure-semitrifluoracetat

Hergestellt analog Beispiel 9 aus 4′-[(2-n-Butyl-6-(N-cyclohexylaminocarbonyl-cyclohexylamino)-benzimidazol-1-yl)-me thyl]biphenyl-2-carbonsäure-tert.butylester und Trifluoressigsäure.
Ausbeute: 91,9 % der Theorie,
Schmelzpunkt: 130-132°C (amorph)

| $C_{38}H_{46}N_4O_3$ x 0,5 $CF_3COOH$ (663,82) | | | | | | |
|---|---|---|---|---|---|---|
| Ber.: | C | 70,57 | H | 7,06 | N | 8,44 |
| Gef.: | | 70,48 | | 7,13 | | 8,60 |

Beispiel 201

4′-[(2-n-Butyl-6-(N-(n-butylaminocarbonyl)-cyclohexylamino)-benzimidazol-1-yl)-methyl]biphenyl-2-carbonsäure

Hergestellt analog Beispiel 9 aus 4′-[(2-n-Butyl-6-(N-(n-butylaminocarbonyl)-cyclohexylamino)-benzimidazol-1-yl)-methyl]biphenyl-2-carbonsäure-tert.butylester und Trifluoressigsäure.
Ausbeute: 85,7 % der Theorie,

Schmelzpunkt: 227-228°C

| $C_{36}H_{44}N_4O_3$ (580,77) | | | | | | |
|---|---|---|---|---|---|---|
| Ber.: | C | 74,45 | H | 7,64 | N | 9,65 |
| Gef.: | | 74,32 | | 7,70 | | 9,50 |

Beispiel 202

4′-[(2-n-Butyl-6-(N-methylaminocarbonyl-cyclohexylamino)-benzimidazol-1-yl)-methyl]biphenyl-2-carbonsäure

Hergestellt analog Beispiel 9 aus 4′-[(2-n-Butyl-6-(N-methylaminocarbonyl-cyclohexylamino)-benzimidazol-1-yl)-methyl]biphenyl-2-carbonsäure-tert.butylester und Trifluoressigsäure.
Ausbeute: 88,3 % der Theorie,
Schmelzpunkt: 204-206°C

| $C_{33}H_{38}N_4O_3$ (538,69) | | | | | | |
|---|---|---|---|---|---|---|
| Ber.: | C | 73,58 | H | 7,11 | N | 10,40 |
| Gef.: | | 73,65 | | 6,99 | | 10,49 |

Beispiel 203

4′-[(2-n-Butyl-6-(N-ethoxycarbonyl-cyclohexylamino)-benzimidazol-1-yl)-methyl]biphenyl-2-carbonsäure

Hergestellt analog Beispiel 9 aus 4′-[(2-n-Butyl-6-(N-ethoxycarbonyl-cyclohexylamino)-benzimidazol-1-yl)-methyl]biphenyl-2-carbonsäure-tert.butylester und Trifluoressigsäure.
Ausbeute: 89,0 % der Theorie,
Schmelzpunkt: 239-240°C

| $C_{34}H_{39}N_3O_4$ (553,70) | | | | | | |
|---|---|---|---|---|---|---|
| Ber.: | C | 73,75 | H | 7,10 | N | 7,59 |
| Gef.: | | 73,76 | | 7,25 | | 7,68 |

Beispiel 204

4′-[(2-n-Butyl-6-cyclohexylacetamino-benzimidazol-1-yl)-methyl]biphenyl-2-carbonsäure x 0,7 Trifluoressigsäure

Hergestellt analog Beispiel 9 aus 4′-[(2-n-Butyl-6-cyclohexylacetamino-benzimidazol-1-yl)-methyl]biphenyl-2-carbonsäure-tert.butylester und Trifluoressigsäure.
Ausbeute: 97,5 % der Theorie,

Schmelzpunkt: 117-119°C

| $C_{33}H_{37}N_3O_3$ x 0,7 $CF_3COOH$ (603,49) | | | | | | |
|---|---|---|---|---|---|---|
| Ber.: | C | 68,46 | H | 6,30 | N | 6,96 |
| Gef.: | | 68,80 | | 6,60 | | 6,73 |

Beispiel 205

4'-[(2-n-Butyl-6-phthalimino-benzimidazol-1-yl)-methyl]biphenyl-2-carbonsäure-semitrifluoracetat

Hergestellt analog Beispiel 9 aus 4'-[(2-n-Butyl-6-phthalimino-benzimidazol-1-yl)-methyl]biphenyl-2-carbonsäure-tert.butylester und Trifluoressigsäure in Methylenchlorid.
Ausbeute: 85,5 % der Theorie,
Schmelzpunkt: 181-183°C

| $C_{33}H_{27}N_3O_4$ x 0,5 $CF_3COOH$ (586,61) | | | | | | |
|---|---|---|---|---|---|---|
| Ber.: | C | 69,62 | H | 4,73 | N | 7,16 |
| Gef.: | | 69,70 | | 4,81 | | 7,31 |

Beispiel 206

4'-[(2-n-Butyl-6-((5-trifluoracetoxy-n-pentyl)-aminocarbonylamino)-benzimidazol-1-yl)-methyl]biphenyl-2-carbonsäure-semitrifluoracetat-semihydrat

1,92 g (3,3 mMol) 4'-[(2-n-Butyl-6-(tetrahydropyran-2-yl-aminocarbonylamino)-benzimidazol-1-yl)-methyl]biphenyl-2-carbonsäure-tert.butylester werden in 100 ml Ethanol gelöst und mit 1,9 g Raney-Nickel versetzt. Die Reaktionslösung wird nun 4 Stunden bei 120°C und 100 bar Wasserstoff hydriert. Anschließend wird vom Katalysator abgesaugt und das Lösungsmittel im Vakuum abdestilliert. Der ölige Rückstand wird über eine Säule gereinigt (Kieselgel; Korngröße: 0,063-0,2 mm), wobei mit Methylenchlorid und ansteigenden Teilen von 2-5 % Ethanol eluiert wird. Nach dem Einrotieren der ensprechenden Fraktionen, löst man das erhaltene Öl in einem Gemisch aus 10 ml Methylenchlorid und 10 ml Trifluoressigsäure und beläßt die Lösung 15 Stunden bei Raumtemperatur. Anschließend wird vom Lösungsmittel abrotiert, der Rückstand in ca. 50 ml Essigester gelöst und die organische Phase 3 mal mit ca. 50 ml Wasser gewaschen. Die organische Lösung wird mit ca. 20 g Magnesiumsulfat getrocknet, abfiltriert und einrotiert. Das so erhaltene Produkt trocknet man bei 50°C im Vakuum.
Ausbeute: 1,5 g (66,0 % der Theorie),
Schmelzpunkt: 80-82°C (amorph)

| | | | | | | |
|---|---|---|---|---|---|---|
| Ber.: | C | 59,13 | H | 5,33 | N | 8,11 |
| Gef.: | | 59,22 | | 5,52 | | 7,95 |

Beispiel 207

4'-[(2-n-Butyl-6-(N-ethoxycarbonyl-benzylamino)-benzimidazol-1-yl)-methyl]biphenyl-2-carbonsäure

Hergestellt analog Beispiel 9 aus 4'-[(2-n-Butyl-6-(N-ethoxycarbonyl-benzylamino)-benzimidazol-1-yl)-methyl]biphenyl-2-carbonsäure-tert.butylester und Trifluoressigsäure.
Ausbeute: 86,4 % der Theorie,

Schmelzpunkt: 218-220°C

| $C_{35}H_{35}N_3O_4$ (561,68) | | | | | | |
|---|---|---|---|---|---|---|
| Ber.: | C | 74,84 | H | 6,28 | N | 7,48 |
| Gef.: | | 74,57 | | 6,14 | | 7,59 |

Beispiel 208

4'-[(2-n-Butyl-6-(N-methylaminocarbonyl-benzylamino)-benzimidazol-1-yl)-methyl]biphenyl-2-carbonsäure

Hergestellt analog Beispiel 9 aus 4'-[(2-n-Butyl-6-(N-methylaminocarbonyl-benzylamino)-benzimidazol-1-yl)-methyl]biphenyl-2-carbonsäure-tert.butylester und Trifluoressigsäure.
Ausbeute: 83,3 % der Theorie,
Schmelzpunkt: 247-249°C

| $C_{34}H_{34}N_4O_3$ (546,67) | | | | | | |
|---|---|---|---|---|---|---|
| Ber.: | C | 74,70 | H | 6,27 | N | 10,25 |
| Gef.: | | 74,95 | | 6,37 | | 10,12 |

Beispiel 209

4'-[(2-n-Butyl-6-(n-hexyloxycarbonylamino)-benzimidazol-1-yl)-methyl]biphenyl-2-carbonsäure

Hergestellt analog Beispiel 9 aus 4'-[(2-n-Butyl-6-(n-hexyloxycarbonylamino)-benzimidazol-1-yl)-methyl]biphenyl-2-carbonsäure-tert.butylester und Trifluoressigsäure.
Ausbeute: 89,5 % der Theorie,
Schmelzpunkt: 243-244°C

| $C_{32}H_{37}N_3O_4$ (527,66) | | | | | | |
|---|---|---|---|---|---|---|
| Ber.: | C | 72,84 | H | 7,07 | N | 7,96 |
| Gef.: | | 72,65 | | 7,15 | | 7,98 |

Beispiel 210 4'-[(2-n-Butyl-6-(N-(n-hexylaminocarbonyl)-benzylamino)-benz

imidazol-1-yl)-methyl]biphenyl-2-carbonsäure

Hergestellt analog Beispiel 9 aus 4'-[(2-n-Butyl-6-(N-(n-hexylaminocarbonyl)-benzylamino)-benzimidazol-1-yl)-methyl]biphenyl-2-carbonsäure-tert.butylester und Trifluoressigsäure.
Ausbeute: 69,2 % der Theorie,

Schmelzpunkt: 186-187°C

| $C_{39}H_{44}N_4O_3$ (616,80) | | | | | | |
|---|---|---|---|---|---|---|
| Ber.: | C | 75,94 | H | 7,19 | N | 9,08 |
| Gef.: | | 75,85 | | 7,24 | | 9,14 |

Beispiel 211

4'-[(2-n-Butyl-6-(n-pentylamino)-benzimidazol-1-yl)-methyl]biphenyl-2-carbonsäure

Hergestellt analog Beispiel 9 aus 4'-[(2-n-Butyl-6-(n-pentylamino)-benzimidazol-1-yl)-methyl]biphenyl-2-carbonsäure-tert.butylester und Trifluoressigsäure/Methylenchlorid.
Ausbeute: 75 % der Theorie,
Schmelzpunkt: 108-113°C

| $C_{30}H_{35}N_3O_2$ (469,62) | | | | | | |
|---|---|---|---|---|---|---|
| Ber.: | C | 76,73 | H | 7,51 | N | 8,94 |
| Gef.: | | 76,56 | | 7,40 | | 8,91 |

Beispiel 212

4'-[(2-n-Butyl-6-(5,7-dioxo-1H,3H-imidazo[1,5-c]thiazol-6-yl)-benzimidazol-1-yl)-methyl]biphenyl-2-carbonsäure

Hergestellt analog Beispiel 9 aus 4'-[(2-n-Butyl-6-(5,7-dioxo-1H,3H-imidazo[1,5-c]thiazol-6-yl)-benzimidazol-1-yl)-methyl]biphenyl-2-carbonsäure-tert.butylester und Trifluoressigsäure.
Ausbeute: 95,1 % der Theorie, Schmelzpunkt: 229-230°C

| $C_{30}H_{28}N_4O_4S$ (540,64) | | | | | | | | |
|---|---|---|---|---|---|---|---|---|
| Ber.: | C | 66,65 | H | 5,22 | N | 10,36 | S | 5,93 |
| Gef.: | | 66,42 | | 5,29 | | 10,15 | | 6,01 |

Beispiel 213

4'-[(2-n-Butyl-6-((5-hydroxy-n-pentyl)-aminocarbonylamino)-benzimidazol-1-yl)-methyl]biphenyl-2-carbonsäure-monohydrat

Hergestellt analog Beispiel 72 aus 4'-[(2-n-Butyl-6-((5-hydroxy-n-pentyl)-aminocarbonylamino)-benzimidazol-1-yl)-methyl]biphenyl-2-carbonsäure-ethylester und 2 n NaOH/Ethanol.
Ausbeute: 50,0 % der Theorie,

Schmelzpunkt: 158-160°C

| $C_{31}H_{36}N_4O_4$ x $H_2O$ (546,67) | | | | | | |
|---|---|---|---|---|---|---|
| Ber.: | C | 68,11 | H | 7,01 | N | 10,25 |
| Gef.: | | 68,01 | | 6,90 | | 10,30 |

Beispiel 214

4'-[(2-n-Butyl-6-((5-hydroxy-n-pentyl)-aminocarbonylamino)-benzimidazol-1-yl)-methyl]biphenyl-2-carbonsäure-ethylester

7,5 g (13,5 mMol) 4'-[(2-n-Butyl-6-(tetrahydropyran-2-yl-aminocarbonylamino)-benzimidazol-1-yl)-methyl]biphenyl-2-carbonsäure-ethylester werden in 250 ml Ethanol gelöst und mit 7,5 g Raney-Nickel versetzt. Die Lösung wird nun 2 Stunden bei 120°C und 100 bar Wasserstoff in einem Autoklaven hydriert. Anschließend saugt man vom Katalysator ab und destilliert vom Lösungsmittel im Vakuum ein. Das anfallende ölige Produkt reinigt man über eine Säule (Kieselgel; Korngröße: 0,063-0,2 mm), wobei mit Essigester/Petrolether (9/1) sowie mit Essigester/Ethanol (99/1) eluiert wird. Die entsprechenden Fraktionen werden im Vakuum eingeengt und im Vakuumtrockenschrank bei 50°C getrocknet.
Ausbeute: 2,6 g (34,7 % der Theorie),
Öl, $R_f$: 0,50 (Kieselgel: Essigester/Ethanol = 9:1)

Beispiel 215

4'-[(2-n-Butyl-6-(N-(n-butanoyl)-n-butylamino)-benzimidazol-1-yl)-methyl]biphenyl-2-carbonsäure

Hergestellt analog Beispiel 9 aus 4'-[(2-n-Butyl-6-(N-(n-butanoyl)-n-butylamino)-benzimidazol-1-yl)-methyl]biphenyl-2-carbonsäure-tert.butylester und Trifluoressigsäure.
Ausbeute: 90,6 % der Theorie,
Schmelzpunkt: 234-235°C

| $C_{33}H_{39}N_3O_3$ (525,69) | | | | | | |
|---|---|---|---|---|---|---|
| Ber.: | C | 75,40 | H | 7,48 | N | 7,99 |
| Gef.: | | 75,55 | | 7,49 | | 8,02 |

Beispiel 216

4'-[(2-n-Butyl-6-(N-(4-methoxycarbonyl-thiazolidin-3-yl-carbonyl)-n-butylamino)-benzimidazol-1-yl)-methyl]biphenyl-2-carbonsäure

Hergestellt analog Beispiel 9 aus 4'-[(2-n-Butyl-6-(N-(4-methoxycarbonyl-thiazolidin-3-yl-carbonyl)-n-butylamino)-benzimidazol-1-yl)-methyl]biphenyl-2-carbonsäure-tert.butylester und Trifluoressigsäure.
Ausbeute: 92,6 % der Theorie,
Schmelzpunkt: 86-88°C (amorph)

| $C_{35}H_{40}N_4O_5S$ (628,79) | | | | | | | | |
|---|---|---|---|---|---|---|---|---|
| Ber.: | C | 66,86 | H | 6,41 | N | 8,91 | S | 5,10 |
| Gef.: | | 66,68 | | 6,40 | | 8,50 | | 5,41 |

Beispiel 217

4'-[(2-n-Butyl-6-(N-cyclohexylaminocarbonyl-n-butylamino)-benzimidazol-1-yl)-methyl]biphenyl-2-carbonsäure

Hergestellt analog Beispiel 9 aus 4'-[(2-n-Butyl-6-(N-cyclohexylaminocarbonyl-n-butylamino)-benzimidazol-1-yl)-methyl]biphenyl-2-carbonsäure-tert.butylester und Trifluoressigsäure.
Ausbeute: 96,8 % der Theorie,
Schmelzpunkt: 106-108°C (amorph)

| $C_{36}H_{44}N_4O_3$ (580,77) | | | | | | |
|---|---|---|---|---|---|---|
| Ber.: | C | 74,45 | H | 7,64 | N | 9,65 |
| Gef.: | | 74,54 | | 7,65 | | 9,50 |

Beispiel 218

4'-[(2-n-Butyl-6-(N-cyclohexylaminocarbonyl-benzylamino)-benzimidazol-1-yl)-methyl]biphenyl-2-carbonsäure

Hergestellt analog Beispiel 9 aus 4'-[(2-n-Butyl-6-(N-cyclohexylaminocarbonyl-benzylamino)-benzimidazol-1-yl)-methyl]biphenyl-2-carbonsäure-tert.butylester.
Ausbeute: 95,0 % der Theorie,
Schmelzpunkt: 203-204°C

| $C_{39}H_{42}N_4O_3$ (614,79) | | | | | | |
|---|---|---|---|---|---|---|
| Ber.: | C | 76,19 | H | 6,89 | N | 9,11 |
| Gef.: | | 75,93 | | 7,04 | | 9,34 |

Beispiel 219

4'-[(2-n-Butyl-6-(N-(2-trifluormethylphenylaminocarbonyl)-methylamino)-benzimidazol-1-yl)-methyl]biphenyl-2-carbonsäure

Hergestellt analog Beispiel 9 aus 4'-[(2-n-Butyl-6-(N-(2-trifluormethylphenylaminocarbonyl)-methylamino)-benzimidazol-1-yl)-methyl]biphenyl-2-carbonsäure-tert.butylester und Trifluoressigsäure/Methylenchlorid.
Ausbeute: 72,2 % der Theorie,
Schmelzpunkt: 142-144°C

| $C_{34}H_{31}F_3N_4O_3$ x $CF_3COOH$ (714,67) | | | | | | |
|---|---|---|---|---|---|---|
| Ber.: | C | 60,52 | H | 4,51 | N | 7,84 |
| Gef.: | | 60,53 | | 4,48 | | 8,03 |

Beispiel 220

4'-[(2-n-Butyl-6-(N-cyclohexylaminocarbonyl-n-hexylamino)-benzimidazol-1-yl)-methyl]biphenyl-2-carbonsäure

Hergestellt analog Beispiel 9 aus 4'-[(2-n-Butyl-6-(N-cyclohexylaminocarbonyl-n-hexylamino)-benzimidazol-1-yl)-methyl]biphenyl-2-carbonsäure-tert.butylester und Trifluoressigsäure.
Ausbeute: 94,1 % der Theorie,

Schmelzpunkt: 177-178°C

| $C_{38}H_{48}N_4O_3$ (608,82) | | | | | | |
|---|---|---|---|---|---|---|
| Ber.: | C | 74,97 | H | 7,95 | N | 9,20 |
| Gef.: | | 74,75 | | 8,04 | | 9,09 |

Beispiel 221

4'-[(2-n-Butyl-6-(N-cyclohexylcarbonyl-n-hexylamino)-benzimidazol-1-yl)-methyl]biphenyl-2-carbonsäure

Hergestellt analog Beispiel 9 aus 4'-[(2-n-Butyl-6-(N-cyclohexylcarbonyl-n-hexylamino)-benzimidazol-1-yl)-methyl]biphenyl-2-carbonsäure-tert.butylester und Trifluoressigsäure.
Ausbeute: 92,8 % der Theorie,
Schmelzpunkt: 195-197°C

| $C_{38}H_{47}N_3O_3$ (593,81) | | | | | | |
|---|---|---|---|---|---|---|
| Ber.: | C | 76,86 | H | 7,98 | N | 7,08 |
| Gef.: | | 76,66 | | 7,94 | | 7,16 |

Beispiel 222

4'-[(2-n-Butyl-6-(N-cyclohexylaminocarbonyl-n-propylamino)-benzimidazol-1-yl)-methyl]biphenyl-2-carbonsäure

Hergestellt analog Beispiel 9 aus 4'-[(2-n-Butyl-6-(N-cyclohexylaminocarbonyl-n-propylamino)-benzimidazol-1-yl)-methyl]biphenyl-2-carbonsäure-tert.butylester und Trifluoressigsäure.
Ausbeute: 93,5 % der Theorie,
Schmelzpunkt: 180-182°C

| $C_{35}H_{42}N_4O_3$ (566,74) | | | | | | |
|---|---|---|---|---|---|---|
| Ber.: | C | 74,18 | H | 7,47 | N | 9,89 |
| Gef.: | | 73,98 | | 7,54 | | 10,05 |

Beispiel 223

4'-[(2-n-Butyl-6-(N-cyclohexylcarbonyl-methylamino)-benzimidazol-1-yl)-methyl]biphenyl-2-carbonsäure-semihydrat

Hergestellt analog Beispiel 9 aus 4'-[(2-n-Butyl-6-(N-cyclohexylcarbonyl-methylamino)-benzimidazol-1-yl)-methyl]biphenyl-2-carbonsäure-tert.butylester und Trifluoressigsäure.
Ausbeute: 93,8 % der Theorie,

Schmelzpunkt: 230-231°C

| $C_{33}H_{37}N_3O_3$ x 0,5 $H_2O$ (532,68) | | | | | | |
|---|---|---|---|---|---|---|
| Ber.: | C | 74,41 | H | 7,19 | N | 7,89 |
| Gef.: | | 74,59 | | 7,14 | | 7,70 |

Beispiel 224

4'-[(2-n-Butyl-6-(N-cyclohexylaminocarbonyl-ethylamino)-benzimidazol-1-yl)-methyl]biphenyl-2-carbonsäure

Hergestellt analog Beispiel 9 aus 4'-[(2-n-Butyl-6-(N-cyclohexylaminocarbonyl-ethylamino)-benzimidazol-1-yl)-methyl]biphenyl-2-carbonsäure-tert.butylester und Trifluoressigsäure.
Ausbeute: 95,5 % der Theorie,
Schmelzpunkt: 185-186°C

| $C_{34}H_{40}N_4O_3$ (552,72) | | | | | | |
|---|---|---|---|---|---|---|
| Ber.: | C | 73,89 | H | 7,29 | N | 10,14 |
| Gef.: | | 73,79 | | 7,13 | | 10,11 |

Beispiel 225

4'-[(2-n-Butyl-6-(N-(n-butanoyl)-n-pentylamino)-benzimidazol-1-yl)-methyl]-biphenyl-2-carbonsäure

Hergestellt analog Beispiel 9 aus 4'-[(2-n-Butyl-6-(N-(n-butanoyl)-n-pentylamino)-benzimidazol-1-yl)-methyl]biphenyl-2-carbonsäure-tert.butylester und Trifluoressigsäure.
Ausbeute: 77,8 % der Theorie,
Schmelzpunkt: 206-208°C

| $C_{34}H_{41}N_3O_3$ (539,72) | | | | | | |
|---|---|---|---|---|---|---|
| Ber.: | C | 75,66 | H | 7,66 | N | 7,79 |
| Gef.: | | 75,54 | | 7,47 | | 7,67 |

Beispiel 226

4'-[(2-n-Butyl-6-isopropylcarbonylamino-benzimidazol-1-yl)-methyl]biphenyl-2-carbonsäure

Hergestellt analog Beispiel 9 aus 4'-[(2-n-Butyl-6-isopropylcarbonylamino-benzimidazol-1-yl)-methyl]biphenyl-2-carbonsäure-tert.butylester und Trifluoressigsäure.
Ausbeute: 73,3 % der Theorie,

Schmelzpunkt: 273-275°C

| $C_{29}H_{31}N_3O_3$ (469,58) | | | | | | |
|---|---|---|---|---|---|---|
| Ber.: | C | 74,18 | H | 6,65 | N | 8,95 |
| Gef.: | | 73,93 | | 6,66 | | 8,84 |

Beispiel 227

4'-[(2-n-Butyl-6-(N-ethoxycarbonyl-methylamino)-benzimidazol-1-yl)-methyl]biphenyl-2-carbonsäure

Hergestellt analog Beispiel 9 aus 4'-[(2-n-Butyl-6-(N-ethoxycarbonyl-methylamino)-benzimidazol-1-yl)-methyl]biphenyl-2-carbonsäure-tert.butylester und Trifluoressigsäure.
Ausbeute: 91,8 % der Theorie,
Schmelzpunkt: 190-192°C

| $C_{29}H_{31}N_3O_4$ (485,58) | | | | | | |
|---|---|---|---|---|---|---|
| Ber.: | C | 71,73 | H | 6,43 | N | 8,65 |
| Gef.: | | 71,60 | | 6,40 | | 8,69 |

Beispiel 228

4'-[(2-n-Butyl-6-(N-ethoxycarbonyl-n-pentylamino)-benzimidazol-1-yl)-methyl]biphenyl-2-carbonsäure

Hergestellt analog Beispiel 9 aus 4'-[(2-n-Butyl-6-(N-ethoxycarbonyl-n-pentyl-amino)-benzimidazol-1-yl)-methyl]biphenyl-2-carbonsäure-tert.butylester und Trifluoressigsäure/Methylenchlorid.
Ausbeute: 86,9 % der Theorie,
Schmelzpunkt: 201-203°C

| $C_{33}H_{39}N_3O_4$ (541,69) | | | | | | |
|---|---|---|---|---|---|---|
| Ber.: | C | 73,17 | H | 7,26 | N | 7,76 |
| Gef.: | | 72,97 | | 6,98 | | 7,69 |

Beispiel 229

4'-[(2-n-Butyl-6-(N-(dimethylaminocarbonyl)-benzylamino)-benzimidazol-1-yl)-methyl]-biphenyl-2-carbonsäure

Hergestellt analog Beispiel 9 aus 4'-[(2-n-Butyl-6-(N-(dimethylaminocarbonyl)-benzylamino)-benzimidazol-1-yl)-methyl]biphenyl-2-carbonsäure-tert.butylester und Trifluoressigsäure.
Ausbeute: 75,3 % der Theorie,

Schmelzpunkt: 202-203°C

| $C_{35}H_{36}N_4O_3$ (560,69) | | | | | | |
|---|---|---|---|---|---|---|
| Ber.: | C | 74,98 | H | 6,47 | N | 9,99 |
| Gef.: | | 74,89 | | 6,24 | | 10,02 |

Beispiel 230

4'-[(2-n-Butyl-6-(N-cyclohexylaminocarbonyl-N-(2-phenylethyl)-amino)-benzimidazol-1-yl)-methyl]biphenyl-2-carbonsäure

Hergestellt analog Beispiel 9 aus 4'-[(2-n-Butyl-6-(N-cyclohexylaminocarbonyl-N-(2-phenylethyl)-amino)-benzimidazol-1-yl)-methyl]biphenyl-2-carbonsäure-tert.butylester und Trifluoressigsäure.
Ausbeute: 70,0 % der Theorie,
Schmelzpunkt: 168-170°C

| $C_{40}H_{44}N_4O_3$ (628,81) | | | | | | |
|---|---|---|---|---|---|---|
| Ber.: | C | 76,40 | H | 7,05 | N | 8,91 |
| Gef.: | | 76,31 | | 7,26 | | 8,79 |

Beispiel 231

4'-[(2-n-Butyl-6-diethylaminocarbonylamino-benzimidazol-1-yl)-methyl]biphenyl-2-carbonsäure-semitrifluoracetat

Hergestellt analog Beispiel 9 aus 4'-[(2-n-Butyl-6-diethylaminocarbonylamino-benzimidazol-1-yl)-methyl]biphenyl-2-carbonsäure-tert.butylester und Trifluoressigsäure.
Ausbeute: 79,4 % der Theorie,
Schmelzpunkt: 235-237°C

| $C_{30}H_{34}N_4O_3$ x 0,5 $CF_3COOH$ (555,64) | | | | | | |
|---|---|---|---|---|---|---|
| Ber.: | C | 67,01 | H | 6,26 | N | 10,08 |
| Gef.: | | 66,99 | | 6,02 | | 10,12 |

Beispiel 232

4'-[(2-n-Butyl-6-dimethylaminoacetamino-benzimidazol-1-yl)-methyl]biphenyl-2-carbonsäure-ditrifluoracetat

Hergestellt analog Beispiel 9 aus 4'-[(2-n-Butyl-6-(dimethylaminoacetamino)-benzimidazol-1-yl)-methyl]biphenyl-2-carbonsäure-tert.butylester und Trifluoressigsäure/Methylenchlorid.
Ausbeute: 75,5 % der Theorie,

Schmelzpunkt: 218-220°C

| $C_{29}H_{32}N_4O_3$ x 2 $CF_3COOH$ (712,65) | | | | | | |
|---|---|---|---|---|---|---|
| Ber.: | C | 55,61 | H | 4,81 | N | 7,86 |
| Gef.: | | 55,61 | | 5,05 | | 8,19 |

Beispiel 233

4'-[(2-n-Butyl-6-(2,2-dimethyl-propionylamino)-benzimidazol-1-yl)-methyl]biphenyl-2-carbonsäure

Hergestellt analog Beispiel 9 aus 4'-[(2-n-Butyl-6-(2,2-dimethyl-propionylamino)-benzimidazol-1-yl)-methyl]biphenyl-2-carbonsäure-tert.butylester und Trifluoressigsäure.
Ausbeute: 67,5 % der Theorie,
Schmelzpunkt: 326-327°C

| $C_{30}H_{33}N_3O_3$ (483,61) | | | | | | |
|---|---|---|---|---|---|---|
| Ber.: | C | 74,51 | H | 6,88 | N | 8,69 |
| Gef.: | | 74,32 | | 7,06 | | 8,58 |

Beispiel 234

4'-[(2-n-Butyl-6-(N-(n-hexylaminocarbonyl)-N-(2-phenylethyl)-amino)-benzimidazol-1-yl)-methyl]biphenyl-2-carbonsäure

Hergestellt analog Beispiel 9 aus 4'-[(2-n-Butyl-6-(N-(n-hexylaminocarbonyl)-N-(2-phenylethyl)-amino)-benzimidazol-1-yl)-methyl]biphenyl-2-carbonsäure-tert.butylester und Trifluoressigsäure.
Ausbeute: 66,7 % der Theorie,
Schmelzpunkt: 176-177°C

| $C_{40}H_{46}N_4O_3$ (630,83) | | | | | | |
|---|---|---|---|---|---|---|
| Ber.: | C | 76,16 | H | 7,35 | H | 8,88 |
| Gef.: | | 75,97 | | 7,44 | | 8,98 |

Beispiel 235

4'-[(2-n-Butyl-6-cyclopentylcarbonylamino-benzimidazol-1-yl)-methyl]biphenyl-2-carbonsäure-semihydrat

Hergestellt analog Beispiel 9 aus 4'-[(2-n-Butyl-6-cyclopentylcarbonylamino-benzimidazol-1-yl)-methyl]biphenyl-2-carbonsäure-tert.butylester und Trifluoressigsäure.
Ausbeute: 72,2 % der Theorie,

Schmelzpunkt: 272-274°C

| $C_{31}H_{33}N_3O_3$ x 0,5 $H_2O$ (504,63) | | | | | | |
|---|---|---|---|---|---|---|
| Ber.: | C | 73,79 | H | 6,79 | N | 8,33 |
| Gef.: | | 74,00 | | 6,91 | | 8,25 |

Beispiel 236

4'-[(2-n-Butyl-6-cyclopropylcarbonylamino-benzimidazol-1-yl)-methyl]biphenyl-2-carbonsäure-semihydrat

Hergestellt analog Beispiel 9 aus 4'-[(2-n-Butyl-6-cyclopropylcarbonylamino-benzimidazol-1-yl)-methyl]biphenyl-2-carbonsäure-tert.butylester und Trifluoressigsäure.
Ausbeute: 91,7 % der Theorie,
Schmelzpunkt: 275-277°C

| $C_{29}H_{29}N_3O_3$ x 0,5 $H_2O$ (476,57) | | | | | | |
|---|---|---|---|---|---|---|
| Ber.: | C | 73,09 | H | 6,34 | N | 8,82 |
| Gef.: | | 72,97 | | 6,50 | | 8,52 |

Beispiel 237

4'-[(2-n-Butyl-6-(1-oxo-isoindolin-2-yl)-benzimidazol-1-yl)-methyl]biphenyl-2-carbonsäure-trifluoracetat

1,6 g (3 mMol) 4'-[(2-n-Butyl-6-phthalimino-benzimidazol-1-yl)-methyl]biphenyl-2-carbonsäure-trifluoracetat werden in 30 ml Eisessig unter Rückfluß mit 1,6 g Zinkstaub versetzt. Nach je einer Stunde werden 2 mal je 1,6 g Zinkstaub zugefügt und eine Stunde am Rückfluß gekocht. Es wird vom überschüssigen Zink abgesaugt und das Filtrat zur Trockene einrotiert. Das Rohprodukt wird über eine Kieselgelsäule (Korngröße: 0,063-0,2 mm) mit Essigester/Ethanol/Ammoniak (90:10:0,1 bis 80:20:0,1) gereinigt und aus Ether kristallisiert.
Ausbeute: 0,45 g (64,1 % der Theorie),
Schmelzpunkt: 214-216°C

| $C_{33}H_{29}N_3O_3$ x $CF_3COOH$ (629,64) | | | | | | |
|---|---|---|---|---|---|---|
| Ber.: | C | 66,76 | H | 4,80 | N | 6,67 |
| Gef.: | | 66,62 | | 5,08 | | 6,69 |

Beispiel 238

4'-[(2-(1-trans-Butenyl)-6-dimethylaminocarbonylamino-benzimidazol-1-yl)-methyl]biphenyl-2-carbonsäure-trifluoracetat

a) 4'-[2-(1-Brombutyl)-6-phthalimino-benzimidazol-1-yl)-methyl]biphenyl-2-carbonsäure-tert.butylester

10,3 g (17,6 mMol) 4'-[(2-n-Butyl-6-phthalimino-benzimidazol-1-yl)-methyl]biphenyl-2-carbonsäure-tert.butylester` 1 l Tetrachlorkohlenstoff und 3,1 g (17,5 mMol) N-Bromsuccinimid werden 3 Stunden unter Rühren mit einer Quecksilbertauchlampe bestrahlt. Hierbei steigt die Innentemperatur auf 50°C an. Anschließend wird vom ausgefallenen schmierigen Produkt abfiltriert, der Rückstand in Methylenchlorid gelöst und mit Wasser ausgeschüttelt. Die organische Phase wird über Natriumsulfat getrocknet und einrotiert. Das Rohprodukt wird über eine Kieselgelsäule (Korngröße: 0,063-0,2

mm) Essigester/Petrolether (1:4) gereinigt.
Ausbeute: 4,5 g (38,4 % der Theorie)

b) 4'-[2-(1-trans-Butenyl)-6-phthalimino-benzimidazol-1-yl)-methyl]biphenyl-2-carbonsäure-tert.butylester

10,8 g (16,25 mMol) 4'-[2-(1-Brombutyl)-6-phthalimino-benzimidazol-1-yl)-methyl]biphenyl-2-carbonsäure-tert.butylester, 110 ml Dimethylformamid und 5,1 ml (5,2 g = 34,1 mMol) 1,8-Diazabicyclo[5,4,0]undec-7-en werden über Nacht bei Raumtemperatur gerührt. Es wird mit 200 ml Ether verdünnt und mit 2 n Salzsäure und Wasser ausgeschüttelt. Die vereinigte saure Phase wird mit Essigester ausgeschüttelt. Die Essigesterphase wird über Natriumsulfat getrocknet und nach dem Einrotieren über eine Kieselgelsäule (Korngröße: 0,063-0,2 mm) mit Essigester/Petrolether (20:80 bis 30:70) gereinigt. Beim Eindampfen der entsprechenden Fraktionen kristallisiert das Produkt aus. Es wird abgesaugt und mit Ether gewaschen.
Ausbeute: 2,30 g (24,2 % der Theorie),
Schmelzpunkt: 232-234°C

| $C_{37}H_{33}N_3O_4$ (583,69) | | | | | | |
|---|---|---|---|---|---|---|
| Ber.: | C | 76,14 | H | 5,70 | N | 7,20 |
| Gef.: | | 75,92 | | 5,69 | | 7,34 |

c) 4'-[6-Amino-(2-(1-trans-Butenyl)-benzimidazol-1-yl)-methyl]biphenyl-2-carbonsäure-tert.butylester

2,3 g (3,94 mMol) 4'-[(2-(1-trans-Butenyl)-6-phthalimino-benzimidazol-1-yl)-methyl]biphenyl-2-carbonsäure-tert.butylester, 80 ml Ethanol und 25 ml 40%iges wässriges Methylamin werden 3 Stunden bei Raumtemperatur gerührt. Man erhitzt 1/4 Stunde auf dem Dampfbad, kühlt ab und rotiert ein. Der Rückstand wird in Aceton aufgeschlämmt, gekühlt und vom ungelösten N-Methylphthalimid abfiltriert. Das Filtrat wird zur Trockene eingedampft.
Ausbeute: 1,78 g (100 % der Theorie),
Schmelzpunkt: 174-176°C

d) 4'-[(2-(1-trans-Butenyl)-6-dimethylaminocarbonylamino-benzimidazol-1-yl)-methyl]biphenyl-2-carbonsäure-tert.butylester

Hergestellt analog Beispiel 8 aus 4'-[6-Amino-(2-(1-trans-butenyl)-benzimidazol-1-yl)-methyl]biphenyl-2-carbonsäure-tert.butylester und Dimethylcarbamoylchlorid.
Ausbeute: 82,8 % der Theorie,
Öl, $R_f$-Wert: 0,35 (Kieselgel; Methylenchlorid/Ethanol = 19:1)

e) 4'-[(2-(1-trans-Butenyl)-6-dimethylaminocarbonylamino-benzimidazol-1-yl)-methyl]biphenyl-2-carbonsäure-trifluoracetat

Hergestellt analog Beispiel 9 aus 4'-[(2-(1-trans-Butenyl)-6-dimethylaminocarbonylamino-benzimidazol-1-yl)-methyl]biphenyl-2-carbonsäure-tert.butylester und Trifluoressigsäure in Methylenchlorid.
Ausbeute: 66,6 % der Theorie,
Schmelzpunkt: 221-223°C

| $C_{28}H_{28}N_4O_3$ x $CF_3COOH$ x 0,5 $H_2O$ (591,59) | | | | | | |
|---|---|---|---|---|---|---|
| Ber.: | C | 60,90 | H | 5,11 | N | 9,47 |
| Gef.: | | 60,83 | | 4,96 | | 9,53 |

Beispiel 239

4'-[(2-(1-trans-Butenyl)-6-cyclohexylaminocarbonylamino-benzimidazol-1-yl)-methyl]biphenyl-2-carbonsäure-trifluoracetat

Hergestellt analog Beispiel 238 aus 4'-[(2-(1-trans-Butenyl)-6-cyclohexylaminocarbonylamino-benzimidazol-1-yl)-methyl]bi phenyl-2-carbonsäure-tert.butylester und Trifluoressigsäure in Methylenchlorid.
Ausbeute: 93,7 % der Theorie,
Schmelzpunkt: 171-173°C

| $C_{32}H_{34}N_4O_3 \times CF_3COOH$ (636,67) | | | | | | |
|---|---|---|---|---|---|---|
| Ber.: | C | 64,14 | H | 5,54 | N | 8,80 |
| Gef.: | | 64,00 | | 5,49 | | 8,97 |

Beispiel 240

4'-[(2-n-Butyl-6-(cis-hexahydrophthalimino)-benzimidazol-1-yl)-methyl]biphenyl-2-carbonsäure-tert.butylester

1,8 g (4 mMol) 4'-[(6-Amino-2-n-butyl-benzimidazol-1-yl)-methyl]biphenyl-2-carbonsäure-tert.butylester, 30 ml Eisessig und 1,85 g (1,2 mMol) cis-Cyclohexan-1,2-dicarbonsäureanhydrid werden eine Stunde unter Rühren am Rückfluß gekocht. Das Reaktionsgemisch wird zur Trockene eingedampft. Der Rückstand wird in Methylenchlorid, gelöst mit gesättigter Natriumbicarbonatlösung neutral gewaschen, über Natriumsulfat getrocknet und einrotiert. Das Rohprodukt wird über eine Kieselgelsäule (Korngröße: 0,063-0,2 mm) mit Essigester/Petrolether (10:90; 20:80 und 30:70) gereinigt.
Ausbeute: 1,5 g (64,1 % der Theorie),

| $C_{37}H_{41}N_3O_4$ (591,80) | | | | | | |
|---|---|---|---|---|---|---|
| Ber.: | C | 75,10 | H | 6,98 | N | 7,10 |
| Gef.: | | 74,92 | | 7,10 | | 6,99 |

Analog werden folgende Verbindungen erhalten:
4'-[(2-n-Butyl-6-(4,5-dimethyl-1,2,3,6-tetrahydrophthalimino)-benzimidazol-1-yl)-methyl]biphenyl-2-carbonsäure-tert.butylester
Öl, $R_f$-Wert: 0,30 (Kieselgel: Essigester/Petrolether = 1:1)
4'-[(2-n-Butyl-6-(3,4-dimethoxy-phthalimino)-benzimidazol-1-yl)-methyl]biphenyl-2-carbonsäure-tert.butylester
Schmelzpunkt: 238-239°C
4'-[(2-n-Butyl-6-(cis-hexahydrophthalimino)-benzimidazol-1-yl)-methyl]biphenyl-2-carbonsäure-tert.butylester
Öl, $R_f$-Wert: 0,45 (Kieselgel: Essigester/Petrolether = 4:1)
4'-[(2-n-Butyl-6-(cis-hexahydro-phthalimino)-benzimidazol-1-yl)-methyl]-2-(1-triphenylmethyl-tetrazol-5-yl)-biphenyl
Öl, $R_f$-Wert: 0,55 (Kieselgel: Essigester/Petrolether/Ammoniak = 80:20:1)
4'-[(2-n-Butyl-6-(endo-bicyclo[2.2.2]oct-5-en-2,3-dicarbonsäureimino)-benzimidazol-1-yl)-methyl]biphenyl-2-carbonsäure-tert.butylester
Schmelzpunkt: 180-182,5°C
4'-[(2-n-Butyl-6-(cis-hexahydrophthalimino)-benzimidazol-1-yl)-methyl]biphenyl-2-carbonsäure-tert.butylester
Öl, $R_f$-Wert: 0,43 (Kieselgel: Essigester/Petrolether = 2:1)
4'-[(2-n-Butyl-6-(methyl-5-norbornen-2,3-dicarbonsäureimino)-benzimidazol-1-yl)-methyl]biphenyl-2-carbonsäure-tert.butylester
4'-[(2-n-Butyl-6-(trans-hexahydro-phthalimino)-benzimidazol-1-yl)-methyl]biphenyl-2-carbonsäure-tert.butylester
Schmelzpunkt: 168-170°C
4'-[(2-n-Butyl-6-(3,6-endoxo-1,2,3,6-tetrahydro-phthalimino)-benzimidazol-1-yl)-methyl]biphenyl-2-carbonsäure-tert.butylester
4'-[(2-n-Butyl-6-(cis-5-norbornen-endo-2,3-dicarbonsäureimino)-benzimidazol-1-yl)-methyl]biphenyl-2-carbonsäure-tert.butylester

Schmelzpunkt: 179-180°C
4'-[(2-n-Butyl-5-(N-cyclohexylaminocarbonyl-methylamino)-benzimidazol-1-yl)-methyl]-2-(1-triphenylmethyl-tetrazol-5-yl)-biphenyl
Öl, $R_f$-Wert: 0,45 (Kieselgel: Essigester/Petrolether/Ammoniak = 90:10:1)
4'-[(2-n-Butyl-5-(cis-hexahydrophthalimino)-benzimidazol-1-yl)-methyl]-2-(1-triphenylmethyl-tetrazol-5-yl)-biphenyl
Öl, $R_f$-Wert: 0,35 (Kieselgel: Essigester/Petrolether = 4:1)

Beispiel 241

4'-[(2-n-Butyl-6-(cis-hexahydrophthalimino)-benzimidazol-1-yl)-methyl]biphenyl-2-carbonsäure-trifluoracetat

Hergestellt analog Beispiel 9 aus 4'-[(2-n-Butyl-6-(cis-hexahydrophthalimino)-benzimidazol-1-yl)-methyl]biphenyl-2-carbonsäure-tert.butylester und Trifluoressigsäure in Methylenchlorid.
Ausbeute: 58,8 % der Theorie,
Schmelzpunkt: 270°C (Zers.)

| $C_{33}H_{33}N_3O_4$ x $CF_3COOH$ (649,68) | | | | | | |
|---|---|---|---|---|---|---|
| Ber.: | C | 64,71 | H | 5,28 | N | 6,47 |
| Gef.: | | 64,76 | | 5,41 | | 6,65 |

Beispiel 242

4'-[(2-n-Butyl-6-(4,5-dimethyl-1,2,3,6-tetrahydrophthalimino)-benzimidazol-1-yl)-methyl]biphenyl-2-carbonsäure-hydrat

Hergestellt analog Beispiel 9 aus 4'-[(2-n-Butyl-6-(4,5-dimethyl-1,2,3,6-tetrahydrophthalimino)-benzimidazol-1-yl)-methyl]biphenyl-2-carbonsäure-tert.butylester und Trifluoressigsäure in Methylenchlorid.
Ausbeute: 30 % der Theorie,
Schmelzpunkt: 219-221°C

| $C_{35}H_{35}N_3O_4$ x $H_2O$ (579,69) | | | | | | |
|---|---|---|---|---|---|---|
| Ber.: | C | 72,52 | H | 6,43 | N | 7,23 |
| Gef.: | | 72,66 | | 6,49 | | 7,43 |

Beispiel 243

4'-[(2-n-Butyl-6-(3,4-dimethoxy-phthalimino)-benzimidazol-1-yl)-methyl]biphenyl-2-carbonsäure-dihydrat

Hergestellt analog Beispiel 9 aus 4'-[(2-n-Butyl-6-(3,4-dimethoxy-phthalimino)-benzimidazol-1-yl)-methyl]biphenyl-2-carbonsäure-tert.butylester und Trifluoressigsäure in Methylenchlorid.
Ausbeute: 24,3 % der Theorie,
Schmelzpunkt: 206-208°C

| $C_{35}H_{31}N_3O_6$ x 2 $H_2O$ (625,68) | | | | | | |
|---|---|---|---|---|---|---|
| Ber.: | C | 67,19 | H | 5,64 | N | 6,72 |
| Gef.: | | 67,22 | | 5,84 | | 6,97 |

Beispiel 244

4'-[(2-n-Butyl-6-(N-(dimethylaminocarbonyl)-n-pentylamino)-benzimidazol-1-yl)-methyl]biphenyl-2-carbonsäure-trifluoracetat

Hergestellt analog Beispiel 9 aus 4'-[(2-n-Butyl-6-(N-(dimethylaminocarbonyl)-n-pentylamino)-benzimidazol-1-yl)-methyl]biphenyl-2-carbonsäure-tert.butylester und Trifluoressigsäure in Methylenchlorid.
Ausbeute: 81,8 % der Theorie,
Schmelzpunkt: 174-176°C

| $C_{33}H_{40}N_4O_3$ x $CF_3COOH$ (654,73) | | | | | | |
|---|---|---|---|---|---|---|
| Ber.: | C | 64,21 | H | 6,31 | N | 8,55 |
| Gef.: | | 64,38 | | 6,28 | | 8,64 |

Beispiel 245

4'-[(2-n-Butyl-6-(N-(4-phenylamino-n-butyl)-n-pentylamino)-benzimidazol-1-yl)-methyl]biphenyl-2-carbonsäure

Hergestellt analog Beispiel 9 aus 4'-[(2-n-Butyl-6-(N-(4-phenylamino-n-butyl)-n-pentylamino)-benzimidazol-1-yl)-methyl]biphenyl-2-carbonsäure-tert.butylester und Trifluoressigsäure in Methylenchlorid.

Beispiel 246

4'-[(2-n-Butyl-6-cyclohexylaminocarbonylamino-benzimidazol-1-yl)-methyl]-2-(1H-tetrazol-5-yl)-biphenyl

Hergestellt analog Beispiel 58 aus 4'-[(6-Amino-2-n-butyl-benzimidazol-1-yl)-methyl]-2-(1H-tetrazol-5-yl)-biphenyl und Cyclohexylisocyanat in Methylenchlorid.
Ausbeute: 55,1 % der Theorie,
Schmelzpunkt: 232-234°C

| $C_{32}H_{36}N_8O$ (548,69) | | | | | | |
|---|---|---|---|---|---|---|
| Ber.: | C | 70,05 | H | 6,61 | N | 20,42 |
| Gef.: | | 69,86 | | 6,66 | | 20,25 |

Beispiel 247

4'-[(2-n-Butyl-6-dimethylaminoaminocarbonylamino-benzimidazol-1-yl)-methyl]-2-(1H-tetrazol-5-yl)-biphenyl

Hergestellt analog Beispiel 58b aus 4'-[(2-n-Butyl-6-dimethylaminoaminocarbonylamino-benzimidazol-1-yl)-methyl]-2-(1-triphenylmethyl-tetrazol-5-yl)-biphenyl und Salzsäure in Ether/Methanol/Methylenchlorid.
Ausbeute: 69,8 % der Theorie,
Schmelzpunkt: 239-241°C

| $C_{28}H_{30}N_8O$ (494,60) | | | | | | |
|---|---|---|---|---|---|---|
| Ber.: | C | 67,99 | H | 6,11 | N | 22,65 |
| Gef.: | | 67,85 | | 6,08 | | 22,55 |

Beispiel 248

4'-[(2-n-Butyl-6-(N-cyclohexylaminoaminocarbonyl-methylamino)-benzimidazol-1-yl)-methyl]-2-(1H-tetrazol-5-yl)-biphenyl-hydrat

Hergestellt analog Beispiel 58b aus 4'-[(2-n-Butyl-6-(N-cyclohexylaminoaminocarbonyl-methylamino)-benzimidazol-1-yl)-methyl]-2-(1-triphenylmethyl-tetrazol-5-yl)-biphenyl und Salzsäure in Methylenchlorid/Ether/Methanol.
Ausbeute: 97,5 % der Theorie,
Schmelzpunkt: 163-165°C

| $C_{33}H_{38}H_8O$ x $H_20$ (580,73) | | | | | | |
|---|---|---|---|---|---|---|
| Ber.: | C | 68,25 | H | 6,94 | N | 19,30 |
| Gef.: | | 68,43 | | 6,90 | | 19,15 |

Beispiel 249

4'-[(2-n-Butyl-6-cyclohexylaminoaminocarbonylamino-benzimidazol-1-yl)-methyl]-2-trifluormethansulfonamino-biphenyl

Hergestellt analog Beispiel 7 aus 4'-[(6-Amino-2-n-butyl-benzimidazol-1-yl)-methyl]-2-trifluormethansulfonamino-biphenyl und Cyclohexylisocyanat.
Ausbeute: 90,9 % der Theorie,
Schmelzpunkt: 163-165°C

| $C_{32}H_{36}F_3N_5O_3S$ (627,73) | | | | | | |
|---|---|---|---|---|---|---|
| Ber.: | C | 61,23 | H | 5,78 | N | 11,15 |
| Gef.: | | 61,12 | | 5,67 | | 11,29 |

Beispiel 250

4'-[(2-n-Butyl-6-cyclohexylaminocarbonyloxy-benzimidazol-1-yl)-methyl]biphenyl-2-carbonsäure

Hergestellt analog Beispiel 9 aus 4'-[(2-n-Butyl-6-cyclohexylaminocarbonyloxy-benzimidazol-1-yl)-methyl]biphenyl-2-carbonsäure-tert.butylester und Trifluoressigsäure in Methylenchlorid.
Ausbeute: 80,0 % der Theorie,
Schmelzpunkt: 218-219°C

| $C_{32}H_{35}N_3O_4$ (525,65) | | | | | | |
|---|---|---|---|---|---|---|
| Ber.: | C | 73,12 | H | 6,71 | N | 7,99 |
| Gef.: | | 73,04 | | 6,84 | | 7,92 |

Beispiel 251

4'-[(2-n-Butyl-6-cyclohexyloxycarbonylamino-benzimidazol-1-yl)-methyl]biphenyl-2-carbonsäure

Hergestellt analog Beispiel 9 aus 4'-[(2-n-Butyl-6-cyclohexyloxycarbonylamino-benzimidazol-1-yl)-methyl]biphenyl-2-carbonsäure-tert.butylester und Trifluoressigsäure in Methylenchlorid.

Ausbeute: 90,9 % der Theorie,
Schmelzpunkt: 238-240°C

| $C_{32}H_{35}N_3O_4$ (525,65) | | | | | | |
|---|---|---|---|---|---|---|
| Ber.: | C | 73,12 | H | 6,71 | N | 7,99 |
| Gef.: | | 73,18 | | 6,74 | | 8,11 |

Beispiel 252

4'-[(2-n-Butyl-6-morpholinocarbonylamino-benzimidazol-1-yl)-methyl]biphenyl-2-carbonsäure

Hergestellt analog Beispiel 9 aus 4'-[(2-n-Butyl-6-morpholinocarbonylamino-benzimidazol-1-yl)-methyl]biphenyl-2-carbonsäure-tert.butylester und Trifluoressigsäure in Methylenchlorid.
Ausbeute: 85,0 % der Theorie,
Schmelzpunkt: 278-279°C

| $C_{30}H_{32}N_4O_4$ (512,61) | | | | | | |
|---|---|---|---|---|---|---|
| Ber.: | C | 70,29 | H | 6,29 | N | 10,93 |
| Gef.: | | 70,28 | | 6,28 | | 11,00 |

Beispiel 253

4'-[(2-n-Butyl-6-pyrrolidinocarbonylamino-benzimidazol-1-yl)-methyl]biphenyl-2-carbonsäure

Hergestellt analog Beispiel 9 aus 4'-[(2-n-Butyl-6-pyrrolidinocarbonylamino-benzimidazol-1-yl)-methyl]biphenyl-2-carbonsäure-tert.butylester und Trifluoressigsäure in Methylenchlorid.

Beispiel 254

4'-[(2-n-Butyl-6-(N-methylaminocarbonyl-N-(3-cyclohexyl-n-propyl)-amino)-benzimidazol-1-yl)-methyl]biphenyl-2-carbonsäure

Hergestellt analog Beispiel 9 aus 4'-[(2-n-Butyl-6-(N-methylaminocarbonyl-N-(3-cyclohexyl-n-propyl)-amino)-benzimidazol-1-yl)-methyl]biphenyl-2-carbonsäure-tert.butylester und Trifluoressigsäure in Methylenchlorid.
Ausbeute: 84,8 % der Theorie,
Schmelzpunkt: 215-216°C

| $C_{36}H_{44}N_4O_3$ (580,72) | | | | | | |
|---|---|---|---|---|---|---|
| Ber.: | C | 74,45 | H | 7,64 | N | 9,65 |
| Gef.: | | 74,52 | | 7,75 | | 9,76 |

Beispiel 255

4'-[(2-n-Butyl-6-(cis-hexahydro-phthalimino-benzimidazol-1-yl)-methyl]-1-(1H-tetrazol-5-yl)-2-phenylnaphthalin

Hergestellt analog Beispiel 59 aus 4'-[(2-n-Butyl-(cis-hexahydro-phthalimino-benzimidazol-1-yl)-methyl]-1-cyano-2-phenylnaphthalin und Ammoniumchlorid/Natriumazid.

Beispiel 256

4'-[(2-n-Propyl-6-(N-cyclohexylaminocarbonyl-methylamino)-benzimidazol-1-yl)-methyl]-1-(1H-tetrazol-5-yl)-2-phenylnaphthalin

Hergestellt analog Beispiel 59 aus 4'-[(2-n-Propyl-6-(N-cyclohexylaminocarbonyl-methylamino)-benzimidazol-1-yl)-methyl]-1-cyano-2-phenylnaphthalin und Ammoniumchlorid/Natriumazid.

Beispiel 257

4'-[(2-n-Propyl-6-(N-cyclohexylaminocarbonyl-methylamino)-benzimidazol-1-yl)-methyl]-2-phenylnaphthalin-1-carbonsäure

Hergestellt analog Beispiel 9 aus 4'-[(2-n-Propyl-6-(N-cyclohexylaminocarbonyl-methylamino)-benzimidazol-1-yl)-methyl]-2-phenylnaphthalin-1-carbonsäure-tert.butylester und Trifluoressigsäure.
Ausbeute: 73,0 % der Theorie,
Schmelzpunkt: 193-195°C

| $C_{36}H_{38}N_4O_3$ (574,70) | | | | | | |
|---|---|---|---|---|---|---|
| Ber.: | C | 75,28 | H | 6,67 | N | 9,75 |
| Gef.: | | 75,10 | | 6,71 | | 9,72 |

Beispiel 258

4'-[(2-n-Butyl-6-(cis-hexahydro-phthalimino)-benzimidazol-1-yl)-methyl]-2-phenylnaphthalin-1-carbonsäure

Hergestellt analog Beispiel 9 aus 4'-[(2-n-Butyl-6-(cis-hexahydro-phthalimino)-benzimidazol-1-yl)-methyl]-2-phenylnaphthalin-1-carbonsäure-tert.butylester und Trifluoressigsäure.
Ausbeute: 37,0 % der Theorie,
Schmelzpunkt: 215-218°C

| $C_{36}H_{33}N_3O_4$ (571,65) | | | | | | |
|---|---|---|---|---|---|---|
| Ber.: | C | 75,63 | H | 5,82 | N | 7,35 |
| Gef.: | | 75,47 | | 5,63 | | 7,11 |

Beispiel 259

4'-[(2-n-Butyl-6-(N-(n-dodecylaminocarbonyl)-methylamino)-benzimidazol-1-yl)-methyl]biphenyl-2-carbonsäure-trifluoracetat

Hergestellt analog Beispiel 9 aus 4'-[(2-n-Butyl-6-(N-(n-dodecylaminocarbonyl)-methylamino)-benzimidazol-1-yl)-methyl]biphenyl-2-carbonsäure-tert.butylester in Trifluoressigsäure/Methylenchlorid.
Ausbeute: 85,7 % der Theorie,

Schmelzpunkt: 61-62°C (amorph)

| $C_{39}H_{52}H_4O_3$ x $CF_3COOH$ (738,89) | | | | | | |
|---|---|---|---|---|---|---|
| Ber.: | C | 66,65 | H | 7,23 | N | 7,58 |
| Gef.: | | 66,68 | | 7,47 | | 7,83 |

Beispiel 260

4'-[(2-n-Butyl-6-(N-(cyclohexylaminocarbonyl)-n-octylamino)-benzimidazol-1-yl)-methyl]biphenyl-2-carbonsäure

Hergestellt analog Beispiel 72 aus 4'-[(2-n-Butyl-6-(N-(cyclohexylaminocarbonyl)-n-octylamino)-benzimidazol-1-yl)-methyl]biphenyl-2-carbonsäure-ethylester in Ethanol/2n Natronlauge.
Ausbeute: 94,9 % der Theorie,
Schmelzpunkt: 151-152°C

| $C_{40}H_{52}N_4O_3$ (636,88) | | | | | | |
|---|---|---|---|---|---|---|
| Ber.: | C | 75,44 | H | 8,23 | N | 8,80 |
| Gef.: | | 75,63 | | 8,37 | | 8,92 |

Beispiel 261

4'-[(2-n-Butyl-6-(N-(n-octylaminocarbonyl)-methylamino)-benzimidazol-1-yl)-methyl]biphenyl-2-carbonsäure

Hergestellt analog Beispiel 9 aus 4'-[(2-n-Butyl-6-(N-(n-octylaminocarbonyl)-methylamino)-benzimidazol-1-yl)-methyl]biphenyl-2-carbonsäure-tert.butylester in Trifluoressigsäure/Methylenchlorid.
Ausbeute: 87,5 % der Theorie,
Schmelzpunkt: 169-170°C

| $C_{35}H_{44}N_4O_3$ (568,76) | | | | | | |
|---|---|---|---|---|---|---|
| Ber.: | C | 73,91 | H | 7,80 | N | 9,85 |
| Gef.: | | 73,98 | | 7,95 | | 9,78 |

Beispiel 262

4'-[(2-n-Butyl-6-(cis-hexahydro-phthalimino)-benzimidazol-1-yl)-methyl]-2-(1H-tetrazol-5-yl)-biphenyl

Hergestellt analog Beispiel 58 aus 4'-[(2-n-Butyl-6-(cis-hexahydro-phthalimino)-benzimidazol-1-yl)-methyl]-2-(1-triphenylmethyl-tetrazol-5-yl)-biphenyl und Salzsäure in Ether/Methanol/Methylenchlorid.
Ausbeute: 47,6 % der Theorie,

Schmelzpunkt: 147-149°C

| $C_{33}H_{33}N_7O_2$ (559,67) | | | | | | |
|---|---|---|---|---|---|---|
| Ber.: | C | 70,82 | H | 5,94 | N | 17,52 |
| Gef.: | | 70,61 | | 6,12 | | 17,63 |

Beispiel 263

4'-[(2-n-Butyl-6-(endo-bicyclo[2.2.2]oct-5-en-2,3-dicarbonsäureimino)-benzimidazol-1-yl)-methyl]biphenyl-2-carbonsäure-trifluoracetat

Hergestellt analog Beispiel 9 aus 4'-[(2-n-Butyl-6-(endo-bicyclo[2.2.2]oct-5-en-2,3-dicarbonsäureimino)-benzimidazol-1-yl)-methyl]biphenyl-2-carbonsäure-tert.butylester und Trifluoressigsäure in Methylenchlorid.
Ausbeute: 88,6 % der Theorie,
Schmelzpunkt: 161-163°C

| $C_{35}H_{33}N_3O_4$ x $CF_3COOH$ (673,69) | | | | | | |
|---|---|---|---|---|---|---|
| Ber.: | C | 65,96 | H | 5,08 | N | 6,23 |
| Gef.: | | 65,72 | | 4,99 | | 6,11 |

Beispiel 264

4'-[(2-n-Butyl-6-(methyl-5-norbornen-2,3-dicarbonsäureimino)-benzimidazol-1-yl)-methyl]biphenyl-2-carbonsäure (Isomerengemisch)

Hergestellt analog Beispiel 9 aus 4'-[(2-n-Butyl-6-(methyl-5-norbornen-2,3-dicarbonsäureimino)-benzimidazol-1-yl)-methyl]biphenyl-2-carbonsäure-tert.butylester (Isomerengemisch) und Trifluoressigsäure in Methylenchlorid.

Beispiel 265

4'-[(2-n-Butyl-6-(trans-hexahydro-phthalimino)-benzimidazol-1-yl)-methyl]biphenyl-2-carbonsäure-dihydrat

Hergestellt analog Beispiel 9 aus 4'-[(2-n-Butyl-6-(transhexahydro-phthalimino)-benzimidazol-1-yl)-methyl]biphenyl- 2-carbonsäure-tert.butylester und Trifluoressigsäure in Methylenchlorid.
Ausbeute: 60,7 % der Theorie,
Schmelzpunkt: 188-190°C (Zers.)

| $C_{33}H_{33}N_3O_4$ x 2 $H_2O$ (571,67) | | | | | | |
|---|---|---|---|---|---|---|
| Ber.: | C | 69,33 | H | 6,52 | N | 7,35 |
| Gef.: | | 69,48 | | 6,40 | | 7,57 |

Beispiel 266

4'-[(2-n-Butyl-6-(3,6-endoxo-1,2,3,6-tetrahydro-phthalimino)-benzimidazol-1-yl)-methyl]biphenyl-2-carbonsäure

Hergestellt analog Beispiel 9 aus 4'-[(2-n-Butyl-6-(3,6-endoxo-1,2,3,6-tetrahydro-phthalimino)-benzimidazol-1-yl)-methyl]biphenyl-2-carbonsäure-tert.butylester und Trifluoressigsäure in Methylenchlorid.

Beispiel 267

4'-[(2-n-Butyl-6-(cis-5-norbornen-endo-2,3-dicarbonsäureimino)-benzimidazol-1-yl)-methyl]biphenyl-2-carbonsäure-monohydrat

Hergestellt analog Beispiel 9 aus 4'-[(2-n-Butyl-6-(cis-5-norbornen-endo-2,3-dicarbonsäureimino)-benzimidazol-1-yl)-methyl]biphenyl-2-carbonsäure-tert.butylester und Trifluoressigsäure in Methylenchlorid.
Ausbeute: 22,1 % der Theorie,
Schmelzpunkt: 263-265°C

| $C_{34}H_{31}N_3O_4$ x $H_2O$ (563,65) | | | | | | |
|---|---|---|---|---|---|---|
| Ber.: | C | 72,44 | H | 5,90 | N | 7,45 |
| Gef.: | | 72,28 | | 5,81 | | 7,46 |

Beispiel 268

4'-[(2-n-Butyl-6-(2-carboxy-cyclohexylcarbonylamino)-benzimidazol-1-yl)-methyl]biphenyl-2-carbonsäure

0,6 g (1,5 mMol) 4'-[(6-Amino-2-n-butyl-benzimidazol-1-yl)-methyl]biphenyl-2-carbonsäure werden unter Erwärmen in 150 ml Methylenchlorid gelöst und auf Raumtemperatur abgekühlt. Nach Zugabe von 240 mg (1,55 mMol) cis-Cyclohexan-1,2-dicarbonsäureanhydrid wird 5 Stunden bei Raumtemperatur gerührt. Das auskristallisierte Reaktionsprodukt wird abgesaugt, mit Methylenchlorid gewaschen und im Vakuumtrockenschrank bei 50°C getrocknet.
Ausbeute: 0,58 g (69,8 % der Theorie),
Schmelzpunkt: 186-188°C

| $C_{33}H_{35}N_3O_5$ (553,66) | | | | | | |
|---|---|---|---|---|---|---|
| Ber.: | C | 71,59 | H | 6,37 | N | 7,59 |
| Gef.: | | 71,42 | | 6,56 | | 7,56 |

Beispiel 269

4'-[(2-n-Butyl-6-(3-carboxy-cis-5-norbornen-endo-2-carbonylamino)-benzimidazol-1-yl)-methyl]biphenyl-2-carbonsäure

Hergestellt analog Beispiel 9 aus 4'-[(2-n-Butyl-6-(cis-5-norbornen-endo-2,3-dicarbonsäureimino)-benzimidazol-1-yl)-methyl]biphenyl-2-carbonsäure-tert.butylester und Trifluoressigsäure in Methylenchlorid.
Ausbeute: 17,1 % der Theorie,
Schmelzpunkt: 199-201°C

| $C_{34}H_{33}N_3O_5$ (563,66) | | | | | | |
|---|---|---|---|---|---|---|
| Ber.: | C | 72,44 | H | 5,90 | N | 7,45 |
| Gef.: | | 72,26 | | 5,82 | | 7,44 |

Beispiel 270

4'-[(2-n-Butyl-6-dimethylaminocarbonyloxy-benzimidazol-1-yl)-methyl]biphenyl-2-carbonsäure

Hergestellt analog Beispiel 9 aus 4'-[(2-n-Butyl-6-dimethylaminocarbonyloxy-benzimidazol-1-yl)-methyl]biphenyl-2-carbonsäure-tert.butylester und Trifluoressigsäure in Methylenchlorid.
Ausbeute: 81,9 % der Theorie,
Schmelzpunkt: 241-242°C

| $C_{28}H_{29}N_3O_4$ (471,56) | | | | | | |
|---|---|---|---|---|---|---|
| Ber.: | C | 71,32 | H | 6,20 | N | 8,91 |
| Gef.: | | 71,15 | | 6,28 | | 8,89 |

Beispiel 271

4'-[(2-n-Butyl-6-(N-acetyl-n-octylamino)-benzimidazol-1-yl)-methyl]biphenyl-2-carbonsäure

Hergestellt analog Beispiel 9 aus 4'-[(2-n-Butyl-6-(N-acetyl-n-octylamino)-benzimidazol-1-yl)-methyl]biphenyl-2-carbonsäure-tert.butylester und Trifluoressigsäure in Methylenchlorid.
Ausbeute: 77,2 % der Theorie,
Schmelzpunkt: 192-193°C

| $C_{35}H_{43}N_3O_3$ (553,74) | | | | | | |
|---|---|---|---|---|---|---|
| Ber.: | C | 75,92 | H | 7,83 | N | 7,59 |
| Gef.: | | 75,75 | | 8,03 | | 7,45 |

Beispiel 272

4'-[(2-n-Butyl-6-(2-phenylethylaminocarbonyl)-benzimidazol-1-yl)-methyl]biphenyl-2-carbonsäure-tert.butylester

484 mg (1 mMol) 4'-[(2-n-Butyl-6-carboxy-benzimidazol-1-yl)-methyl]biphenyl-2-carbonsäure-tert.butylester werden in 10 ml absolutem Tetrahydrofuran gelöst und unter Rühren und Trockeneis/Isopropanol-Kühlung auf -20°C abgekühlt. Bei dieser Temperatur werden 101 mg (1 mMol) N-Methylmorpholin zugegeben. Anschließend wird auf -30°C abgekühlt und eine Lösung von 136 mg (1 mMol) Chlorameisensäureisobutylester, gelöst in 5 ml absolutem Tetrahydrofuran, langsam zugetropft. Zur vollständigen Bildung des gemischten Anhydrides wird eine Stunde bei -40°C nachgerührt. Bei -40°C tropft man 134 mg (1,1 mMol) 2-Phenylethylamin, gelöst in 2 ml absolutem Tetrahydrofuran, langsam zu. Danach läßt man die Temperatur langsam auf Raumtemperatur ansteigen und rührt 16 Stunden lang bei Raumtemperatur. Das Lösungsmittel wird abdestilliert, das zurückbleibende viskose Öl in gesättigter Kochsalzlösung/Essigester aufgenommen und insgesamt 3 x mit Essigester extrahiert. Die Essigesterphasen werden über Natriumsulfat getrocknet und eingeengt. Das viskose Öl wird in Methylenchlorid gelöst und über eine Kieselgel-Säule (Elutionsmittel: Methylenchlorid/Ethanol = 50:1 und 25:1) gereinigt.
Ausbeute: 505 mg (86 % der Theorie),
Öl, $R_f$-Wert: 0,5 (Kieselgel: Ethanol/Methylenchlorid = 1:19)

| $C_{38}H_{41}N_3O_3$ (587,80) | | | | | | |
|---|---|---|---|---|---|---|
| Ber.: | C | 77,65 | H | 7,03 | N | 7,15 |
| Gef.: | | 77,58 | | 7,24 | | 7,34 |

Analog werden folgende Verbindungen erhalten:
4'-[(2-n-Butyl-6-(2-phenylamino-ethylaminocarbonyl)-benzimidazol-1-yl)-methyl]biphenyl-2-carbonsäure-tert.butylester
Öl, $R_f$-Wert: 0,45 (Kieselgel: Ethanol/Methylenchlorid = 1:19)
4'-[(2-n-Butyl-6-(5-carboxy-n-pentylaminocarbonyl)-benzimidazol-1-yl)-methyl]biphenyl-2-carbonsäure-tert.butylester
Schmelzpunkt: 166-167°C

Beispiel 273

4'-[(2-n-Butyl-6-(2-phenylethylaminocarbonyl)-benzimidazol-1-yl)-methyl]biphenyl-2-carbonsäure

Hergestellt analog Beispiel 9 aus 4'-[(2-n-Butyl-6-(2-phenylethylaminocarbonyl)-benzimidazol-1-yl)-methyl]biphenyl-2-carbonsäure-tert.butylester und Trifluoressigsäure in Methylenchlorid.
Ausbeute: 19,7 % der Theorie,
Schmelzpunkt: 208-210°C

| $C_{34}H_{33}N_3O_3$ (531,65) | | | | | | |
|---|---|---|---|---|---|---|
| Ber.: | C | 76,81 | H | 6,26 | N | 7,90 |
| Gef.: | | 76,69 | | 6,48 | | 7,92 |

Beispiel 274

4'-[(2-n-Butyl-6-(2-phenylaminoethylaminocarbonyl)-benzimidazol-1-yl)-methyl]biphenyl-2-carbonsäure x 1,5 HCl

Hergestellt analog Beispiel 9 aus 4'-[(2-n-Butyl-6-(2-phenylaminoethylaminocarbonyl)-benzimidazol-1-yl)-methyl]biphenyl-2-carbonsäure-tert.butylester und Trifluoressigsäure in Methylenchlorid.
Ausbeute: 30 % der Theorie,
Schmelzpunkt: 240-242°C

| $C_{34}H_{34}N_4O_3$ x 1,5 HCl (601,36) | | | | | | |
|---|---|---|---|---|---|---|
| Ber.: | C | 67,91 | H | 5,95 | N | 9,32 |
| Gef.: | | 67,88 | | 5,93 | | 9,15 |

Beispiel 275

4'-[(2-n-Butyl-6-(5-carboxy-n-pentylaminocarbonyl)-benzimidazol-1-yl)-methyl]biphenyl-2-carbonsäure

Hergestellt analog Beispiel 9 aus 4'-[(2-n-Butyl-6-(5-carboxy-n-pentylaminocarbonyl)-benzimidazol-1-yl)-methyl]biphenyl-2-carbonsäure-tert.butylester und Trifluoressigsäure in Methylenchlorid.
Ausbeute: 37,4 % der Theorie,
Schmelzpunkt: ab 116°C (Zers.)

| $C_{37}H_{35}N_3O_5$ (541,70) | | | | | | |
|---|---|---|---|---|---|---|
| Ber.: | C | 70,96 | H | 6,51 | N | 7,76 |
| Gef.: | | 70,84 | | 6,42 | | 7,85 |

Beispiel 276

4'-[(2-n-Butyl-6-(2-carboxy-propionyl)-benzimidazol-1-yl)-methyl]biphenyl-2-carbonsäure

Hergestellt analog Beispiel 72 aus 4'-[(2-n-Butyl-6-(2-carboxy-propionyl)-benzimidazol-1-yl)-methyl]biphenyl-2-carbonsäure-methylester und Natronlauge/Methanol.

Beispiel 277

4'-[(2-n-Butyl-6-homophthalimino-benzimidazol-1-yl)-methyl]biphenyl-2-carbonsäure-tert.butylester

1,5 g (3,3 mMol) 4'-[(6-Amino-2-n-butyl-benzimidazol-1-yl)-methyl]biphenyl-2-carbonsäure-tert.butylester und 0,6 g (3,63 mMol) Homophthalsäureanhydrid werden in 20 ml Pyridin 20 Stunden am Rückfluß gekocht. Anschließend wird das Lösungsmittel im Vakuum entfernt und der Rückstand über eine Kieselgelsäule (Korngröße: 0,063-0,2 mm; Elutionsmittel: Methylenchlorid/Ethanol = 100:2, 100:3 und 100:5) gereinigt.
Ausbeute: 15,2 % der Theorie,
Schmelzpunkt: 109-112°C

| $C_{38}H_{37}N_3O_4$ (599,73) | | | | | | |
|---|---|---|---|---|---|---|
| Ber.: | C | 76,10 | H | 6,22 | N | 7,01 |
| Gef.: | | 75,90 | | 6,28 | | 6,90 |

Analog werden folgende Verbindungen erhalten:
4'-[(2-n-Butyl-6-(3,3-tetramethylen-glutarimino)-benzimidazol-1-yl)-methyl]biphenyl-2-carbonsäure-tert.butylester
4'-[(2-n-Butyl-6-(3,3-pentamethylen-glutarimino)-benzimidazol-1-yl)-methyl]biphenyl-2-carbonsäure-tert.butylester
4'-[(2-n-Butyl-6-(7-methoxy-homophthalimino)-benzimidazol-1-yl)-methyl]biphenyl-2-carbonsäure-tert.butylester
4'-[(2-n-Butyl-6-(4,4-dimethyl-7-methoxy-homophthalimino)-benzimidazol-1-yl)-methyl]biphenyl-2-carbonsäure-tert.butylester
4'-[(2-n-Butyl-6-(6,7-dimethoxy-homophthalimino)-benzimidazol-1-yl)-methyl]biphenyl-2-carbonsäure-tert.butylester
4'-[(2-n-Butyl-6-(6,7-dimethoxy-4,4-dimethyl-homophthalimino)-benzimidazol-1-yl)-methyl]biphenyl-2-carbonsäure-tert.butylester
4'-[(2-n-Butyl-6-glutarimino-benzimidazol-1-yl)-methyl]biphenyl-2-carbonsäure-tert.butylester
4'-[(2-n-Butyl-6-(3-methyl-glutarimino)-benzimidazol-1-yl)-methyl]biphenyl-2-carbonsäure-tert.butylester
4'-[(2-n-Butyl-6-(3,3-dimethyl-glutarimino)-benzimidazol-1-yl)-methyl]biphenyl-2-carbonsäure-tert.butylester
4'-[(2-n-Butyl-6-(3-ethyl-3-methyl-glutarimino)-benzimidazol-1-yl)-methyl]biphenyl-2-carbonsäure-tert.butylester
4'-[(2-n-Butyl-6-(cis-hexahydrophthalimino)-benzimidazol-1-yl)-methyl]-2-(1-triphenylmethyl-tetrazol-5-yl)biphenyl
4'-[(2-n-Butyl-6-(4,4-dimethyl-homophthalimino)-benzimidazol-1-yl)-methyl]biphenyl-2-carbonsäure-tert.butylester
4'-[(2-n-Butyl-6-(5-methyl-hexahydrophthalimino)-benzimidazol-1-yl)-methyl]biphenyl-2-carbonsäure-tert.butylester
Öl, $R_f$-Wert: 0,75 (Kieselgel; Essigester/Petrolether = 4:1)

Beispiel 278

4'-[(2-n-Butyl-6-homophthalimino-benzimidazol-1-yl)-methyl]biphenyl-2-carbonsäure

Hergestellt analog Beispiel 9 aus 4'-[(2-n-Butyl-6-homophtalimino-benzimidazol-1-yl)-methyl]biphenyl-2-carbonsäure-tert.butylester und Trifluoressigsäure in Methylenchlorid.
Ausbeute: 79 % der Theorie,
Schmelzpunkt: 171-173°C

| $C_{34}H_{29}N_3O_4$ (543,62) | | | | | | |
|---|---|---|---|---|---|---|
| Ber.: | C | 75,12 | H | 5,38 | N | 7,73 |
| Gef.: | | 74,95 | | 5,18 | | 7,64 |

Beispiel 279

4'-[(2-n-Butyl-6-(4,4-dimethyl-homophthalimino)-benzimidazol-1-yl)-methyl]biphenyl-2-carbonsäure-monohydrat

Hergestellt analog Beispiel 9 aus 4'-[(2-n-Butyl-6-(4,4-dimethyl-homophthalimino)-benzimidazol-1-yl)-methyl]biphenyl-2-carbonsäure-tert.butylester und Trifluoressigsäure in Methylenchlorid.
Ausbeute: 77,4 % der Theorie,
Schmelzpunkt: 299-301°C

| $C_{36}H_{33}N_3O_4$ x $H_2O$ (589,69) | | | | | | |
|---|---|---|---|---|---|---|
| Ber.: | C | 73,33 | H | 5,98 | N | 7,13 |
| Gef.: | | 73,60 | | 6,14 | | 7,33 |

Beispiel 280

4'-[(2-n-Butyl-6-(3,3-tetramethylen-glutarimino)-benzimidazol-1-yl)-methyl]biphenyl-2-carbonsäure

Hergestellt analog Beispiel 9 aus 4'-[(2-n-Butyl-6-(3,3-tetramethylen-glutarimino)-benzimidazol-1-yl)-methyl]biphenyl-2-carbonsäure-tert.butylester und Trifluoressigsäure in Methylenchlorid.
Ausbeute: 40,0 % der Theorie,
Schmelzpunkt: 191-194°C

| $C_{34}H_{35}N_3O_4$ (549,64) | | | | | | |
|---|---|---|---|---|---|---|
| Ber.: | C | 74,29 | H | 6,42 | N | 7,65 |
| Gef.: | | 74,08 | | 6,31 | | 7,52 |

Beispiel 281

4'-[(2-n-Butyl-6-(3,3-pentamethylen-glutarimino)-benzimidazol-1-yl)-methyl]biphenyl-2-carbonsäure

Hergestellt analog Beispiel 9 aus 4'-[(2-n-Butyl-6-(3,3-pentamethylen-glutarimino)-benzimidazol-1-yl)-methyl]biphenyl-2-carbonsäure-tert.butylester und Trifluoressigsäure in Methylenchlorid.
Ausbeute: 31,0 % der Theorie,
Schmelzpunkt: 182-185°C

| $C_{35}H_{37}N_3O_4$ (563,67) | | | | | | |
|---|---|---|---|---|---|---|
| Ber.: | C | 74,57 | H | 6,62 | N | 7,46 |
| Gef.: | | 74,43 | | 6,52 | | 7,32 |

Beispiel 282

4'-[(2-n-Butyl-6-(7-methoxy-homophthalimino)-benzimidazol-1-yl)-methyl]biphenyl-2-carbonsäure

Hergestellt analog Beispiel 9 aus 4'-[(2-n-Butyl-6-(7-methoxy-homophthalimino)-benzimidazol-1-yl)-methyl]biphenyl-2-carbonsäure-tert.butylester und Trifluoressigsäure.

Beispiel 283

4'-[(2-n-Butyl-6-(4,4-dimethyl-7-methoxy-homophthalimino)-benzimidazol-1-yl)-methyl]biphenyl-2-carbonsäure

Hergestellt analog Beispiel 9 aus 4'-[(2-n-Butyl-6-(4,4-dimethyl-7-methoxy-homophthalimino)-benzimidazol-1-yl)-methyl]biphenyl-2-carbonsäure-tert.butylester und Trifluoressigsäure in Methylenchlorid.
Ausbeute: 60,1 % der Theorie,
Schmelzpunkt: 104-105°C

| $C_{37}H_{35}N_3O_5$ (601,70) | | | | | | |
|---|---|---|---|---|---|---|
| Ber.: | C | 73,86 | H | 5,86 | N | 6,98 |
| Gef.: | | 73,92 | | 6,04 | | 7,11 |

Beispiel 284

4'-[(2-n-Butyl-6-(6,7-dimethoxy-homophthalimino)-benzimidazol-1-yl)-methyl]biphenyl-2-carbonsäure

Hergestellt analog Beispiel 9 aus 4'-[(2-n-Butyl-6-(6,7-dimethoxy-homophthalimino)-benzimidazol-1-yl)-methyl]biphenyl-2-carbonsäure-tert.butylester und Trifluoressigsäure in Methylenchlorid.

Beispiel 285

4'-[(2-n-Butyl-6-(6,7-dimethoxy-4,4-dimethyl-homophthalimino)-benzimidazol-1-yl)-methyl]biphenyl-2-carbonsäure

Hergestellt analog Beispiel 9 aus 4'-[(2-n-Butyl-6-(6,7-dimethoxy-4,4-dimethyl-homophthalimino)-benzimidazol-1-yl)-methyl]biphenyl-2-carbonsäure-tert.butylester und Trifluoressigsäure in Methylenchlorid.

Beispiel 286

4'-[(2-n-Butyl-6-(3,3-pentamethylen-glutarimino)-benzimidazol-1-yl)-methyl]-2-(1H-tetrazol-5-yl)-biphenyl

Hergestellt analog Beispiel 59 aus 4'-[(2-n-Butyl-6-(3,3-pentamethylen-glutarimino)-benzimidazol-1-yl)-methyl]-2-cyano- biphenyl und Natriumazid/Ammoniumchlorid.
Ausbeute: 29,0 % der Theorie,
Schmelzpunkt: ab 140°C (sintern)

| $C_{35}H_{37}N_7O_2$ (587,70) | | | | | | |
|---|---|---|---|---|---|---|
| Ber.: | C | 71,52 | H | 6,35 | N | 16,68 |
| Gef.: | | 71,37 | | 6,20 | | 16,71 |

Beispiel 287

4'-[(2-n-Butyl-6-(3,3-tetramethylen-glutarimino)-benzimidazol-1-yl)-methyl]-2-(1H-tetrazol-5-yl)-biphenyl

Hergestellt analog Beispiel 59 aus 4'-[(2-n-Butyl-6-(3,3-tetramethylen-glutarimino)-benzimidazol-1-yl)-methyl]-2-cyano-biphenyl und Natriumazid/Ammoniumchlorid.
Ausbeute: 31,0 % der Theorie,

Schmelzpunkt: ab 166°C (sintern)

| $C_{34}H_{35}N_7O_2$ (573,68) | | | | | | |
|---|---|---|---|---|---|---|
| Ber.: | C | 71,18 | H | 6,15 | N | 17,09 |
| Gef.: | | 71,10 | | 6,22 | | 17,20 |

Beispiel 288

4'-[(2-n-Butyl-6-glutarimino-benzimidazol-1-yl)-methyl]biphenyl-2-carbonsäure

Hergestellt analog Beispiel 9 aus 4'-[(2-n-Butyl-6-glutarimino-benzimidazol-1-yl)-methyl]biphenyl-2-carbonsäure-tert.butylester und Trifluoressigsäure in Methylenchlorid. Ausbeute: 82,0 % der Theorie,
Schmelzpunkt: >220°C

| $C_{30}H_{29}N_3O_4$ (495,56) | | | | | | |
|---|---|---|---|---|---|---|
| Ber.: | C | 72,71 | H | 5,90 | N | 8,48 |
| Gef.: | | 72,56 | | 5,79 | | 8,22 |

Beispiel 289

4'-[(2-n-Butyl-6-glutarimino-benzimidazol-1-yl)-methyl]-2-(1H-tetrazol-5-yl)-biphenyl

Hergestellt analog Beispiel 59 aus 4'-[(2-n-Butyl-6-glutarimino-benzimidazol-1-yl)-methyl]-2-cyano-biphenyl und Natriumazid/Ammoniumchlorid.
Ausbeute: 47,0 % der Theorie,
Schmelzpunkt: ab 139°C (sintern)

| $C_{30}H_{29}N_7O_2$ (519,59) | | | | | | |
|---|---|---|---|---|---|---|
| Ber.: | C | 69,34 | H | 5,62 | N | 18,87 |
| Gef.: | | 69,30 | | 5,52 | | 18,69 |

Beispiel 290

4'-[(2-n-Butyl-6-(3-methyl-glutarimino)-benzimidazol-1-yl)-methyl]biphenyl-2-carbonsäure

Hergestellt analog Beispiel 9 aus 4'-[(2-n-Butyl-6-(3-methyl-glutarimino)-benzimidazol-1-yl)-methyl]biphenyl-2-carbonsäure-tert.butylester und Trifluoressigsäure in Methylenchlorid.
Ausbeute: 39,0 % der Theorie,
Schmelzpunkt: >220°C

| $C_{31}H_{31}N_3O_4$ (509,58) | | | | | | |
|---|---|---|---|---|---|---|
| Ber.: | C | 73,06 | H | 6,13 | N | 8,25 |
| Gef.: | | 72,91 | | 5,88 | | 8,02 |

Beispiel 291

4'-[(2-n-Butyl-6-(3-methyl-glutarimino)-benzimidazol-1-yl)-methyl]-2-(1H-tetrazol-5-yl)-biphenyl

Hergestellt analog Beispiel 59 aus 4'-[(2-n-Butyl-6-(3-methyl-glutarimino)-benzimidazol-1-yl)-methyl]-2-cyano-biphenyl und Natriumazid/Ammoniumchlorid.
Ausbeute: 28,0 % der Theorie,
Schmelzpunkt: ab 157°C (sintern)

| $C_{31}H_{31}N_7O_2$ (533,61) | | | | | | |
|---|---|---|---|---|---|---|
| Ber.: | C | 69,77 | H | 5,86 | N | 18,38 |
| Gef.: | | 69,69 | | 5,81 | | 18,16 |

Beispiel 292

4'-[(2-n-Butyl-6-(3,3-dimethyl-glutarimino)-benzimidazol-1-yl)-methyl]biphenyl-2-carbonsäure

Hergestellt analog Beispiel 9 aus 4'-[(2-n-Butyl-6-(3,3-dimethyl-glutarimino)-benzimidazol-1-yl)-methyl]biphenyl-2-carbonsäure-tert.butylester und Trifluoressigsäure in Methylenchlorid.
Ausbeute: 41,0 % der Theorie,
Schmelzpunkt: >220°C

| $C_{32}H_{33}N_3O_4$ (523,61) | | | | | | |
|---|---|---|---|---|---|---|
| Ber.: | C | 73,40 | H | 6,35 | N | 8,03 |
| Gef.: | | 73,29 | | 6,21 | | 7,91 |

Beispiel 293

4'-[(2-n-Butyl-6-(3,3-dimethyl-glutarimino)-benzimidazol-1-yl)-methyl]-2-(1H-tetrazol-5-yl)-biphenyl

Hergestellt analog Beispiel 59 aus 4'-[(2-n-Butyl-6-(3,3-dimethyl-glutarimino)-benzimidazol-1-yl)-methyl]-2-cyano-biphenyl und Natriumazid/Ammoniumchlorid.
Ausbeute: 29,0 % der Theorie,
Schmelzpunkt: ab 151°C (sintern)

| $C_{32}H_{33}N_7O_2$ (547,64) | | | | | | |
|---|---|---|---|---|---|---|
| Ber.: | C | 70,18 | H | 6,07 | N | 17,91 |
| Gef.: | | 69,91 | | 5,98 | | 17,85 |

Beispiel 294

4'-[(2-n-Butyl-6-(3-ethyl-3-methyl-glutarimino)-benzimidazol-1-yl)-methyl]biphenyl-2-carbonsäure

Hergestellt analog Beispiel 9 aus 4'-[(2-n-Butyl-6-(3-ethyl-3-methyl-glutarimino)-benzimidazol-1-yl)-methyl]biphenyl-2-carbonsäure-tert.butylester und Trifluoressig in Methylenchlorid.
Ausbeute: 38,0 % der Theorie,

Schmelzpunkt: >220°C

| $C_{33}H_{35}N_3O_4$ (537,63) | | | | | | |
|---|---|---|---|---|---|---|
| Ber.: | C | 73,72 | H | 6,56 | N | 7,82 |
| Gef.: | | 73,55 | | 6,41 | | 7,76 |

Beispiel 295

4'-[(2-n-Butyl-6-(3-ethyl-3-methyl-glutarimino)-benzimidazol-1-yl)-methyl]-2-(1H-tetrazol-5-yl)-biphenyl

Hergestellt analog Beispiel 59 aus 4'-[(2-n-Butyl-6-(3-ethyl-3-methyl-glutarimino)-benzimidazol-1-yl)-methyl]-2-cyano-biphenyl und Natriumazid/Ammoniumchlorid.
Ausbeute: 26,0 % der Theorie,
Schmelzpunkt: ab 138°C (sintern)

| $C_{33}H_{35}N_7O_2$ (561,67) | | | | | | |
|---|---|---|---|---|---|---|
| Ber.: | C | 70,56 | H | 6,28 | N | 17,46 |
| Gef.: | | 70,47 | | 6,02 | | 17,33 |

Beispiel 296

4'-[(2-n-Butyl-5-(N-cyclohexylaminocarbonyl-methylamino)-benzimidazol-1-yl)-methyl]-2-(1H-tetrazol-5-yl)-biphenyl

Hergestellt analog Beispiel 58b aus 4'-[(2-n-Butyl-5-(N-cyclohexylaminocarbonyl-methylamino)-benzimidazol-1-yl)-methyl]-2-(1-triphenylmethyl-tetrazol-5-yl)-biphenyl und Salzsäure in Methylenchlorid/Ether/Methanol.
Ausbeute: 76,2 % der Theorie,
Schmelzpunkt: 150-152°C

| $C_{33}H_{38}N_8O$ (562,72) | | | | | | |
|---|---|---|---|---|---|---|
| Ber.: | C | 70,44 | H | 6,80 | N | 19,91 |
| Gef.: | | 70,32 | | 7,05 | | 19,76 |

Beispiel 297

4'-[(2-n-Butyl-6-(5-methyl-hexahydrophthalimino)-benzimidazol-1-yl)-methyl]biphenyl-2-carbonsäure

Hergestellt analog Beispiel 9 aus 4'-[(2-n-Butyl-6-(5-methyl-hexahydrophthalimino)-benzimidazol-1-yl)-methyl]biphenyl-2-carbonsäure-tert.butylester und Trifluoressigsäure in Methylenchlorid.
Ausbeute: 37 % der Theorie,

Schmelzpunkt: 143-146°C

| $C_{34}H_{35}N_3O_4$ (549,67) | | | | | | |
|---|---|---|---|---|---|---|
| Ber.: | C | 74,29 | H | 6,42 | N | 7,62 |
| Gef.: | | 74,47 | | 6,67 | | 7,55 |

Beispiel 298

4'-[(2-n-Butyl-5-(cis-hexahydrophthalimino)-benzimidazol-1-yl)-methyl]-2-(1H-tetrazol-5-yl)-biphenyl

Hergestellt analog Beispiel 58 aus 4'-[(2-n-Butyl-5-(cis-hexahydrophthalimino)-benzimidazol-1-yl)-methyl]-2-(1-triphenylmethyl-tetrazol-5-yl)-biphenyl und Salzsäure/Ethanol.
Ausbeute: 59,5 % der Theorie,
Schmelzpunkt: 230-232°C

| $C_{33}H_{33}N_7O_2$ (559,67) | | | | | | |
|---|---|---|---|---|---|---|
| Ber.: | C | 70,82 | H | 5,94 | N | 17,52 |
| Gef.: | | 70,64 | | 6,10 | | 17,72 |

Bei den nachfolgenden pharmazeutischen Anwendungsbeispielen kann als Wirksubstanz jede geeignete Verbindung der Formel I eingesetzt werden, z.B. die Verbindungen der vorstehenden Beispiele:

Beispiel I

| Ampullen, enthaltend 50 mg Wirkstoff pro 5 ml | |
|---|---|
| Wirkstoff | 50 mg |
| $KH_2PO_4$ | 2 mg |
| $Na_2HPO_4$ x $2H_20$ | 50 mg |
| NaCl | 12 mg |
| Wasser für Injektionszwecke ad | 5 ml |

Herstellung:

In einem Teil des Wassers werden die Puffersubstanzen und das Isotonans gelöst. Der Wirkstoff wird zugegeben und nach vollständiger Lösung mit Wasser auf das Nennvolumen aufgefüllt.

Beispiel II

| Ampullen, enthaltend 100 mg Wirkstoff pro 5 ml | |
|---|---|
| Wirkstoff | 100 mg |
| Methylglucamin | 35 mg |
| Glykofurol | 1000 mg |
| Polyethylenglykol-Polypropylenglykol-Blockpolymer | 250 mg |
| Wasser für Injektionszwecke ad | 5 ml |

Herstellung:

In einem Teil des Wassers wird Methylglucamin gelöst und der Wirkstoff unter Rühren und Erwarmen in Lösung gebracht. Nach Zugabe der Lösungsmittel wird mit Wasser auf das Nennvolumen aufgefüllt.

Beispiel III

| Tabletten, enthaltend 50 mg Wirkstoff | |
|---|---|
| Wirkstoff | 50,0 mg |
| Calciumphosphat | 70,0 mg |
| Milchzucker | 40,0 mg |
| Maisstärke | 35,0 mg |
| Polyvinylpyrrolidon | 3,5 mg |
| Magnesiumstearat | 1,5 mg |
| | 200,0 mg |

Herstellung:

Der Wirkstoff, $CaHPO_4$, Milchzucker und Maisstärke werden mit einer wässrigen PVP-Lösung gleichmäßig befeuchtet. Die Masse wird durch ein 2-mm-Sieb gegeben, im Umlufttrockenschrank bei 50°C getrocknet und erneut gesiebt.

Nach Zumischen des Schmiermittels wird das Granulat auf einer Tablettiermaschine verpresst.

Beispiel IV

| Dragees, enthaltend 50 mg Wirkstoff | |
|---|---|
| Wirkstoff | 50,0 mg |
| Lysin | 25,0 mg |
| Milchzucker | 60,0 mg |
| Maisstärke | 34,0 mg |
| Gelatine | 10,0 mg |
| Magnesiumstearat | 1,0 mg |
| | 180,0 mg |

Herstellung:

Der Wirkstoff wird mit den Hilfsstoffen gemischt und mit einer wässrigen Gelatine-Lösung befeuchtet. Nach Siebung und Trocknung wird das Granulat mit Magnesiumstearat vermischt und zu Kernen verpresst.

Die so hergestellten Kerne werden nach bekannten Verfahren mit einer Hülle überzogen. Der Dragiersuspension oder -lösung kann Farbstoff zugegeben werden.

Beispiel V

| Dragees, enthaltend 100 mg Wirkstoff | |
|---|---|
| Wirkstoff | 100,0 mg |
| Lysin | 50,0 mg |
| Milchzucker | 86,0 mg |
| Maisstärke | 50,0 mg |
| Polyvinylpyrrolidon | 2,8 mg |
| Mikrokristalline Cellulose | 60,0 mg |
| Magnesiumstearat | 1,2 mg |
| | 350,0 mg |

Herstellung:

Der Wirkstoff wird mit den Hilfsstoffen gemischt und mit einer wässrigen PVP-Lösung befeuchtet. Die feuchte Masse wird durch ein 1,5-mm-Sieb gegeben und bei 45°C getrocknet. Nach dem Trocknen wird erneut gesiebt und das Magnesiumstearat zugemischt. Diese Mischung wird zu Kernen verpreßt.

Die so hergestellten Kerne werden nach bekannten Verfahren mit einer Hülle überzogen. Der Dragiersuspension oder -lösung können Farbstoffe zugegeben werden.

Beispiel VI

| Kapseln, enthaltend 250 mg Wirkstoff | |
|---|---|
| Wirkstoff | 250,0 mg |
| Maisstärke | 68,5 mg |
| Magnesiumstearat | 1,5 mg |
| | 320,0 mg |

Herstellung:

Wirkstoff und Maisstärke werden gemischt und mit Wasser befeuchtet. Die feuchte Masse wird gesiebt und getrocknet. Das trockene Granulat wird gesiebt und mit Magnesiumstearat gemischt. Die Endmischung wird in Hartgelatinekapseln Größe 1 abgefüllt.

Beispiel VII

| Orale Suspension, enthaltend 50 mg Wirkstoff pro 5 ml | |
|---|---|
| Wirkstoff | 50,0 mg |
| Hydroxyethylcellulose | 50,0 mg |
| Sorbinsäure | 5,0 mg |
| Sorbit 70%ig | 600,0 mg |
| Glycerin | 200,0 mg |
| Aroma | 15,0 mg |
| Wasser ad | 5,0 ml |

Herstellung:

Destilliertes Wasser wird auf 70°C erhitzt. Hierin wird unter Rühren Hydroxyethylcellulose gelöst. Durch Zugabe von Sorbitlösung und Glycerin wird auf Raumtemperatur abgekühlt. Bei Raumtemperatur werden Sorbinsäure, Aroma und Wirkstoff zugegeben. Zur Entlüftung der Suspension wird unter Rühren evakuiert. Eine Dosis = 50 mg ist enthalten in 5,0 ml.

Beispiel VIII

| Suppositorien, enthaltend 100 mg Wirkstoff | |
|---|---|
| Wirkstoff | 100,0 mg |
| Adeps solidus | 1600,0 mg |
| | 1700,0 mg |

Herstellung:

Das Hartfett wird geschmolzen. Bei 40°C wird die gemahlene Wirksubstanz in der Schmelze homogen dispergiert. Es wird auf 38°C abgekühlt und in schwach vorgekühlte Suppositorienformen ausgegossen.

## Patentansprüche

**1.** Benzimidazole der Formel

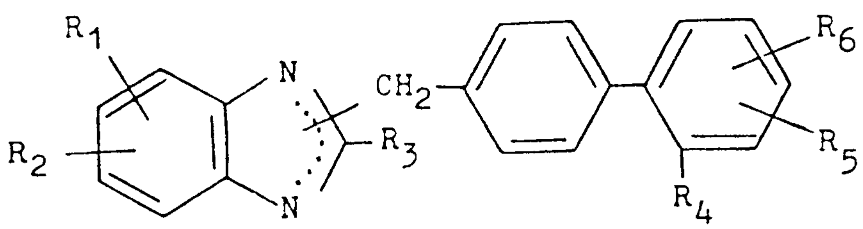

, (I)

in der

$R_1$ ein Wasserstoff-, Fluor-, Chlor- oder Bromatom,

eine Alkylgruppe mit 1 bis 4 Kohlenstoffatomen, die durch eine Hydroxy-, Alkoxy-, Amino-, Alkylamino-, Dialkylamino- oder Acylaminogruppe substituiert sein kann,

eine Alkoxygruppe mit 1 bis 4 Kohlenstoffatomen, die in 2-, 3- oder 4-Stellung durch eine Hydroxy-, Alkoxy-, Amino-, Alkylamino-, Dialkylamino- oder Imidazolylgruppe substituiert sein kann,

eine Hydroxy-, Phenylalkoxy-, Acyloxy-, Trifluormethylsulfonyloxy-, Alkylaminocarbonyloxy-, Dialkylaminocarbonyloxy-, Cycloalkylaminocarbonyloxy-, Cycloalkylalkylaminocarbonyloxy-, Arylaminocarbonyloxy-, Aralkylaminocarbonyloxy-, Alkoxycarbonyloxy-, Cycloalkoxycarbonyloxy-, Cycloalkylalkoxycarbonyloxy-, Aryloxycarbonyloxy-, Aralkoxycarbonyloxy-, Trifluormethyl-, Cyano-, Nitro-, Alkylmercapto-, Alkylsulfinyl-, Alkylsulfonyl-, Aminosulfonyl-, Alkylaminosulfonyl-, Dialkylaminosulfonyl-, Arylaminosulfonyl-, Acylaminosulfonyl- oder Acylgruppe,

eine Aminogruppe, die durch eine Imidazolylalkyl-, Dialkylaminoalkanoyl-, Acyl-, Cycloalkoxycarbonyl-, Cycloalkylalkoxycarbonyl-, Aryloxycarbonyl-, Aralkoxycarbonyl- oder Trifluoracetylgruppe, durch eine bi- oder tricyclische Alkylgruppe mit 7 bis 11 Kohlenstoffatomen, durch eine Alkylsulfonylgruppe mit 1 bis 4 Kohlenstoffatomen, durch eine Alkoxycarbonylgruppe mit insgesamt 2 bis 7 Kohlenstoffatomen oder durch eine durch eine Alkoxycarbonylgruppe substituierte Thiazolidin-3-yl-carbonylgruppe monosubstituiert sein kann,

eine Alkylaminogruppe mit 1 bis 6 Kohlenstoffatomen, die am Stickstoffatom durch eine Alkyl-, Alkylsulfonyl- oder Acylgruppe substituiert sein kann, wobei, falls die Acylgruppe eine Alkanoylgruppe darstellt, diese zusätzlich durch eine Alkoxygruppe substituiert sein kann, und der Alkylsubstituent ab Position 2 durch eine Hydroxy-, Alkoxy- oder Arylaminogruppe substituiert sein kann,

eine durch eine Cycloalkyl-, Cycloalkylalkyl-, Phenylalkyl- oder Phenylgruppe mono- oder disubstituierte Aminogruppe, wobei die Substituenten gleich oder verschieden sein können, eine N-Alkyl-cycloalkylamino-, N-Alkylcycloalkylalkylamino-, N-Alkyl-phenylalkylamino- oder N-Alkyl-phenylaminogruppe,

eine gegebenenfalls durch eine Alkyl-, Cycloalkyl- oder Phenylgruppe substituierte Pyrrolidino-, Piperidino- oder Hexamethyleniminogruppe,

eine N-Alkoxycarbonyl-alkylamino-, N-Cycloalkoxycarbonylalkylamino-, N-Cycloalkylalkoxycarbonyl-alkylamino-,

N-Aryloxycarbonyl-alkylamino- oder N-Aralkoxycarbonyl-alkylaminogruppe, in der die Alkylgruppe jeweils 1 bis 6 Kohlenstoffatome enthalten kann, eine am Stickstoffatom durch eine Cycloalkyl-, Cycloalkylalkyl- oder Aralkylgruppe substituierte Alkoxycarbonylamino-, Cycloalkoxycarbonylamino-, Cycloalkylalkoxycarbonylamino-, Aryloxycarbonylamino-, Aralkoxycarbonylamino-, Acylamino- oder Alkylsulfonylaminogruppe,

eine Carbonylgruppe, die durch eine Alkylgruppe, die in 2- oder 3-Stellung durch eine Hydroxy-, Alkanoyloxy- oder Alkylaminogruppe substituiert ist, durch eine Hydroxycarbonylalkyl-, Hydroxy-, Alkoxy-, Amino-, Cycloalkylamino-, Cycloalkylalkylamino-, Arylamino- oder Aralkylaminogruppe, durch eine in 2- oder 3-Stellung durch eine Arylaminogruppe substituierte Alkylaminogruppe oder durch eine gegebenenfalls im Alkylteil durch eine Carboxygruppe substituierte Alkylaminogruppe mit 1 bis 5 Kohlenstoffatomen, die jeweils zusätzlich am Stickstoffatom durch eine Alkylgruppe substituiert sein kann, substituiert ist,

eine gegebenenfalls durch eine Alkoxycarbonylgruppe mit insgesamt 2 bis 5 Kohlenstoffatomen substituierte Aminoacetylaminogruppe,

eine Aminocarbonylamino- oder Aminothiocarbonylaminogruppe, welche durch eine Alkylgruppe mit 1 bis 20 Kohlenstoffatomen, durch eine Alkenyl- oder Alkinylgruppe mit jeweils 3 bis 5 Kohlenstoffatomen, durch eine bi- oder tricyclische Alkylgruppe mit 7 bis 11 Kohlenstoffatomen, durch eine Cycloalkyl-, Cycloalkylalkyl-, Aralkyl- oder Arylgruppe mono-, di- oder trisubstituiert sein kann, wobei die Substituenten gleich oder verschieden sein können sowie eine Methylengruppe in einem Cycloalkylrest mit 5 bis 7 Kohlenstoffatomen durch ein Sauerstoffatom ersetzt und eine Alkylgruppe in 4-, 5- oder 6-Stellung durch eine Hydroxy-, Alkanoyl- oder Trifluoracetylgruppe substituiert sein kann,

eine Cycloalkyleniminocarbonylaminogruppe mit 4 bis 6 Kohlenstoffatomen im Cycloalkyleniminoteil oder eine Morpholinocarbonylaminogruppe, die beide am Aminostickstoff durch eine Alkylgruppe mit 1 bis 20 Kohlenstoffatomen, durch eine Cycloalkyl-, Cycloalkylalkyl-, Aralkyl- oder Arylgruppe substituiert sein können,

eine Phthalimino-, Homophthalimino-, 2-Carboxyphenylcarbonylamino-, 2-Carboxyphenylmethylamino-, 2-Carboxyphenylmethylencarbonylamino- oder 2-Carboxymethylenphenylcarbonylaminogruppe, wobei eine Carbonylgruppe in einer Phthaliminogruppe durch eine Methylengruppe ersetzt sowie eine Methylengruppe in einer Homophthalimino-, 2-Carboxyphenylmethylencarbonylamino- oder 2-Carboxymethylenphenylcarbonylaminogruppe durch eine oder zwei Alkylgruppen substituiert sein kann, und zusätzlich die vorstehend erwähnten Phenylkerne durch Alkyl- oder Alkoxygruppen mono- oder disubstituiert, wobei die Substituenten gleich oder verschieden sein können, und gleichzeitig ganz oder teilweise hydriert sein können, eine in 2-Stellung durch eine Carboxygruppe substituierte Bicycloalkan-3-carbonsäureamino- oder Bicycloalken-3-carbonsäureaminogruppe, eine Bicycloalkan-2,3-dicarbonsäureimino- oder Bicycloalken-2,3-dicarbonsäureiminogruppe, in denen der Bicycloalkan- und Bicycloalkenteil jeweils 9 oder 10 Kohlenstoffatome enthalten, durch 1, 2 oder 3 Methylgruppen substituiert und eine Endomethylengruppe durch ein Sauerstoffatom ersetzt sein kann, eine Glutarsäureimino- oder 3-Carboxy-n-propylencarbonylgruppe, in denen jeweils die n-Propylengruppe perfluoriert, durch ein oder zwei Alkylgruppen oder durch eine Tetramethylen- oder Pentamethylengruppe substituiert sein kann, oder

eine 5,7-Dioxa-1H,3H-imidazo[1,5-c]thiazolylgruppe,

$R_2$ die für $R_1$ vorstehend erwähnten Bedeutungen, eine gegebenenfalls in 3-Stellung durch eine Alkyl-, Cycloalkyl-, Cycloalkylalkyl- oder Aralkylgruppe substituierte 2-Imidazolidon-1-yl- oder 3,4,5,6-Tetrahydro-2-pyrimidon-1-ylgruppe oder eine Tetrazolylgruppe oder

$R_1$ und $R_2$ zusammen mit 2 Kohlenstoffatomen des benachbarten Phenylringes eine Phenyl- oder 1-Alkyl-3,3-dialkyl-2,3-dihydro-pyrrol-2-on-gruppe,

$R_3$ ein Wasserstoff-, Fluor-, Chlor- oder Bromatom,

eine Alkylgruppe mit 1 bis 6 Kohlenstoffatomen, in welcher eine Methylengruppe durch ein Sauerstoff- oder Schwefelatom, eine Sulfinyl-, Sulfonyl- oder Alkylaminogruppe ersetzt und eine Methylgruppe durch eine Hydroxy-, Alkoxy-, Amino-, Alkylamino- oder Dialkylaminogruppe substituiert sein kann, wobei jedoch die zum Benzimidazolring benachbarte Methylengruppe durch keine Sulfinyl- oder Sulfonylgruppe ersetzt sein kann und bei gleichzeitigem Ersatz einer Methylengruppe und Substitution einer Methylgruppe diese durch mindestens 2 Kohlenstoffatome voneinander getrennt sein müssen,

eine Alkenyl- oder Alkinylgruppe mit jeweils 3 bis 5 Kohlenstoffatomen,

eine Phenylalkylgruppe,

eine Cycloalkyl- oder Cycloalkyl-alkylgruppe, in welcher der Cycloalkylteil jeweils 5 bis 7 Kohlenstoffatome enthalten kann,

eine Aryl-, Hydroxy- oder Imidazolylalkylaminogruppe,

eine Alkylaminogruppe mit 1 bis 4 Kohlenstoffatomen,

eine Aminocarbonylgruppe, welche durch eine Alkyl- oder Cycloalkylgruppe mit 5 bis 7 Kohlenstoffatomen substituiert sein kann,

einen 5-gliedrigen heteroaromatischen Ring, welcher eine NH-Gruppe, ein Sauerstoff- oder Schwefelatom enthält, wobei dieser vorstehend erwähnte 5-gliedrige heteroaromatische Ring zusätzlich noch 1 bis 3 N-Atome enthalten kann, oder einen 6-gliedrigen heteroaromatischen Ring, welcher 1 oder 2 Stickstoffatome enthalten kann, wobei

die vorstehend erwähnten 5- und 6-gliedrigen heteroaromatischen Ringe durch eine oder mit Ausnahme der Tetrazolylgruppe durch zwei Alkylgruppen substituiert sein können,
$R_4$ eine Amino-, Phthalimino-, Aminomethyl-, Cyano-, tert.Butoxycarbonyl- oder 1-(Triphenylmethyl)-tetrazolylgruppe, wobei diese Verbindungen Zwischenprodukte darstellen,
eine eine Brönsted-Säure enthaltende Gruppe oder einen Rest, der in vivo in eine eine Brönsted-Säure enthaltende Gruppe übergeführt werden kann, wobei diese Verbindungen Angiotensin-II-Antagonisten darstellen
$R_5$ und $R_6$ jeweils ein Wasserstoffatom oder
$R_5$ und $R_6$ zusammen mit den beiden ortho-ständigen Kohlenstoffatomen eine Phenylgruppe bedeuten, wobei, sofern nichts anderes erwähnt wurde,
unter der bei der Definition der Reste $R_1$ bis $R_6$ erwähnten Acylgruppe eine Alkanoylgruppe mit 1 bis 7 Kohlenstoffatomen, eine Cycloalkylcarbonylgruppe mit insgesamt 4 bis 8 Kohlenstoffatomen, eine Cycloalkylalkanoylgruppe mit insgesamt 5 bis 10 Kohlenstoffatomen oder eine gegebenenfalls durch ein Fluor-, Chlor- oder Bromatom, eine Alkyl- oder Alkoxygruppe substituierte Arylcarbonyl-, Aralkanoyl- oder Phenylsulfonylgruppe,
unter der Arylgruppe eine gegebenenfalls durch ein Fluor-, Chlor- oder Bromatom, durch eine Hydroxy-, Alkyl-, Alkoxy-, Phenylalkoxy- oder Trifluormethylgruppe substituierte Phenylgruppe, wobei der Alkylteil jeweils 1 bis 4 Kohlenstoffatome enthalten kann, oder eine Naphthylgruppe,
unter der Cycloalkylgruppe eine Cycloalkylgruppe mit 3 bis 7 Kohlenstoffatomen, welche durch eine oder zwei Alkylgruppen substituiert sein kann, und
unter den bei der Definition der Reste $R_1$ bis $R_6$ erwähnten übrigen Alkyl- und Alkanoylgruppen jeweils Alkyl- oder Alkanoylgruppen mit 1 bis 3 Kohlenstoffatomen zu verstehen sind, deren 1-, 3-Isomerengemische und deren Salze mit anorganischen oder organischen Säuren oder Basen.

2. Benzimidazole der Formel I gemäß Anspruch 1, in der $R_1$ bis $R_3$, $R_5$ und $R_6$ wie im Anspruch 1 definiert sind und $R_4$ eine Carboxy-, Aminoacetylamino-, Trifluormethylcarbonylamino-, Trifluormethylcarbonylaminomethyl-, Trifluormethylsulfonylamino-, Trifluormethylsulfonylaminomethyl- oder 1H-Tetrazolylgruppe, eine Alkylcarbonylamino-, Alkylcarbonylaminomethyl-, Arylcarbonylamino-, Arylcarbonylaminomethyl-, Aralkylcarbonylamino-, Aralkylcarbonylaminomethyl-, Alkylsulfonylamino-, Alkylsulfonylaminomethyl-, Arylsulfonylamino-, Arylsulfonylaminomethyl-, Aralkylsulfonylamino-, Aralkylsulfonylaminomethyl-, Arylsulfonylaminocarbonyl- oder Benzylsulfonylaminocarbonylgruppe, eine Alkylsulfonylaminocarbonyl- oder Perfluoralkylsulfonylaminocarbonylgruppe mit jeweils 1 bis 6 Kohlenstoffatomen im Alkylteil, eine Alkoxycarbonylgruppe mit insgesamt 2 bis 7 Kohlenstoffatomen, eine Aralkoxycarbonylgruppe, eine Pivaloyloxymethoxycarbonyl-, Phthalidylmethoxycarbonyl-, Ethoxycarbonyloxyethoxycarbonyl-, Methoxymethoxycarbonyl-, Cyclohexyloxycarbonylmethoxycarbonyl- oder (1,3-Dioxa-2-oxo-4-methyl-cyclopenten-5-yl)-methoxycarbonylgruppe darstellt, deren 1-, 3-Isomerengemische und deren Salze mit anorganischen oder organischen Säuren oder Basen.

3. Benzimidazole der Formel I gemäß Anspruch 1, in der
$R_1$ ein Wasserstoff-, Fluor- oder Chloratom, eine Trifluormethyl-, Hydroxy-, Benzyloxy-, Carboxy-, Cyano-, Amino-, Nitro-, Aminosulfonyl-, Methylaminosulfonyl-, Dimethylaminosulfonyl-, Trifluormethylsulfonyloxy- oder Tetrazolylgruppe,
eine Alkylgruppe mit 1 bis 4 Kohlenstoffatomen, wobei die Methylgruppe zusätzlich durch eine Hydroxy- oder Alkylaminogruppe mit 1 bis 4 Kohlenstoffatomen substituiert sein kann,
eine Alkoxygruppe mit 1 bis 4 Kohlenstoffatomen, die in 2-, 3- oder 4-Stellung durch eine Hydroxy-, Alkoxy-, Alkylamino-, Dialkylamino- oder Imidazolylgruppe substituiert sein und der Alkylsubstituent jeweils 1 bis 3 Kohlenstoffatome enthalten kann,
eine Alkanoyloxygruppe mit 1 bis 4 Kohlenstoffatomen oder eine Cycloalkylaminocarbonyloxygruppe mit 5 bis 7 Kohlenstoffatomen im Cycloalkylteil,
eine Aminogruppe, die durch eine Benzyl-, Decalin-, Trifluormethylcarbonyl-, Benzylcarbonyl-, Benzyloxycarbonyl-, 2-Ethyl-5-methylcyclohexyloxy- oder tert.Butoxycarbonylaminoacetylgruppe, durch eine Alkylgruppe mit 1 bis 5 Kohlenstoffatomen, durch eine Cycloalkyl-, Cycloalkylalkyl-, Cycloalkoxycarbonyl-, Cycloalkylcarbonyl- oder Cycloalkylalkanoylgruppe, wobei der Cycloalkylteil jeweils 5 bis 7 Kohlenstoffatome, der Alkylteil 1 bis 3 Kohlenstoffatome und der Alkanoylteil 1 bis 3 Kohlenstoffatome enthalten kann, durch eine Alkanoylgruppe mit 1 bis 6 Kohlenstoffatomen, durch eine Aminoalkanoyl- oder Dialkylaminoalkanoylgruppe, in welcher jeweils der Alkylteil und der Alkanoylteil 1 bis 3 Kohlenstoffatome enthalten kann, durch eine Alkoxycarbonylgruppe mit insgesamt 2 bis 7 Kohlenstoffatomen, durch eine Alkylsulfonylgruppe mit 1 bis 4 Kohlenstoffatomen oder durch eine durch eine Alkoxycarbonylgruppe mit insgesamt 2 bis 4 Kohlenstoffatomen substituierte Thiazolidin-3-yl-carbonylgruppe substituiert ist,
eine Benzylamino- oder Alkylaminogruppe mit 1 bis 5 Kohlenstoffatomen, die am Stickstoffatom zusätzlich durch eine Benzyl- oder Cyclohexylgruppe, durch eine Alkylgruppe mit 1 bis 3 Kohlenstoffatomen, die in 2- oder 3-Stellung durch eine Alkoxygruppe mit 1 bis 3 Kohlenstoffatomen oder durch eine Phenylaminogruppe substituiert sein kann,

durch eine Alkanoylgruppe mit 1 bis 5 Kohlenstoffatomen, welche durch eine Alkoxygruppe mit 1 bis 3 Kohlenstoffatomen substituiert sein kann, durch eine Cycloalkylcarbonylgruppe mit insgesamt 6 bis 8 Kohlenstoffatomen oder durch eine Alkoxycarbonylgruppe mit insgesamt 2 bis 4 Kohlenstoffatomen substituiert ist,

eine Carbonylgruppe, die durch eine Alkylgruppe, die in 2- oder 3-Stellung durch eine Hydroxy-, Alkoxycarbonyl- oder Alkylaminogruppe substituiert sein kann, durch eine Alkylaminogruppe mit 1 bis 5 Kohlenstoffatomen, die im Alkylteil durch eine Carboxygruppe substituiert sein kann, durch eine Alkylaminogruppe, die in 2- oder 3-Stellung durch eine Phenylaminogruppe substituiert ist, durch eine Phenyl-, Alkoxy-, Amino-, Benzylamino-, Phenylethylamino-, Cycloalkylamino- oder Cycloalkylalkylamino-, in welchen jeweils der Alkylteil 1 bis 3 Kohlenstoffatome und der Cycloalkylteil 5 bis 7 Kohlenstoffatome enthalten kann sowie zusätzlich eine Alkylaminogruppe mit 1 bis 5 Kohlenstoffatomen am Stickstoffatom durch eine Alkylgruppe mit 1 bis 4 Kohlenstoffatomen substituiert sein kann, substituiert ist,

eine Aminocarbonylamino- oder Aminothiocarbonylaminogruppe, welche durch eine Alkylgruppe mit 1 bis 12 Kohlenstoffatomen, durch eine Alkenyl- oder Alkinylgruppe mit jeweils 3 bis 5 Kohlenstoffatomen, durch eine bi- oder tricyclische Alkylgruppe mit 7 bis 11 Kohlenstoffatomen, durch eine Cycloalkyl-, Cycloalkylalkyl- oder Phenylalkylgruppe mono-, di- oder trisubstituiert sein kann, wobei die Substituenten gleich oder verschieden sein können sowie jeweils der Alkylteil 1 bis 3 Kohlenstoffatome und der Cycloalkylteil 5 bis 7 Kohlenstoffatome enthalten kann sowie eine Methylengruppe in einem Cycloalkylrest mit 5 bis 7 Kohlenstoffatomen durch ein Sauerstoffatom ersetzt und eine Alkylgruppe in 4-, 5- oder 6-Stellung durch eine Hydroxy- oder Trifluoracetylgruppe substituiert sein kann,

eine Cycloalkyleniminocarbonylaminogruppe mit 4 bis 6 Kohlenstoffatomen im Cycloalkyleniminoteil oder eine Morpholinocarbonylaminogruppe, die beide am Aminostickstoff durch eine Alkylgruppe mit 1 bis 12 Kohlenstoffatomen, durch eine Cycloalkyl-, Cycloalkylalkyl- oder Phenylalkylgruppe substituiert sein können, in denen jeweils der Alkylteil 1 bis 3 Kohlenstoffatome und der Cycloalkylteil 5 bis 7 Kohlenstoffatme enthalten kann,

eine Phthalimino-, Homophthalimino-, 2-Carboxyphenylcarbonylamino-, 2-Carboxyphenylmethylamino-, 2-Carboxyphenylmethylencarbonylamino- oder 2-Carboxymethylenphenylcarbonylaminogruppe, wobei eine Carbonylgruppe in einer Phthaliminogruppe durch eine Methylengruppe ersetzt sowie eine Methylengruppe in einer Homophthalimino-, 2-Carboxyphenylmethylencarbonylamino- oder 2-Carboxymethylenphenylcarbonylaminogruppe durch eine oder zwei Methylgruppen substituiert sein kann, und zusätzlich die vorstehend erwähnten Phenylkerne durch Methyl- oder Methoxygruppen mono- oder disubstituiert, wobei die Substituenten gleich oder verschieden sein können, und gleichzeitig ganz oder teilweise hydriert sein können, eine in 2-Stellung durch eine Carboxygruppe substituierte Bicycloalkan-3-carbonsäureamino- oder Bicycloalken-3-carbonsäureaminogruppe, eine Bicycloalkan-2,3-dicarbonsäureimino- oder Bicycloalken-2,3-dicarbonsäureiminogruppe, in denen der Bicycloalkan- und Bicycloalkenteil jeweils 9 oder 10 Kohlenstoffatome enthalten, durch 1, 2 oder 3 Methylgruppen substituiert und eine Endomethylengruppe durch ein Sauerstoffatom ersetzt sein kann, eine Glutarsäureimino- oder 3-Carboxy-n-propylencarbonylgruppe, in denen jeweils die n-Propylengruppe perfluoriert, durch ein oder zwei Alkylgruppen oder durch eine Tetramethylen- oder Pentamethylengruppe substituiert sein kann, oder

eine 5,7-Dioxa-1H,3H-imidazo[1,5-c]thiazolylgruppe, eine gegebenfalls in 3-Stellung durch eine Alkylgruppe mit 1 bis 3 Kohlenstoffatomen substituierte 2-Imidazolidon-1-yl-gruppe oder eine 3,4,5,6-Tetrahydro-2-pyrimidon-1-ylgruppe,

$R_2$ ein Wasserstoff-, Fluor- oder Chloratom, eine Methyl-, Hydroxy- oder Methoxygruppe, oder

$R_1$ und $R_2$ zusammen mit den beiden ortho-ständigen Kohlenstoffatomen des benachbarten Phenylringes eine Phenyl- oder 1-Alkyl-3,3-dialkyl-2,3-dihydro-pyrrol-2-on-gruppe, in welcher der Alkylteil jeweils 1 bis 3 Kohlenstoffatme enthalten kann,

$R_3$ ein Wasserstoff-, Fluor- oder Chloratom,

eine Hydroxy-, Benzyl- oder Aminocarbonylgruppe,

eine Alkylgruppe mit 1 bis 5 Kohlenstoffatomen, wobei die Methylgruppe zusätzlich durch eine Hydroxygruppe, durch eine Alkoxy-, Alkylsulfenyl-, Alkylsulfinyl- oder Alkylsulfonylgruppe mit jeweils 1 bis 3 Kohlenstoffatomen substituiert sein kann, eine Cycloalkyl- oder Cycloalkylalkylgruppe, in welcher der Cycloalkylteil jeweils 5 bis 7 Kohlenstoffatome und der Alkylteil 1 bis 3 Kohlenstoffatome enthalten kann, eine Alkenyl- oder Alkinylgruppe mit jeweils 3 bis 5 Kohlenstoffatomen, eine Phenylalkylgruppe mit 1 bis 3 Kohlenstoffatomen,

eine Alkylaminogruppe mit 1 bis 4 Kohlenstoffatomen,

eine Pyridyl-, Furyl-, Thiazolyl- oder Pyrazolylgruppe, welche durch eine oder zwei Methylgruppen substituiert sein können,

$R_4$ eine Alkoxycarbonyl mit insgesamt 1 bis 5 Kohlenstoffatomen, eine Amino-, Phthalimino-, Aminomethyl-, Carboxy-, Cyano-, Methylsulfonylaminocarbonyl-, Trifluormethylsulfonylaminocarbonyl-, Benzolsulfonylaminocarbonyl-, Trifluormethylcarbonylaminomethyl-, Trifluormethylaminomethyl-, Tetrazolyl- oder 1-(Triphenylmethyl)-tetrazolylgruppe,

$R_5$ und $R_6$ jeweils ein Wasserstoffatom oder

$R_5$ und $R_6$ zusammen mit den beiden ortho-ständigen Kohlenstoffatomen eine Phenylgruppe bedeuten, deren 1-, 3-Isomerengemische und deren Salze mit anorganischen oder organischen Säuren oder Basen.

4. Benzimidazole der Formel I gemäß Anspruch 1, in der
$R_1$ eine Aminogruppe, die durch eine Benzyl-, Decalin-, Trifluormethylcarbonyl-, Benzylcarbonyl-, Benzyloxycarbonyl-, 2-Ethyl-5-methylcyclohexyloxy- oder tert.Butoxycarbonylaminoacetylgruppe, durch eine Alkylgruppe mit 1 bis 5 Kohlenstoffatomen, durch eine Cycloalkyl-, Cycloalkylalkyl-, Cycloalkoxycarbonyl-, Cycloalkylcarbonyl- oder Cycloalkylalkanoylgruppe, wobei der Cycloalkylteil jeweils 5 bis 7 Kohlenstoffatome, der Alkylteil 1 bis 3 Kohlenstoffatome und der Alkanoylteil 1 bis 3 Kohlenstoffatome enthalten kann, durch eine Alkanoylgruppe mit 1 bis 6 Kohlenstoffatomen, durch eine Aminoalkanoyl- oder Dialkylaminoalkanoylgruppe, in welcher jeweils der Alkylteil und der Alkanoylteil 1 bis 3 Kohlenstoffatome enthalten kann, durch eine Alkoxycarbonylgruppe mit insgesamt 2 bis 7 Kohlenstoffatomen, durch eine Alkylsulfonylgruppe mit 1 bis 4 Kohlenstoffatomen oder durch eine durch eine Alkoxycarbonylgruppe mit insgesamt 2 bis 4 Kohlenstoffatomen substituierte Thiazolidin-3-yl-carbonylgruppe substituiert ist,
eine Benzylamino- oder Alkylaminogruppe mit 1 bis 5 Kohlenstoffatomen, die am Stickstoffatom zusätzlich durch eine Benzyl- oder Cyclohexylgruppe, durch eine Alkylgruppe mit 1 bis 3 Kohlenstoffatomen, die in 2- oder 3-Stellung durch eine Alkoxygruppe mit 1 bis 3 Kohlenstoffatomen oder durch eine Phenylaminogruppe substituiert sein kann, durch eine Alkanoylgruppe mit 1 bis 5 Kohlenstoffatomen, welche durch eine Alkoxygruppe mit 1 bis 3 Kohlenstoffatomen substituiert sein kann, durch eine Cycloalkylcarbonylgruppe mit insgesamt 6 bis 8 Kohlenstoffatomen oder durch eine Alkoxycarbonylgruppe mit insgesamt 2 bis 4 Kohlenstoffatomen substituiert ist,
eine Aminocarbonylamino- oder Aminothiocarbonylaminogruppe, welche durch eine Alkylgruppe mit 1 bis 12 Kohlenstoffatomen, durch eine Alkenyl- oder Alkinylgruppe mit jeweils 3 bis 5 Kohlenstoffatomen, durch eine bi- oder tricyclische Alkylgruppe mit 7 bis 11 Kohlenstoffatomen, durch eine Cycloalkyl-, Cycloalkylalkyl- oder Phenylalkylgruppe mono-, di- oder trisubstituiert sein kann, wobei die Substituenten gleich oder verschieden sein können sowie jeweils der Alkylteil 1 bis 3 Kohlenstoffatome und der Cycloalkylteil 5 bis 7 Kohlenstoffatome enthalten kann sowie eine Methylengruppe in einem Cycloalkylrest mit 5 bis 7 Kohlenstoffatomen durch ein Sauerstoffatom ersetzt und eine Alkylgruppe in 4-, 5- oder 6-Stellung durch eine Hydroxy- oder Trifluoracetylgruppe substituiert sein kann,
eine Cycloalkyleniminocarbonylaminogruppe mit 4 bis 6 Kohlenstoffatomen im Cycloalkyleniminoteil oder eine Morpholinocarbonylaminogruppe, die beide am Aminostickstoff durch eine Alkylgruppe mit 1 bis 12 Kohlenstoffatomen, durch eine Cycloalkyl-, Cycloalkylalkyl- oder Phenylalkylgruppe substituiert sein können, in denen jeweils der Alkylteil 1 bis 3 Kohlenstoffatome und der Cycloalkylteil 5 bis 7 Kohlenstoffatme enthalten kann,
eine Phthalimino-, Homophthalimino-, 2-Carboxyphenylcarbonylamino-, 2-Carboxyphenylmethylamino-, 2-Carboxyphenylmethylencarbonylamino- oder 2-Carboxymethylenphenylcarbonylaminogruppe, wobei eine Carbonylgruppe in einer Phthaliminogruppe durch eine Methylengruppe ersetzt sowie eine Methylengruppe in einer Homophthalimino-, 2-Carboxyphenylmethylencarbonylamino- oder 2-Carboxymethylenphenylcarbonylaminogruppe durch eine oder zwei Methylgruppen substituiert sein kann, und zusätzlich die vorstehend erwähnten Phenylkerne durch Methyl- oder Methoxygruppen mono- oder disubstituiert, wobei die Substituenten gleich oder verschieden sein können, und gleichzeitig ganz oder teilweise hydriert sein können, eine in 2-Stellung durch eine Carboxygruppe substituierte Bicycloalkan-3-carbonsäureamino- oder Bicycloalken-3-carbonsäureaminogruppe, eine Bicycloalkan-2,3-dicarbonsäureimino- oder Bicycloalken-2,3-dicarbonsäureiminogruppe, in denen der Bicycloalkan- und Bicycloalkenteil jeweils 9 oder 10 Kohlenstoffatome enthalten, durch 1, 2 oder 3 Methylgruppen substituiert und eine Endomethylengruppe durch ein Sauerstoffatom ersetzt sein kann, eine Glutarsäureimino- oder 3-Carboxy-n-propylencarbonylgruppe, in denen jeweils die n-Propylengruppe perfluoriert, durch ein oder zwei Alkylgruppen oder durch eine Tetramethylen- oder Pentamethylengruppe substituiert sein kann, oder
eine 5,7-Dioxa-1H,3H-imidazo[1,5-c]thiazolylgruppe,
$R_2$ ein Wasserstoffatom, eine Methyl- oder Hydroxygruppe oder
$R_1$ und $R_2$ zusammen mit den beiden ortho-ständigen Kohlenstoffatomen des benachbarten Phenylringes eine Phenylgruppe,
$R_3$ eine Alkylgruppe mit 3 bis 5 Kohlenstoffatomen, eine Alkenyl- oder Alkinylgruppe mit jeweils 3 bis 5 Kohlenstoffatomen,
$R_4$ eine Carboxy-, Methylsulfonylaminocarbonyl-, Trifluormethylsulfonylaminocarbonyl-, Benzolsulfonylaminocarbonyl-, Trifluormethylcarbonylaminomethyl-, Trifluormethylaminomethyl- oder Tetrazolylgruppe,
$R_5$ und $R_6$ jeweils ein Wasserstoffatom oder
$R_5$ und $R_6$ zusammen mit den beiden ortho-ständigen Kohlenstoffatomen eine Phenylgruppe bedeuten, deren 1-, 3-Isomerengemische und deren Salze mit anorganischen oder organischen Säuren oder Basen.

5. Benzimidazole der Formel I gemäß Anspruch 1, in der
$R_1$ eine Aminogruppe, die durch eine Alkanoylgruppe mit 1 bis 6 Kohlenstoffatomen, durch eine Aminoalkanoyl- oder Dialkylaminoalkanoylgruppe, in welcher der Alkylteil und der Alkanoylteil jeweils 1 bis 3 Kohlenstoffatome enthalten kann, durch eine Alkoxycarbonylgruppe mit insgesamt 2 bis 7 Kohlenstoffatomen oder durch eine durch eine Alkoxycarbonylgruppe mit insgesamt 2 bis 4 Kohlenstoffatomen substituierte Thiazolidin-3-yl-carbonylgruppe substituiert ist,

eine Benzylamino- oder Alkylaminogruppe mit 1 bis 5 Kohlenstoffatomen, die am Stickstoffatom durch eine Alkanoylgruppe mit 1 bis 5 Kohlenstoffatomen, welche durch eine Alkoxygruppe mit 1 bis 3 Kohlenstoffatomen substituiert sein kann, durch eine Cycloalkylcarbonylgruppe mit insgesamt 6 bis 8 Kohlenstoffatomen oder durch eine Alkoxycarbonylgruppe mit insgesamt 2 bis 4 Kohlenstoffatomen substituiert ist,
eine Aminocarbonylamino- oder Aminothiocarbonylaminogruppe, welche durch eine Alkylgruppe mit 1 bis 8 Kohlenstoffatomen, durch eine Alkenyl- oder Alkinylgruppe mit jeweils 3 bis 5 Kohlenstoffatomen, durch eine bi- oder tricyclische Alkylgruppe mit 7 bis 11 Kohlenstoffatomen, durch eine Cycloalkyl-, Cycloalkylalkyl- oder Phenylalkylgruppe mono-, di- oder trisubstituiert sein kann, wobei die Substituenten gleich oder verschieden sein können sowie jeweils der Alkylteil 1 bis 3 Kohlenstoffatome und der Cycloalkylteil 5 bis 7 Kohlenstoffatome enthalten kann sowie eine Methylengruppe in einem Cycloalkylrest mit 5 bis 7 Kohlenstoffatomen durch ein Sauerstoffatom ersetzt und eine Alkylgruppe in 4-, 5- oder 6-Stellung durch eine Hydroxy- oder Trifluoracetylgruppe substituiert sein kann,
eine Cycloalkyleniminocarbonylaminogruppe mit 4 bis 6 Kohlenstoffatomen im Cycloalkyleniminoteil oder eine Morpholinocarbonylaminogruppe, die beide am Aminostickstoff durch eine Alkylgruppe mit 1 bis 8 Kohlenstoffatomen, durch eine Cycloalkyl-, Cycloalkylalkyl- oder Phenylalkylgruppe substituiert sein können, in denen jeweils der Alkylteil 1 bis 3 Kohlenstoffatome und der Cycloalkylteil 5 bis 7 Kohlenstoffatme enthalten kann,
eine Phthalimino-, Homophthalimino-, 2-Carboxyphenylcarbonylamino-, 2-Carboxyphenylmethylamino-, 2-Carboxyphenylmethylencarbonylamino- oder 2-Carboxymethylenphenylcarbonylaminogruppe, wobei eine Carbonylgruppe in einer Phthaliminogruppe durch eine Methylengruppe ersetzt sowie eine Methylengruppe in einer Homophthalimino-, 2-Carboxyphenylmethylencarbonylamino- oder 2-Carboxymethylenphenylcarbonylaminogruppe durch eine oder zwei Methylgruppen substituiert sein kann, und zusätzlich die vorstehend erwähnten Phenylkerne durch Methyl- oder Methoxygruppen mono- oder disubstituiert, wobei die Substituenten gleich oder verschieden sein können, und gleichzeitig ganz oder teilweise hydriert sein können, eine in 2-Stellung durch eine Carboxygruppe substituierte Bicycloalkan-3-carbonsäureamino- oder Bicycloalken-3-carbonsäureaminogruppe, eine Bicycloalkan-2,3-dicarbonsäureimino- oder Bicycloalken-2,3-dicarbonsäureiminogruppe, in denen der Bicycloalkan- und Bicycloalkenteil jeweils 9 oder 10 Kohlenstoffatome enthalten, durch 1, 2 oder 3 Methylgruppen substituiert und eine Endomethylengruppe durch ein Sauerstoffatom ersetzt sein kann, eine Glutarsäureimino- oder 3-Carboxy-n-propylencarbonylgruppe, in denen jeweils die n-Propylengruppe perfluoriert, durch ein oder zwei Alkylgruppen oder durch eine Tetramethylen- oder Pentamethylengruppe substituiert sein kann, oder
eine 5,7-Dioxa-1H,3H-imidazo[1,5-c]thiazolylgruppe,
$R_2$ ein Wasserstoffatom,
$R_1$ und $R_2$ zusammen mit den beiden ortho-ständigen Kohlenstoffatomen des benachbarten Phenylringes eine Phenylgruppe,
$R_3$ eine Alkylgruppe mit 3 bis 5 Kohlenstoffatomen, eine Alkenyl- oder Alkinylgruppe mit jeweils 3 bis 5 Kohlenstoffatomen,
$R_4$ eine Carboxy- oder Tetrazolylgruppe,
$R_5$ und $R_6$ jeweils ein Wasserstoffatom oder
$R_5$ und $R_6$ zusammen mit den beiden ortho-ständigen Kohlenstoffatomen eine Phenylgruppe bedeuten, deren 1-, 3-Isomerengemische und deren Salze mit anorganischen oder organischen Säuren oder Basen.

**6.** Benzimidazole der Formel I gemäß Anspruch 1:
4'-[(2-n-Butyl-6-(N-(n-hexylaminocarbonyl)-benzylamino)-benzimidazol-1-yl)-methyl]biphenyl-2-carbonsäure,
4'-[(2-n-Butyl-6-(N-cyclohexylaminocarbonyl-n-butylamino)-benzimidazol-1-yl)-methyl]biphenyl-2-carbonsäure,
4'-[(2-n-Butyl-6-(N-cyclohexylaminocarbonyl-ethylamino)-benzimidazol-1-yl)-methyl]biphenyl-2-carbonsäure,
4'-[(2-n-Butyl-6-(N-cyclohexylaminocarbonyl-n-propylamino)-benzimidazol-1-yl)-methyl]biphenyl-2-carbonsäure,
4'-[(2-n-Butyl-6-(N-cyclohexylaminocarbonyl-methylamino)-benzimidazol-1-yl)-methyl]biphenyl-2-carbonsäure,
4'-[(2-n-Butyl-6-cyclohexylaminocarbonylamino-benzimidazol-1-yl)-methyl]biphenyl-2-carbonsäure,
4'-[(2-n-Butyl-6-(N-cyclohexylaminocarbonyl-n-pentylamino)-benzimidazol-1-yl)-methyl]biphenyl-2-carbonsäure,
4'-[(2-n-Butyl-6-(N-methylaminocarbonyl-benzylamino)-benzimidazol-1-yl)-methyl]biphenyl-2-carbonsäure,
4'-[(2-n-Butyl-6-(1-oxo-isoindolin-2-yl)-benzimidazol-1-yl)-methyl]biphenyl-2-carbonsäure,
4'-[(2-n-Butyl-6-(N-cyclohexylaminocarbonyl-benzylamino)-benzimidazol-1-yl)-methyl]biphenyl-2-carbonsäure
4'-[(2-n-Butyl-6-n-pentanoylamino-benzimidazol-1-yl)-methyl]biphenyl-2-carbonsäure,
4'-[(2-n-Butyl-6-propionylamino-benzimidazol-1-yl)-methyl]biphenyl-2-carbonsäure,
4'-[(2-n-Butyl-6-isopropylcarbonylamino-benzimidazol-1-yl)-methyl]biphenyl-2-carbonsäure,
4'-[(2-n-Butyl-6-(tetrahydropyran-2-yl-aminocarbonylamino)-benzimidazol-1-yl)-methyl]biphenyl-2-carbonsäure,
4'-[(2-n-Butyl-6-(n-butylaminocarbonylamino)-benzimidazol-1-yl)-methyl]biphenyl-2-carbonsäure,
4'-[(6-n-Butanoylamino-2-n-butyl-benzimidazol-1-yl)-methyl]biphenyl-2-carbonsäure,
4'-[(2-n-Butyl-6-(N-ethoxycarbonyl-benzylamino)-benzimidazol-1-yl)-methyl]biphenyl-2-carbonsäure,
4'-[(2-n-Butyl-6-(N-(dimethylaminocarbonyl)-amino)-benzimidazol-1-yl)-methyl]biphenyl-2-carbonsäure,
4'-[(2-n-Butyl-6-(N-cyclohexylaminocarbonyl-n-hexylamino)-benzimidazol-1-yl)-methyl]biphenyl-2-carbonsäure,

4'-[(2-n-Butyl-6-((5-hydroxy-n-pentyl)-aminocarbonylamino)-benzimidazol-1-yl)-methyl]biphenyl-2-carbonsäure,
4'-[(2-n-Butyl-6-(N-methylaminocarbonyl-n-pentylamino)-benzimidazol-1-yl)-methyl]biphenyl-2-carbonsäure,
4'-[(2-n-Butyl-6-cyclohexylcarbonylamino-benzimidazol-1-yl)-methyl]biphenyl-2-carbonsäure,
4'-[(2-n-Butyl-6-(N-(n-butylaminocarbonyl)-methylamino)-benzimidazol-1-yl)-methyl]biphenyl-2-carbonsäure,
4'-[(2-n-Butyl-7-hydroxy-4-methyl-benzimidazol-1-yl)-methyl]-2-(1H-tetrazol-5-yl)-biphenyl,
4'-[(2-n-Butyl-6-(cis-hexahydrophthalimino)-benzimidazol-1-yl)-methyl]biphenyl-2-carbonsäure,
4'-[(2-n-Butyl-6-(N-(dimethylaminocarbonyl)-benzylamino)-benzimidazol-1-yl)-methyl]-biphenyl-2-carbonsäure,
4'-[(2-n-Butyl-6-(N-(n-hexylaminocarbonyl)-N-(2-phenylethyl)-amino)-benzimidazol-1-yl)-methyl]biphenyl-2-carbonsäure und
4'-[(2-n-Butyl-6-cyclopentylcarbonylamino-benzimidazol-1-yl)-methyl]biphenyl-2-carbonsäure,
deren 1-, 3-Isomerengemische und deren Salze mit anorganischen oder organischen Säuren oder Basen.

7. Physiologisch verträgliche Salze der Verbindungen nach mindestens einem der Ansprüche 1 bis 6 mit anorganischen oder organischen Säuren oder Basen.

8. Arzneimittel, enthaltend eine Verbindung nach mindestens einem der Ansprüche 1 bis 6, in denen $R_4$ eine eine Brönsted-Säure enthaltende Gruppe oder einen Rest, der in vivo in eine eine Brönsted-Säure enthaltende Gruppe übergeführt werden kann, darstellt, oder ein physiologisch verträgliches Salz hiervon neben gegebenenfalls einem oder mehreren inerten Trägerstoffen und/oder Verdünnungsmitteln.

9. Verwendung einer Verbindung nach mindestens einem der Ansprüche 1 bis 6, in denen $R_4$ eine eine Brönsted-Säure enthaltende Gruppe oder einen Rest, der in vivo in eine eine Brönsted-Säure enthaltende Gruppe übergeführt werden kann, darstellt, oder ein physiologisch verträgliches Salz hiervon zur Herstellung eines Arzneimittels, das für die Behandlung der Hypertonie, Herzinsuffizienz, von ischämischen peripheren Durchblutungsstörungen, der myokardialen Ischämie (Angina), zur Prävention der Herzinsuffizienzprogression nach Myokard-Infarkt, zur Behandlung der diabetischen Nephropathie, des Glaukoms, von gastrointestinalen Erkrankungen und Blasenerkrankungen geeignet ist.

10. Verfahren zur Herstellung eines Arzneimittels gemäß Anspruch 8, dadurch gekennzeichnet, daß auf nichtchemischem Wege eine Verbindung nach mindestens einem der Ansprüche 1 bis 6, in denen $R_4$ eine eine Brönsted-Säure enthaltende Gruppe oder einen Rest, der in vivo in eine eine Brönsted-Säure enthaltende Gruppe übergeführt werden kann, darstellt, oder ein physiologisch verträgliches Salz hiervon in einen oder mehrere inerte Trägerstoffe und/oder Verdünnungsmittel eingearbeitet wird.

11. Verfahren zur Herstellung der Benzimidazole gemäß den Ansprüchen 1 bis 7, dadurch gekennzeichnet, daß

a) eine Verbindung der Formel

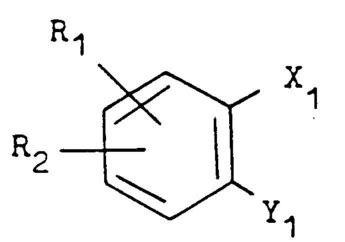

,(II)

in der
$R_1$ und $R_2$ wie mindestens in einem der Ansprüche 1 bis 6 definiert sind,

einer der Reste $X_1$ oder $Y_1$ eine Gruppe der Formel

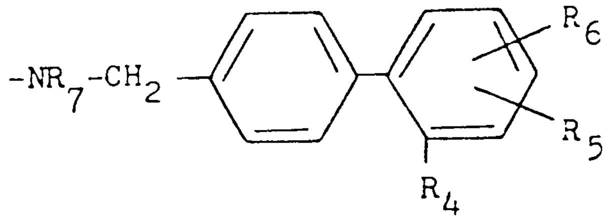

und der andere der Reste $X_1$ oder $Y_1$ eine Gruppe der Formel

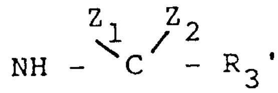

darstellen, wobei

$R_3$' mit Ausnahme des Fluor-, Chlor- oder Bromatoms, der Hydroxy-, Mercapto- oder Aminocarbonylgruppe, wobei die letztere durch eine Alkylgruppe mit 1 bis 3 Kohlenstoffatomen oder durch eine Cycloalkylgruppe mit 5 bis 7 Kohlenstoffatomen substituiert sein kann, die für $R_3$ mindestens in einem der Ansprüche 1 bis 6 erwähnten Bedeutungen besitzt,

$R_4$ bis $R_6$ wie mindestens in einem der Ansprüche 1 bis 6 definiert sind,

$R_7$ ein Wasserstoffatom, eine Hydroxygruppe oder eine $R_3$'CO-Gruppe, wobei $R_3$' wie vorstehend erwähnt definiert ist,

$Z_1$ und $Z_2$, die gleich oder verschieden sein können, gegebenenfalls substituierte Aminogruppen oder gegebenenfalls durch niedere Alkylgruppen substituierte Hydroxy- oder Mercaptogruppen oder

$Z_1$ und $Z_2$, zusammen ein Sauerstoff- oder Schwefelatom, eine gegebenenfalls durch eine Alkylgruppe mit 1 bis 3 Kohlenstoffatomen substituierte Iminogruppe, eine Alkylendioxy- oder Alkylendithiogruppe mit jeweils 2 oder 3 Kohlenstoffatomen bedeuten, wobei jedoch einer der Reste $X_1$ oder $Y_1$ eine Gruppe der Formel

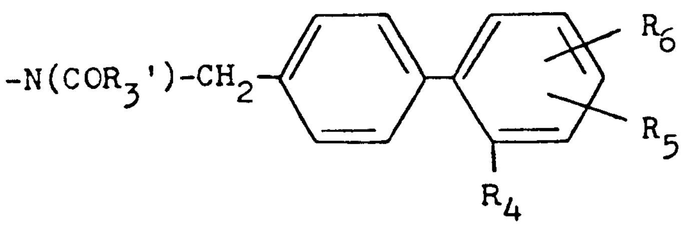

oder

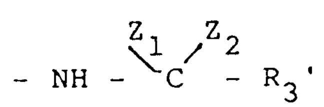

darstellen muß, cyclisiert wird oder

b) zur Herstellung der Verbindungen der Formel I, in der $R_3$ die für $R_3$ mindestens in einem der Ansprüche 1 bis 6 erwähnten aromatischen oder heteroaromatischen Reste darstellt, ein Phenylendiamin der Formel

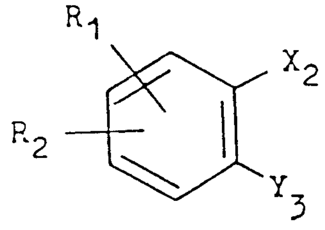

,(III)

in der
$R_1$ und $R_2$ wie mindestens in einem der Ansprüche 1 bis 6 definiert sind,
einer der Reste $X_2$ oder $Y_2$ eine Gruppe der Formel

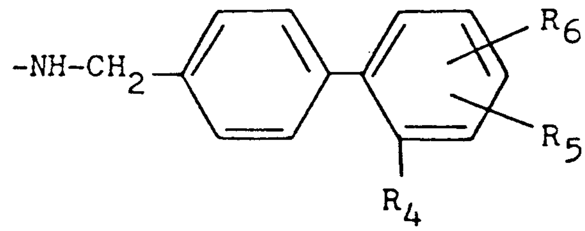

in der
$R_4$ bis $R_6$ wie mindestens in einem der Ansprüche 1 bis 6 definiert sind,
und der andere der Reste $X_2$ oder $Y_2$ eine Aminogruppe, bedeuten, mit einem Aldehyd der Formel

$$OCH - R_3'' ,(IV)$$

in der
$R_3''$ die für $R_3$ mindestens in einem der Ansprüche 1 bis 6 erwähnten aromatischen oder heteroaromatischen Reste bedeutet, in Gegenwart eines Oxidationsmittels umgesetzt wird oder
c) zur Herstellung von Verbindungen der Formel I, in der min
destens einer der Reste $R_1$, $R_2$ und/oder $R_3$ einen der für $R_1$, $R_2$ und/oder $R_3$ mindestens in einem der Ansprüche 1 bis 6 erwähnten eine Sulfinyl- oder Sulfonylgruppe enthaltenden Reste darstellt und die übrigen der Reste $R_1$, $R_2$ und/oder $R_3$ wie mindestens in einem der Ansprüche 1 bis 6 definiert sind, eine Verbindung der Formel

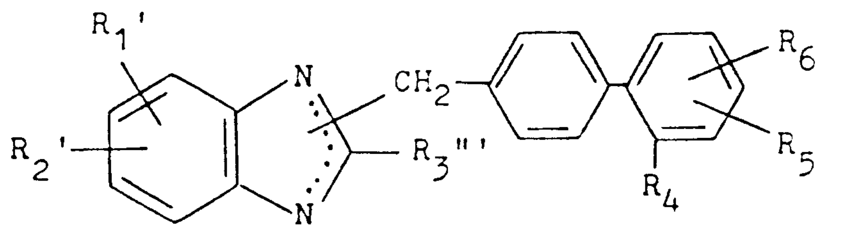

,(V)

in der
$R_4$ bis $R_6$ wie mindestens in einem der Ansprüche 1 bis 6 definiert sind,
mindestens einer der Reste $R_1'$, $R_2'$ und/oder $R_3'''$ einen der für $R_1$, $R_2$ und/oder $R_3$ mindestens in einem der Ansprüche 1 bis 6 erwähnten ein Schwefelatom oder eine Sulfinylgruppe enthaltenden Reste darstellt und die übrigen der Reste $R_1'$, $R_2'$ und/oder $R_3'''$ die für $R_1$, $R_2$ und/oder $R_3$ mindestens in einem der Ansprüche 1 bis 6 erwähnten Bedeutungen besitzen, oxydiert wird oder

d) zur Herstellung einer Verbindung der Formel I, in der $R_4$ eine Carboxy- oder Aminogruppe darstellt, eine Verbindung der Formel

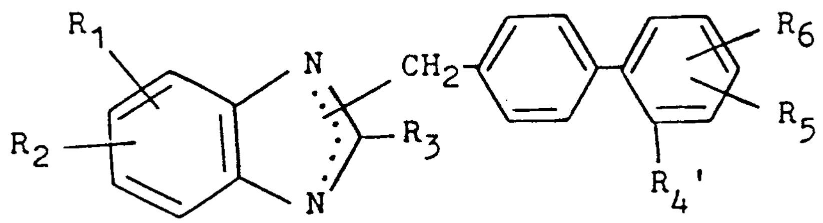

, (VI)

in der
$R_1$ bis $R_3$, $R_5$ und $R_6$ wie mindestens in einem der Ansprüche 1 bis 6 definiert sind und
$R_4$' eine in eine Carboxy- oder Aminogruppe überführbare Gruppe darstellt, in eine entsprechende Carboxy- oder Aminoverbindung übergeführt wird oder
e) ein Benzimidazol der Formel

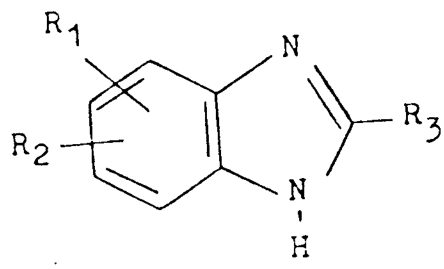

, (VII)

in der
$R_1$ bis $R_3$ wie mindestens in einem der Ansprüche 1 bis 6 definiert sind, mit einer Biphenylverbindung der Formel

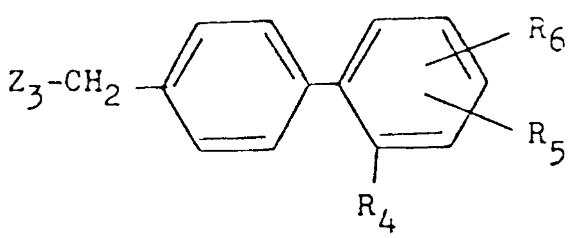

, (VIII)

in der
$R_4$ bis $R_6$ wie mindestens in einem der Ansprüche 1 bis 6 definiert sind und
$Z_3$ eine nukleophile Austrittsgruppe wie ein Halogenatom, z.B. ein Chlor-, Brom- oder Jodatom, oder eine substituierte Sulfonyloxygruppe, z.B. eine Methansulfonyloxy-, Phenylsulfonyloxy- oder p-Toluolsulfonyloxygruppe, darstellt, umgesetzt wird oder
f) zur Herstellung einer Verbindung der Formel I, in der $R_4$ eine 1H-Tetrazolylgruppe darstellt, ein Schutzrest von einer Verbindung der Formel

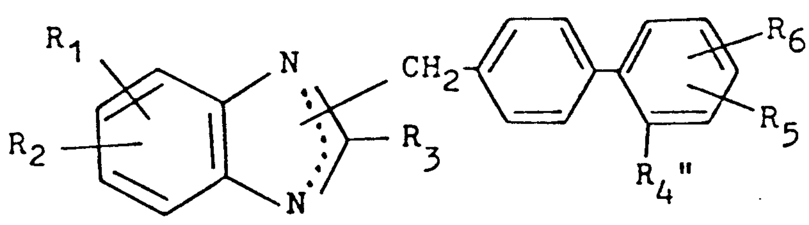

, (IX)

in der
$R_1$ bis $R_3$, $R_5$ und $R_6$ wie mindestens in einem der Ansprüche 1 bis 6 definiert sind und
$R_4$" eine in 1-Stellung durch einen Schutzrest geschützte 1H-Tetrazolylgruppe darstellt, abgespalten wird oder
g) zur Herstellung einer Verbindung der Formel I, in der $R_4$ eine 1H-Tetrazolylgruppe darstellt, eine Verbindung der Formel

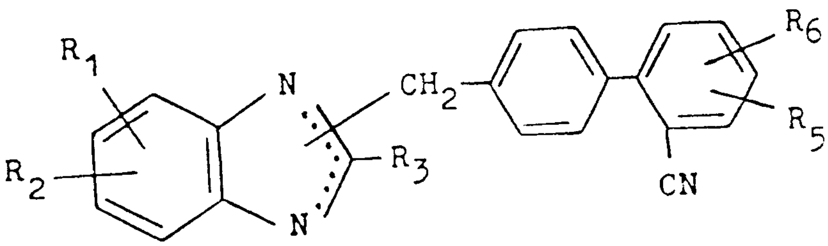
,(X)

in der
$R_1$ bis $R_3$, $R_5$ und $R_6$ wie mindestens in einem der Ansprüche 1 bis 6 definiert sind, mit Stickstoffwasserstoffsäure umgesetzt wird oder
h) zur Herstellung einer Verbindung der Formel I, in der $R_1$ und/oder $R_2$ eine Aminocarbonylaminogruppe darstellt, welche durch eine bi- oder tricyclische Alkylgruppe oder durch eine Alkyl-, Alkenyl-, Alkinyl-, Cycloalkyl-, Cycloalkylalkyl-, Aralkyl- oder Arylgruppe mono-, di- oder tribsubstituiert sein kann, wobei eine Methylengruppe in einem Cycloalkylrest mit 4 bis 7 Kohlenstoffatomen durch ein Sauerstoffatom ersetzt sein kann, eine Verbindung der Formel

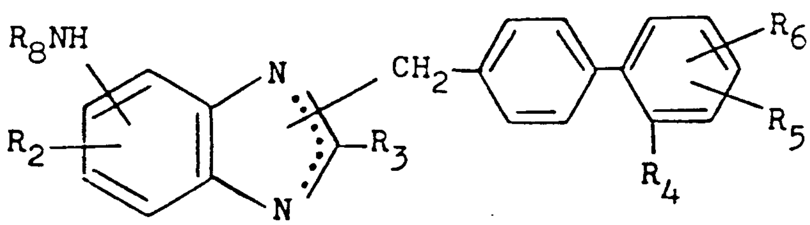
,(XI)

in der
$R_2$ bis $R_6$ wie mindestens in einem der Ansprüche 1 bis 6 definiert sind und
$R_8$ ein Wasserstoffatom, eine Alkylgruppe mit 1 bis 20 Koh
lenstoffatomen, eine Alkenyl- oder Alkinylgruppe mit jeweils 3 bis 5 Kohlenstoffatomen, eine Cycloalkylgruppe mit 3 bis 7 Kohlenstoffatomen, eine Cycloalkyl-alkylgruppe, in welcher der Cycloalkylteil 3 bis 7 Kohlenstoffatome, der Alkylteil 1 bis 3 Kohlenstoffatome enthalten und in einem Cycloalkylteil mit 4 bis 7 Kohlenstoffatomen eine Methylengruppe durch ein Sauerstoffatom ersetzt sein kann, eine bi- oder tricyclische Alkylgruppe mit 7 bis 10 Kohlenstoffatomen, eine Aryl- oder Aralkylgruppe mit 1 bis 3 Kohlenstoffatomen im Alkylteil, wobei die Arylgruppe eine gegebenenfalls durch eine Fluor-, Chlor- oder Bromatom, eine Hydroxy-, Alkyl- oder Alkoxygruppe mit jeweils 1 bis 4 Kohlenstoffatomen substituierte Phenylgruppe darstellen kann, bedeutet, mit einer Verbindung der Formel

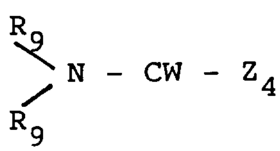
,(XII)

in der
$R_9$, die gleich oder verschieden sein können, die vorstehend für $R_8$ erwähnten Bedeutungen aufweisen,
W ein Sauerstoff- oder Schwefelatom,
$Z_4$ eine nukleophile Austrittsgruppe wie ein Chlor- oder Bromatom oder auch, wenn mindestens einer der Reste $R_9$ ein Wasserstoffatom darstellt,

$Z_4$ und $R_9$ zusammen eine Stickstoff-Kohlenstoff-Bindung oder eine Cycloalkyleniminogruppe mit 4 bis 6 Kohlenstoffatomen oder eine Morpholinogruppe darstellen, umgesetzt wird oder

i) zur Herstellung einer Verbindung der Formel I, in der $R_4$ eine Trifluormethylcarbonylamino-, Trifluormethylcarbonylaminomethyl-, Trifluormethylsulfonylamino-, Trifluormethylsulfonylaminomethyl- oder 1H-Tetrazolylgruppe, eine Alkylcarbonylamino-, Alkylcarbonylaminomethyl-, Arylcarbonylamino-, Arylcarbonylaminomethyl-, Aralkylcarbonylamino-, Aralkylcarbonylaminomethyl-, Alkylsulfonylamino-, Alkylsulfonylaminomethyl-, Arylsulfonylamino-, Arylsulfonylaminomethyl-, Aralkylsulfonylamino- oder Aralkylsulfonylaminomethylgruppe, in denen der Alkylteil jeweils 1 bis 3 Kohlenstoffatome enthalten kann, darstellt, eine Verbindung der Formel

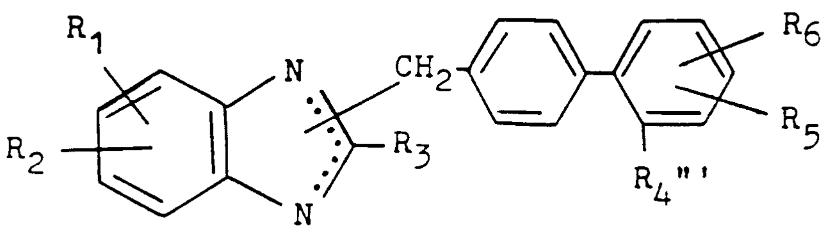

,(XIII)

in der
$R_1$ bis $R_3$, $R_5$ und $R_6$ wie mindestens in einem der Ansprüche 1 bis 6 definiert sind und
$R_4'''$ eine Amino- oder Aminomethylgruppe darstellt, mit einer Verbindung der Formel

$$HO - U - R_{10} ,(XIV)$$

in der
$R_{10}$ eine Trifluormethylgruppe, eine Alkyl-, Aryl- oder Aralkylgruppe, wobei der Alkylteil jeweils 1 bis 3 Kohlenstoffatome enthalten kann, und
U eine Carbonyl- oder Sulfonylgruppe bedeuten, oder mit deren reaktionsfähigen Derivaten wie deren Säurehalogeniden, Säureestern oder Säureanhydriden acyliert wird oder

k) zur Herstellung einer Verbindung der Formel I, in der $R_4$ eine Alkylsulfonylaminocarbonyl- oder Perfluoralkylsulfonylaminogruppe mit jeweils 1 bis 4 Kohlenstoffatomen im Alkylteil oder eine Benzylsulfonylaminocarbonylgruppe darstellt, ein reaktionsfähiges Derivat einer Carbonsäure der Formel

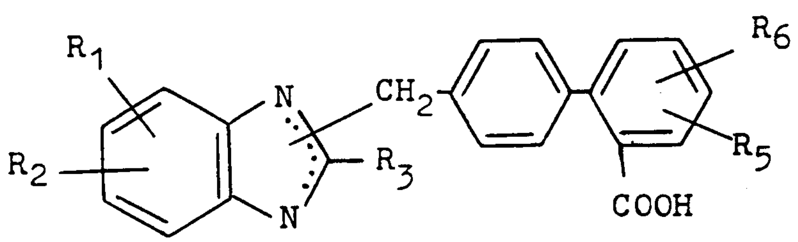

,(XV)

in der
$R_1$ bis $R_3$, $R_5$ und $R_6$ wie mindestens in einem der Ansprüche 1 bis 6 definiert sind, mit einem Sulfonamid der Formel

$$H_2N - SO_2 - R_{11} ,(XVI)$$

in der
$R_{11}$ eine Alkyl- oder Perfluoralkylgruppe mit jeweils 1 bis 4 Kohlenstoffatomen oder eine Benzylgruppe darstellt, oder mit dessen Alkalisalz umgesetzt wird oder

l) zur Herstellung einer Verbindung der Formel I, in der $R_2$ eine gegebenfalls in 3-Stellung durch eine Alkyl-, Cycloalkyl-, Cycloalkylalkyl- oder Aralkylgruppe substituierte 2-Imidazolidon-1-yl- oder 3,4,5,6-Tetrahydro-2-pyrimidon-1-yl-gruppe darstellt, eine Verbindung der Formel

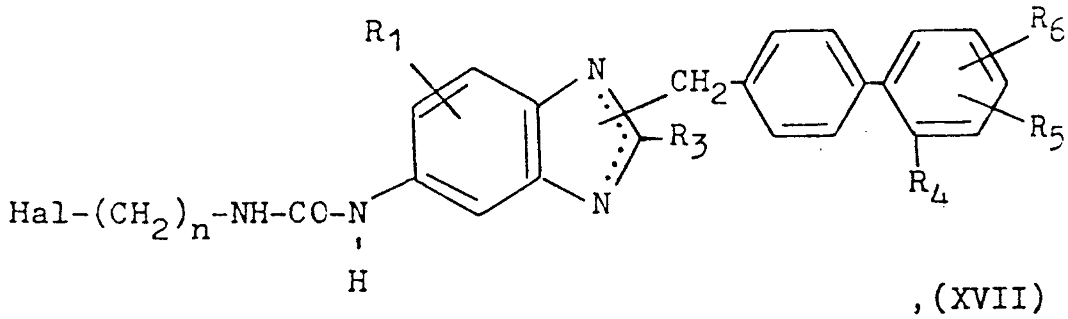

, (XVII)

in der
$R_1$, $R_3$ und $R_4$ bis $R_6$ wie mindestens in einem der Ansprüche 1 bis 6 definiert sind,
Hal ein Chlor-, Brom- oder Jodatom und
n die Zahl 2 oder 3 darstellen, cyclisiert und gewünschtenfalls eine so erhaltene NH-Verbindung mit einer Verbindung der Formel

$R_{12}$ - Hal ,(XVIII)

in der
$R_{12}$ eine Alkyl-, Cycloalkyl-, Cycloalkylalkyl- oder Aralkylgruppe und
Hal ein Chlor-, Brom- oder Jodatom darstellt, alkyliert wird oder
m) zur Herstellung von Verbindungen der allgemeinen Formel I, in der $R_1$ eine Aminogruppe, die durch eine Dialkylaminoalkanoyl-, Acyl-, Cycloalkoxycarbonyl-, Cycloalkylalkoxycarbonyl-, Aryloxycarbonyl-, Aralkoxycarbonyl- oder Trifluoracetylgruppe, durch eine Alkylsulfonylgruppe mit 1 bis 4 Kohlenstoffatomen, durch eine Alkoxycarbonylgruppe mit insgesamt 2 bis 7 Kohlenstoffatomen oder durch eine durch eine Alkoxycarbonylgruppe substituierte Thiazolidin-3-yl-carbonylgruppe monosubstituiert ist,
eine Alkylaminogruppe mit 1 bis 6 Kohlenstoffatomen, die am Stickstoffatom durch eine Alkylsulfonyl- oder Acylgruppe substituiert sein kann, wobei, falls die Acylgruppe eine Alkanoylgruppe darstellt, diese zusätzlich durch eine Alkoxygruppe substituiert sein kann, und der Alkylsubstituent ab Position 2 durch eine Hydroxy-, Alkoxy- oder Arylaminogruppe substituiert sein kann,
eine N-Alkoxycarbonyl-alkylamino-, N-Cycloalkoxycarbonylalkylamino-, N-Cycloalkylalkoxycarbonyl-alkylamino-, N-Aryloxycarbonyl-alkylamino- oder N-Aralkoxycarbonyl-alkylaminogruppe, in der die Alkylgruppe jeweils 1 bis 6 Kohlenstoffatome enthalten kann, eine am Stickstoffatom durch eine Cycloalkyl-, Cycloalkylalkyl- oder Aralkylgruppe substituierte Alkoxycarbonylamino-, Cycloalkoxycarbonylamino-, Cycloalkylalkoxycarbonylamino-, Aryloxycarbonylamino-, Aralkoxycarbonylamino-, Acylamino- oder Alkylsulfonylaminogruppe, oder eine Phthalimino-, Homophthalimino-, 2-Carboxyphenylcarbonylamino-, 2-Carboxyphenylmethylamino-, 2-Carboxyphenylmethylencarbonylamino- oder 2-Carboxymethylenphenylcarbonylaminogruppe, wobei eine Carbonylgruppe in einer Phthaliminogruppe durch eine Methylengruppe ersetzt sowie eine Methylengruppe in einer Homophthalimino-, 2-Carboxyphenylmethylencarbonylamino- oder 2-Carboxymethylenphenylcarbonylaminogruppe durch eine oder zwei Alkylgruppen substituiert sein kann, und zusätzlich die vorstehend erwähnten Phenylkerne durch Alkyl- oder Alkoxygruppen mono- oder disubstituiert, wobei die Substituenten gleich oder verschieden sein können, und gleichzeitig ganz oder teilweise hydriert sein können, eine in 2-Stellung durch eine Carboxygruppe substituierte Bicycloalkan-3-carbonsäureamino- oder Bicycloalken-3-carbonsäureaminogruppe, eine Bicycloalkan-2,3-dicarbonsäureimino- oder Bicycloalken-2,3-dicarbonsäureiminogruppe, in denen der Bicycloalkan- und Bicycloalkenteil jeweils 9 oder 10 Kohlenstoffatome enthalten, durch 1, 2 oder 3 Methylgruppen substituiert und eine Endomethylengruppe durch ein Sauerstoffatom ersetzt sein kann, eine Glutarsäureimino- oder 3-Carboxy-n-propylencarbonylgruppe, in denen jeweils die n-Propylengruppe perfluoriert, durch ein oder zwei Alkylgruppen oder durch eine Tetramethylen- oder Pentamethylengruppe substituiert sein kann, bedeutet und
$R_4$ mit Ausnahme der Aminogruppe die für $R_4$ mindestens in einem der Ansprüche 1 bis 6 erwähnten Bedeutungen besitzt, eine Verbindung der Formel

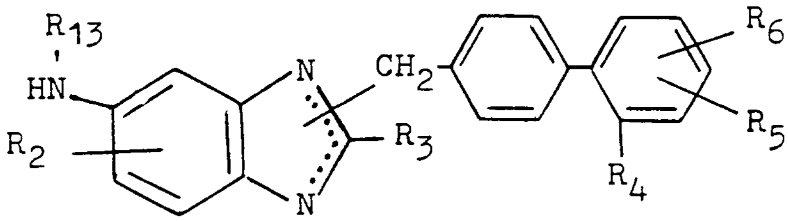

, (XIX)

in der
$R_2$, $R_3$, $R_5$ und $R_6$ wie mindestens in einem der Ansprüche 1 bis 6 definiert sind,
$R_4$ mit Ausnahme der Aminogruppe wie mindestens in einem der Ansprüche 1 bis 6 definiert ist und
$R_{13}$ ein Wasserstoffatom, eine Alkylgruppe mit 1 bis 6 Kohlenstoffatomen, welche ab Position 2 durch eine Hydroxy-, Alkoxy- oder Arylaminogruppe substituiert sein kann, eine Cycloalkyl-, Cycloalkylalkyl- oder Aralkylgruppe bedeutet, mit einer Verbindung der Formel

$$R_{14} - Z_5 ,(XX)$$

in der
$R_{14}$ eine Dialkylaminoalkanoyl-, Cycloalkoxycarbonyl-, Cycloalkylalkoxycarbonyl-, Aryloxycarbonyl-, Aralkoxycarbonyl- oder Trifluoracetylgruppe, eine Alkylsulfonylgruppe mit 1 bis 4 Kohlenstoffatomen, eine Alkoxycarbonylgruppe mit insgesamt 2 bis 7 Kohlenstoffatomen, eine durch eine Alkoxycarbonylgruppe substituierte Thiazolidin-3-yl-carbonylgruppe oder eine Acylgruppe, in welcher, falls die Acylgruppe eine Alkanoylgruppe darstellt, diese zusätzlich durch eine Alkoxygruppe substituiert sein kann, oder eine 2-Carboxyphenylcarbonyl-, 2-Carboxyphenylmethyl-, 2-Carboxyphenylmethylencarbonyl- oder 2-Carboxymethylenphenylcarbonylgruppe, wobei eine Methylengruppe in einer 2-Carboxyphenylmethylencarbonyl- oder 2-Carboxymethylenphenylcarbonylgruppe durch eine oder zwei Alkylgruppen substituiert sein kann, und zusätzlich die vorstehend erwähnten Phenylkerne durch Alkyl- oder Alkoxygruppen mono- oder disubstituiert, wobei die Substituenten gleich oder verschieden sein können, und gleichzeitig ganz oder teilweise hydriert sein können, eine in 2-Stellung durch eine Carboxygruppe substituierte Bicycloalkan-3-carbonyl- oder Bicycloalken-3-carbonylgruppe, in denen der Bicycloalkan- und Bicycloalkenteil jeweils 9 oder 10 Kohlenstoffatome enthalten, durch 1, 2 oder 3 Methylgruppen substituiert und eine Endomethylengruppe durch ein Sauerstoffatom ersetzt sein kann, eine 3-Carboxy-n-propylencarbonylgruppe, in der die n-Propylengruppe perfluoriert, durch ein oder zwei Alkylgruppen oder durch eine Tetramethylen- oder Pentamethylengruppe substituiert sein kann, und $Z_5$ eine nukleophile Austrittsgruppe bedeuten, oder mit deren
reaktionsfähigen Derivaten wie deren Säurehalogeniden, Säureestern oder Säureanhydriden acyliert wird oder
n) zur Herstellung von Verbindungen der allgemeinen Formel I, in der mindestens einer der Reste $R_1$ oder $R_2$ eine 2-Carboxyphenylcarbonylamino-, 2-Carboxyphenylmethylamino-, 2-Carboxyphenylmethylencarbonylamino- oder 2-Carboxymethylenphenylcarbonylaminogruppe, wobei eine Methylengruppe in einer 2-Carboxyphenylmethylencarbonylamino- oder 2-Carboxymethylenphenylcarbonylaminogruppe durch eine oder zwei Alkylgruppen substituiert sein kann, und zusätzlich die vorstehend erwähnten Phenylkerne durch Alkyl- oder Alkoxygruppen mono- oder disubstituiert, wobei die Substituenten gleich oder verschieden sein können, und gleichzeitig ganz oder teilweise hydriert sein können, eine in 2-Stellung durch eine Carboxygruppe substituierte Bicycloalkan-3-carbonsäureamino- oder Bicycloalken-3-carbonsäureaminogruppe, in denen der Bicycloalkan- und Bicycloalkenteil jeweils 9 oder 10 Kohlenstoffatome enthalten, durch 1, 2 oder 3 Methylgruppen substituiert und eine Endomethylengruppe durch ein Sauerstoffatom ersetzt sein kann, eine 3-Carboxy-n-propylencarbonylgruppe, in der die n-Propylengruppe perfluoriert, durch ein oder zwei Alkylgruppen oder durch eine Tetramethylen- oder Pentamethylengruppe substituiert sein kann, bedeutet, eine Verbindung der allgemeinen Formel

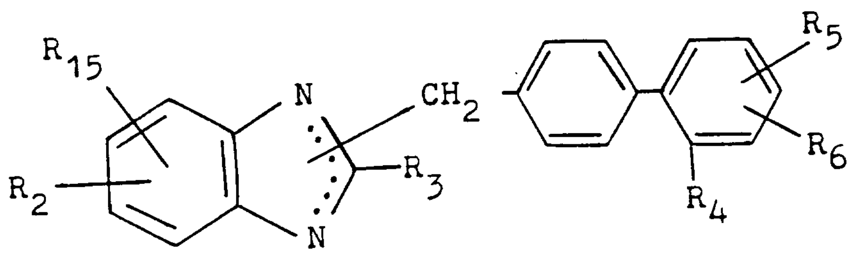

, (XXI)

in der

$R_2$ bis $R_6$ wie mindestens in einem der Ansprüche 1 bis 6 definiert sind und

$R_{15}$ eine Phthalimino- oder Homophthaliminogruppe, wobei eine Carbonylgruppe in einer Phthaliminogruppe durch eine Methylengruppe ersetzt sowie eine Methylengruppe in einer Homophthaliminogruppe durch eine oder zwei Alkylgruppen substituiert sein kann, und zusätzlich die vorstehend erwähnten Phenylkerne durch Alkyl- oder Alkoxygruppen mono- oder disubstituiert, wobei die Substituenten gleich oder verschieden sein können, und gleichzeitig ganz oder teilweise hydriert sein können, eine Bicycloalkan-2,3-dicarbonsäureimino- oder Bicycloalken-2,3-dicarbonsäureiminogruppe, in denen der Bicycloalkan- und Bicycloalkenteil jeweils 9 oder 10 Kohlenstoffatome enthalten, durch 1, 2 oder 3 Methylgruppen substituiert und eine Endomethylengruppe durch ein Sauerstoffatom ersetzt sein kann, eine Glutarsäureiminogruppe, in der die n-Propylengruppe perfluoriert, durch ein oder zwei Alkylgruppen oder durch eine Tetramethylen- oder Pentamethylengruppe substituiert sein kann, bedeutet, partielle hydrolysiert wird und

erforderlichenfalls ein während der Umsetzungen a) bis n) zum Schutze von reaktiven Gruppen verwendeter Schutzrest abgespalten wird und/oder

gewünschtenfalls anschließend eine so erhaltene Verbindung der Formel I, in der $R_1$ und/oder $R_2$ eine Nitrogruppe darstellt, mittels Reduktion in eine entsprechende Aminoverbindung der Formel I übergeführt wird oder

eine so erhaltene Verbindung der Formel I, in der $R_1$ und/oder $R_2$ eine Hydroxy-, Amino-, Alkylamino-, Phenylalkylamino-, Alkylsulfonylamino- oder Acylaminogruppe darstellt, mittels Alkylierung in eine entsprechende alkylierte Verbindung der Formel I übergeführt wird oder

eine so erhaltene Verbindung der Formel I, in der $R_1$ und/oder $R_2$ eine Benzyloxygruppe darstellt, mittels Entbenzylierung in eine entsprechende Verbindung der Formel I, in der $R_1$ und/oder $R_2$ eine Hydroxygruppe darstellt, übergeführt wird oder

eine so erhaltene Verbindung der Formel I, in der $R_1$ und/oder $R_2$ eine Carboxygruppe darstellt, mittels Amidierung in eine entsprechende Verbindung der Formel I, in der $R_1$ und/oder $R_2$ eine gegebenenfalls durch eine oder zwei Alkylgruppen mit jeweils 1 bis 3 Kohlenstoffatomen substituierte Aminocarbonylgruppe darstellt, übergeführt wird oder

eine so erhaltene Verbindung der Formel I, in der $R_1$ und/oder $R_2$ eine Carboxygruppe und/oder $R_4$ eine Cyanogruppe darstellt, mittels Reduktion in eine entsprechende Verbindung der Formel I, in der $R_1$ und/oder $R_2$ eine Hydroxymethylgruppe und/oder $R_4$ eine Aminomethylgruppe darstellt, übergeführt wird oder

eine so erhaltene Verbindung der Formel I, in der $R_1$ und/oder $R_2$ eine Alkoxycarbonylgruppe darstellt, mittels Hydrolyse in eine entsprechende Verbindung der Formel I, in der $R_1$ und/oder $R_2$ eine Carboxygruppe darstellt, übergeführt wird oder

eine so erhaltene Verbindung der Formel I, in der $R_1$ und/oder $R_2$ eine Alkoxy- oder Phenylalkoxygruppe darstellt, mittels Etherspaltung in eine entsprechende Verbindung der Formel I, in der $R_1$ und/oder $R_2$ eine Hydroxygruppe darstellt, übergeführt wird oder

eine so erhaltene Verbindung der Formel I, in der $R_1$ und/oder $R_2$ eine Aminocarbonylamino- oder Aminothiocarbonylgruppe darstellt, die durch eine Cycloalkylgruppe mit 5 bis 10 Kohlenstoffatomen, in der eine Methylengruppe in 2-Stellung durch ein Sauerstoffatom ersetzt ist, substituiert ist und zusätzlich durch eine Alkylgruppe mit 1 bis 20 Kohlenstoffatomen, durch eine Alkenyl- oder Alkinylgruppe mit jeweils 3 bis 5 Kohlenstoffatomen, durch eine bi- oder tricyclische Alkylgruppe mit 7 bis 11 Kohlenstoffatomen, durch eine Cycloalkyl-, Cycloalkylalkyl-, Aralkyl- oder Arylgruppe mono- oder disubstituiert sein kann, mittels Reduktion in eine entsprechende Verbindung der Formel I, in der $R_1$ und/oder $R_2$ eine Aminocarbonylamino- oder Aminothiocarbonylgruppe darstellt, die durch eine 4-Hydroxy-n-butyl-, 5-Hydroxy-n-pentyl- oder 6-Hydroxy-n-hexylgruppe substituiert ist und zusätzlich durch eine Alkylgruppe mit 1 bis 20 Kohlenstoffatomen, durch eine Alkenyl- oder Alkinylgruppe mit jeweils 3 bis 5 Kohlenstoffatomen, durch eine bi- oder tricyclische Alkylgruppe mit 7 bis 11 Kohlenstoffatomen, durch eine Cycloalkyl-, Cycloalkylalkyl-, Aralkyl- oder Arylgruppe mono- oder disubstituiert

sein kann, darstellt, übergeführt wird oder
eine so erhaltene Verbindung der Formel I, in der $R_1$ und oder $R_2$ eine Phthaliminogruppe darstellt, welche durch eine Alkyl- oder Alkoxygruppe mono- oder disubstituiert sein kann, obei die Substituenten gleich oder verschieden sein können, mittels Reduktion in eine entsprechende Verbindung der Formel I in der $R_1$ und/oder $R_2$ eine 1-Oxo-isoindolin-2-yl-gruppe, welche durch eine Alkyl- oder Alkoxygruppe mono- oder disubstituiert sein kann, darstellt, wobei die Substituenten gleich oder verschieden sein können, übergeführt wird oder
ein so erhaltenes 1-, 3-Isomerengemisch einer Verbindung der Formel I mittels Isomerentrennung in ihr 1- und 3-Isomer aufgetrennt wird oder
eine so erhaltene Verbindung der Formel I in ihr Salz, insbesondere für die pharmazeutische Anwendung in ihr physiologisch verträgliches Salz mit einer anorganischen oder organischen Säure oder Base, übergeführt wird.

## Claims

1. Benzimidazoles of the formula

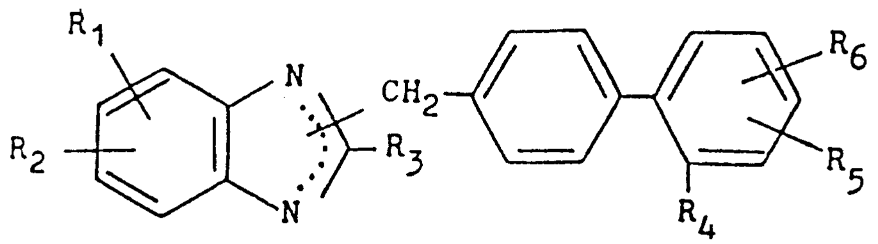 ,(I)

in which
$R_1$ denotes a hydrogen, fluorine, chlorine or bromine atom,
an alkyl group having 1 to 4 carbon atoms, which may be substituted by a hydroxy, alkoxy, amino, alkylamino, dialkylamino or acylamino group,
an alkoxy group having 1 to 4 carbon atoms, which may be substituted in the 2, 3 or 4 position by a hydroxy, alkoxy, amino, alkylamino, dialkylamino or imidazolyl group,
a hydroxy, phenylalkoxy, acyloxy, trifluoromethylsulphonyloxy, alkylaminocarbonyloxy, dialkylaminocarbonyloxy, cycloalkylaminocarbonyloxy, cycloalkylalkylaminocarbonyloxy, arylaminocarbonyloxy, aralkylaminocarbonyloxy, alkoxycarbonyloxy, cycloalkoxycarbonyloxy, cycloalkylalkoxycarbonyloxy, aryloxycarbonyloxy, aralkoxycarbonyloxy, trifluoromethyl, cyano, nitro, alkylmercapto, alkylsulphinyl, alkylsulphonyl, aminosulphonyl, alkylaminosulphonyl, dialkylaminosulphonyl, arylaminosulphonyl, acylaminosulphonyl or acyl group,
an amino group, which may be monosubstituted by an imidazolylalkyl, dialkylaminoalkanoyl, acyl, cycloalkoxycarbonyl, cycloalkylalkoxycarbonyl, aryloxycarbonyl, aralkoxycarbonyl or trifluoroacetyl group, by a bicyclic or tricyclic alkyl group having 7 to 11 carbon atoms, by an alkylsulphonyl group having 1 to 4 carbon atoms, by an alkoxycarbonyl group having a total of 2 to 7 carbon atoms or by a thiazolidin-3-yl-carbonyl group substituted by an alkoxycarbonyl group,
an alkylamino group having 1 to 6 carbon atoms, which may be substituted at the nitrogen atom by an alkyl, alkylsulphonyl or acyl group, wherein, if the acyl group is an alkanoyl group, it may be additionally substituted by an alkoxy group, and the alkyl substituent may be substituted at position 2 by a hydroxy, alkoxy or arylamino group,
an amino group monosubstituted or disubstituted by a cycloalkyl, cycloalkylalkyl, phenylalkyl or phenyl group, wherein the substituents may be the same or different, an N-alkyl-cycloalkylamino, N-alkyl-cycloalkylalkyl-amino, N-alkyl-phenylalkylamino or N-alkyl-phenylamino group,
a pyrrolidino, piperidino or hexamethyleneimino group optionally substituted by an alkyl, cycloalkyl or phenyl group,
an N-alkoxycarbonyl-alkylamino, N-cycloalkoxycarbonyl-alkylamino, N-cycloalkylalkoxycarbonyl-alkylamino, N-aryloxycarbonyl-alkylamino or N-aralkoxycarbonyl-alkylamino group, in which the alkyl group may contain 1 to 6 carbon atoms in each case, an alkoxycarbonylamino, cycloalkoxycarbonylamino, cycloalkylalkoxycarbonylamino, aryloxycarbonylamino, aralkoxycarbonylamino, acylamino or alkylsulphonylamino group substituted at the nitrogen atom by a cycloalkyl, cycloalkylalkyl or aralkyl group,
a carbonyl group which is substituted by an alkyl group substituted in the 2 or 3 position by a hydroxy, alkanoyloxy or alkylamino group, by a hydroxycarbonylalkyl, hydroxy, alkoxy, amino, cycloalkylamino, cycloalkylalkylamino, arylamino or aralkylamino group, by an alkylamino group substituted in the 2 or 3 position by an arylamino group, or by an alkylamino group having 1 to 5 carbon atoms optionally substituted in the alkyl moiety by a carboxy group, which may be substituted in each case additionally at the nitrogen atom by an alkyl group.

an aminoacetylamino group optionally substituted by an alkoxycarbonyl group having a total of 2 to 5 carbon atoms, an aminocarbonylamino or aminothiocarbonylamino group, which may be monosubstituted, disubstituted or trisubstituted by an alkyl group having 1 to 20 carbon atoms, by an alkenyl or alkynyl group having 3 to 5 carbon atoms in each case, by a bicyclic or tricyclic alkyl group having 7 to 11 carbon atoms, by a cycloalkyl, cycloalkylalkyl, aralkyl or aryl group, wherein the substituents may be the same or different, and a methylene group in a cycloalkyl radical having 5 to 7 carbon atoms may be replaced by an oxygen atom, and an alkyl group may be substituted in the 4, 5 or 6 position by a hydroxy, alkanoyl or trifluoroacetyl group,

a cycloalkyleneiminocarbonylamino group having 4 to 6 carbon atoms in the cycloalkyleneimino moiety or a morpholinocarbonylamino group, which may both be substituted at the amino nitrogen by an alkyl group having 1 to 20 carbon atoms, or by a cycloalkyl, cycloalkylalkyl, aralkyl or aryl group,

a phthalimino, homophthalimino, 2-carboxyphenylcarbonyl amino, 2-carboxyphenylmethylamino, 2-carboxyphenylmethylenecarbonylamino, or 2-carboxymethylenephenylcarbonyl amino group, wherein a carbonyl group in a phthalimino group may be replaced by a methylene group, and a methylene group in a homopthalimino, 2-carboxyphenylmethylenecarbonyl amino or 2-carboxymethylenephenylcarbonylamino group may be substituted by one or two alkyl groups, and additionally the above phenyl nuclei may be mono or disubstituted by alkyl or alkoxy groups, whilst the substituents may be the same or different, and may at the same time be completely or partially hydrogenated, a bicycloalkane-3-carboxylic acid amino or bicycloalkane-3-carboxylic acid amino group, a bicycloalkane-2,3-dicarboxylic acid imino or bicycloalkane-2,3,-dicarboxylic acid imino group substituted in the 2 position by a carboxy group, wherein the bicycloalkane and bicycloalkene moiety each contain 9 or 10 carbon atoms, may be substituted by 1, 2 or 3 methyl groups and an endomethylene group may be replaced by an oxygen atom, a glutaric acid imino or 3-carboxy-n-propylenecarbonyl group, in which the n-propylene group may be perfluorinated in each case, may be substituted by one or two alkyl groups or by a tetramethylene or pentamethylene group, or

a 5,7-dioxa-1H,3H-imidazo[1,5-c]thiazolyl group,

$R_2$ denotes the meanings for $R_1$ mentioned above, a 2-imidazolidon-1-yl or 3,4,5,6-tetrahydro-2-pyrimidon-1-yl group optionally substituted in the 3 position by an alkyl, cycloalkyl, cycloalkylalkyl or aralkyl group, or a tetrazolyl group or

$R_1$ and $R_2$ together with 2 carbon atoms of the neighbouring phenyl ring denote a phenyl or 1-alkyl-3,3-dialkyl-2,3-dihydropyrrol-2-one group,

$R_3$ denotes a hydrogen, fluorine, chlorine or bromine atom,

an alkyl group having 1 to 6 carbon atoms, in which a methylene group may be replaced by an oxygen or sulphur atom, a sulphinyl, sulphonyl or alkylamino group, and a methyl group may be substituted by a hydroxy, alkoxy, amino, alkylamino or dialkylamino group, but wherein the methylene group next to the benzimidazole ring may not be replaced by a sulphinyl or sulphonyl group, and when a methylene group is replaced and a methyl group is substituted at the same time, they must be separated from one another by at least 2 carbon atoms,

an alkenyl or alkynyl group each having 3 to 5 carbon atoms,

a phenylalkyl group,

a cycloalkyl or cycloalkylalkyl group, in which the cycloalkyl moiety may contain 5 to 7 carbon atoms in each case,

an aryl, hydroxy or imidazolylalkylamino group,

an alkylamino group having 1 to 4 carbon atoms,

an aminocarbonyl group, which may be substituted by an alkyl or cycloalkyl group having 5 to 7 carbon atoms,

a 5-membered heteroaromatic ring, which contains an NH group, an oxygen or sulphur atom, wherein this 5-membered heteroaromatic ring mentioned above may additionally contain a further 1 to 3 N atoms, or a 6-membered heteroaromatic ring, which may contain 1 or 2 nitrogen atoms, wherein the 5-membered and 6-membered heteroaromatic rings mentioned above may be substituted by one or, with the exception of the tetrazolyl group, by two alkyl groups,

$R_4$ denotes an amino, phthalimino, aminomethyl, cyano, tert.-butoxycarbonyl or 1-(triphenylmethyl)-tetrazolyl group, these compounds being intermediate products, a group containing a Brönsted acid or a radical which can be converted in vivo to a group containing a Brönsted acid, these compounds being angiotension-II-antagonists.

$R_5$ and $R_6$ each denote a hydrogen atom or

$R_5$ and $R_6$ together with the two ortho position carbon atoms denote a phenyl group, wherein, provided that nothing else has been mentioned,

the acyl group mentioned in the definition of the radicals $R_1$ to $R_6$ means an alkanoyl group having 1 to 7 carbon atoms, a cycloalkylcarbonyl group having a total of 4 to 8 carbon atoms, a cycloalkylalkanoyl group having a total of 5 to 10 carbon atoms, or an arylcarbonyl, aralkanoyl or phenylsulphonyl group optionally substituted by a fluorine, chlorine, or bromine atom or an alkyl or alkoxy group,

the aryl group means a phenyl group optionally substituted by a fluorine, chlorine or bromine atom, by a hydroxy, alkyl, alkoxy, phenylalkoxy or trifluoromethyl group, wherein the alkyl moiety may contain 1 to 4 carbon atoms in each case, or a naphthyl group,

the cycloalkyl group means a cycloalkyl group having 3 to 7 carbon atoms, which may be substituted by one or two alkyl groups, and

the remaining alkyl and alkanoyl groups mentioned in the definition of the radicals $R_1$ to $R_6$ mean in each case alkyl or alkanoyl groups having 1 to 3 carbon atoms, mixtures of the 1- and 3-isomers thereof and the salts thereof with inorganic or organic acids or bases.

**2.** Benzimidazoles of the formula I according to claim 1, in which $R_1$ to $R_3$, $R_5$ and $R_6$ are as defined in claim 1, and $R_4$ is a carboxy, aminoacetylamino, trifluoromethylcarbonylamino, trifluoromethylcarbonylaminomethyl, trifluoromethylsulphonylamino, trifluoromethylsulphonylaminomethyl or 1H-tetrazolyl group, an alkylcarbonylamino, alkylcarbonylaminomethyl, arylcarbonylamino, arylcarbonylaminomethyl, aralkylcarbonylamino, aralkylcarbonylaminomethyl, alkylsulphonylamino, alkylsulphonylaminomethyl, arylsulphonylamino, arylsulphonylaminomethyl, aralkylsulphonylamino, aralkylsulphonylaminomethyl, arylsulphonylaminocarbonyl or benzylsulphonylaminocarbonyl group, an alkylsulphonylaminocarbonyl or perfluoroalkylsulphonylaminocarbonyl group each having 1 to 6 carbon atoms in the alkyl moiety, an alkoxycarbonyl group having a total of 2 to 7 carbon atoms, an aralkoxycarbonyl group, a pivaloyloxymethoxycarbonyl, phthalidylmethoxycarbonyl, ethoxycarbonyloxyethoxycarbonyl, methoxymethoxycarbonyl, cyclohexyloxycarbonylmethoxycarbonyl or (1,3-dioxa-2-oxo-4-methyl-cyclopenten-5-yl)-methoxycarbonyl group, mixtures of the 1- and 3-isomers thereof and the salts thereof with inorganic or organic acids or bases.

**3.** Benzimidazoles of the formula I according to claim 1, in which

$R_1$ denotes a hydrogen, fluorine or chlorine atom, a trifluoromethyl, hydroxy, benzyloxy, carboxy, cyano, amino, nitro, aminosulphonyl, methylaminosulphonyl, dimethylaminosulphonyl, trifluoromethylsulphonyloxy or tetrazolyl group, an alkyl group having 1 to 4 carbon atoms, wherein the methyl group may be additionally substituted by a hydroxy or alkylamino group having 1 to 4 carbon atoms,

an alkoxy group having 1 to 4 carbon atoms, which may be substituted in the 2, 3 or 4 position by a hydroxy, alkoxy, alkylamino, dialkylamino or imidazolyl group and the alkyl substituent may contain 1 to 3 carbon atoms in each case, an alkanoyloxy group having 1 to 4 carbon atoms or a cycloalkylaminocarbonyloxy group having 5 to 7 carbon atoms in the cycloalkyl moiety,

an amino group, which is substituted by a benzyl, decalin, trifluoromethylcarbonyl, benzylcarbonyl, benzyloxycarbonyl, 2-ethyl-5-methyl-cyclohexyloxy or tert.butoxycarbonylaminoacetyl group, by an alkyl group having 1 to 5 carbon atoms, by a cycloalkyl, cycloalkylalkyl, cycloalkoxycarbonyl, cycloalkylcarbonyl or cycloalkylalkanoyl group, wherein the cycloalkyl moiety may contain 5 to 7 carbon atoms, the alkyl moiety may contain 1 to 3 carbon atoms and the alkanoyl moiety may contain 1 to 3 carbon atoms, by an alkanoyl group having 1 to 6 carbon atoms, by an aminoalkanoyl or dialkylaminoalkanoyl group, in which the alkyl moiety and the alkanoyl moiety may each contain 1 to 3 carbon atoms, by an alkoxycarbonyl group having a total of 2 to 7 carbon atoms, by an alkylsulphonyl group having 1 to 4 carbon atoms or by a thiazolidin-3-ylcarbonyl group substituted by an alkoxycarbonyl group having a total of 2 to 4 carbon atoms,

a benzylamino or alkylamino group having 1 to 5 carbon atoms, which is substituted at the nitrogen atom additionally by a benzyl or cyclohexyl group, by an alkyl group having 1 to 3 carbon atoms, which may be substituted in the 2 or 3 position by an alkoxy group having 1 to 3 carbon atoms or by a phenylamino group, by an alkanoyl group having 1 to 5 carbon atoms, which may be substituted by an alkoxy group having 1 to 3 carbon atoms, by a cycloalkylcarbonyl group having a total of 6 to 8 carbon atoms or by an alkoxycarbonyl group having a total of 2 to 4 carbon atoms,

a carbonyl group which is substituted by an alkyl group which may be substituted in the 2 or 3 position by a hydroxy, alkoxycarbonyl or alkylamino group, by an alkylamino group having 1 to 5 carbon atoms which may be substituted in the alkyl moiety by a carboxy group, by an alkylamino group which is substituted in the 2 or 3 position by a phenylamino group, by a phenyl, alkoxy, amino, benzylamino, phenylethylamino, cycloalkylamino, or cycloalkylalkylamino group, wherein the alkyl moiety may contain 1 to 3 carbon atoms and the cycloalkyl moiety may contain 5 to 7 carbon atoms, in each case, and additionally an alkylamino group having 1 to 5 carbon atoms may be substituted at the nitrogen atom by an alkyl group having 1 to 4 carbon atoms,

an aminocarbonylamino or aminothiocarbonylamino group, which may be monosubstituted, disubstituted or trisubstituted by an alkyl group having 1 to 12 carbon atoms, by an alkenyl or alkynyl group having 3 to 5 carbon atoms in each case, by a bicyclic or tricyclic alkyl group having 7 to 11 carbon atoms, by a cycloalkyl, cycloalkylalkyl or phenylalkyl group, wherein the substituents may be the same or different, and the alkyl moiety may contain 1 to 3 carbon atoms and the cycloalkyl moiety may contain 5 to 7 carbon atoms, and a methylene group in a cycloalkyl radical having 5 to 7 carbon atoms may be replaced by an oxygen atom, and an alkyl group may be substituted in the 4, 5 or 6 position by a hydroxy or trifluoroacetyl group,

a cycloalkyleneiminocarbonylamino group having 4 to 6 carbon atoms in the cycloalkyleneimino moiety or a morpholinocarbonylamino group, which may both be substituted at the amino nitrogen by an alkyl group having 1 to 12 carbon atoms, by a cycloalkyl, cycloalkylalkyl or phenylalkyl group, in which the alkyl moiety may contain 1 to 3 carbon atoms and the cycloalkyl moiety may contain 5 to 7 carbon atoms,

a phthalimino, homophthalimino, 2-carboxyphenylcarbonyl amino, 2-carboxyphenylmethylamino, 2-carboxyphenylmethylenecarbonylamino, or 2-carboxycarboxymethylenephenylcarbonyl amino group, wherein a carbonyl group in a phthalimino group may be replaced by a methylene group, and a methylene group in a homopthalimino, 2-carboxyphenylmethylenecarbonyl amino or 2-carboxymethylenephenylcarbonylamino group may be substituted by one or two methyl groups, and additionally the above phenyl nuclei may be mono or disubstituted by methyl or methoxy groups, whilst the substituents may be the same or different, and may at the same time be completely or partially hydrogenated, a bicycloalkane-3-carboxylic acid amino or bicycloalkane-3-carboxylic acid amino group, a bicycloalkane-2,3-dicarboxylic acid imino or bicycloalkane-2,3,-dicarboxylic acid imino group substituted in the 2 position by a carboxy group, wherein the bicycloalkane and bicycloalkene moiety each contain 9 or 10 carbon atoms, may be substituted by 1, 2 or 3 methyl groups and an endomethylene group may be replaced by an oxygen atom, a glutaric acid imino or 3-carboxy-n-propylenecarbonyl group, in which the n-propylene group may be perfluorinated in each case, may be substituted by one or two alkyl groups or by a tetramethylene or pentamethylene group, or a 5,7-dioxa-1H,3H-imidazo[1,5-c]thiazolyl group, a 2-imidazolidon-1-yl group optionally substituted in the 3 position by an alkyl group having 1 to 3 carbon atoms, or a 3,4,5,6-tetrahydro-2-pyrimidon-1-yl group,

$R_2$ denotes a hydrogen, fluorine or chlorine atom, a methyl, hydroxy or methoxy group, or

$R_1$ and $R_2$ together with the two ortho position carbon atoms of the neighbouring phenyl ring denote a phenyl or 1-alkyl-3,3-dialkyl-2,3-dihydropyrrol-2-one group, in which the alkyl moiety may contain 1 to 3 carbon atoms in each case,

$R_3$ denotes a hydrogen, fluorine or chlorine atom,

a hydroxy, benzyl or aminocarbonyl group,

an alkyl group having 1 to 5 carbon atoms, wherein the methyl group may be additionally substituted by a hydroxy group, by an alkoxy, alkylsulphenyl, alkylsulphinyl or alkylsulphonyl group each having 1 to 3 carbon atoms, a cycloalkyl or cycloalkylalkyl group, in which the cycloalkyl moiety may contain 5 to 7 carbon atoms and the alkyl moiety may contain 1 to 3 carbon atoms, an alkenyl or alkynyl group each having 3 to 5 carbon atoms, a phenylalkyl group having 1 to 3 carbon atoms,

an alkylamino group having 1 to 4 carbon atoms,

a pyridyl, furyl, thiazolyl or pyrazolyl group, which may be substituted by one or two methyl groups,

$R_4$ denotes an alkoxycarbonyl having a total of 1 to 5 carbon atoms, an amino, phthalimino, aminomethyl, carboxy, cyano, methylsulphonylaminocarbonyl, trifluoromethylsulphonylaminocarbonyl, benzenesulphonylaminocarbonyl, trifluoro-methylcarbonylaminomethyl, trifluoro-methylaminomethyl, tetrazolyl or 1-(triphenylmethyl)-tetrazolyl group,

$R_5$ and $R_6$ denote a hydrogen atom or

$R_5$ and $R_6$ together with the two ortho position carbon atoms denote a phenyl group, mixtures of the 1- and 3-isomers thereof and addition salts thereof with inorganic or organic acids or bases.

**4.** Benzimidazoles of the formula I according to claim 1, in which

$R_1$ denotes an amino group, which is substituted by a benzyl, Decalin, trifluoromethylcarbonyl, benzylcarbonyl, benzyloxycarbonyl, 2-ethyl-5-methylcyclohexyloxy or tert.butoxycarbonylaminoacetyl group, by an alkyl group having 1 to 5 carbon atoms, by a cycloalkyl, cycloalkylalkyl, cycloalkoxycarbonyl, cycloalkylcarbonyl or cycloalkylalkanoyl group, wherein the cycloalkyl moiety may contain 5 to 7 carbon atoms, the alkyl moiety may contain 1 to 3 carbon atoms and the alkanoyl moiety may contain 1 to 3 carbon atoms, by an alkanoyl group having 1 to 6 carbon atoms, by an aminoalkanoyl or dialkylaminoalkanoyl group, in which the alkyl moiety and the alkanoyl moiety may each contain 1 to 3 carbon atoms, by an alkoxycarbonyl group having a total of 2 to 7 carbon atoms, by an alkylsulphonyl group having 1 to 4 carbon atoms or by a thiazolidin-3-ylcarbonyl group substituted by an alkoxycarbonyl group having a total of 2 to 4 carbon atoms,

a benzylamino or alkylamino group having 1 to 5 carbon atoms, which is additionally substituted at the nitrogen atom by a benzyl or cyclohexyl group, by an alkyl group having 1 to 3 carbon atoms, which may be substituted in the 2 or 3 position by an alkoxy group having 1 to 3 carbon atoms or by a phenylamino group, by an alkanoyl group having 1 to 5 carbon atoms, which may be substituted by an alkoxy group having 1 to 3 carbon atoms, by a cycloalkylcarbonyl group having a total of 6 to 8 carbon atoms or by an alkoxycarbonyl group having a total of 2 to 4 carbon atoms,

an aminocarbonylamino or aminothiocarbonylamino group, which may be monosubstituted, disubstituted or trisubstituted by an alkyl group having 1 to 12 carbon atoms, by an alkenyl or alkynyl group each having 3 to 5 carbon atoms, by a bicyclic or tricyclic alkyl group having 7 to 11 carbon atoms, by a cycloalkyl, cycloalkylalkyl or phenylalkyl group, wherein the substituents may be the same or different, and the alkyl moiety may contain 1 to 3 carbon atoms and the cycloalkyl moiety may contain 5 to 7 carbon atoms, and a methylene group in a cycloalkyl radical having 5 to 7 carbon atoms may be replaced by an oxygen atom, and an alkyl group may be substituted in the 4, 5 or 6 position by a hydroxy or trifluoroacetyl group,

a cycloalkyleneiminocarbonylamino group having 4 to 6 carbon atoms in the cycloalkyleneimino moiety or a morpholinocarbonylamino group, which may both be substituted at the amino nitrogen by an alkyl group having 1 to 12 carbon atoms, by a cycloalkyl, cycloalkylalkyl or phenylalkyl group, in which the alkyl moiety may contain 1 to 3

carbon atoms and the cycloalkyl moiety may contain 5 to 7 carbon atoms,
a phthalimino, homophthalimino, 2-carboxyphenylcarbonyl amino, 2-carboxyphenylmethylamino, 2-carboxyphenylmethylenecarbonylamino, or 2-carboxy-carboxymethylenephenylcarbonyl amino group, wherein a carbonyl group in a phthalimino group may be replaced by a methylene group, and a methylene group in a homopthalimino, 2-carboxyphenylmethylenecarbonyl amino or 2-carboxymethylenephenylcarbonylamino group may be substituted by one or two methyl groups, and additionally the above phenyl nuclei may be mono or disubstituted by methyl or methoxy groups, whilst the substituents may be the same or different, and may at the same time be completely or partially hydrogenated, a bicycloalkane-3-carboxylic acid amino or bicycloalkane-3-carboxylic acid amino group, a bicycloalkane-2,3-dicarboxylic acid imino or bicycloalkane-2,3,-dicarboxylic acid imino group substituted in the 2 position by a carboxy group, wherein the bicycloalkane and bicycloalkene moiety each contain 9 or 10 carbon atoms, may be substituted by 1, 2 or 3 methyl groups and an endomethylene group may be replaced by an oxygen atom, a glutaric acid imino or 3-carboxy-n-propylenecarbonyl group, in which the n-propylene group may be perfluorinated in each case, may be substituted by one or two alkyl groups or by a tetramethylene or pentamethylene group, or
a 5,7-dioxa-1H,3H-imidazo[1,5-c]thiazolyl group,
$R_2$ denotes a hydrogen atom, a methyl or hydroxy group, or
$R_1$ and $R_2$ together with the two ortho position carbon atoms of the neighbouring phenyl ring denote a phenyl group,
$R_3$ denotes an alkyl group having 3 to 5 carbon atoms, an alkenyl or alkynyl group each having 3 to 5 carbon atoms,
$R_4$ denotes a carboxy, methylsulphonylaminocarbonyl, trifluoromethylsulphonylaminocarbonyl, benzenesulphonylaminocarbonyl, trifluorocarbonylaminomethyl, trifluoromethylaminomethyl or tetrazolyl group,
$R_5$ and $R_6$ each denote a hydrogen atom or
$R_5$ and $R_6$ together with the two ortho position carbon atoms denote a phenyl group, mixtures of the 1- and 3-isomers thereof and addition salts thereof with inorganic or organic acids or bases.

**5.** Benzimidazoles of the formula I according to claim 1, in which
$R_1$ denotes an amino group, which is substituted by an alkanoyl group having 1 to 6 carbon atoms, by an aminoalkanoyl or dialkylaminoalkanoyl group, in which the alkyl moiety and the alkanoyl moiety may each contain 1 to 3 carbon atoms, by an alkoxycarbonyl group having a total of 2 to 7 carbon atoms or by a thiazolidin-3-ylcarbonyl group substituted by an alkoxycarbonyl group having a total of 2 to 4 carbon atoms,
a benzylamino or alkylamino group having 1 to 5 carbon atoms, which is substituted at the nitrogen atom by an alkanoyl group having 1 to 5 carbon atoms, which may be substituted by an alkoxy group having 1 to 3 carbon atoms, by a cycloalkylcarbonyl group having a total of 6 to 8 carbon atoms or by an alkoxycarbonyl group having a total of 2 to 4 carbon atoms,
an aminocarbonylamino or aminothiocarbonylamino group, which may be monosubstituted, disubstituted or trisubstituted by an alkyl group having 1 to 8 carbon atoms, by an alkenyl or alkynyl group each having 3 to 5 carbon atoms, by a bicyclic or tricyclic alkyl group having 7 to 11 carbon atoms, by a cycloalkyl, cycloalkylalkyl or phenylalkyl group, wherein the substituents may be the same or different, and the alkyl moiety may contain 1 to 3 carbon atoms and the cycloalkyl moiety may contain 5 to 7 carbon atoms, and a methylene group in a cycloalkyl radical having 5 to 7 carbon atoms may be replaced by an oxygen atom, and an alkyl group may be substituted in the 4, 5 or 6 position by a hydroxy or trifluoroacetyl group,
a cycloalkyleneiminocarbonylamino group having 4 to 6 carbon atoms in the cycloalkyleneimino moiety or a morpholinocarbonylamino group, which may both be substituted at the amino nitrogen by an alkyl group having 1 to 8 carbon atoms, by a cycloalkyl, cycloalkylalkyl or phenylalkyl group, in which the alkyl moiety may contain 1 to 3 carbon atoms and the cycloalkyl moiety may contain 5 to 7 carbon atoms,
a phthalimino, homophthalimino, 2-carboxyphenylcarbonyl amino, 2-carboxyphenylmethylamino, 2-carboxyphenylmethylenecarbonylamino, or 2-carboxy-carboxymethylenephenylcarbonyl amino group, wherein a carbonyl group in a phthalimino group may be replaced by a methylene group, and a methylene group in a homopthalimino, 2-carboxyphenylmethylenecarbonyl amino or 2-carboxymethylenephenylcarbonylamino group may be substituted by one or two methyl groups, and additionally the above phenyl nuclei may be mono or disubstituted by methyl or methoxy groups, whilst the substituents may be the same or different, and may at the same time be completely or partially hydrogenated, a bicycloalkane-3-carboxylic acid amino or bicycloalkane-3-carboxylic acid amino group, a bicycloalkane-2,3-dicarboxylic acid imino or bicycloalkane-2,3,-dicarboxylic acid imino group substituted in the 2 position by a carboxy group, wherein the bicycloalkane and bicycloalkene moiety each contain 9 or 10 carbon atoms, may be substituted by 1, 2 or 3 methyl groups and an endomethylene group may be replaced by an oxygen atom, a glutaric acid imino or 3-carboxy-n-propylenecarbonyl group, in which the n-propylene group may be perfluorinated in each case, may be substituted by one or two alkyl groups or by a tetramethylene or pentamethylene group, or
a 5,7-dioxa-1H,3H-imidazo[1,5-c]thiazolyl group,
$R_2$ denotes a hydrogen atom,
$R_1$ and $R_2$ together with the two ortho position carbon atoms of the neighbouring phenyl ring denote a phenyl group,
$R_3$ denotes an alkyl group having 3 to 5 carbon atoms, an alkenyl or alkynyl group each having 3 to 5 carbon atoms,

$R_4$ denotes a carboxy or tetrazolyl group,
$R_5$ and $R_6$ denote a hydrogen atom or
$R_5$ and $R_6$ together with the two ortho position carbon atoms denote a phenyl group, mixtures of the 1- and 3-isomers thereof and addition salts thereof with inorganic or organic acids or bases.

**6.** Benzimidazoles of the formula I according to claim 1:
4'-[(2-n-butyl-6-(N-(n-hexylaminocarbonyl)-benzylamino)-benzimidazol-1-yl)-methyl]biphenyl-2-carboxylic acid,
4'-[(2-n-butyl-6-(N-cyclohexylaminocarbonyl-n-butylamino)-benzimidazol-1-yl)-methyl]biphenyl-2-carboxylic acid,
4'-[(2-n-butyl-6-(N-cyclohexylaminocarbonyl-ethylamino)-benzimidazol-1-yl)-methyl]biphenyl-2-carboxylic acid,
4'-[(2-n-butyl-6-(N-cyclohexylaminocarbonyl-n-propylamino)-benzimidazol-1-yl)-methyl]biphenyl-2- carboxylic acid,
4'-[(2-n-butyl-6-(N-cyclohexylaminocarbonylmethylamino)-benzimidazol-1-yl)-methyl]biphenyl-2-carboxylic acid,
4'-[(2-n-butyl-6-cyclohexylaminocarbonylamino-benzimidazol-1-yl)-methyl]biphenyl-2-carboxylic acid,
4'-[(2-n-butyl-6-(N-cyclohexylaminocarbonyl-n-pentylamino)-benzimidazol-1-yl)-methyl]biphenyl-2-carboxylic acid,
4'-[(2-n-butyl-6-(N-methylaminocarbonyl-benzylamino)-benzimidazol-1-yl)-methyl]biphenyl-2-carboxylic acid,
4'-[(2-n-butyl-6-(1-oxo-isoindolin-2-yl)-benzimidazol-1-yl)-methyl]biphenyl-2-carboxylic acid,
4'-[(2-n-butyl-6-(N-cyclohexylaminocarbonyl-benzylamino)-benzimidazol-1-yl)-methyl]biphenyl-2-carboxylic acid,
4'-[(2-n-butyl-6-n-pentanoylamino-benzimidazol-1-yl)-methyl]biphenyl-2-carboxylic acid,
4'-[(2-n-butyl-6-propionylamino-benzimidazol-1-yl)-methyl]biphenyl-2-carboxylic acid,
4'-[(2-n-butyl-6-isopropylcarbonylamino-benzimidazol-1-yl)-methyl]biphenyl-2-carboxylic acid,
4'-[(2-n-butyl-6-(tetrahydropyran-2-yl-aminocarbonylamino)-benzimidazol-1-yl)-methyl]biphenyl-2-carboxylic acid,
4'-[(2-n-butyl-6-(n-butylaminocarbonylamino)-benzimidazol-1-yl)-methyl]biphenyl-2-carboxylic acid,
4'-[(6-n-butanoylamino-2-n-butyl-benzimidazol-1-yl)-methyl]biphenyl-2-carboxylic acid,
4'-[(2-n-butyl-6-(N-ethoxycarbonyl-benzylamino)-benzimidazol-1-yl)-methyl]biphenyl-2-carboxylic acid,
4'-[(2-n-butyl-6-(N-(dimethylaminocarbonyl)-amino)-benzimidazol-1-yl)-methyl]biphenyl-2-carboxylic acid,
4'-[(2-n-butyl-6-(N-cyclohexylaminocarbonyl-n-hexylamino)-benzimidazol-1-yl)-methyl]biphenyl-2-carboxylic acid,
4'-[(2-n-butyl-6-((5-hydroxy-n-pentyl)-aminocarbonylamino)-benzimidazol-1-yl)-methyl]biphenyl-2-carboxylic acid,
4'-[(2-n-butyl-6-(N-methylaminocarbonyl-n-pentylamino)-benzimidazol-1-yl)-methyl]biphenyl-2-carboxylic acid,
4'-[(2-n-butyl-6-cyclohexylcarbonylamino-benzimidazol-1-yl)-methyl]biphenyl-2-carboxylic acid,
4'-[(2-n-butyl-6-(N-(n-butylaminocarbonyl)-methylamino)-benzimidazol-1-yl)-methyl]biphenyl-2-carboxylic acid,
4'-[(2-n-butyl-7-hydroxy-4-methyl-benzimidazol-1-yl)-methyl]-2-(1H-tetrazol-5-yl)-biphenyl,
4'-[(2-n-butyl-6-(cis-hexahydrophthalimino)-benzimidazol-1-yl)-methyl]biphenyl-2-carboxylic acid,
4'-[(2-n-butyl-6-(N-(dimethylaminocarbonyl)-benzylamino)-benzimidazol-1-yl)-methyl]biphenyl-2-carboxylic acid,
4'-[(2-n-butyl-6-(N-(n-hexylaminocarbonyl)-N-(2-phenylethyl)-amino)-benzimidazol-1-yl)-methyl]biphenyl- 2-carboxylic acid and
4'-[(2-n-butyl-6-cyclopentylcarbonylamino-benzimidazol-1-yl)-methyl]biphenyl-2-carboxylic acid,
mixtures of the 1- and 3-isomers thereof and addition salts thereof with inorganic or organic acids or bases.

**7.** Physiologically acceptable addition salts of the compounds according to at least one of claims 1 to 6 with inorganic or organic acids or bases.

**8.** Medicaments containing a compound according to at least one of claims 1 to 6, in which $R_4$ is a group containing a Brönsted acid or a radical which may be converted in vivo to a group containing a Brönsted acid, or a physiologically acceptable addition salt thereof in addition to one or more optional inert excipients and/or diluents.

**9.** Use of a compound according to at least one of claims 1 to 6, in which $R_4$ is a group containing a Brönsted acid or a radical which may be converted in vivo to a group containing a Brönsted acid, or a physiologically acceptable addition salt thereof, for the preparation of a medicament which is suitable for the treatment of hypertonia, cardiac insufficiency, ischaemic peripheral circulatory disorders, myocardial ischaemia (Angina), for the prevention of cardiac insufficiency progression after myocardial infarction, for the treatment of diabetic nephropathy, glaucoma, gastrointestinal illnesses and diseases of the bladder.

**10.** Process for the preparation of a medicament according to claim 8, characterised in that a compound according to at least one of claims 1 to 6 in which $R_4$ is a group containing a Brönsted acid or a radical which may be converted in vivo to a group containing a Brönsted acid, or a physiologically acceptable addition salt thereof is incorporated in one or more inert excipients and/or diluents in non-chemical manner.

**11.** Process for the preparation of the benzimidazoles according to claims 1 to 7, characterised in that

a) a compound of the formula

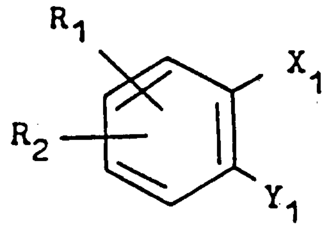

,(II)

in which
$R_1$ and $R_2$ are as defined in at least one of claims 1 to 6,
one of the radicals $X_1$ or $Y_1$ is a group of the formula

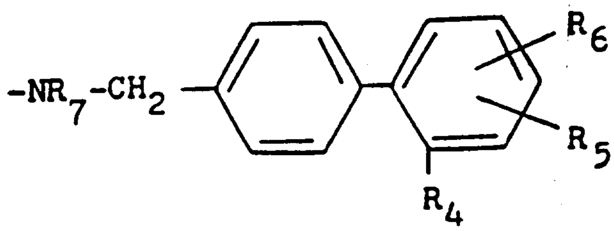

and the other of the radicals $X_1$ or $Y_1$ is a group of the formula

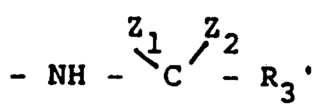

wherein
$R_3$' has the meanings mentioned in at least one of claims 1 to 6 for $R_3$, with the exception of the fluorine, chlorine or bromine atom, the hydroxy, mercapto or aminocarbonyl group, wherein the latter may be substituted by an alkyl group having 1 to 3 carbon atoms or by a cycloalkyl group having 5 to 7 carbon atoms, $R_4$ to $R_6$ are as defined in at least one of claims 1 to 6, $R_7$ denotes a hydrogen atom, a hydroxy group or an $R_3$'CO group, wherein $R_3$' is defined as mentioned above, $Z_1$ and $Z_2$, which may be the same or different, denote optionally substituted amino groups or hydroxy or mercapto groups optionally substituted by lower alkyl groups, or
$Z_1$ and $Z_2$, together denote an oxygen atom or sulphur atom, an imino group optionally substituted by an alkyl group having 1 to 3 carbon atoms, an alkylenedioxy or alkylenedithio group each having 2 or 3 carbon atoms,

but wherein one of the radicals $X_1$ or $Y_1$ must be a group of the formula

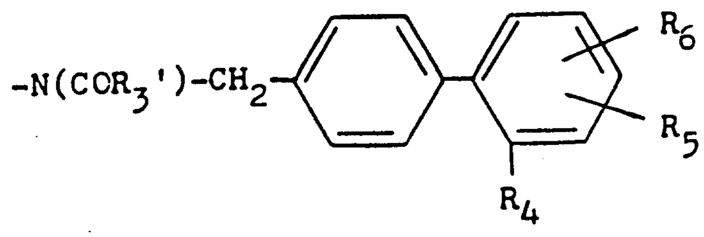

or

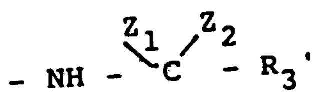

is cyclised, or

b) for the preparation of the compounds of the formula I, in which $R_3$ represents the aromatic or heteroaromatic radicals mentioned in at least one of claims 1 to 6 for $R_3$,
a phenylenediamine of the formula

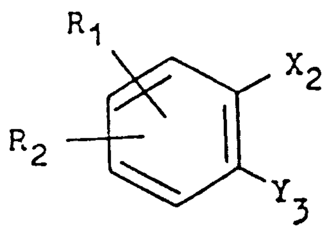

,(III)

in which
$R_1$ and $R_2$ are as defined in at least one of claims 1 to 6, one of the radicals $X_2$ or $Y_2$ denotes a group of the formula

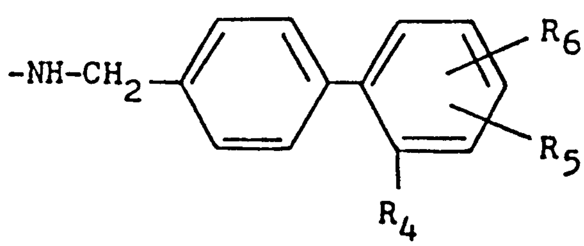

in which
$R_4$ to $R_6$ are as defined in at least one of claims 1 to 6, and the other of the radicals $X_2$ or $Y_2$ denotes an amino group, is reacted with an aldehyde of the formula

OCH -$R_3''$ (IV)

in which

$R_3$" denotes the aromatic or heteroaromatic radicals mentioned in at least one of claims 1 to 6 for $R_3$, in the presence of an oxidising agent, or

c) for the preparation of compounds of the formula I, in which at least one of the radicals $R_1$, $R_2$ and/or $R_3$ is one of the radicals containing a sulphinyl or sulphonyl group mentioned in at least one of claims 1 to 6 for $R_1$, $R_2$ and/or $R_3$, and the remainder of the radicals $R_1$, $R_2$ and/or $R_3$ are as defined in at least one of claims 1 to 6, a compound of the formula

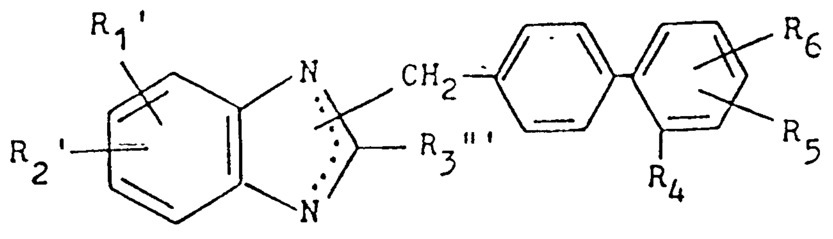
, (V)

in which

$R_4$ to $R_6$ are as defined in at least one of claims 1 to 6, at least one of the radicals $R_1$', $R_2$' and/or $R_3$''' is one of the radicals containing a sulphur atom or a sulphinyl group mentioned in at least one of claims 1 to 6 for $R_1$, $R_2$ and/or $R_3$, and the remainder of the radicals $R_1$', $R_2$' and/or $R_3$''' have the meanings mentioned in at least one of claims 1 to 6 for $R_1$, $R_2$ and/or $R_3$, is oxidised, or

d) for the preparation of a compound of the formula I, in which $R_4$ is a carboxy or amino group, a compound of the formula

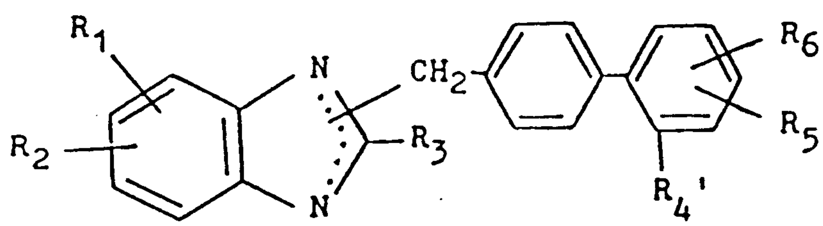
, (VI)

in which

$R_1$ to $R_3$, $R_5$ and $R_6$ are as defined in at least one of claims 1 to 6, and

$R_4$' is a group which can be converted to a carboxy or amino group, is converted to a corresponding carboxy or amino compound, or

e) a benzimidazole of the formula

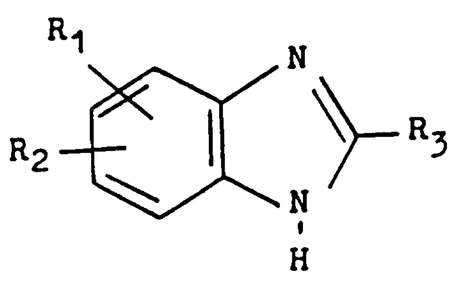
, (VII)

in which

$R_1$ to $R_3$ are as defined in at least one of claims 1 to 6, is reacted with a biphenyl compound of the formula

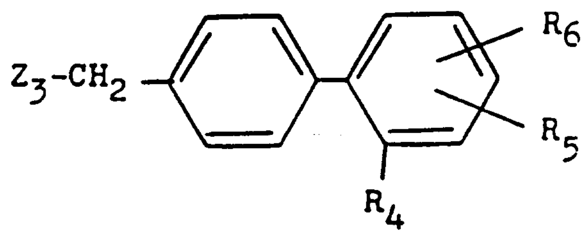

, (VIII)

in which
$R_4$ to $R_6$ are as defined in at least one of claims 1 to 6, and
$Z_3$ is a nucleophilic leaving group, such as a halogen atom, for example a chlorine, bromine or iodine atom, or a substituted sulphonyloxy group, for example a methanesulphonyloxy, phenylsulphonyloxy or p-toluenesulphonyloxy group, or

f) for the preparation of a compound of the formula I, in which $R_4$ is a 1H-tetrazolyl group, a protective radical is cleaved off from a compound of the formula

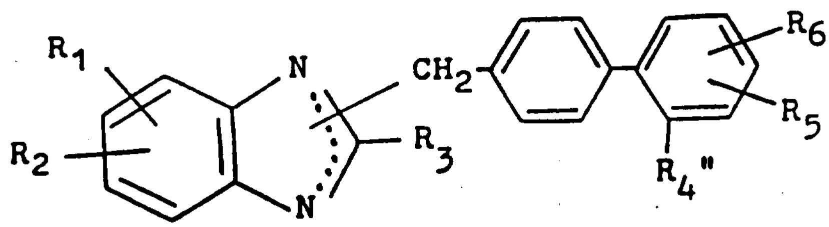

, (IX)

in which
$R_1$ to $R_3$, $R_5$ and $R_6$ are as defined in at least one of claims 1 to 6, and
$R_4$" is a 1H-tetrazolyl group protected in the 1 position by a protective radical, or
g) for the preparation of a compound of the formula I, in which $R_4$ is a 1H-tetrazolyl group, a compound of the formula

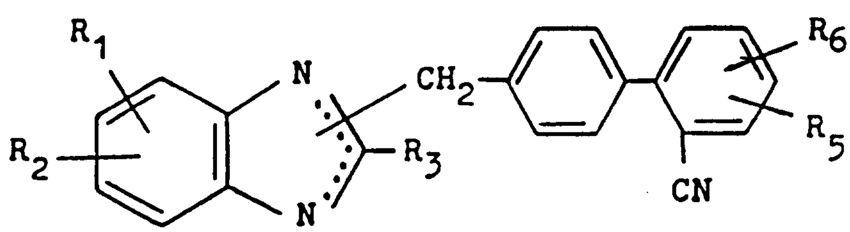

, (X)

in which
$R_1$ to $R_3$, $R_5$ and $R_6$ are as defined in at least one of claims 1 to 6, is reacted with hydrazoic acid, or
h) for the preparation of a compound of the formula I, in which $R_1$ and/or $R_2$ is an aminocarbonylamino group, which may be monosubstituted, disubstituted or trisubstituted by a bicyclic or tricyclic alkyl group or by an alkyl, alkenyl, alkynyl, cycloalkyl, cycloalkylalkyl, aralkyl or aryl group, wherein a methylene group in a cycloalkyl

radical having 4 to 7 carbon atoms may be replaced by an oxygen atom, a compound of the formula

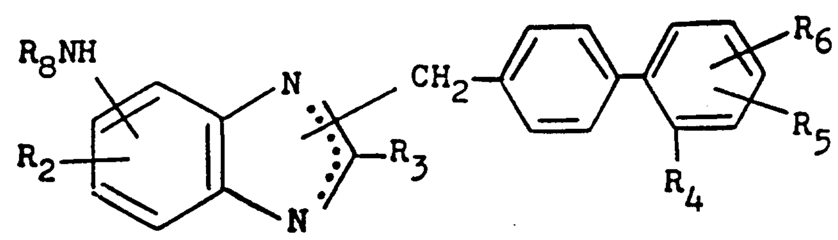

,(XI)

in which
$R_2$ to $R_6$ are as defined in at least one of claims 1 to 6, and $R_8$ denotes a hydrogen atom, an alkyl group having 1 to 20 carbon atoms, an alkenyl or alkynyl group each having 3 to 5 carbon atoms, a cycloalkyl group having 3 to 7 carbon atoms, a cycloalkylalkyl group, in which the cycloalkyl moiety may contain 3 to 7 carbon atoms, the alkyl moiety may contain 1 to 3 carbon atoms and a methylene group in a cycloalkyl moiety having 4 to 7 carbon atoms may be replaced by an oxygen atom, a bicyclic or tricyclic alkyl group having 7 to 10 carbon atoms, an aryl or aralkyl group having 1 to 3 carbon atoms in the alkyl moiety, wherein the aryl group may be a phenyl group optionally substituted by a fluorine, chlorine or bromine atom, or a hydroxy, alkyl or alkoxy group each having 1 to 4 carbon atoms, is reacted with a compound of the formula

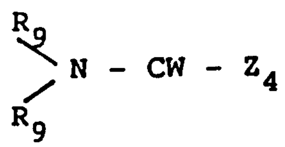

,(XII)

in which
$R_9$, which may be the same or different, have the meanings mentioned above for $R_8$,
W is an oxygen atom or sulphur atom,
$Z_4$ is a nucleophilic leaving group, such as a chlorine or bromine atom, or also, if at least one of the radicals $R_9$ is a hydrogen atom,
$Z_4$ and $R_9$ together are a nitrogen-carbon bond or a cycloalkyleneimino group having 4 to 6 carbon atoms, or a morpholino group, or

i) for the preparation of a compound of the formula I, in which $R_4$ is a trifluoromethylcarbonylamino, trifluoromethylcarbonylaminomethyl, trifluoromethylsulphonylamino, trifluoromethylsulphonylaminomethyl or 1H-tetrazolyl group, an alkylcarbonylamino, alkylcarbonylaminomethyl, arylcarbonylamino, arylcarbonylaminomethyl, aralkylcarbonylamino, aralkylcarbonylaminomethyl, alkylsulphonylamino, alkylsulphonylaminomethyl, arylsulphonylamino, arylsulphonylaminomethyl, aralkylsulphonylamino or aralkylsulphonylaminomethyl group, in which the alkyl moiety may contain 1 to 3 carbon atoms in each case, a compound of the formula

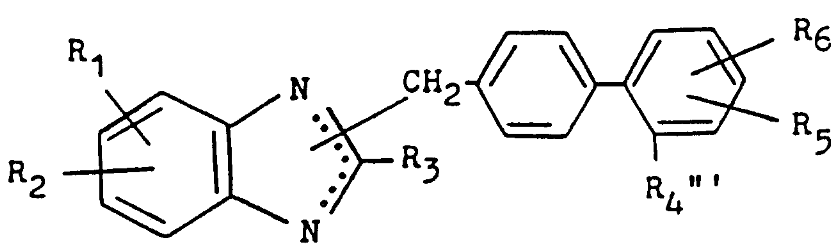

,(XIII)

in which
$R_1$ to $R_3$, $R_5$ and $R_6$ are as defined in at least one of claims 1 to 6, and
$R_4'''$ is an amino or aminomethyl group, is acylated using a compound of the formula

$$HO - U - R_{10} \quad (XIV)$$

(in which

$R_{10}$ denotes a trifluoromethyl group, an alkyl, aryl or aralkyl group, wherein the alkyl moiety may contain 1 to 3 carbon atoms in each case, and

U denotes a carbonyl or sulphonyl group) or using reactive derivatives thereof, such as acid halides, acid esters or acid anhydrides thereof, or

k) for the preparation of a compound of the formula I, in which $R_4$ is an alkylsulphonylaminocarbonyl or perfluoroalkylsulphonylamino group each having 1 to 4 carbon atoms in the alkyl moiety, or is a benzylsulphonylaminocarbonyl group, a reactive derivative of a carboxylic acid of the formula

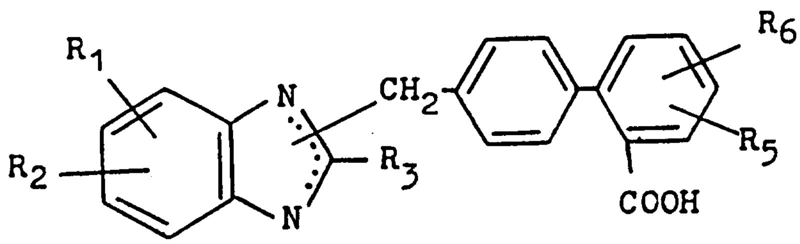

, (XV)

in which

$R_1$ to $R_3$, $R_5$ and $R_6$ are as defined in at least one of claims 1 to 6, is reacted with a sulphonamide of the formula

$$H_2N - SO_2 - R_{11} \quad (XVI)$$

(in which

$R_{11}$ is an alkyl or perfluoroalkyl group each having 1 to 4 carbon atoms, or is a benzyl group) or with alkali metal salt thereof, or

l) for the preparation of a compound of the formula I, in which $R_2$ is a 2-imidazolidon-1-yl or 3,4,5,6-tetrahydro-2-pyrimidon-1-yl group optionally substituted in the 3 position by an alkyl, cycloalkyl, cycloalkylalkyl or aralkyl group, a compound of the formula

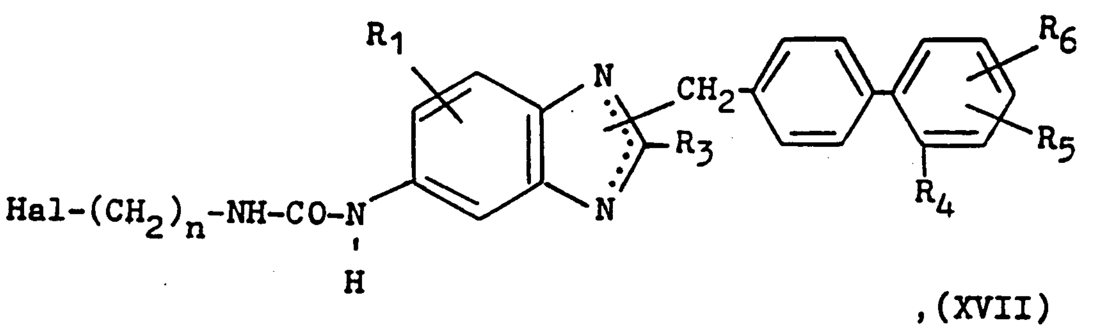

, (XVII)

in which

$R_1$, $R_3$ and $R_4$ to $R_6$ are as defined in at least one of claims 1 to 6,

Hal is a chlorine, bromine or iodine atom, and

n is the number 2 or 3, is cyclised and, if required, an NH compound thus obtained is alkylated using a compound of the formula

$$R_{12} - Hal \quad (XVIII)$$

in which

$R_{12}$ is an alkyl, cycloalkyl, cycloalkylalkyl or aralkyl group, and

Hal is a chlorine, bromine or iodine atom, or

m) for the preparation of compounds of the general formula I, in which $R_1$ denotes an amino group, which is monosubstituted by a dialkylaminoalkanoyl, acyl, cycloalkoxycarbonyl, cycloalkylalkoxycarbonyl, aryloxycarbonyl, aralkoxycarbonyl or trifluoroacetyl group, by an alkylsulphonyl group having 1 to 4 carbon atoms, by an alkoxycarbonyl group having a total of 2 to 7 carbon atoms, or by a thiazolidin-3-yl-carbonyl group substituted by an alkoxycarbonyl group,

an alkylamino group having 1 to 6 carbon atoms, which may be substituted at the nitrogen atom by an alkylsulphonyl or acyl group, wherein, if the acyl group is an alkanoyl group, it may additionally be substituted by an alkoxy group, and the alkyl substituent may be substituted at position 2 by a hydroxy, alkoxy or arylamino group, an N-alkoxycarbonyl-alkylamino, N-cycloalkoxycarbonylalkylamino, N-cycloalkylalkoxycarbonyl-alkylamino, N-aryloxycarbonyl-alkylamino or N-aralkoxycarbonylalkylamino group, in which the alkyl group may contain 1 to 6 carbon atoms in each case, an alkoxycarbonylamino, cycloalkoxycarbonylamino, cycloalkylalkoxycarbonylamino, aryloxycarbonylamino, aralkoxycarbonylamino, acylamino or alkylsulphonylamino group substituted at the nitrogen atom by a cycloalkyl, cycloalkylalkyl or aralkyl group, or

a phthalimino, homophthalimino, 2-carboxyphenylcarbonyl amino, 2-carboxyphenylmethylamino, 2-carboxyphenylmethylenecarbonylamino, or 2-carboxymethylenephenylcarbonyl amino group, wherein a carbonyl group in a phthalimino group may be replaced by a methylene group, and a methylene group in a homopthalimino, 2-carboxyphenylmethylenecarbonyl amino or 2-carboxymethylenephenylcarbonylamino group may be substituted by one or two alkyl groups, and additionally the above phenyl nuclei may be mono or disubstituted by alkyl or alkoxy groups, whilst the substituents may be the same or different, and may at the same time be completely or partially hydrogenated, a bicycloalkane-3-carboxylic acid amino or bicycloalkane-3-carboxylic acid amino group, a bicycloalkane-2,3-dicarboxylic acid imino or bicycloalkane-2,3,-dicarboxylic acid imino group substituted in the 2 position by a carboxy group, wherein the bicycloalkane and bicycloalkene moiety each contain 9 or 10 carbon atoms, may be substituted by 1, 2 or 3 methyl groups and an endomethylene group may be replaced by an oxygen atom, a glutaric acid imino or 3-carboxy-n-propylenecarbonyl group, in which the n-propylene group may be perfluorinated in each case, may be substituted by one or two alkyl groups or by a tetramethylene or pentamethylene group, or

and

$R_4$ has the meanings mentioned in at least one of claims 1 to 6 for $R_4$, with the exception of the amino group, a compound of the formula

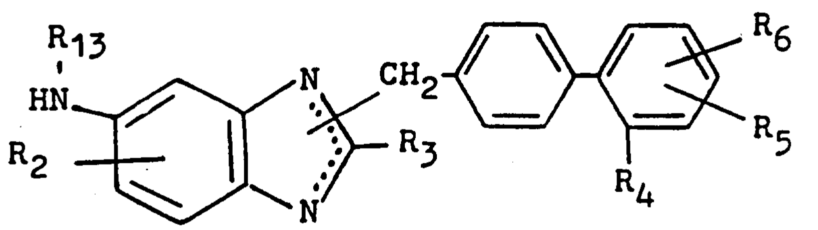

, (XIX)

in which

$R_2$, $R_3$, $R_5$ and $R_6$ are as defined in at least one of claims 1 to 6,

$R_4$ is as defined in at least one of claims 1 to 6, with the exception of the amino group, and

$R_{13}$ denotes a hydrogen atom, an alkyl group having 1 to 6 carbon atoms, which may be substituted at position 2 by a hydroxy, alkoxy or arylamino group, a cycloalkyl, cycloalkylalkyl or aralkyl group, is acylated using a compound of the formula

$$R_{14}\text{-}Z_5 \quad (XX)$$

(in which

$R_{14}$ denotes a dialkylaminoalkanoyl, cycloalkoxycarbonyl, cycloalkylalkoxycarbonyl, aryloxycarbonyl, aralkoxycarbonyl or trifluoroacetyl group, an alkylsulphonyl group having 1 to 4 carbon atoms, an alkoxycarbonyl group having a total of 2 to 7 carbon atoms, a thiazolidin-3-ylcarbonyl group substituted by an alkoxycarbonyl group, or an acyl group, in which, if the acyl group is an alkanoyl group, it may additionally be substituted by an alkoxy group, or a 2-carboxyphenylcarbonyl, 2-carboxyphenylmethyl, 2-carboxyphenylmethylenecarbonyl or 2-carboxymethylenephenylcarbonyl group, whilst a methylene group in a 2-carboxyphenylmethylenecarbonyl or 2-carboxy-methylenephenylcarbonyl group may be substituted by one or two alkenyl groups, and additionally the above phenyl nuclei may be mono or disubstituted by alkyl or alkoxy groups, while the substituents may be the same or different, and may at the same time be completely or partially hydrogenated, a bicycloalkane-3-carbonyl or bicycloalkene-3-carbonyl group substituted in the 2 position by a carboxy group, wherein the bicycloalkane and bicycloalkene moiety each have 9 or 10 carbon atoms, may be substituted by 1, 2 or 3 methyl groups, and

an endomethylene group may be replaced by an oxygen atom, a 3-carboxy-n-propylenecarbonyl group, wherein the n-propylene group may be perfluorinated, may be substituted by one or two alkyl groups or by a tetramethylene or pentamethylene group
$Z_5$ denotes a nucleophilic leaving group) or using reactive derivatives thereof, such as acid halides, acid esters or acid anhydrides thereof, and

n) for the preparation of compounds of the general Formula I, in which at least one of the groups $R_1$ or $R_2$ is a 2-carboxyphenylcarbonylamino, 2-carboxyphenylmethylamino, 2-carboxyphenylmethylenecarbonylamino, or 2-carboxymethylenephenylcarbonylamino group, whilst a methylene group in a 2-carboxyphenylmethylenecarbonylamino or 2-carboxymethyleneamino group may be substituted by one or two alkyl groups, and additionally the above phenyl nuclei may be mono or disubstituted by alkyl or alkoxy groups, whilst the substituents may be the same or different, and at the same time may be completely or partially hydrogenated, a bicycloalkane-3-carboxylic acidamino group or bicycloalkene-3-carboxylic acid amino group substituted in the 2 position by a carboxy group, wherein the bicycloalkane and bicycloalkene moiety each contain 9 or 10 carbon atoms, may be substituted by 1, 2 or 3 methyl groups, and an endomethylene group may be replaced by an oxygen atom, a 3-carboxy-n-propylenecarbonyl group, wherein the n-propylene group may be perfluorinated, may be substituted by one or two alkyl groups or by a tetramethylene or a pentamethylene group, a compound of the general formula

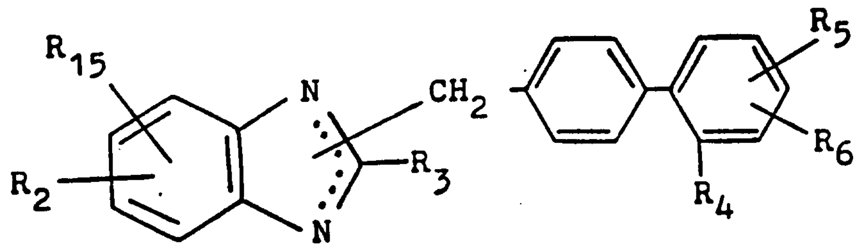
, (XXI)

in which
$R_2$ to $R_6$ are as defined in at least one of Claims 1 to 6, and $R_{15}$ a phthalimino or homophthalimino group, whilst a carbonyl group in a phthalimino group may be replaced by a methylene group and a methylene group in a homophthalimino group may be substituted by one or two alkyl groups, and additionally the above phenyl nuclei may be mono or disubstituted by alkyl or alkoxy groups, whilst the substituents may be the same or different, and at the same time may be completely or partially hydrogenated, a bicycloalkane-2,3-dicarboxylic acid imino or bicycloalkene-2,3-dicarboxylic acid imino group, wherein the bicycloalkane and bicycloalkane moiety each contain 9 or 10 carbon atoms, may be substituted by 1, 2 or 3 methyl groups and an endomethylene group may be replaced by an oxygen atom, a glutaricacidimino group, wherein the n-propylene group may be perfluorinated, may be substituted by one or two alkyl groups or by a tetramethylene or pentamethylene group, is partially hydrolysed, and
if required, a protective radical used during the reactions a) to n) to protect reactive groups is cleaved and/or if desired, a compound of the formula I thus obtained, in which $R_1$ and/or $R_2$ is a nitro group, is then converted to a corresponding amino compound of the formula I by means of reduction, or
a compound of the formula I thus obtained, in which $R_1$ and/or $R_2$ is a hydroxy, amino, alkylamino, phenylalkylamino, alkylsulphonylamino or acylamino group, is converted to a corresponding alkylated compound of the formula I by means of alkylation, or
a compound of the formula I thus obtained, in which $R_1$ and/or $R_2$ is a benzyloxy group, is converted to a corresponding compound of the formula I, in which $R_1$ and/or $R_2$ is a hydroxy group, by means of debenzylation, or
a compound of the formula I thus obtained, in which $R_1$ and/or $R_2$ is a carboxy group, is converted to a corresponding compound of the formula I, in which $R_1$ and/or $R_2$ is an aminocarbonyl group optionally substituted by one or two alkyl groups each having 1 to 3 carbon atoms, by means of amidation, or
a compound of the formula I thus obtained, in which $R_1$ and/or $R_2$ is a carboxy group and/or $R_4$ is a cyano group, is converted to a corresponding compound of the formula I, in which $R_1$ and/or $R_2$ is a hydroxymethyl group and/or $R_4$ is an aminomethyl group, by means of reduction, or
a compound of the formula I thus obtained, in which $R_1$ and/or $R_2$ is an alkoxycarbonyl group, is converted to a corresponding compound of the formula I, in which $R_1$ and/or $R_2$ is a carboxy group, by means of hydrolysis, or
a compound of the formula I thus obtained, in which $R_1$ and/or $R_2$ is an alkoxy or phenylalkoxy group, is converted to a corresponding compound of the formula I, in which $R_1$ and/or $R_2$ is a hydroxy group, by means of ether cleaving, or

a compound of the formula I thus obtained, in which $R_1$ and/or $R_2$ is an aminocarbonylamino or aminothiocarbonyl group, which is substituted by a cycloalkyl group having 5 to 10 carbon atoms, in which a methylene group is replaced in the 2 position by an oxygen atom, and may be additionally monosubstituted or disubstituted by an alkyl group having 1 to 20 carbon atoms, by an alkenyl or alkynyl group each having 3 to 5 carbon atoms, by a bicyclic or tricyclic alkyl group having 7 to 11 carbon atoms, by a cycloalkyl, cycloalkylalkyl, aralkyl or aryl group, is converted to a corresponding compound of the formula I, in which $R_1$ and/or $R_2$ is an aminocarbonylamino or aminothiocarbonyl group, which is substituted by a 4-hydroxy-n-butyl, 5-hydroxy-n-pentyl or 6-hydroxy-n-hexyl group and may additionally be monosubstituted or disubstituted by an alkyl group having 1 to 20 carbon atoms, by an alkenyl or alkynyl group each having 3 to 5 carbon atoms, by a bicyclic or tricyclic alkyl group having 7 to 11 carbon atoms, by a cycloalkyl, cycloalkylalkyl, aralkyl or aryl group, by means of reduction, or

a compound of the formula I thus obtained, in which $R_1$ and/or $R_2$ is a phthalimino group, which may be monosubstituted or disubstituted by an alkyl or alkoxy group, wherein the substituents may be the same or different, is converted to a corresponding compound of the formula I, in which $R_1$ and/or $R_2$ is a 1-oxo-isoindolin-2-yl group, which may be monosubstituted or disubstituted by an alkyl or alkoxy group, wherein the substituents may be the same or different, by means of reduction, or

a mixture of the 1- and 3-isomers of a compound of the formula I thus obtained is separated into its 1-isomer and 3-isomer by means of isomer separation, or

a compound of the formula I thus obtained is converted to its addition salt, in particular for the pharmaceutical application to its physiologically acceptable salt with an inorganic or organic acid or base.

## Revendications

1. Benzimidazoles de formule

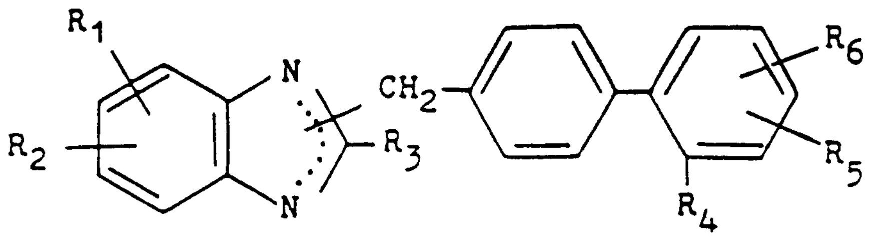

(I)

dans laquelle

$R_1$ représente un atome d'hydrogène, de fluor, de chlore ou de brome,

un groupe alkyle de 1 à 4 atomes de carbone qui peut être substitué par un groupe hydroxyle, alcoxy, amino, alkylamino, dialkylamino ou acylamino,

un groupe alcoxy de 1 à 4 atomes de carbone qui peut être substitué en position 2, 3 ou 4 par un groupe hydroxyle, alcoxy, amino, alkylamino, dialkylamino ou imidazolyle, un groupe hydroxyle, phénylalcoxy, acyloxy, trifluorométhylsulfonyloxy, alkylaminocarbonyloxy, dialkylaminocarbonyloxy, cycloalkylaminocarbonyloxy, cycloalkylalkylaminocarbonyloxy, arylaminocarbonyloxy, aralkylaminocarbonyloxy, alcoxycarbonyloxy, cycloalcoxycarbonyloxy, cycloalkylalcoxycarbonyloxy, aryloxycarbonyloxy, aralcoxycarbonyloxy, trifluorométhyle, cyano, nitro, alkylmercapto, alkylsulfinyle, alkylsulfonyle, aminosulfonyle, alkylaminosulfonyle, dialkylaminosulfonyle, arylaminosulfonyle, acylaminosulfonyle ou acyle,

un groupe amino qui peut être monosubstitué par un groupe imidazolylalkyle, dialkylaminoalkyle, acyle, cycloalcoxycarbonyle, cycloalkylalcoxycarbonyle, aryloxycarbonyle, aralcoxycarbonyle ou trifluoroacétyle, par un groupe alkyle bi- ou tricyclique de 7 à 11 atomes de carbone, par un groupe alkylsulfonyle de 1 à 4 atomes de carbone, par un groupe alcoxycarbonyle de 2 à 7 atomes de carbone au total ou par un groupe thiazolidin-3-ylcarbonyle substitué par un groupe alcoxycarbonyle,

un groupe alkylamino de 1 à 6 atomes de carbone qui peut être substitué sur l'atome d'azote par un groupe alkyle, alkylsulfonyle ou acyle, auquel cas, lorsque le groupe acyle représente un groupe alcanoyle, celui-ci peut être substitué en outre par un groupe alcoxy, et le substituant alkyle peut être substitué à partir de la position 2 par un groupe hydroxyle, alcoxy ou arylamino,

un groupe amino mono- ou disubstitué par un groupe cycloalkyle, cycloalkylalkyle, phénylalkyle ou phényle, les substituants pouvant être identiques ou différents, un groupe N-alkyl-cycloalkylamino, N-alkyl-cycloalkylalkylamino, N-alkyl-phénylalkylamino ou N-alkyl-phénylamino,

un groupe pyrrolidino, pipéridino ou hexaméthylèneimino éventuellement substitué par un groupe alkyle, cycloalkyle ou phényle,

un groupe N-alcoxycarbonyl-alkylamino, N-cycloalcoxycarbonyl-alkylamino, N-cycloalkylalcoxycarbonyl-alkylamino, N-aryloxycarbonyl-alkylamino ou N-aralcoxycarbonylalkylamino dans lequel le groupe alkyle peut contenir dans chaque cas 1 à 6 atomes de carbone, un groupe alcoxycarbonylamino, cycloalcoxycarbonylamino, cycloalkylalcoxycarbonylamino, aryloxycarbonylamino, aralcoxycarbonylamino, acylamino ou alkylsulfonylamino substitué sur l'atome d'azote par un groupe cycloalkyle, cycloalkylalkyle ou aralkyle,

un groupe carbonyle qui est substitué par un groupe alkyle qui est substitué en position 2 ou 3 par un groupe hydroxyle, alcanoyloxy ou alkylamino, par un groupe hydroxycarbonylalkyle, hydroxyle, alcoxy, amino, cycloalkylamino, cycloalkylalkylamino, arylamino ou aralkylamino, par un groupe alkylamino substitué en position 2 ou 3 par un groupe arylamino ou par un groupe alkylamino de 1 à 5 atomes de carbone éventuellement substitué dans la partie alkyle par un groupe carboxyle, qui, dans chaque cas, peut être substitué en plus sur l'atome d'azote par un groupe alkyle,

un groupe aminoacétylamino éventuellement substitué par un groupe alcoxycarbonyle de 2 à 5 atomes de carbone au total,

un groupe aminocarbonylamino ou aminothiocarbonylamino qui peut être mono-, di- ou trisubstitué par un groupe alkyle de 1 à 20 atomes de carbone, par un groupe alcényle ou alcynyle de 3 à 5 atomes de carbone dans chaque cas, par un groupe alkyle bi- ou tricyclique de 7 à 11 atomes de carbone, par un groupe cycloalkyle, cycloalkylalkyle, aralkyle ou aryle, les substituants pouvant être identiques ou différents et un groupe méthylène pouvant être remplacé dans un reste cycloalkyle de 5 à 7 atomes de carbone par un atome d'oxygène et un groupe alkyle pouvant être substitué en position 4, 5 ou 6 par un groupe hydroxyle, alcanoyle ou trifluoroacétyle,

un groupe cycloalkylèneiminocarbonylamino de 4 à 6 atomes de carbone dans la partie cycloalkylèneimino ou un groupe morpholinocarbonylamino qui peuvent être substitués l'un et l'autre sur l'azote de l'amino par un groupe alkyle de 1 à 20 atomes de carbone, par un groupe cycloalkyle, cycloalkylalkyle, aralkyle ou aryle,

un groupe phtalimino, homophtalimino, 2-carboxyphénylcarbonylamino, 2-carboxyphénylméthylamino, 2-carboxyphénylméthylènecarbonylamino ou 2-carboxyméthylènephénylcarbonylamino, auquel cas un groupe carbonyle dans un groupe phtalimino peut être remplacé par un groupe méthylène et un groupe méthylène dans un groupe homophtalimino, 2-carboxyphénylméthylènecarbonylamino ou 2-carboxyméthylènephénylcarbonylamino peut être substitué par un ou deux groupes alkyle, et en outre les noyaux phényle cités précédemment peuvent être mono- ou disubstitués par des groupes alkyle ou alcoxy, les substituants pouvant être identiques ou différents, et peuvent être en même temps hydrogénés totalement ou partiellement, un groupe bicycloalcane-3-carboxamido ou bicycloalcène-3-carboxamido substitué en position 2 par un groupe carboxyle, un groupe bicycloalcane-2,3-dicarboximido ou bicycloalcène-2,3-dicarboximido, dans lesquels, dans chaque cas, les parties bicycloalcane et bicycloalcène peuvent contenir 9 ou 10 atomes de carbone, peuvent être substituées par 1, 2 ou 3 groupes méthyle et un groupe endométhylène peut être remplacé par un atome d'oxygène, un groupe glutarimido ou 3-carboxy-n-propylènecarbonyle où dans chaque cas le groupe n-propylène peut être perfluoré ou peut être substitué par un ou deux groupes alkyle ou par un groupe tétraméthylène ou pentaméthylène, ou

un groupe 5,7-dioxa-1H,3H-imidazo[1,5-c]thiazolyle,

$R_2$ a les significations indiqués précédemment pour $R_1$, représente un groupe 2-imidazolidon-1-yle ou 3,4,5,6-tétrahydro-2-pyrimidon-1-yle éventuellement substitué en position 3 par un groupe alkyle, cycloalkyle, cycloalkylalkyle ou aralkyle ou un groupe tétrazolyle ou

$R_1$ et $R_2$ représentent avec deux atomes de carbone du cycle phényle voisin un groupe phényle ou 1-alkyl-3,3-dialkyl-2,3-dihydro-pyrrol-2-one,

$R_3$ représente un atome d'hydrogène, de fluor, de chlore ou de brome,

un groupe alkyle de 1 à 6 atomes de carbone dans lequel un groupe méthylène peut être remplacé par un atome d'oxygène ou de soufre, un groupe sulfinyle, sulfonyle ou alkylamino et un groupe méthyle peut être substitué par un groupe hydroxyle, alcoxy, amino, alkylamino ou dialkylamino, auquel cas cependant le groupe méthylène voisin du cycle benzimidazole ne peut pas être remplacé par un groupe sulfinyle ou sulfonyle et lors d'un remplacement d'un groupe méthylène et d'une substitution simultanée d'un groupe méthyle ceux-ci doivent être séparés l'un de l'autre par au moins 2 atomes de carbone,

un groupe alcényle ou alcynyle de 3 à 5 atomes de carbone dans chaque cas,

un groupe phénylalkyle,

un groupe cycloalkyle ou cycloalkylalkyle dans lequel la partie cycloalkyle peut contenir dans chaque cas 5 à 7 atomes de carbone,

un groupe aryle, hydroxyle ou imidazolylalkylamino,

un groupe alkylamino de 1 à 4 atomes de carbone,

un groupe aminocarbonyle qui peut être substitué par un groupe alkyle ou cycloalkyle de 5 à 7 atomes de carbone,

un cycle hétéroaromatique à 5 sommets qui contient un groupe NH, un atome d'oxygène ou un atome de soufre, ce cycle hétéroaromatique à 5 sommets cité précédemment pouvant contenir encore 1 à 3 atomes de N, ou un

cycle hétéroaromatique à 6 sommets qui peut contenir 1 ou 2 atomes d'azote, les cycles hétéroaromatiques à 5 ou 6 sommets cités précédemment pouvant être substitués par un ou, à l'exception du groupe tétrazolyle, par deux groupes alkyle,
$R_4$ représente un groupe amino, phtalimino, aminométhyle, cyano, tert.butoxycarbonyle ou 1-(triphénylméthyl)-tétrazolyle, ces composés représentant des produits intermédiaires, un groupe contenant un acide de Brönsted ou un reste qui, in vivo, peut être converti en un groupe contenant un acide de Brönsted, ces composés représentant des antagonistes de l'angiotensine II,
$R_5$ et $R_6$ représentent chacun un atome d'hydrogène ou
$R_5$ et $R_6$ représentent avec les deux atomes de carbone en ortho un groupe phényle, dans lesquels, sauf indication contraire
le groupe acyle cité lors de la définition des restes $R_1$ à $R_6$ est un groupe alcanoyle de 1 à 7 atomes de carbone, un groupe cycloalkylcarbonyle de 4 à 8 atomes de carbone au total, un groupe cycloalkylalcanoyle de 5 à 10 atomes de carbone au total ou un groupe arylcarbonyle, aralcanoyle ou phénylsulfonyle éventuellement substitué par un atome de fluor, de chlore ou de brome, un groupe alkyle ou alcoxy,
le groupe aryle est un groupe phényle éventuellement substitué par un atome de fluor, de chlore ou de brome, par un groupe hydroxyle, alkyle, alcoxy, phénylalcoxy ou trifluorométhyle, la partie alkyle pouvant contenir dans chaque cas 1 à 4 atomes de carbone, ou un groupe naphtyle,
le groupe cycloalkyle est un groupe cycloalkyle de 3 à 7 atomes de carbone qui peut être substitué par un ou deux groupes alkyle, et
les autres groupes alkyle et alcanoyle cités lors de la définition des restes $R_1$ à $R_6$ sont dans chaque cas des groupes alkyle ou alcanoyle de 1 à 3 atomes de carbone,
leurs mélanges d'isomères 1, 3 et leurs sels avec des acides ou des bases inorganiques ou organiques.

**2.** Benzimidazoles de formule I selon la revendication 1, dans laquelle $R_1$ à $R_3$, $R_5$ et $R_6$ sont définis comme dans la revendication 1 et
$R_4$ représente un groupe carboxyle, aminoacétylamino, trifluorométhylcarbonylamino, trifluorométhylcarbonylaminométhyle, trifluorométhylsulfonylamino, trifluorométhylsulfonylaminométhyle ou 1H-tétrazolyle, un groupe alkylcarbonylamino, alkylcarbonylaminométhyle, arylcarbonylamino, arylcarbonylaminométhyle, aralkylcarbonylamino, aralkylcarbonylaminométhyle, alkylsulfonylamino, alkylsulfonylaminométhyle, arylsulfonylamino, arylsulfonylaminométhyle, aralkylsulfonylamino, aralkylsulfonylaminométhyle, arylsulfonylaminocarbonyle ou benzylsulfonylaminocarbonyle, un groupe alkylsulfonylaminocarbonyle ou perfluoroalkylsulfonylaminocarbonyle de 1 à 6 atomes de carbone dans la partie alkyle dans chaque cas, un groupe alcoxycarbonyle de 2 à 7 atomes de carbone au total, un groupe aralcoxycarbonyle, un groupe pivaloyloxyméthoxycarbonyle, phtalidylméthoxycarbonyle, éthoxycarbonyloxyéthoxycarbonyle, méthoxyméthoxycarbonyle, cyclohexyloxycarbonylméthoxycarbonyle ou (1,3-dioxa-2-oxo-4-méthylcyclopentén-5-yl)méthoxycarbonyle, leurs mélanges d'isomères 1, 3 et leurs sels avec des acides ou des bases inorganiques ou organiques.

**3.** Benzimidazoles de formule I selon la revendication 1, dans laquelle
$R_1$ représente un atome d'hydrogène, de fluor ou de chlore, un groupe trifluorométhyle, hydroxyle, benzyloxy, carboxyle, cyano, amino, nitro, aminosulfonyle, méthylaminosulfonyle, diméthylaminosulfonyle, trifluorométhylsulfonyloxy ou tétrazolyle,
un groupe alkyle de 1 à 4 atomes de carbone, le groupe méthyle pouvant être substitué en outre par un groupe hydroxyle ou alkylamino de 1 à 4 atomes de carbone,
un groupe alcoxy de 1 à 4 atomes de carbone qui peut être substitué en position 2, 3 ou 4 par un groupe hydroxyle, alcoxy, alkylamino, dialkylamino ou imidazolyle et le substituant alkyle peut contenir dans chaque cas 1 à 3 atomes de carbone,
un groupe alcanoyloxy de 1 à 4 atomes de carbone ou un groupe cycloalkylaminocarbonyloxy de 5 à 7 atomes de carbone dans la partie cycloalkyle,
un groupe amino qui est substitué par un groupe benzyle, décaline, trifluorométhylcarbonyle, benzylcarbonyle, benzyloxycarbonyle, 2-éthyl-5-méthylcyclohexyloxy ou tert.butoxycarbonylaminoacétyle, par un groupe alkyle de 1 à 5 atomes de carbone, par un groupe cycloalkyle, cycloalkylalkyle, cycloalcoxycarbonyle, cycloalkylcarbonyle ou cycloalkylalcanoyle, la partie cycloalkyle pouvant contenir dans chaque cas 5 à 7 atomes de carbone, la partie alkyle pouvant contenir 1 à 3 atomes de carbone et la partie alcanoyle pouvant contenir 1 à 3 atomes de carbone, par un groupe alcanoyle de 1 à 6 atomes de carbone, par un groupe aminoalcanoyle ou dialkylaminoalcanoyle dans lequel dans chaque cas la partie alkyle et la partie alcanoyle peuvent contenir 1 à 3 atomes de carbone, par un groupe alcoxycarbonyle de 2 à 7 atomes de carbone au total, par un groupe alkylsulfonyle de 1 à 4 atomes de carbone ou par un groupe thiazolidin-3-ylcarbonyle substitué par un groupe alcoxycarbonyle de 2 à 4 atomes de carbone au total,
un groupe benzylamino ou alkylamino de 1 à 5 atomes de carbone qui est substitué en outre sur l'atome d'azote

par un groupe benzyle ou cyclohexyle, par un groupe alkyle de 1 à 3 atomes de carbone qui peut être substitué en position 2 ou 3 par un groupe alcoxy de 1 à 3 atomes de carbone ou par un groupe phénylamino, par un groupe alcanoyle de 1 à 5 atomes de carbone qui peut être substitué par un groupe alcoxy de 1 à 3 atomes de carbone, par un groupe cycloalkylcarbonyle de 6 à 8 atomes de carbone au total ou par un groupe alcoxycarbonyle de 2 à 4 atomes de carbone au total,

un groupe carbonyle qui est substitué par un groupe alkyle qui peut être substitué en position 2 ou 3 par un groupe hydroxyle, alcoxycarbonyle ou alkylamino, par un groupe alkylamino de 1 à 5 atomes de carbone qui peut être substitué dans la partie alkyle par un groupe carboxyle, par un groupe alkylamino qui est substitué en position 2 ou 3 par un groupe phénylamino, par un groupe phényle, alcoxy, amino, benzylamino, phényléthylamino, cycloalkylamino ou cycloalkylalkylamino dans lesquels, dans chaque cas, la partie alkyle peut contenir 1 à 3 atomes de carbone et la partie cycloalkyle peut contenir 5 à 7 atomes de carbone et en outre un groupe alkylamino de 1 à 5 atomes de carbone peut être substitué sur l'atome d'azote par un groupe alkyle de 1 à 4 atomes de carbone,

un groupe aminocarbonylamino ou aminothiocarbonylamino qui peut être mono-, di- ou trisubstitué par un groupe alkyle de 1 à 12 atomes de carbone, par un groupe alcényle ou alcynyle de 3 à 5 atomes de carbone dans chaque cas, par un groupe alkyle bi- ou tricyclique de 7 à 11 atomes de carbone, par un groupe cycloalkyle, cycloalkylalkyle ou phénylalkyle, les substituants pouvant être identiques ou différents et dans chaque cas la partie alkyle pouvant contenir 1 à 3 atomes de carbone et la partie cycloalkyle pouvant contenir 5 à 7 atomes de carbone et un groupe méthylène dans un reste cycloalkyle de 5 à 7 atomes de carbone pouvant être remplacé par un atome d'oxygène et un groupe alkyle en position 4, 5 ou 6 pouvant être substitué par un groupe hydroxyle ou trifluoroacétyle,

un groupe cycloalkylèneiminocarbonylamino de 4 à 6 atomes de carbone dans la partie cycloalkylèneimino ou un groupe morpholinocarbonylamino qui peuvent être substitués l'un et l'autre sur l'azote de l'amino par un groupe alkyle de 1 à 12 atomes de carbone, par un groupe cycloalkyle, cycloalkylalkyle ou phénylalkyle, dans lesquels dans chaque cas la partie alkyle peut contenir 1 à 3 atomes de carbone et la partie cycloalkyle peut contenir 5 à 7 atomes de carbone,

un groupe phtalimino, homophtalimino, 2-carboxyphénylcarbonylamino, 2-carboxyphénylméthylamino, 2-carboxyphénylméthylènecarbonylamino ou 2-carboxyméthylènephénylcarbonylamino, auquel cas un groupe carbonyle dans un groupe phtalimino peut être remplacé par un groupe méthylène et un groupe méthylène dans un groupe homophtalimino, 2-carboxyphénylméthylènecarbonylamino ou 2-carboxyméthylènephénylcarbonylamino peut être substitué par un ou deux groupes méthyle, et en outre les noyaux phényle cités précédemment peuvent être mono- ou disubstitués par des groupes méthyle ou méthoxy, les substituants pouvant être identiques ou différents, et peuvent être en même temps hydrogénés totalement ou partiellement, un groupe bicycloalcane-3-carboxamido ou bicycloalcène-3-carboxamido substitué en position 2 par un groupe carboxyle, un groupe bicycloalcane-2,3-dicarboximido ou bicycloalcène-2,3-dicarboximido, dans lesquels, dans chaque cas, les parties bicycloalcane et bicycloalcène peuvent contenir 9 ou 10 atomes de carbone, peuvent être substituées par 1, 2 ou 3 groupes méthyle et un groupe endométhylène peut être remplacé par un atome d'oxygène, un groupe glutarimido ou 3-carboxy-n-propylènecarbonyle où dans chaque cas le groupe n-propylène peut être perfluoré ou peut être substitué par un ou deux groupes alkyle ou par un groupe tétraméthylène ou pentaméthylène, ou

un groupe 5,7-dioxa-1H,3H-imidazo[1,5-c]thiazolyle, un groupe 2-imidazolidon-1-yle éventuellement substitué en position 3 par un groupe alkyle de 1 à 3 atomes de carbone ou un groupe 3,4,5,6-tétrahydro-2-pyrimidon-1-yle,

$R_2$ représente un atome d'hydrogène, de fluor ou de chlore, un groupe méthyle, hydroxyle ou méthoxy, ou

$R_1$ et $R_2$ représentent avec les deux atomes de carbone en ortho du cycle phényle voisin un groupe phényle ou 1-alkyl-3,3-dialkyl-2,3-dihydro-pyrrol-2-one dans lequel la partie alkyle peut contenir dans chaque cas 1 à 3 atomes de carbone,

$R_3$ représente un atome d'hydrogène, de fluor ou de chlore, un groupe hydroxyle, benzyle ou aminocarbonyle,

un groupe alkyle de 1 à 5 atomes de carbone, le groupe méthyle pouvant être substitué en outre par un groupe hydroxyle, par un groupe alcoxy, alkylsulfényle, alkylsulfinyle ou alkylsulfonyle de 1 à 3 atomes de carbone dans chaque cas, un groupe cycloalkyle ou cycloalkylalkyle dans lequel la partie cycloalkyle peut contenir dans chaque cas 5 à 7 atomes de carbone et la partie alkyle peut contenir 1 à 3 atomes de carbone, un groupe alcényle ou alcynyle de 3 à 5 atomes de carbone dans chaque cas, un groupe phénylalkyle de 1 à 3 atomes de carbone,

un groupe alkylamino de 1 à 4 atomes de carbone,

un groupe pyridyle, furyle, thiazolyle ou pyrazolyle qui peuvent être substitués par un ou deux groupes méthyle,

$R_4$ représente un groupe alcoxycarbonyle de 1 à 5 atomes de carbone au total, un groupe amino, phtalimino, aminométhyle, carboxyle, cyano, méthylsulfonylaminocarbonyle, trifluorométhylsulfonylaminocarbonyle, benzènesulfonylaminocarbonyle, triflurométhylcarbonylaminométhyle, trifluorométhylaminométhyle, tétrazolyle ou 1-(triphénylméthyl)-tétrazolyle,

$R_5$ et $R_6$ représentent chacun un atome d'hydrogène ou

$R_5$ et $R_6$ représentent avec les deux atomes de carbone en ortho un groupe phényle, leurs mélanges d'isomères 1, 3 et leurs sels avec des acides ou des bases inorganiques ou organiques.

**4.** Benzimidazoles de formule I selon la revendication 1, dans laquelle
$R_1$ représente un groupe amino qui est substitué par un groupe benzyle, décaline, trifluorométhylcarbonyle, benzylcarbonyle, benzyloxycarbonyle, 2-éthyl-5-méthylcyclohexyloxy ou tert.butoxycarbonylaminoacétyle, par un groupe alkyle de 1 à 5 atomes de carbone, par un groupe cycloalkyle, cycloalkylalkyle, cycloalcoxycarbonyle, cycloalkylcarbonyle ou cycloalkylalcanoyle, la partie cycloalkyle pouvant contenir dans chaque cas 5 à 7 atomes de carbone, la partie alkyle pouvant contenir 1 à 3 atomes de carbone et la partie alcanoyle pouvant contenir 1 à 3 atomes de carbone, par un groupe alcanoyle de 1 à 6 atomes de carbone, par un groupe aminoalcanoyle ou dialkylaminoalcanoyle dans lequel dans chaque cas la partie alkyle et la partie alcanoyle peuvent contenir 1 à 3 atomes de carbone, par un groupe alcoxycarbonyle de 2 à 7 atomes de carbone au total, par un groupe alkylsulfonyle de 1 à 4 atomes de carbone ou par un groupe thiazolidin-3-ylcarbonyle substitué par un groupe alcoxycarbonyle de 2 à 4 atomes de carbone au total,
un groupe benzylamino ou alkylamino de 1 à 5 atomes de carbone qui est substitué en outre sur l'atome d'azote par un groupe benzyle ou cyclohexyle, par un groupe alkyle de 1 à 3 atomes de carbone qui peut être substitué en position 2 ou 3 par un groupe alcoxy de 1 à 3 atomes de carbone ou par un groupe phénylamino, par un groupe alcanoyle de 1 à 5 atomes de carbone qui peut être substitué par un groupe alcoxy de 1 à 3 atomes de carbone, par un groupe cycloalkylcarbonyle de 6 à 8 atomes de carbone au total ou par un groupe alcoxycarbonyle de 2 à 4 atomes de carbone au total,
un groupe aminocarbonylamino ou aminothiocarbonylamino qui peut être mono-, di- ou trisubstitué par un groupe alkyle de 1 à 12 atomes de carbone, par un groupe alcényle ou alcynyle de 3 à 5 atomes de carbone dans chaque cas, par un groupe alkyle bi- ou tricyclique de 7 à 11 atomes de carbone, par un groupe cycloalkyle, cycloalkylalkyle ou phénylalkyle, les substituants pouvant être identiques ou différents et dans chaque cas la partie alkyle pouvant contenir 1 à 3 atomes de carbone et la partie cycloalkyle pouvant contenir 5 à 7 atomes de carbone et un groupe méthylène dans un reste cycloalkyle de 5 à 7 atomes de carbone pouvant être remplacé par un atome d'oxygène et un groupe alkyle en position 4, 5 ou 6 pouvant être substitué par un groupe hydroxyle ou trifluoroacétyle,
un groupe cycloalkylèneiminocarbonylamino de 4 à 6 atomes de carbone dans la partie cycloalkylèneimino ou un groupe morpholinocarbonylamino qui peuvent être substitués l'un et l'autre sur l'azote de l'amino par un groupe alkyle de 1 à 12 atomes de carbone, par un groupe cycloalkyle, cycloalkylalkyle ou phénylalkyle, dans lesquels dans chaque cas la partie alkyle peut contenir 1 à 3 atomes de carbone et la partie cycloalkyle peut contenir 5 à 7 atomes de carbone,
un groupe phtalimino, homophtalimino, 2-carboxyphénylcarbonylamino, 2-carboxyphénylméthylamino, 2-carboxyphénylméthylènecarbonylamino ou 2-carboxyméthylènephénylcarbonylamino, auquel cas un groupe carbonyle dans un groupe phtalimino peut être remplacé par un groupe méthylène et un groupe méthylène dans un groupe homophtalimino, 2-carboxyphénylméthylènecarbonylamino ou 2-carboxyméthylènephénylcarbonylamino peut être substitué par un ou deux groupes méthyle, et en outre les noyaux phényle cités précédemment peuvent être mono- ou disubstitués par des groupes méthyle ou méthoxy, les substituants pouvant être identiques ou différents, et peuvent être en même temps hydrogénés totalement ou partiellement, un groupe bicycloalcane-3-carboxamido ou bicycloalcène-3-carboxamido substitué en position 2 par un groupe carboxyle, un groupe bicycloalcane-2,3-dicarboximido ou bicycloalcène-2,3-dicarboximido, dans lesquels, dans chaque cas, les parties bicycloalcane et bicycloalcène peuvent contenir 9 ou 10 atomes de carbone, peuvent être substituées par 1, 2 ou 3 groupes méthyle et un groupe endométhylène peut être remplacé par un atome d'oxygène, un groupe glutarimido ou 3-carboxy-n-propylènecarbonyle où dans chaque cas le groupe n-propylène peut être perfluoré ou peut être substitué par un ou deux groupes alkyle ou par un groupe tétraméthylène ou pentaméthylène, ou
un groupe 5,7-dioxa-1H,3H-imidazo[1,5-c]thiazolyle,
$R_2$ représente un atome d'hydrogène, un groupe méthyle ou hydroxyle, ou
$R_1$ et $R_2$ représentent avec les deux atomes de carbone en ortho du cycle phényle voisin un groupe phényle,
$R_3$ représente un groupe alkyle de 3 à 5 atomes de carbone,
un groupe alcényle ou alcynyle de 3 à 5 atomes de carbone dans chaque cas,
$R_4$ représente un groupe carboxyle, méthylsulfonylaminocarbonyle, trifluorométhylsulfonylaminocarbonyle, benzènesulfonylaminocarbonyle, trifluorométhylcarbonylaminométhyle, trifluorométhylaminométhyle ou tétrazolyle,
$R_5$ et $R_6$ représentent chacun un atome d'hydrogène ou
$R_5$ et $R_6$ représentent avec les deux atomes de carbone en ortho un groupe phényle, leurs mélanges d'isomères 1, 3 et leurs sels avec des acides ou des bases inorganiques ou organiques.

**5.** Benzimidazoles de formule I selon la revendication 1, dans laquelle
$R_1$ représente un groupe amino qui est substitué par un groupe alcanoyle de 1 à 6 atomes de carbone, par un groupe aminoalcanoyle ou dialkylaminoalcanoyle dans lequel dans chaque cas la partie alkyle et la partie alcanoyle peuvent contenir 1 à 3 atomes de carbone, par un groupe alcoxycarbonyle de 2 à 7 atomes de carbone au total ou par un groupe thiazolidin-3-ylcarbonyle substitué par un groupe alcoxycarbonyle de 2 à 4 atomes de carbone au total,
un groupe benzylamino ou alkylamino de 1 à 5 atomes de carbone qui est substitué sur l'atome d'azote par un

groupe alcanoyle de 1 à 5 atomes de carbone qui peut être substitué par un groupe alcoxy de 1 à 3 atomes de carbone, par un groupe cycloalkylcarbonyle de 6 à 8 atomes de carbone au total ou par un groupe alcoxycarbonyle de 2 à 4 atomes de carbone au total,
un groupe aminocarbonylamino ou aminothiocarbonylamino qui peut être mono-, di- ou trisubstitué par un groupe alkyle de 1 à 8 atomes de carbone, par un groupe alcényle ou alcynyle de 3 à 5 atomes de carbone dans chaque cas, par un groupe alkyle bi- ou tricyclique de 7 à 11 atomes de carbone, par un groupe cycloalkyle, cycloalkylalkyle ou phénylalkyle, les substituants pouvant être identiques ou différents et dans chaque cas la partie alkyle pouvant contenir 1 à 3 atomes de carbone et la partie cycloalkyle pouvant contenir 5 à 7 atomes de carbone et un groupe méthylène dans un reste cycloalkyle de 5 à 7 atomes de carbone pouvant être remplacé par un atome d'oxygène et un groupe alkyle en position 4, 5 ou 6 pouvant être substitué par un groupe hydroxyle ou trifluoroacétyle,
un groupe cycloalkylèneiminocarbonylamino de 4 à 6 atomes de carbone dans la partie cycloalkylèneimino ou un groupe morpholinocarbonylamino qui peuvent être substitués l'un et l'autre sur l'azote de l'amino par un groupe alkyle de 1 à 8 atomes de carbone, par un groupe cycloalkyle, cycloalkylalkyle ou phénylalkyle, dans lesquels dans chaque cas la partie alkyle peut contenir 1 à 3 atomes de carbone et la partie cycloalkyle peut contenir 5 à 7 atomes de carbone,
un groupe phtalimino, homophtalimino, 2-carboxyphénylcarbonylamino, 2-carboxyphénylméthylamino, 2-carboxyphénylméthylènecarbonylamino ou 2-carboxyméthylènephénylcarbonylamino, auquel cas un groupe carbonyle dans un groupe phtalimino peut être remplacé par un groupe méthylène et un groupe méthylène dans un groupe homophtalimino, 2-carboxyphénylméthylènecarbonylamino ou 2-carboxyméthylènephénylcarbonylamino peut être substitué par un ou deux groupes méthyle, et en outre les noyaux phényle cités précédemment peuvent être mono- ou disubstitués par des groupes méthyle ou méthoxy, les substituants pouvant être identiques ou différents, et peuvent être en même temps hydrogénés totalement ou partiellement, un groupe bicycloalcane-3-carboxamido ou bicycloalcène-3-carboxamido substitué en position 2 par un groupe carboxyle, un groupe bicycloalcane-2,3-dicarboximido ou bicycloalcène-2,3-dicarboximido, dans lesquels, dans chaque cas, les parties bicycloalcane et bicycloalcène peuvent contenir 9 ou 10 atomes de carbone, peuvent être substituées par 1, 2 ou 3 groupes méthyle et un groupe endométhylène peut être remplacé par un atome d'oxygène, un groupe glutarimido ou 3-carboxy-n-propylènecarbonyle où dans chaque cas le groupe n-propylène peut être perfluoré ou peut être substitué par un ou deux groupes alkyle ou par un groupe tétraméthylène ou pentaméthylène, ou
un groupe 5,7-dioxa-1H,3H-imidazo[1,5-c]thiazolyle,
$R_2$ représente un atome d'hydrogène, ou
$R_1$ et $R_2$ représentent avec les deux atomes de carbone en ortho du cycle phényle voisin un groupe phényle,
$R_3$ représente un groupe alkyle de 3 à 5 atomes de carbone,
un groupe alcényle ou alcynyle de 3 à 5 atomes de carbone dans chaque cas,
$R_4$ représente un groupe carboxyle ou tétrazolyle,
$R_5$ et $R_6$ représentent chacun un atome d'hydrogène ou
$R_5$ et $R_6$ représentent avec les deux atomes de carbone en ortho un groupe phényle, leurs mélanges d'isomères 1, 3 et leurs sels avec des acides ou des bases inorganiques ou organiques.

**6.** Benzimidazoles de formule I selon la revendication 1:
acide 4'-[(2-n-butyl-6-(N-(n-hexylaminocarbonyl)benzylamino)benzimidazol-1-yl)méthyl]biphényl-2-carboxylique,
acide 4'-[(2-n-butyl-6-(N-cyclohexylaminocarbonyl-n-butylamino)benzimidazol-1-yl)méthyl]biphényl-2-carboxylique,
acide 4'-[(2-n-butyl-6-(N-cyclohexylaminocarbonyléthylamino)benzimidazol-1-yl)méthyl]biphényl-2-carboxylique,
acide 4'-[(2-n-butyl-6-(N-cyclohexylaminocarbonyl-n-propylamino)benzimidazol-1-yl)méthyl]biphényl-2-carboxylique,
acide 4'-[(2-n-butyl-6-(N-cyclohexylaminocarbonyl-méthylamino)benzimidazol-1-yl)méthyl]biphényl-2-carboxylique,
acide 4'-[(2-n-butyl-6-cyclohexylaminocarbonylamino-benzimidazol-1-yl)méthyl]biphényl-2-carboxylique,
acide 4'-[(2-n-butyl-6-(N-cyclohexylaminocarbonyl-n-pentylamino)benzimidazol-1-yl)méthyl]biphényl-2-carboxylique,
acide 4'-[(2-n-butyl-6-(N-méthylaminocarbonyl-benzylamino)-benzimidazol-1-yl)méthyl]biphényl-2-carboxylique,
acide 4'-[(2-n-butyl-6-(1-oxo-isoindolin-2-yl)benzimidazol-1-yl)méthyl]biphényl-2-carboxylique,
acide 4'-[(2-n-butyl-6-(N-cyclohexylaminocarbonyl-benzylamino)benzimidazol-1-yl)méthyl]biphényl-2-carboxylique,
acide 4'-[(2-n-butyl-6-n-pentanoylamino-benzimidazol-1-yl)méthyl]biphényl-2-carboxylique,
acide 4'-[(2-n-butyl-6-propionylamino-benzimidazol-1-yl)méthyl]biphényl-2-carboxylique,
acide 4'-[(2-n-butyl-6-isopropylcarbonylamino-benzimidazol-1-yl)méthyl]biphényl-2-carboxylique,
acide 4'-[(2-n-butyl-6-(tétrahydropyran-2-yl-aminocarbonylamino)benzimidazol-1-yl)méthyl]biphényl-2-carboxylique,

acide 4'-[(2-n-butyl-6-(n-butylaminocarbonylamino)-benzimidazol-1-yl)méthyl]biphényl-2-carboxylique,
acide 4'-[(6-n-butanoylamino-2-n-butyl-benzimidazol-1-yl)méthyl]biphényl-2-carboxylique,
acide 4'-[(2-n-butyl-6-(N-éthoxycarbonyl-benzylamino)benzimidazol-1-yl)méthyl]biphényl-2-carboxylique,
acide 4'-[(2-n-butyl-6-(N-(diméthylaminocarbonyl)amino)-benzimidazol-1-yl)méthyl]biphényl-2-carboxylique,
acide 4'-[(2-n-butyl-6-(N-cyclohexylaminocarbonyl-n-hexylamino)benzimidazol-1-yl)méthyl]biphényl-2-carboxylique,
acide 4'-[(2-n-butyl-6-((5-hydroxy-n-pentyl)aminocarbonylamino)benzimidazol-1-yl)méthyl]biphényl-2-carboxylique,
acide 4'-[(2-n-butyl-6-(N-méthylaminocarbonyl-n-pentylamino)benzimidazol-1-yl)méthyl]biphényl-2-carboxylique,
acide 4'-[(2-n-butyl-6-cyclohexylcarbonylamino-benzimidazol-1-yl)méthyl]biphényl-2-carboxylique,
acide 4'-[(2-n-butyl-6-(N-(n-butylaminocarbonyl)méthylamino)benzimidazol-1-yl)méthyl]biphényl-2-carboxylique,
4'-[(2-n-butyl-7-hydroxy-4-méthyl-benzimidazol-1-yl)méthyl]-2-(1H-tétrazol-5-yl)biphényle,
acide 4'-[(2-n-butyl-6-(cis-hexahydrophtalimino)-benzimidazol-1-yl)méthyl]biphényl-2-carboxylique,
acide 4'-[(2-n-butyl-6-(N-(diméthylaminocarbonyl)benzylamino)benzimidazol-1-yl)méthyl]biphényl-2-carboxylique,
acide 4'-[(2-n-butyl-6-(N-(n-hexylaminocarbonyl)-N-(2-phényléthyl)amino)benzimidazol-1-yl)méthyl]biphényl-2-carboxylique et
acide 4'-[(2-n-butyl-6-cyclopentylcarbonylamino-benzimidazol-1-yl)méthyl]biphényl-2-carboxylique,
leurs mélanges d'isomères 1, 3 et leurs sels avec des acides ou des bases inorganiques ou organiques.

**7.** Sels physiologiquement acceptables des composés selon au moins l'une des revendications 1 à 6 avec des acides ou des bases inorganiques ou organiques.

**8.** Médicament contenant un composé selon au moins l'une des revendications 1 à 6 où $R_4$ représente un groupe contenant un acide de Brönsted ou un reste qui peut être converti in vivo en un groupe contenant un acide de Brönsted, ou un sel physiologiquement acceptable de celui-ci et éventuellement un ou plusieurs supports et/ou diluants inertes.

**9.** Utilisation d'un composé selon au moins l'une des revendications 1 à 6, où $R_4$ représente un groupe contenant un acide de Brönsted ou un reste qui peut être converti in vivo en un groupe contenant un acide de Brönsted, ou un sel physiologiquement acceptable de celui-ci pour la préparation d'un médicament qui convient pour le traitement de l'hypertonie, de l'insuffisance cardiaque, des troubles ischémiques de l'irrigation sanguine périphérique, de l'ischémie myocardique (angor), pour la prévention de la progression de l'insuffisance cardiaque après un infarctus du myocarde, pour le traitement de la néphropathie diabétique, du glaucome, des maladies gastro-intestinales et des maladies de la vessie.

**10.** Procédé de préparation d'un médicament selon la revendication 8, caractérisé en ce qu'un composé selon au moins l'une des revendications 1 à 6, où $R_4$ représente un groupe contenant un acide de Brönsted ou un reste qui peut être converti in vivo en un groupe contenant un acide de Brönsted, ou un sel physiologiquement acceptable de celui-ci est incorporé par voie non chimique dans un ou plusieurs supports et/ou diluants inertes.

**11.** Procédé de préparation des benzimidazoles selon les revendications 1 à 7, caractérisé en ce que

a) un composé de formule

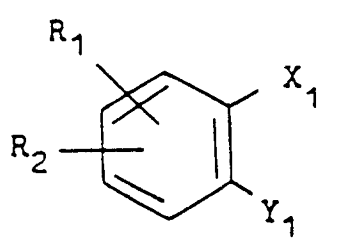

(II)

dans laquelle
$R_1$ et $R_2$ sont définis comme dans au moins l'une des revendications 1 à 6,

l'un des restes $X_1$ et $Y_1$ représente un groupe de formule

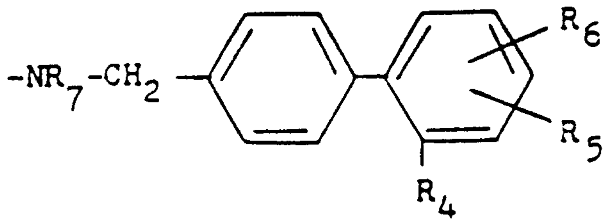

et l'autre des restes $X_1$ et $Y_1$ représente un groupe de formule

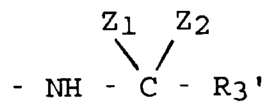

où

$R_3$' possède les significations citées pour $R_3$ dans au moins l'une des revendications 1 à 6, à l'exception de l'atome de fluor, de chlore ou de brome, du groupe hydroxyle, mercapto ou aminocarbonyle, ce dernier pouvant être substitué par un groupe alkyle de 1 à 3 atomes de carbone ou par un groupe cycloalkyle de 5 à 7 atomes de carbone,

$R_4$ à $R_6$ sont définis comme dans au moins l'une des revendications 1 à 6,

$R_7$ représente un atome d'hydrogène, un groupe hydroxyle ou un groupe $R_3$'CO où $R_3$' est défini de la manière citée ci-dessus,

$Z_1$ et $Z_2$, qui peuvent être identiques ou différents, représentent des groupes amino éventuellement substitués ou des groupes hydroxyle ou mercapto éventuellement substitués par des groupes alkyle inférieurs ou $Z_1$ et $Z_2$ représentent ensemble un atome d'oxygène ou de soufre, un groupe imino éventuellement substitué par un groupe alkyle de 1 à 3 atomes de carbone, un groupe alkylènedioxy ou alrylènedithio de 2 ou 3 atomes de carbone dans chaque cas, l'un des restes $X_1$ et $Y_1$ devant cependant représenter un groupe de formule

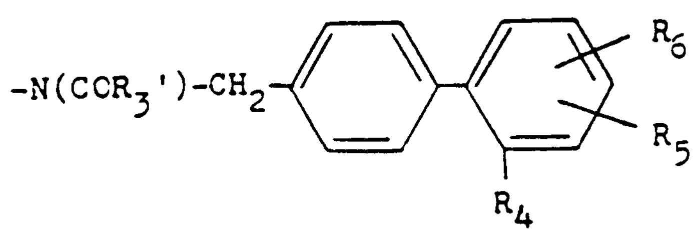

ou

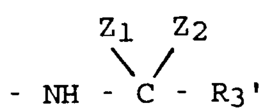

est cyclisé ou

b) pour la préparation des composés de formule I dans laquelle $R_3$ représente les restes aromatiques ou hétéroaromatiques cités pour $R_3$ dans au moins l'une des revendications 1 à 6, une phénylènediamine de

formule

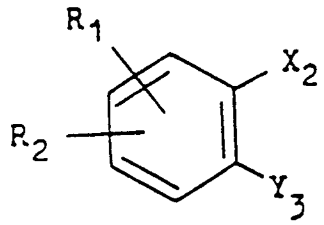

(III)

dans laquelle
$R_1$ et $R_2$ sont définis comme dans au moins l'une des revendications 1 à 6,
l'un des restes $X_2$ et $Y_2$ représente un groupe de formule

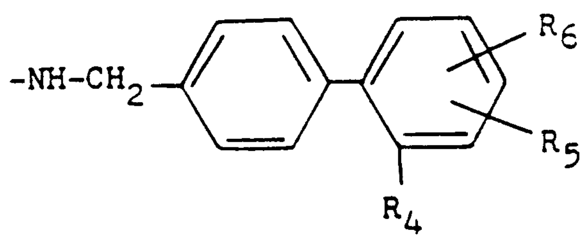

dans laquelle
$R_4$ à $R_6$ sont définis comme dans au moins l'une des revendications 1 à 6,
et l'autre des restes $X_2$ et $Y_2$ représente un groupe amino, est mise à réagir avec un aldéhyde de formule

$$OCH - R_3'' \quad (IV)$$

dans laquelle
$R_3''$ représente les restes aromatiques ou hétéroaromatiques cités pour $R_3$ dans au moins l'une des revendications 1 à 6, en présence d'un oxydant, ou

c) pour la préparation de composés de formule I dans laquelle au moins l'un des restes $R_1$, $R_2$ et/ou $R_3$ représente l'un des restes contenant un groupe sulfinyle ou sulfonyle cités pour $R_1$, $R_2$ et/ou $R_3$ dans au moins l'une des revendications 1 à 6 et les autres restes parmi les restes $R_1$, $R_2$ et/ou $R_3$ sont définis comme dans au moins l'une des revendications 1 à 6, un composé de formule

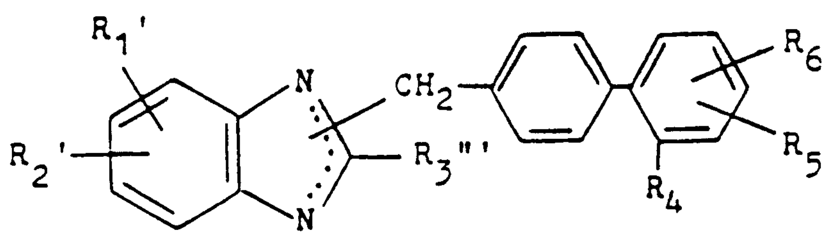

(V)

dans laquelle
$R_4$ à $R_6$ sont définis comme dans au moins l'une des revendications 1 à 6,
au moins l'un des restes $R_1'$, $R_2'$ et/ou $R_3'''$ représente l'un des restes contenant un atome de soufre ou un groupe sulfinyle cités pour $R_1$, $R_2$ et/ou $R_3$ dans au moins l'une des revendications 1 à 6 et les autres restes parmi les restes $R_1'$, $R_2'$ et/ou $R_3'''$ possèdent les significations citées pour $R_1$, $R_2$ et/ou $R_3$ dans au moins l'une des revendications 1 à 6, est oxydé ou

d) pour la préparation d'un composé de formule I dans laquelle $R_4$ représente un groupe carboxyle ou amino, un composé de formule

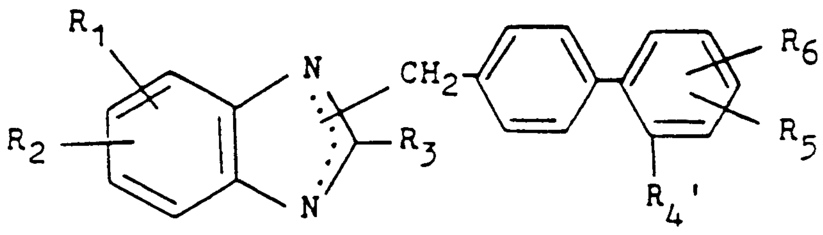

(VI)

dans laquelle
$R_1$ à $R_3$, $R_5$ et $R_6$ sont définis comme dans au moins l'une des revendications 1 à 6 et
$R_4$' représente un groupe convertible en un groupe carboxyle ou amino, est converti en un composé carboxylé ou aminé correspondant ou

e) un benzimidazole de formule

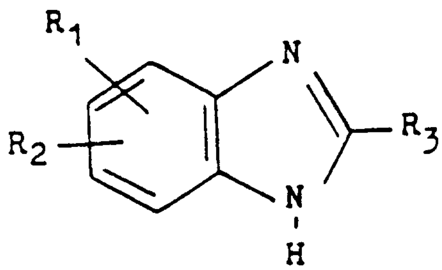

(VII)

dans laquelle
$R_1$ à $R_3$ sont définis comme dans au moins l'une des revendications 1 à 6, est mis à réagir avec un composé biphénylique de formule

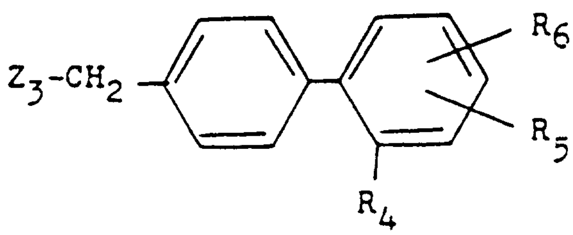

(VIII)

dans laquelle
$R_4$ à $R_6$ sont définis comme dans au moins l'une des revendications 1 à 6 et
$Z_3$ représente un groupe partant nucléophile tel qu'un atome d'halogène, par exemple un atome de chlore, de brome ou d'iode, ou un groupe sulfonyloxy substitué, par exemple un groupe méthanesulfonyloxy, phénylsulfonyloxy ou p-toluènesulfonyloxy ou

f) pour la préparation d'un composé de formule I dans laquelle $R_4$ représente un groupe 1H-tétrazolyle, un reste protecteur est clivé d'un composé de formule

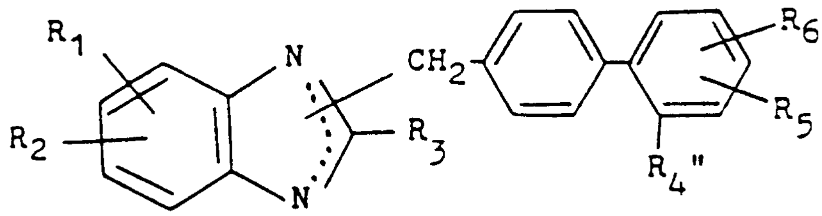

(IX)

dans laquelle
$R_1$ à $R_3$, $R_5$ et $R_6$ sont définis comme dans au moins l'une des revendications 1 à 6 et
$R_4$" représente un groupe 1H-tétrazolyle protégé en position 1 par un reste protecteur, ou

g) pour la préparation d'un composé de formule I dans laquelle $R_4$ représente un groupe 1H-tétrazolyle, un composé de formule

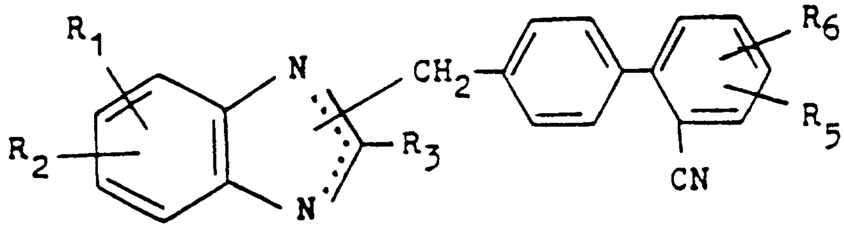

(X)

dans laquelle
$R_1$ à $R_3$, $R_5$ et $R_6$ sont définis comme dans au moins l'une des revendications 1 à 6, est mis à réagir avec l'acide azothydrique ou

h) pour la préparation d'un composé de formule I dans laquelle $R_1$ et/ou $R_2$ représente un groupe aminocarbonylamino qui peut être mono-, di- ou trisubstitué par un groupe alkyle bi- ou tricyclique ou par un groupe alkyle, alcényle, alcynyle, cycloalkyle, cycloalkylalkyle, aralkyle ou aryle, un groupe méthylène dans un reste cycloalkyle de 4 à 7 atomes de carbone pouvant être remplacé par un atome d'oxygène, un composé de formule

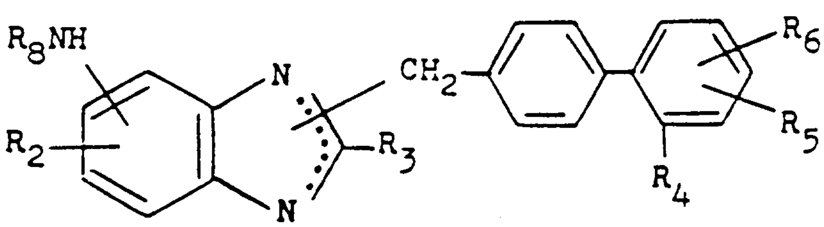

(XI)

dans laquelle
$R_2$ à $R_6$ sont définis comme dans au moins l'une des revendications 1 à 6 et
$R_8$ représente un atome d'hydrogène, un groupe alkyle de 1 à 20 atomes de carbone, un groupe alcényle ou alcynyle de 3 à 5 atomes de carbone dans chaque cas, un groupe cycloalkyle de 3 à 7 atomes de carbone, un groupe cycloalkylalkyle dans lequel la partie cycloalkyle peut contenir 3 à 7 atomes de carbone, la partie alkyle peut contenir 1 à 3 atomes de carbone et dans une partie cycloalkyle de 4 à 7 atomes de carbone un groupe méthylène peut être remplacé par un atome d'oxygène, un groupe alkyle bi- ou tricyclique de 7 à 10 atomes de carbone, un groupe aryle ou aralkyle de 1 à 3 atomes de carbone dans la partie alkyle, le groupe aryle pouvant représenter un groupe phényle éventuellement substitué par un atome de fluor, de chlore ou de brome, un groupe hydroxyle, alkyle ou alcoxy de 1 à 4 atomes de carbone dans chaque cas, est mis à réagir avec un

composé de formule

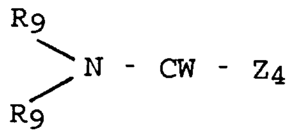

(XII)

dans laquelle
les restes $R_9$, qui peuvent être identiques ou différents, ont les significations citées précédemment pour $R_8$,
W représente un atome d'oxygène ou de soufre,
$Z_4$ représente un groupe partant nucléophile tel qu'un atome de chlore ou de brome ou encore, lorsque l'un au moins des restes $R_9$ représente un atome d'hydrogène,
$Z_4$ et $R_9$ représentent ensemble une liaison azote-carbone ou un groupe cycloalkylèneimino de 4 à 6 atomes de carbone ou un groupe morpholino, ou

i) pour la préparation d'un composé de formule I dans laquelle $R_4$ représente un groupe trifluorométhylcarbonylamino, trifluorométhylcarbonylaminométhyle, trifluorométhylsulfonylamino, trifluorométhylsulfonylaminométhyle ou 1H-tétrazolyle, un groupe alkylcarbonylamino, alkylcarbonylaminométhyle, arylcarbonylamino, arylcarbonylaminométhyle, aralkylcarbonylamino, aralkylcarbonylaminométhyle, alkylsulfonylamino, alkylsulfonylaminométhyle, arylsulfonylamino, arylsulfonylaminométhyle, aralkylsulfonylamino ou aralkylsulfonylaminométhyle, dans lesquels la partie alkyle peut contenir dans chaque cas 1 à 3 atomes de carbone, un composé de formule

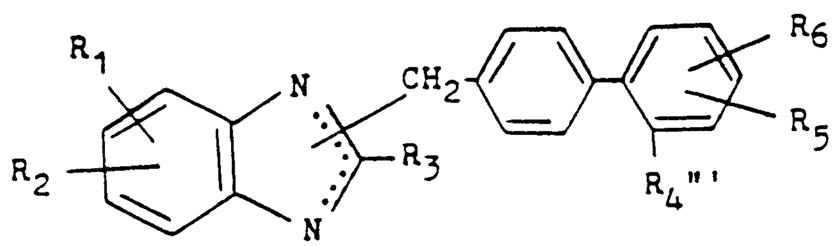

(XIII)

dans laquelle
$R_1$ à $R_3$, $R_5$ et $R_6$ sont définis comme dans au moins l'une des revendications 1 à 6 et
$R_4'''$ représente un groupe amino ou aminométhyle, est acylé avec un composé de formule

$$HO - U - R_{10} \quad (XIV)$$

dans laquelle
$R_{10}$ représente un groupe trifluorométhyle, un groupe alkyle, aryle ou aralkyle, la partie alkyle pouvant contenir dans chaque cas 1 à 3 atomes de carbone, et
U représente un groupe carbonyle ou sulfonyle, ou avec ses dérivés réactifs tels que ses halogénures d'acide, ses esters d'acide ou ses anhydrides d'acide ou

k) pour la préparation d'un composé de formule I dans laquelle $R_4$ représente un groupe alkylsulfonylaminocarbonyle ou perfluoroalkylsulfonylamino de 1 à 4 atomes de carbone dans chaque cas dans la partie alkyle ou

un groupe benzylsulfonylaminocarbonyle, un dérivé réactif d'un acide carboxylique de formule

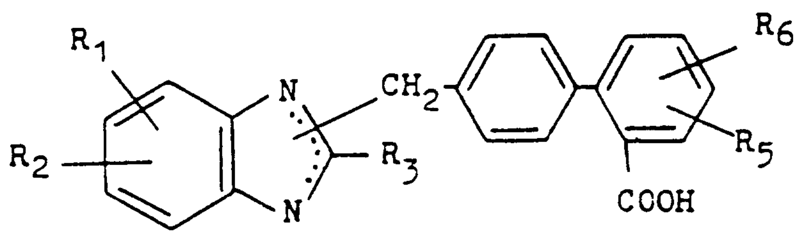

(XV)

dans laquelle

$R_1$ à $R_3$, $R_5$ et $R_6$ sont définis comme dans au moins l'une des revendications 1 à 6, est mis à réagir avec un sulfonamide de formule

$$H_2N - SO_2 - R_{11} \quad (XVI)$$

dans laquelle

$R_{11}$ représente un groupe alkyle ou perfluoroalkyle de 1 à 4 atomes de carbone dans chaque cas ou un groupe benzyle, ou avec son sel alcalin ou

l) pour la préparation d'un composé de formule I dans laquelle $R_2$ représente un groupe 2-imidazolidon-1-yle ou 3,4,5,6-tétrahydro-2-pyrimidon-1-yle éventuellement substitué en position 3 par un groupe alkyle, cycloalkyle, cycloalkylalkyle ou aralkyle, un composé de formule

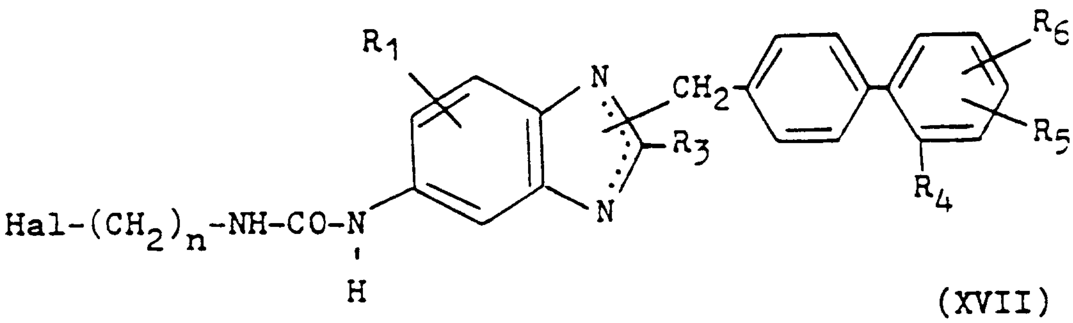

(XVII)

dans laquelle

$R_1$, $R_3$ et $R_4$ à $R_6$ sont définis comme dans au moins l'une des revendications 1 à 6,

Hal représente un atome de chlore, de brome ou d'iode et

n représente le nombre 2 ou 3, est cyclisé et si on le souhaite un composé NH ainsi obtenu est alkylé avec un composé de formule

$$R_{12} - Hal \quad (XVIII)$$

dans laquelle

$R_{12}$ représente un groupe alkyle, cycloalkyle, cycloalkylalkyle ou aralkyle et

Hal représente un atome de chlore, de brome ou d'iode, ou

m) pour la préparation de composés de formule générale I dans laquelle $R_1$ représente un groupe amino qui est monosubstitué par un groupe dialkylaminoalcanoyle, acyle, cycloalcoxycarbonyle, cycloalkylalcoxycarbonyle, aryloxycarbonyle, aralcoxycarbonyle ou trifluoroacétyle, par un groupe alkylsulfonyle de 1 à 4 atomes de carbone, par un groupe alcoxycarbonyle de 2 à 7 atomes de carbone au total ou par un groupe thiazolidin-3-ylcarbonyle substitué par un groupe alcoxycarbonyle,

un groupe alkylamino de 1 à 6 atomes de carbone qui peut être substitué sur l'atome d'azote par un groupe alrylsulfonyle ou acyle, auquel cas, lorsque le groupe acyle représente un groupe alcanoyle, celui-ci peut être substitué en outre par un groupe alcoxy, et le substituant alkyle peut être substitué à partir de la position 2 par un groupe hydroxyle, alcoxy ou arylamino,

un groupe N-alcoxycarbonylalkylamino, N-cycloalcoxycarbonylalkylamino, N-cycloalkylalcoxycarbonylalkylamino, N-aryloxycarbonylalkylamino ou N-aralcoxycarbonylalkylamino dans lequel le groupe alkyle peut contenir dans chaque cas 1 à 6 atomes de carbone, un groupe alcoxycarbonylamino, cycloalcoxycarbonylamino, cycloalkylalcoxycarbonylamino, aryloxycarbonylamino, aralcoxycarbonylamino, acylamino ou alkylsulfonylamino substitué sur l'atome d'azote par un groupe cycloalkyle, cycloalkylalkyle ou aralkyle,

un groupe phtalimino, homophtalimino, 2-carboxyphénylcarbonylamino, 2-carboxyphényiméthylamino, 2-carboxyphénylméthylènecarbonylamino ou 2-carboxyméthylènephénylcarbonylamino, auquel cas un groupe carbonyle dans un groupe phtalimino peut être remplacé par un groupe méthylène et un groupe méthylène dans un groupe homophtalimino, 2-carboxyphénylméthylènecarbonylamino ou 2-carboxyméthylènephénylcarbonylamino peut être substitué par un ou deux groupes alkyle, et en outre les noyaux phényle cités précédemment peuvent être mono- ou disubstitués par des groupes alkyle ou alcoxy, les substituants pouvant être identiques ou différents, et peuvent être en même temps hydrogénés totalement ou partiellement, un groupe bicycloalcane-3-carboxamido ou bicycloalcène-3-carboxamido substitué en position 2 par un groupe carboxyle, un groupe bicycloalcane-2,3-dicarboximido ou bicycloalcène-2,3-dicarboximido, dans lesquels, dans chaque cas, les parties bicycloalcane et bicycloalcène peuvent contenir 9 ou 10 atomes de carbone, peuvent être substituées par 1, 2 ou 3 groupes méthyle et un groupe endométhylène peut être remplacé par un atome d'oxygène, un groupe glutarimido ou 3-carboxy-n-propylènecarbonyle où dans chaque cas le groupe n-propylène peut être perfluoré ou peut être substitué par un ou deux groupes alkyle ou par un groupe tétraméthylène ou pentaméthylène, et

$R_4$ possède les significations citées pour $R_4$ dans au moins l'une des revendications 1 à 6 à l'exception du groupe amino, un composé de formule

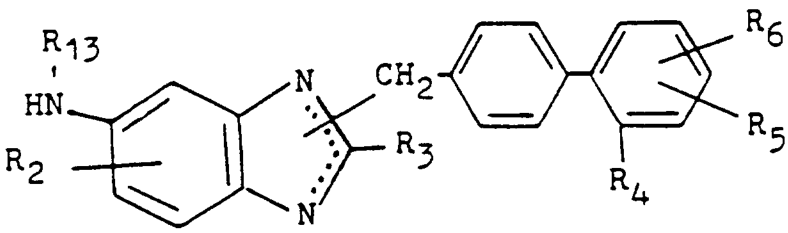

(XIX)

dans laquelle

$R_2$, $R_3$, $R_5$ et $R_6$ sont définis comme dans au moins l'une des revendications 1 à 6,

$R_4$ est défini comme dans au moins l'une des revendications 1 à 6 à l'exception du groupe amino et

$R_{13}$ représente un atome d'hydrogène, un groupe alkyle de 1 à 6 atomes de carbone qui peut être substitué à partir de la position 2 par un groupe hydroxyle, alcoxy ou arylamino, un groupe cycloalkyle, cycloalkylalkyle ou aralkyle, est acylé avec un composé de formule

$$R_{14} - Z_5 \quad (XX)$$

dans laquelle

$R_{14}$ représente un groupe dialkylaminoalcanoyle, cycloalcoxycarbonyle, cycloalkylalcoxycarbonyle, aryloxycarbonyle, aralcoxycarbonyle ou trifluoroacétyle, un groupe alkylsulfonyle de 1 à 4 atomes de carbone, un groupe alcoxycarbonyle de 2 à 7 atomes de carbone au total, un groupe thiazolidin-3-ylcarbonyle substitué par un groupe alcoxycarbonyle ou un groupe acyle dans lequel, lorsque le groupe acyle représente un groupe alcanoyle, celui-ci peut être substitué en outre par un groupe alcoxy, ou un groupe 2-carboxyphénylcarbonyle, 2-carboxyphénylméthyle, 2-carboxyphénylméthylènecarbonyle ou 2-carboxyméthylènephénylcarbonyle, un groupe méthylène dans un groupe 2-carboxyphénylméthylènecarbonyle ou 2-carboxyméthylènephénylcarbonyle pouvant être substitué par un ou deux groupes alkyle, et en outre les noyaux phényle cités précédemment pouvant être mono- ou disubstitués par des groupes alkyle ou alcoxy, les substituants pouvant être identiques ou différents, et pouvant en même temps être hydrogénés totalement ou en partie, un groupe bicycloalcane-3-carbonyle ou bicycloalcène-3-carbonyle substitué en position 2 par un groupe carboxyle dans lequel la partie bicycloalcane et la partie bicycloalcène peuvent contenir chacune 9 ou 10 atomes de carbone, peuvent être substituées par 1, 2 ou 3 groupes méthyle et un groupe endométhylène peut être remplacé par un atome d'oxygène, un groupe 3-carboxy-n-propylènecarbonyle dans lequel le groupe n-propylène peut être perfluoré, peut être substitué par un ou deux groupes alkyle ou par un groupe tétraméthylène ou pentaméthylène, et

$Z_5$ représente un groupe partant nucléophile, ou avec ses dérivés réactifs tels que ses halogénures d'acide, ses esters d'acide ou ses anhydrides d'acide ou

n) pour la préparation de composés de formule générale I dans laquelle au moins l'un des restes $R_1$ et $R_2$ représente un groupe 2-carboxyphénylcarbonylamino, 2-carboxyphénylméthylamino, 2-carboxyphénylméthylènecarbonylamino ou 2-carboxyméthylènephénylcarbonylamino, auquel cas un groupe méthyle dans un groupe 2-carboxyphénylméthylènecarbonylamino ou 2-carboxyméthylènephénylcarbonylamino peut être substitué par un ou deux groupes alkyle, et en outre les noyaux phényle cités précédemment peuvent être mono- ou disubstitués par des groupes alkyle ou alcoxy, les substituants pouvant être identiques ou différents, et peuvent en même temps être hydrogénés totalement ou en partie, un groupe bicycloalcane-3-carboxamido ou bicycloalcène-3-carboxamido substitué en position 2 par un groupe carboxyle, dans lesquels, dans chaque cas, les parties bicycloalcane et bicycloalcène peuvent contenir 9 ou 10 atomes de carbone, peuvent être substituées par 1, 2 ou 3 groupes méthyle et un groupe endométhylène peut être remplacé par un atome d'oxygène, un groupe 3-carboxy-n-propylènecarbonyle dans lequel le groupe n-propylène peut être perfluoré ou peut être substitué par un ou deux groupes alkyle ou par un groupe tétraméthylène ou pentaméthylène, un composé de formule générale

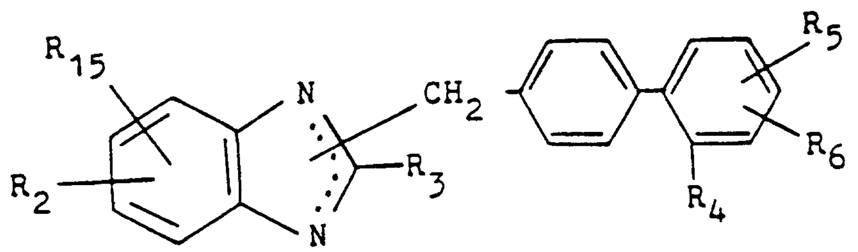

(XXI)

dans laquelle
$R_2$ à $R_6$ sont définis comme dans au moins l'une des revendications 1 à 6 et
$R_{15}$ représente un groupe phtalimino ou homophtalimino, auquel cas un groupe carbonyle dans un groupe phtalimino peut être remplacé par un groupe méthylène et un groupe méthylène dans un groupe homophtalimino peut être substitué par un ou deux groupes alkyle, et en outre les noyaux phényle cités précédemment peuvent être mono- ou disubstitués par des groupes alkyle ou alcoxy, les substituants pouvant être identiques ou différents, et peuvent être en même temps hydrogénés totalement ou partiellement, un groupe bicycloalcane-2,3-dicarboximido ou bicycloalcène-2,3-dicarboximido, dans lesquels, dans chaque cas, les parties bicycloalcane et bicycloalcène peuvent contenir 9 ou 10 atomes de carbone, peuvent être substituées par 1, 2 ou 3 groupes méthyle et un groupe endométhylène peut être remplacé par un atome d'oxygène, un groupe glutarimido dans lequel le groupe n-propylène peut être perfluoré ou peut être substitué par un ou deux groupes alkyle ou par un groupe tétraméthylène ou pentaméthylène, est hydrolysé partiellement et
si nécessaire, un reste protecteur utilisé pendant les réactions a) à n) pour la protection de groupes réactifs est clivé et/ou
si on le souhaite un composé de formule I ainsi obtenu où $R_1$ et/ou $R_2$ représente un groupe nitro est converti par réduction en un composé aminé correspondant de formule I ou
un composé de formule I ainsi obtenu où $R_1$ et/ou $R_2$ représente un groupe hydroxyle, amino, alkylamino, phénylalkylamino, alkylsulfonylamino ou acylamino est converti par alkylation en un composé alkylé correspondant de formule I ou
un composé de formule I ainsi obtenu où $R_1$ et/ou $R_2$ représente un groupe benzyloxy est converti par débenzylation en un composé correspondant de formule I où $R_1$ et/ou $R_2$ représente un groupe hydroxyle ou
un composé de formule I ainsi obtenu où $R_1$ et/ou $R_2$ représente un groupe carboxyle est converti par amidification en un composé correspondant de formule I où $R_1$ et/ou $R_2$ représente un groupe aminocarbonyle éventuellement substitué par un ou deux groupes alkyle de 1 à 3 atomes de carbone dans chaque cas ou
un composé de formule I ainsi obtenu où $R_1$ et/ou $R_2$ représente un groupe carboxyle et/ou $R_4$ représente un groupe cyano est converti par réduction en un composé correspondant de formule I où $R_1$ et/ou $R_2$ représente un groupe hydroxyméthyle et/ou $R_4$ représente un groupe aminométhyle ou un composé de formule I ainsi obtenu où $R_1$ et/ou $R_2$ représente un groupe alcoxycarbonyle est converti par hydrolyse en un composé correspondant de formule I où $R_1$ et/ou $R_2$ représente un groupe carboxyle ou
un composé de formule I ainsi obtenu où $R_1$ et/ou $R_2$ représente un groupe alcoxy ou phénylalcoxy est converti par clivage d'éther en un composé correspondant de formule I où $R_1$ et/ou $R_2$ représente un groupe hydroxyle ou
un composé de formule I ainsi obtenu où $R_1$ et/ou $R_2$ représente un groupe aminocarbonylamino ou aminothiocarbonyle, qui est substitué par un groupe cycloalkyle de 5 à 10 atomes de carbone dans lequel un groupe méthylène est remplacé en position 2 par un atome d'oxygène, et qui, en outre, peut être mono- ou disubstitué par un groupe alkyle de 1 à 20 atomes de carbone, par un groupe alcényle ou alcynyle de 3 à 5 atomes de

carbone dans chaque cas, par un groupe alkyle bi- ou tricyclique de 7 à 11 atomes de carbone, par un groupe cycloalkyle, cycloalkylalkyle, aralkyle ou aryle, est converti par réduction en un composé correspondant de formule I où $R_1$ et/ou $R_2$ représente un groupe aminocarbonylamino ou aminothiocarbonyle qui est substitué par un groupe 4-hydroxy-n-butyle, 5-hydroxy-n-pentyle ou 6-hydroxy-n-hexyle et qui, en outre, peut en être mono- ou disubstitué par un groupe alkyle de 1 à 20 atomes de carbone, par un groupe alcényle ou alcynyle de 3 à 5 atomes de carbone dans chaque cas, par un groupe alkyle bi- ou tricyclique de 7 à 11 atomes de carbone, par un groupe cycloalkyle, cycloalkylalkyle, aralkyle ou aryle, ou

un composé de formule I ainsi obtenu où $R_1$ et/ou $R_2$ représente un groupe phtalimino qui peut être mono- ou disubstitué par un groupe alkyle ou alcoxy, les substituants pouvant être identiques ou différents, est converti par réduction en un composé correspondant de formule I où $R_1$ et/ou $R_2$ représente un groupe 1-oxo-isoindolin-2-yle qui peut être mono- ou disubstitué par un groupe alkyle ou alcoxy, les substituants pouvant être identiques ou différents, ou

un mélange ainsi obtenu d'isomères 1, 3 d'un composé de formule I est résolu par séparation d'isomères en ses isomères 1 et 3 ou

un composé de formule I ainsi obtenu est converti en son sel, en particulier, pour l'utilisation pharmaceutique, en son sel physiologiquement acceptable avec un acide ou une base inorganique ou organique.